# EUROPEAN PATENT APPLICATION

(11) **EP 2 508 526 A1**
(43) Date of publication of application: **10.10.2012**
(21) Application number: 11160690.1
(22) Date of filing: 31.03.2011
(51) Int. Cl.: C07D 491/10, C07D 519/00, A61K 31/407, A61P 29/00, A61P 35/00, A61P 37/00

(54) **Spiroalkene carboxamide derivatives and their use as chemokine receptor modulators**

(71) Applicant: Ares Trading SA, 1170 Aubonne, Vaud (CH)
(72) Inventor: Swinnen, Dominique, 74160 Beaumont (FR); Jorand-Lebrun, Catherine, 74270 Contamine-Sarzin (FR); Gerber, Patrick, 1163 Etoy (CH); Kulkarni, Santosch, 78 Bangalore (IN)
(74) Representative: Merck Serono SA - Geneva Intellectual Property

(57) **Abstract**

The present invention is directed to compounds of Formula (I) below, which are antagonists to the chemoattractant cytokine receptor 2 (CCR2), and/or 5 (CCR5), pharmaceutical compositions, and methods for use thereof.

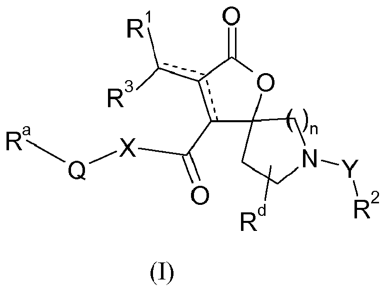

## Description

The present invention provides Oxo-oxa-aza-spiroalkene derivatives of Formula (I) defined below that modulate chemokine receptors, such as CCR2 and/or CCR5. The compounds of the present invention can be used, for example, in the treatment of inflammatory diseases, autoimmune diseases or cancer associated with chemokine receptor expression or activity such as rheumatoid arthritis, arthritis, ankylosing spondylitis, restenosis, osteoarthritis, food allergy, gout disease , systemic lupus erythematosus, lupus nephritis, glomerulonephritis, lupus, multiple sclerosis, Sjogren's Syndrome, bone metastasis, prostate cancer, breast cancer, allergic asthma, asthma, seasonal and perennial allergic rhinitis, transplant rejection, inflammatory bowel disease, Crohn's disease, ulcerative colitis, irritable bowel syndrome, colitis, nephritis, IGa nephropathy, fibrosis, glomerulonephritis fibrosis, liver fibrosis, lung fibrosis, iodiopathic pulmonary fibrosis, fibrotic kidney diseases, systemic sclerosis, scleroderma, liver cirrhosis, liver steatosis, cardiac disease, thrombotic disease, atherosclerosis, myocarditis, stroke, Ischaemia/reperfusion injury in the heart, Ischaemia/reperfusion injury in transplant rejection, pain, neuropathic pain, sarcoidosis, hypertension, psoriasis, atopic dermatitis, contact dermatitis, endometriosis, uveitis, delayed-type hypersensitivity, Behget's disease, diabetes, obesity, insulin resistance, metabolic syndrome, Alzheimer's disease, chronic obstructive pulmonary disease, interstitial lung disease, HIV-infection, HIV with CNS manifestations - Neuro AIDS, organ transplantation, chronic allograph nephropathy, allergic eye disease, age related macular degeneration, acute kidney ischemic disease, (recurrent) Brain infarction, acute coronary syndrome, benign prostatic hyperplasia, trachoma, staphylococcal enterotoxigenic disease (food poisoning), Inner ear inflammation (otitis media), endotoxemia (sepsis), Angiotensin-II-induced cardiac hypertrophy, acute human spinal cord injury..

### Background

CCR2 is a member of the GPCR family of receptors, as are all known chemokine receptors, and are expressed by monocytes and memory T-lymphocytes. The CCR2 signaling cascade involves activation of phospho lipases (PLCβ2), protein kinases (PKC), and lipid kinases (PI-3 kinase).

Chemoattractant cytokines (i.e., chemokines) are relatively small proteins (8-10 kD), which stimulate the migration of cells. The chemokine family is divided into four subfamilies based on the number of amino acid residues between the first and second highly conserved cysteines. Monocyte chemotactic protein- 1 (MCP-I) is a member of the CC chemokine subfamily (wherein CC represents the subfamily having adjacent first and second cysteines) and binds to the cell-surface chemokine receptor 2 (CCR2). MCP-I is a potent chemotactic factor, which, after binding to CCR2, mediates monocyte and lymphocyte migration (i.e., chemotaxis) toward a site of inflammation. MCP-I is also expressed by cardiac muscle cells, blood vessel endothelial cells, fibroblasts, chondrocytes, smooth muscle cells, mesangial cells, alveolar cells, T-lymphocytes, marcophages, and the like. After monocytes enter the inflammatory tissue and differentiate into macrophages, monocyte differentiation provides a secondary source of several proinflammatory modulators, including tumor necrosis factor-α (TNF-α), interleukin-1 (IL-I), IL-8 (a member of the CXC chemokine subfamily, wherein CXC represents one amino acid residue between the first and second cysteines), IL-12, arachidonic acid metabolites (e.g., PGE2 and LTB4), oxygen-derived free radicals, matrix metalloproteinases, and complement components.

Animal model studies of chronic inflammatory diseases have demonstrated that inhibition of binding between MCP-I and CCR2 by an antagonist suppresses the inflammatory response. The interaction between MCP-I and CCR2 has been implicated (see Rollins B J, Monocyte chemoattractant protein 1 : a potential regulator of monocyte recruitment in inflammatory disease, Mol. Med. Today, 1996, 2:198; and Dawson J, et al, Targeting monocyte chemoattractant protein- 1 signaling in disease, Expert Opin. Ther. Targets, 2003 Feb. 7 (I):35-48) in inflammatory disease pathologies such as psoriasis, uveitis, atherosclerosis, rheumatoid arthritis (RA), multiple sclerosis, Crohn's Disease, nephritis, organ allograft rejection, fibroid lung, renal insufficiency, type II diabetes and diabetic complications, diabetic nephropathy, diabetic retinopathy, diabetic retinitis, diabetic microangiopathy, tuberculosis, sarcoidosis, invasive staphylococcia, inflammation after cataract surgery, allergic rhinitis, allergic conjunctivitis, chronic urticaria, Chronic Obstructive Pulmonary Disease (COPD), allergic asthma, periodontal diseases, periodonitis, gingivitis, gum disease, diastolic cardiomyopathies, cardiac infarction, myocarditis, chronic heart failure, angiostenosis, restenosis, reperfusion disorders, glomerulonephritis, solid tumors and cancers, chronic lymphocytic leukemia, chronic myelocytic leukemia, multiple myeloma, malignant myeloma, Hodgkin's disease, and carcinomas of the bladder, breast, cervix, colon, lung, prostate, and stomach.

Monocyte migration is inhibited by MCP-I antagonists (either antibodies or soluble, inactive fragments of MCP-I), which have been shown to inhibit the development of arthritis, asthma, and uveitis. Both MCP-I and CCR2 knockout (KO) mice have demonstrated that monocyte infiltration into inflammatory lesions is significantly decreased. In addition, such KO mice are resistant to the development of experimental allergic encephalomyelitis (EAE, a model of human MS), cockroach allergen-induced asthma, atherosclerosis, and uveitis. Rheumatoid arthritis and Crohn's Disease patients have improved during treatment with TNF-α antagonists (e.g., monoclonal antibodies and soluble receptors) at dose levels correlated with decreases in MCP-I expression and the number of infiltrating macrophages. MCP-I has been implicated in the pathogenesis of seasonal and chronic allergic rhinitis, having been found in the nasal mucosa of most patients with dust mite allergies. MCP-I has also been found to induce histamine release from basophils in vitro. During allergic conditions, both allergens and histamines have been shown to trigger (i.e., to up- regulate) the expression of MCP-I and other chemokines in the nasal mucosa of people with allergic rhinitis, suggesting the presence of a positive feedback loop in such patients. There remains a need for small molecule CCR2 antagonists for preventing, treating or ameliorating a CCR2 mediated inflammatory syndrome, disorder or disease resulting from MCP-I induced monocyte and lymphocyte migration to a site of inflammation.

It has been reported that lung inflammation and granuroma formation are suppressed in CCRI -deficient mice, and that recruitment of macrophages and formation of atherosclerotic lesion decreased in CCR2-deficient mice. See, e.g., Murdoch et al., "Chemokine receptors and their role in inflammation and infectious diseases", Blood 95(10):3032-3043(2000), which is incorporated by reference herein.

CCR2 (also termed CKR-2, MCP-IRA or MCIRB) is predominantly expressed on monocytes and macrophages, and is necessary for macrophage-dependent inflammation (Bruhl et al. 1970). CCR2 is a G protein-coupled receptor (GPCR) which binds with high affinity (Kd of 1 nM) to several members of the MCP family of chemokines (CCL2, CCL7, CCL8, etc.), eliciting a chemotactic signal that results in directed migration of the receptor-bearing cells (Dunzendorfer et al. 2001).

CCR2 is implicated in the pathogenesis of several inflammatory diseases such as rheumatoid arthritis, multiple sclerosis and atherosclerosis (Rodriguez-Frade et al. 2005). The critical role of the CCL2-CCR2 pathway as a modulator of the tissue influx of monocytes was demonstrated in mice deficient in the receptor, CCR2, or the ligand, CCL2, which are phenotypically normal, but show a selective defect in the migration of macrophages to sites of inflammation (Boring et al. 1997; Lu et al. 1998).

It was also recently shown that mRNA levels of CCR2 increase with peak inflammation in rat adjuvant- induced arthritis (AIA), a model for rheumatoid arthritis (Shahrara et al. 2003). Moreover, a small molecule CCR2 antagonist with high affinity for the mouse CCR2 receptor was shown to reduce disease in mice subjected to experimental autoimmune encephalomyelitis, a model of multiple sclerosis, as well as a rat model of inflammatory arthritis (Brodmerkel et al. 2005). See also deBoer, "Perspectives for Cytokine Antagonist therapy in COPD", Drug Discov. Today, 10(2):93-106 (2005),

Taken together, these results support the ability to treat chronic inflammatory diseases with chemical antagonists of CCR2.

Therefore, modulation, such as antagonism or inhibition, of CCR2 and/or CCR5 would be helpful to treat a wide range of diseases selected from inflammatory, autoimmune disease and cancer.

The identification of compounds that modulate the activity of chemokine receptors represents a desirable drug design approach to develop pharmacological agents for the treatment of diseases associated with chemokine receptor activity.

In one aspect, the present invention relates to the compounds of Formula (I) and related Formulae.
In another aspect, the present invention related to the use of the compounds of Formula (I) and related Formula as a medicament. More particularly, the compounds of Formula (I) are useful to treat inflammatory and autoimmune disorders. Compounds of the present invention are also useful to treat cancer. More particularly, the compounds of the present invention are suitable to treat inflammatory diseases or autoimmune diseases associated with chemokine receptor expression or activity such as rheumatoid arthritis, arthritis, Ankylosing spondylitis, restenosis, osteoarthritis, systemic lupus erythematosus, lupus nephritis, glomerulonephritis, lupus, multiple sclerosis, Sjogren's Syndrome, cancer, bone metastasis, prostate cancer, breast cancer, asthma, seasonal and perennial allergic rhinitis, transplant rejection, inflammatory bowel disease, Crohn's disease, ulcerative colitis, irritable bowel syndrome, colitis, nephritis, fibrosis, liver fibrosis, iodiopathic pulmonary fibrosis, scleroderma, liver cirrhosis, liver steatosis, cardiac disease, thrombotic disease, atherosclerosis, stroke, pain, neuropathic pain, hypertension, psoriasis, atopic dermatitis, delayed-type hypersensitivity, Behçet's disease, diabetes, obesity, insulin resistance, metabolic syndrome, Alzheimer's disease, chronic obstructive pulmonary disease, interstitial lung disease, HIV-infection, organ transplantation, chronic allograph nephropathy.

In another aspect, the present invention relates to a pharmaceutical preparation containing at least one of the compounds according to Formula (I) and related Formulae. Such pharmaceutical preparation may also contain additional active agents. The additional active agents may be selected from immunosuppressors or antiinflammatory.

In another aspect, the present invention relates to a process for making the compounds according to Formula (I) and related Formulae.

The present invention further relates to a set or a kit consisting of separate packs of
(a) an effective amount of a compound according to Formula (I) or related Formulae and/or pharmaceutically usable derivatives, tautomers, salts, solvates and stereoisomers thereof, including mixtures thereof in all ratios,
   and
(b) an effective amount of a further medicament active ingredient.

### Detailed description of the invention:

The present invention is directed to compounds of Formula (I) below and related Formulae, which are antagonists to the chemoattractant cytokine receptor 2 (CCR2), and/or 5 (CCR5) pharmaceutical compositions, and methods for use thereof. More particularly, the CCR2 antagonists are compounds useful for preventing, treating or ameliorating a CCR2 mediated syndrome, disorder or disease.

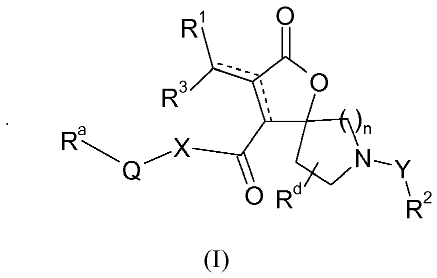

Wherein
One of the two symboles ⁻⁻⁻⁻ independently denotes a single bond and the other one denotes a double bond.
   - R¹, R³: Independently from one another denotes H or a branched or linear alkyl having 1 to 6 carbon atoms,
   - X: is selected from the groups consisting of Het^{X}, -(CH₂)_{q}Het^{X}, NR⁶-Het^{X}, wherein Het^{X} is linked to the adjacent CO group through one N atom contained in its ring, or a group selected from Het^{X}-NR⁶, Ar-NR⁶, -(CH₂)_{q}NR⁶, wherein -NR⁶ is linked to the adjacent CO group, or a group NR⁶CO(CH₂)_{q}N*R⁶, wherein N*R⁶ is linked to the adjacent CO group.
   - Q: denotes a 5- or 6- membered saturated, unsaturated or aromatic ring optionally containing 1 or 2 nitrogen atoms and optionally substituted by 1 or 2 of R^{b} and R^{c}, or
   - Q-X: together denote a fused bicyclic system, containing a N atom linked to the adjacent CO group, which may contain 1 to 3 additional heteroatoms independently selected from N, O, S and/or a group CO, and which may be substituted with 1 or 2 of R^{b} and R^{c},
   - Y: denotes a single bond, a branched or linear alkylene having 1 to 6 carbon atoms or a cyclic alkylene having 3 to 7 carbon atoms, wherein 1 to 5 H atoms, in these cyclic, linear or branched alkylene, may be independently replaced by OR⁶, Hal, or CN,
   - R²: denotes H, Cyc, Hal, Het, Ar, -(CH₂)_{q}COHet, -(CH₂)_{q}COAr, -(CH₂)_{q}COCyc, -(CH₂)_{q}CO₂R⁶, OR⁶,
   - R^{a}: denotes CF₃, OCF₃, Hal, CN, a linear or branched alkyl having 1 to 6 carbon atoms, or when Q denotes a 5- or 6- membered saturated, unsaturated or aromatic ring optionally containing 1 or 2 nitrogen atoms and which is substituted by 1 or 2 of R^{b} and R^{c}, R^{a} also denotes H,
   - R^{b}: denotes Hal, OR⁶, CN, CF₃, or OCF₃,
   - R^{c}: denotes a linear or branched alkyl having 1 to 6 carbon atoms, OR⁶,
   - R^{d}: denotes H or a linear or branched alkyl having 1 to 6 carbon atoms,
   - Hal: denotes F, Cl, Br or I,
   - n: is 1, 2, 3 or 4,
   - q: is 1, 2 or 3,
   - Cyc: denotes a unsaturated or saturated carbocyclic ring having 3 to 7 carbon atoms, which may be substituted by 1 to 5 groups independently selected from a linear or branched alkyl having 1 to 6 carbon atoms, Hal, OR⁶, CF₃, CN, NO₂, CO₂R⁶,
   - Het: denotes a saturated, unsaturated or aromatic monocyclic or fused bicyclic ring having 1 to 3 heteroatoms independently selected from N, O and/or S, which may additionally contain a group selected from CO, SO or SO₂, and which may be substituted with 1 to 5 substituents independently selected from a linear or branched alkyl having 1 to 6 carbon atoms, Hal, OR⁶, CN, NO₂, CO₂R⁶,
   - Ar: denotes a 5-, 6-, or 7-membered saturated, unsaturated or aromatic ring, which may be substituted with 1 to 4 groups independently selected from Hal, CF₃, OR⁶, CN or a linear or branched C₁-C₆-alkyl,
   - R⁶: denotes H, or a branched or linear alkyl having 1 to 6 carbon atoms, or a cycloalkyl having 3 to 6 carbon atoms,
   - Het^{x}: denotes a 5-, 6-, or 7-membered saturated, unsaturated or aromatic heterocyclic ring containing 1 N atom, which may contain 1 to 3 additional heteroatoms independently selected from N, O, S and/or a group CO, and which may be substituted with 1 to 4 groups independently selected from Hal, CF₃, OR⁶, CN or a linear or branched C₁-C₆-alkyl, wherein 2 or more of the linear or branched C₁-C₆-alkyl may be linked to form a bridge.
As well as a pharmaceutically acceptable salt, isomers, diastereoisomers and optically active forms.

The following compounds are preferably not included in the scope of Formula (I) and related Formulae.

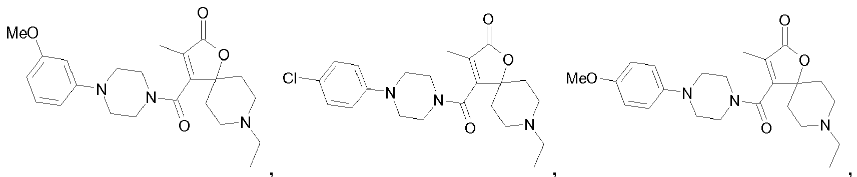

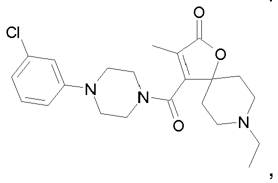

The present invention also encompasses the compounds of Formulae A and B:

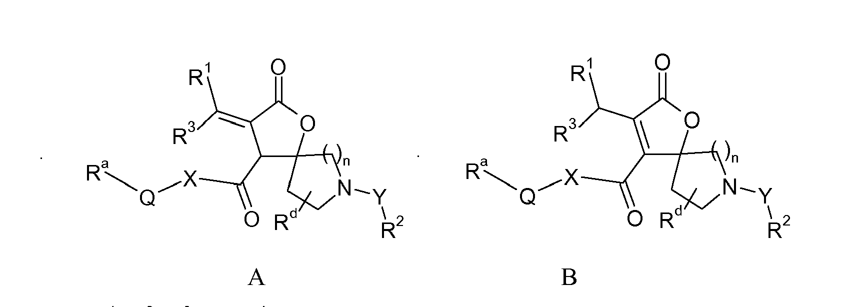

Wherein R¹, R², R³, R^{a}, R^{d}, Y, X, Q and n are as above defined.

In a preferred embodiment, the present invention provides compounds of Formula (Ia): 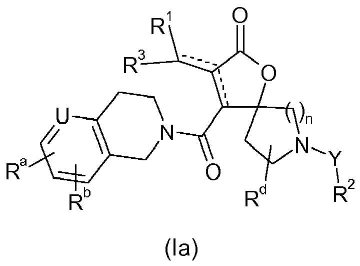 Wherein ⁻⁻⁻⁻ , R^{a}, R^{b}, Y, R¹, R², R^{d}, R³ and n are as above defined, and wherein U is N or C, as well as enantiomers, diastereoisomers thereof.

In another preferred embodiment, the present invention provides compounds of Formula (Ib) 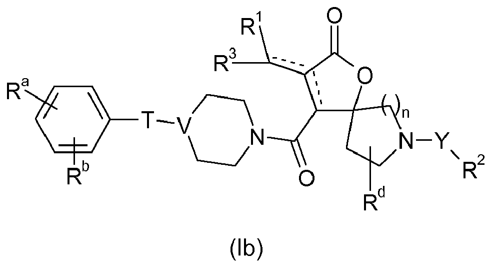 Wherein ⁻⁻⁻⁻ , R^{a}, R^{b}, Y, R¹, R²,R³, R^{d}, Y and n are as above defined, and wherein V is N or C, T is a single bond or NR⁶, (CH₂)_{q} wherein R⁶ and q are as defined above, as well as enantiomers, diastereoisomers thereof.

In another preferred embodiment, the present invention provides compounds of Formula (Ic) 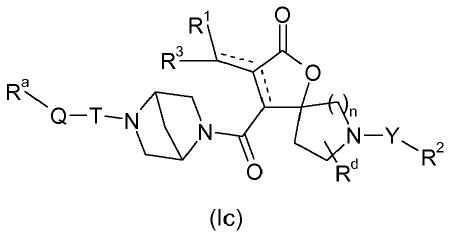 Wherein ⁻⁻⁻⁻ , R^{a}, R^{b}, T, R¹, Y, Q, R^{d}, R² and n are as above defined, as well as enantiomers, diastereoisomers thereof.

In another embodiment, the present invention provides compounds of Formula (I), (Ia), or (Ib) wherein R^{a} is CF₃ or OCF₃.

In another embodiment, the group X in Formulae (I) and related Formulae is Het^{x},

In another embodiment, Het^{x} is fused with Q,

In another embodiment, Het^{X} denotes a 5-, 6-, or 7-membered saturated, unsaturated or aromatic heterocyclic ring containing 1 N atom, which may contain 1 to 3 additional heteroatoms selected from N, O, S and/or a group CO, and which is substituted with 1 to 4 linear or branched C₁-C₆-alkyl, wherein 2 or more of the linear or branched C₁-C₆-alkyl are linked to form a bridge.

In another embodiment, X denotes Het^{X}-NR⁶, Ar-NR⁶, -(CH₂)_{q}NR⁶, wherein -NR⁶ is linked to the adjacent CO group

In another embodiment, R¹ in Formula (I) and related Formulae is H.

In another embodiment, R¹ and R³ in Formula (I) and related Formulae are both H

In another preferred embodiment, Q is selected from phenyl, pyridine, pyrimidine, pyrazine, and pyrazol.

In another embodiment, R^{d} in Formula (I) and related Formulea is H.

In another embodiment, Q-X together denote a fused bicyclic system, containing a N atom linked to the adjacent CO group, which may contain 1 to 3 additional heteroatoms independently selected from N, O, S and/or a group CO, and which may be substituted with 1 or 2 of R^{b} and R^{c}.

Compounds of Formula (I) and related Formulae are obtained as one of the following isomers A and B or as a mixture of both isomers A and B in all ratios: 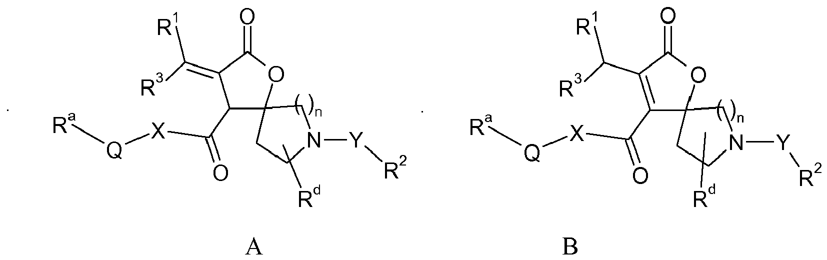 Wherein R¹, R², R³, R^{a}, R^{d}, Y, X, Q and n are as above defined.

Isomer A is preferably obtained when R¹ or R³ is a linear or branched alkyl having 1 to 6 carbon atoms. Isomer B is preferably obtained when R¹ and R³ are both H.

In another embodiment, the group -Y-R² denotes H, CH₃, -CH₂-CH₃, -CH₂-CH₂-CH₃, - CH₂-CH₂-OH, -CH₂-CH₂-CH₂-OH, -CH(CH₃)₂, -CH-(C₂H₅)2, -C(CH₃)₃, -CH₂-CH(CH₃)₂, or one of the following groups:

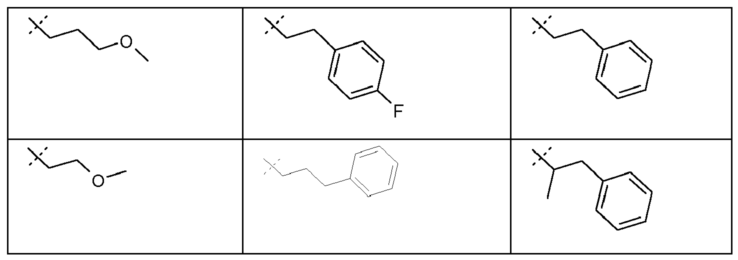

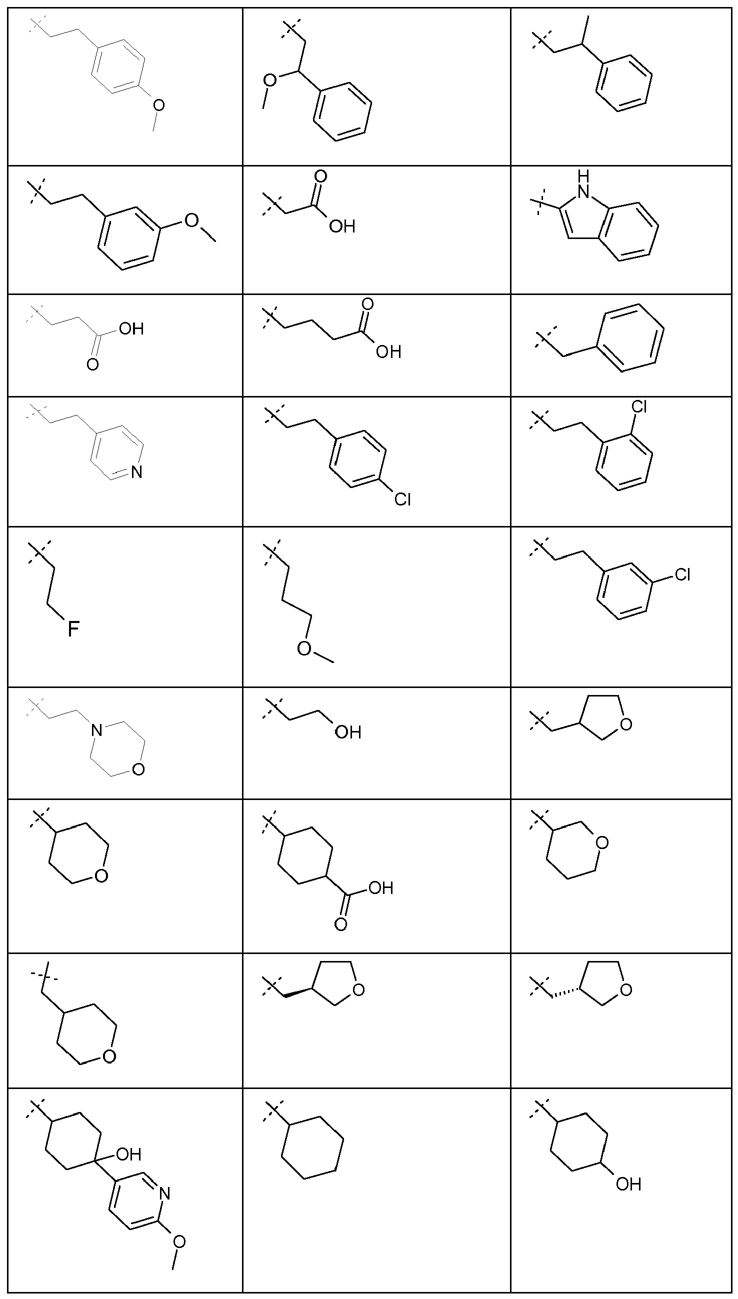

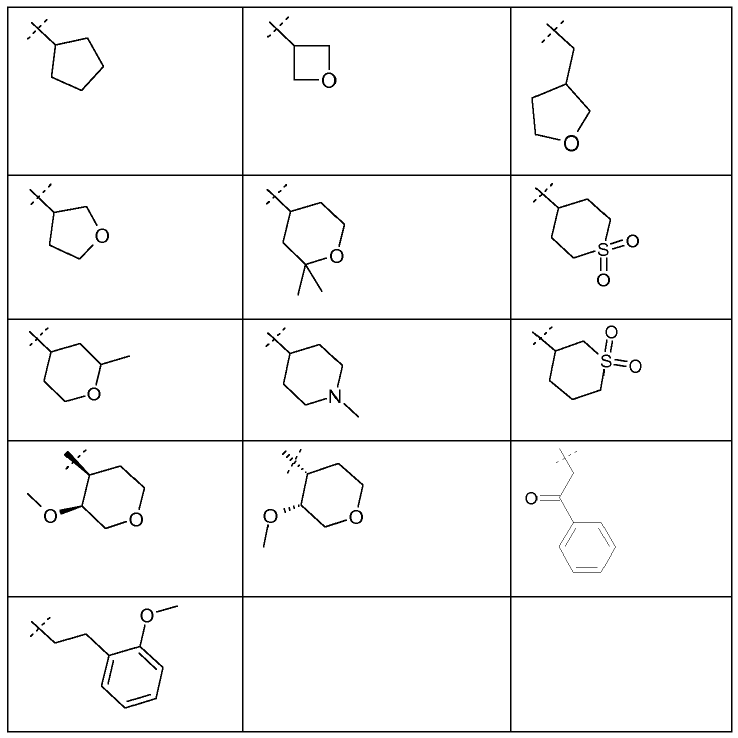

In another embodiment the group X-Q-R^{a} in Formula (I) and related formulae denotes one of the following groups:

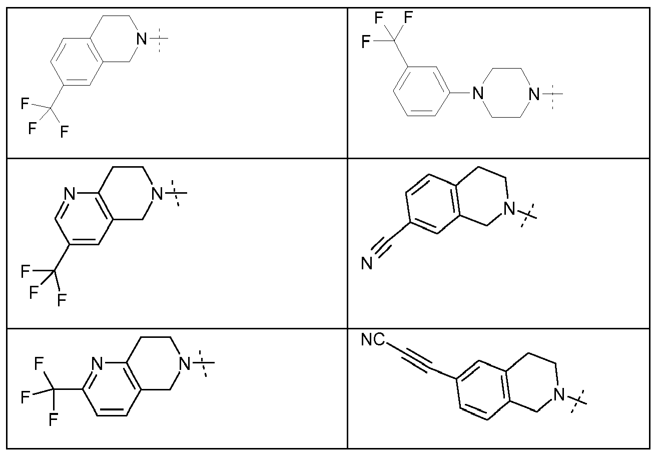

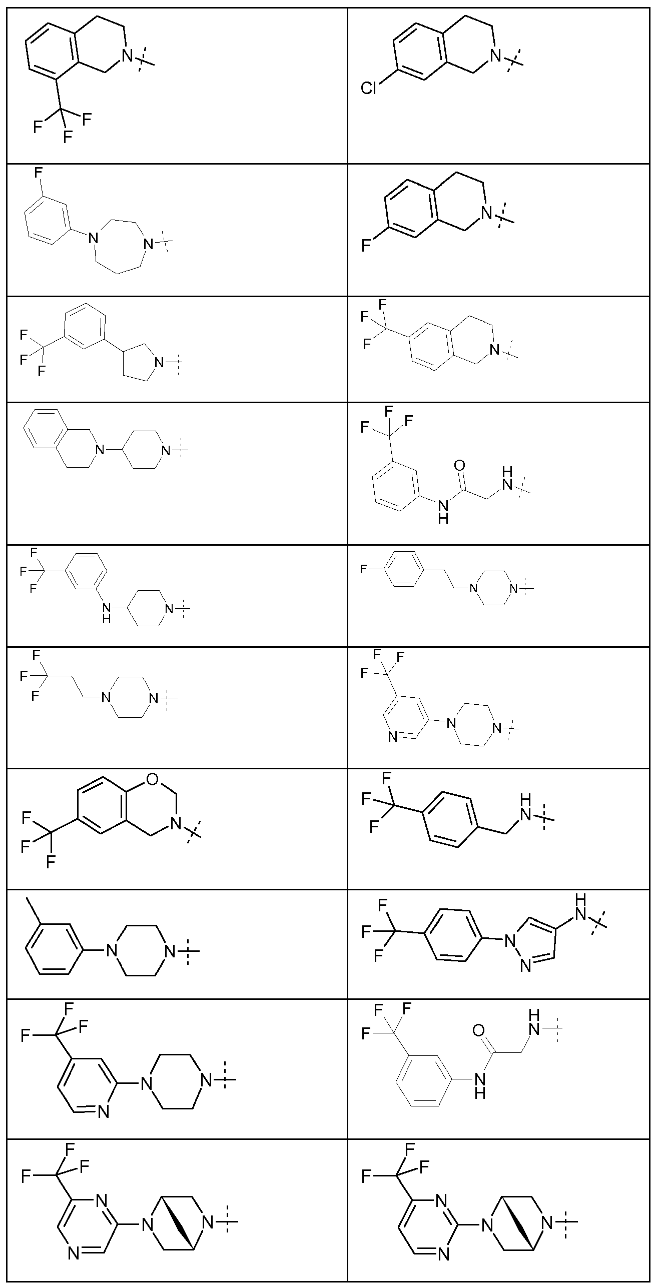

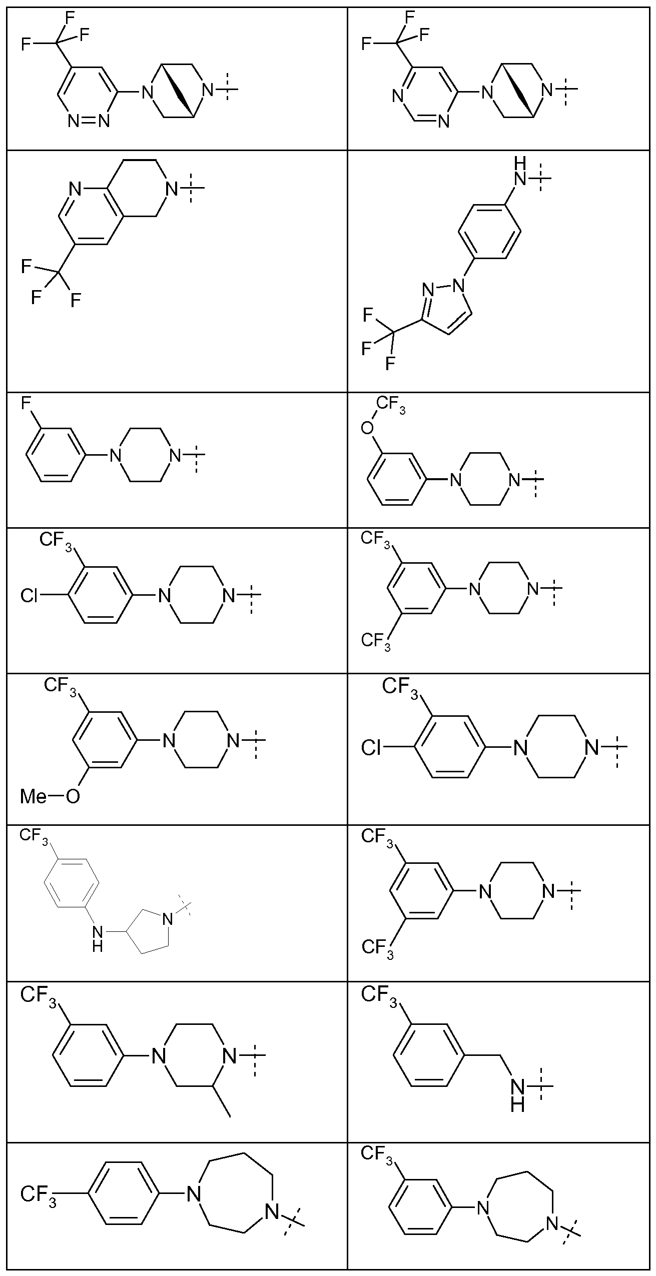

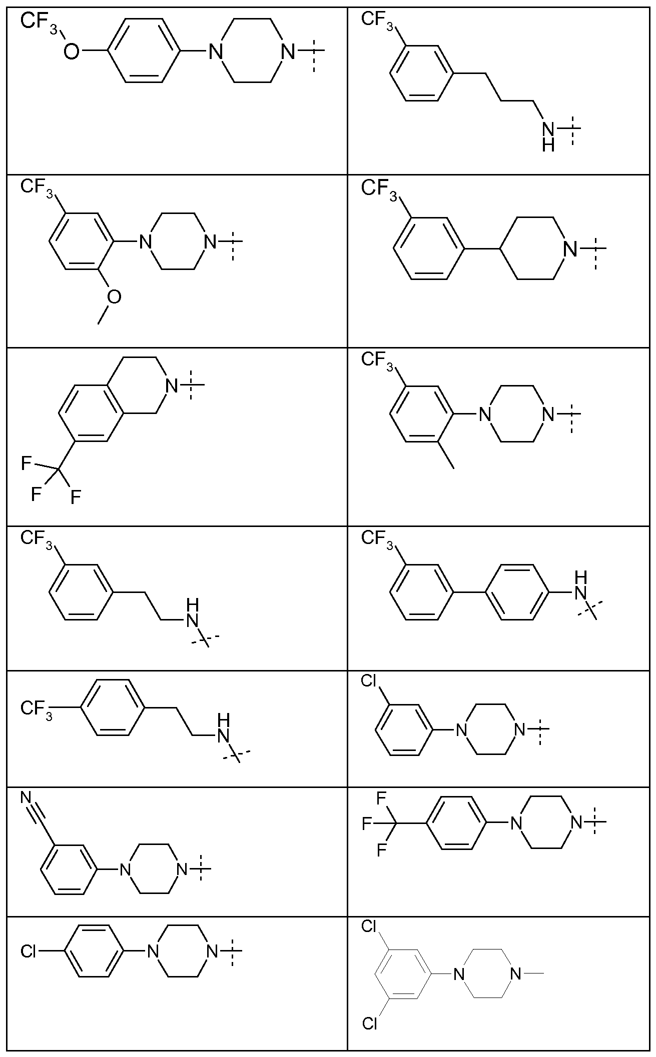

Preferred compounds are selected from the followings:

| **Compound number** | **structure** |
|---|---|
| **1** | 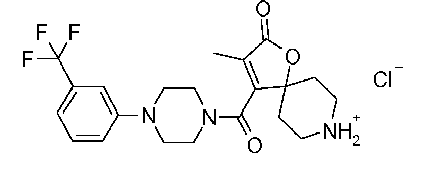 |
| **2** | 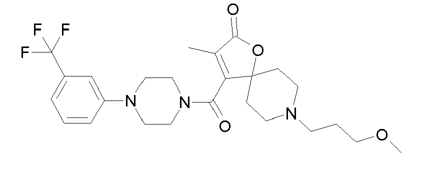 |
| **3** | 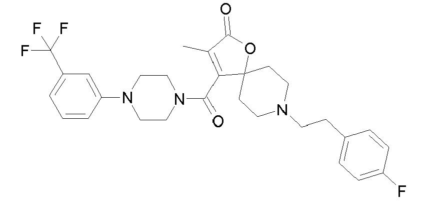 |
| **4** | 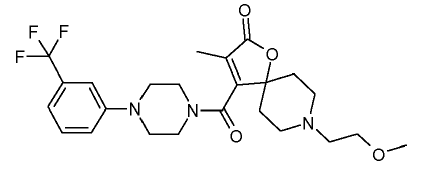 |
| **5** | 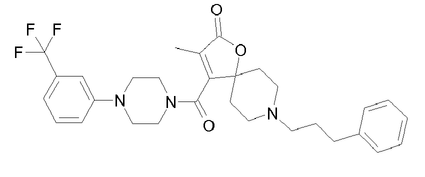 |
| **6** | 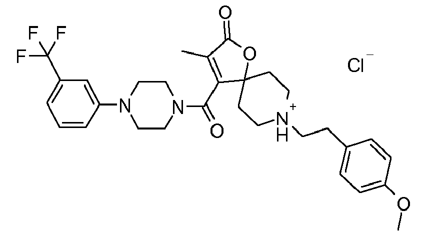 |
| **7** | 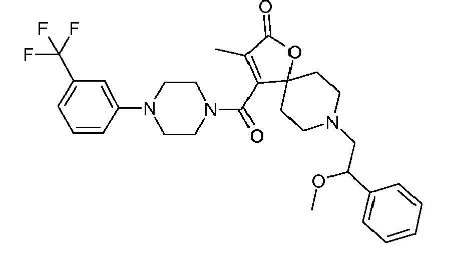 |
| **8** | 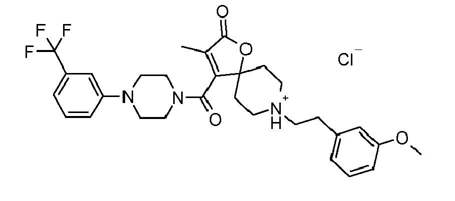 |
| **9** | 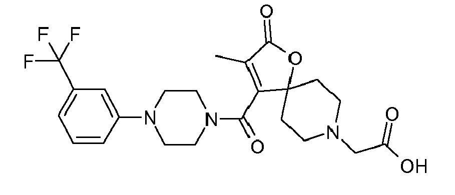 |
| **10** | 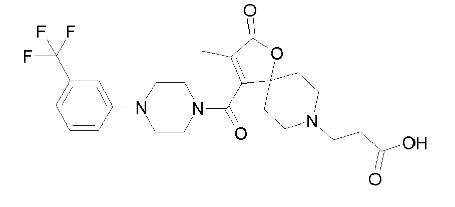 |
| **11** | 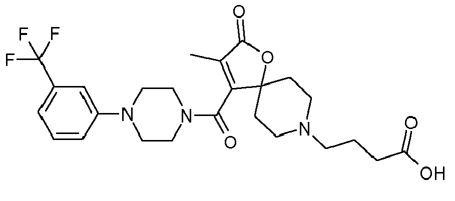 |
| **12** | 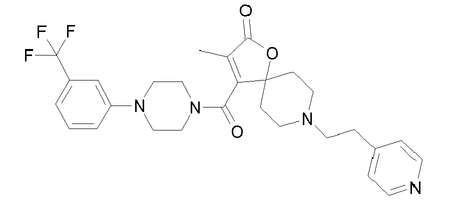 |
| **13** | 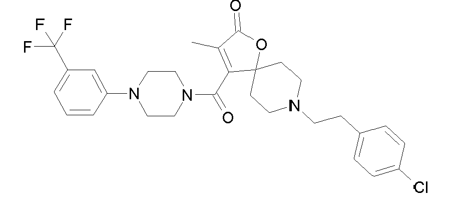 |
| **14** | 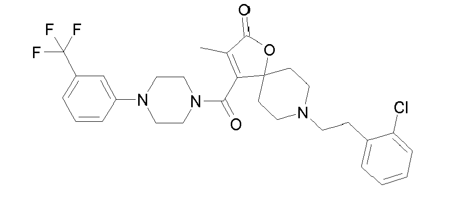 |
| **15** | 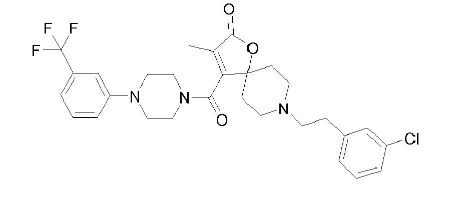 |
| **16** | 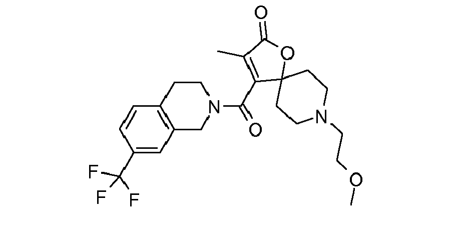 |
| **17** | 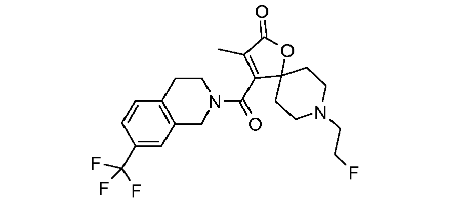 |
| **18** | 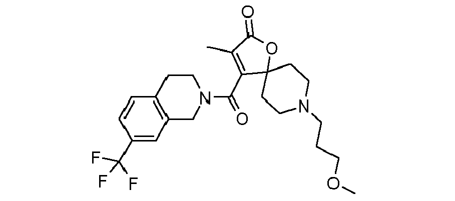 |
| **19** | 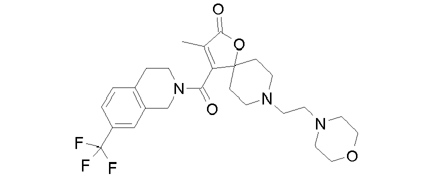 |
| **20** | 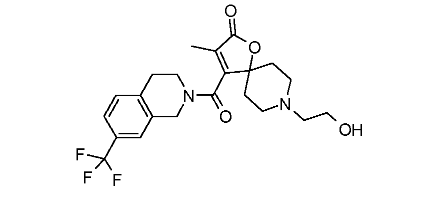 |
| **21** | 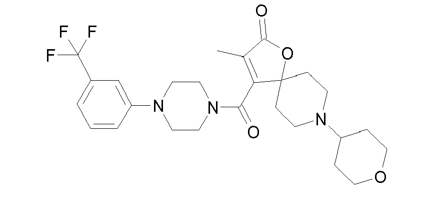 |
| **22** | 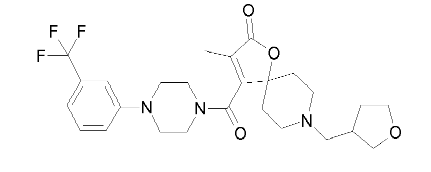 |
| **23** | 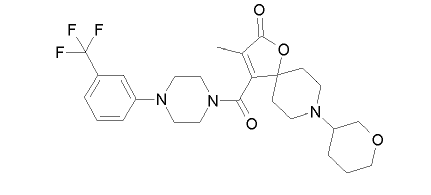 |
| **24** | 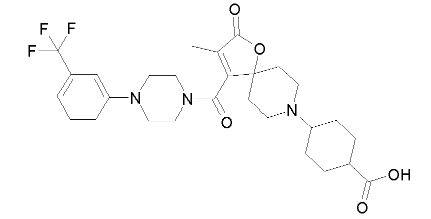 |
| **25** | 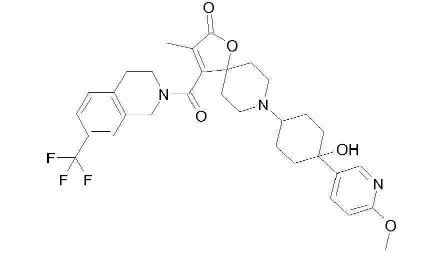 |
| **26** | 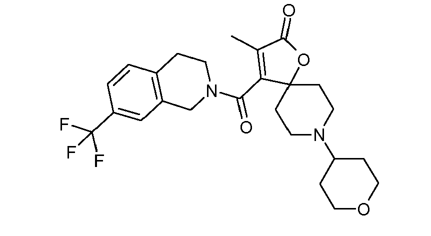 |
| **27** | 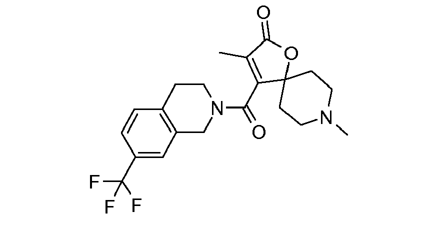 |
| **28** | 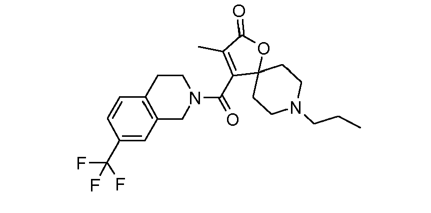 |
| **29** | 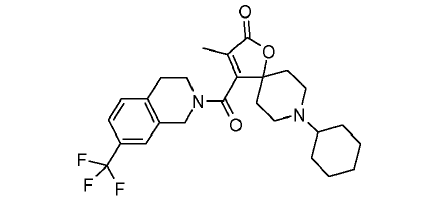 |
| **30** | 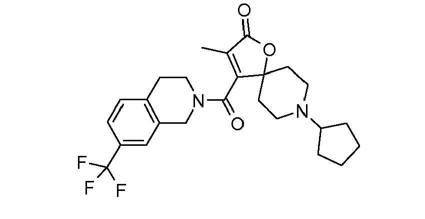 |
| **31** | 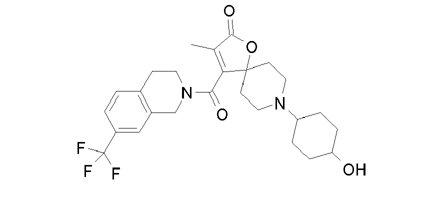 |
| **32** | 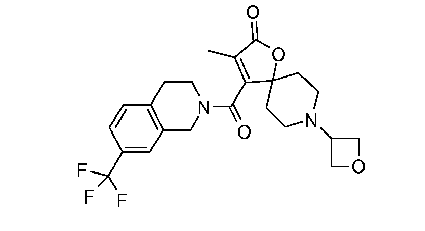 |
| **33** | 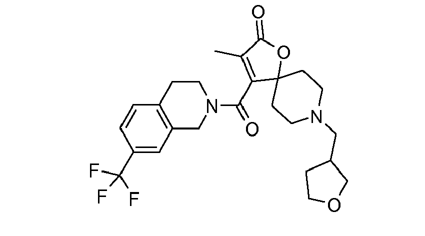 |
| **34** | 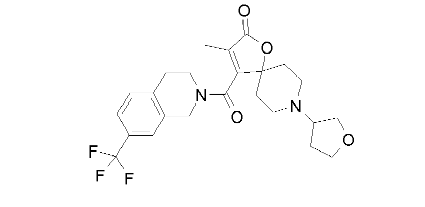 |
| **35** | 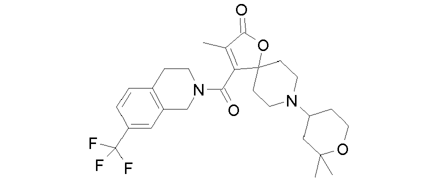 |
| **36** | 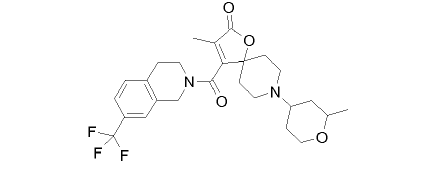 |
| **37** | 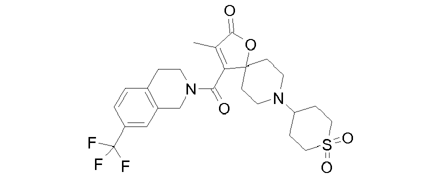 |
| **38** | 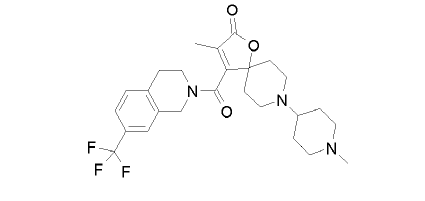 |
| **39** | 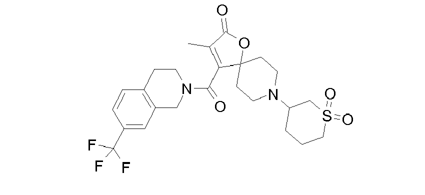 |
| **40** | 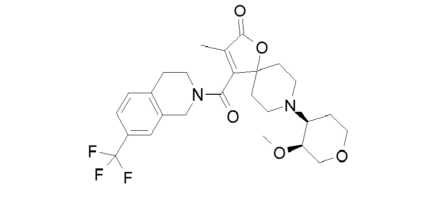 |
| **41** | 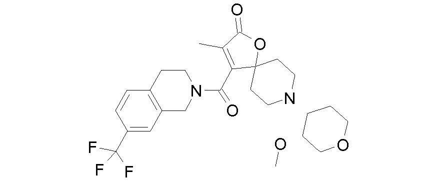 |
| **42** | 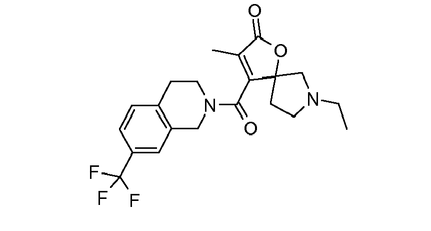 |
| **44** | 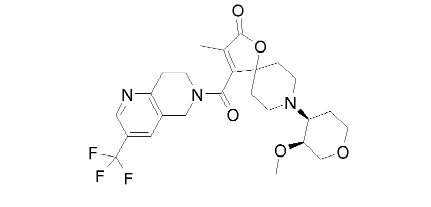 |
| **45** | 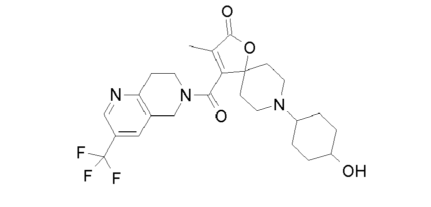 |
| **46** | 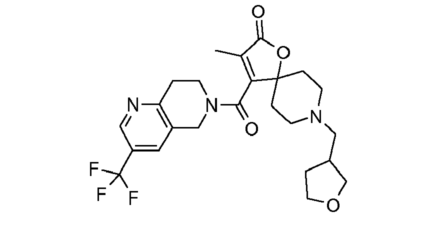 |
| **47** | 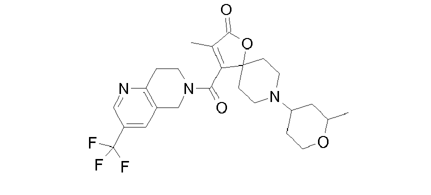 |
| **48** | 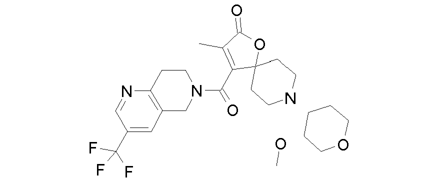 |
| **49** | 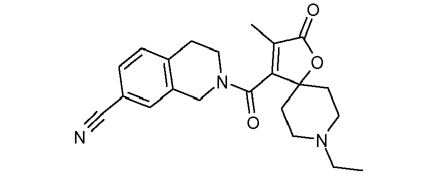 |
| **50** | 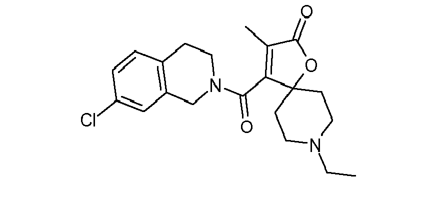 |
| **51** | 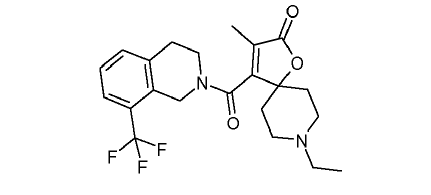 |
| **52** | 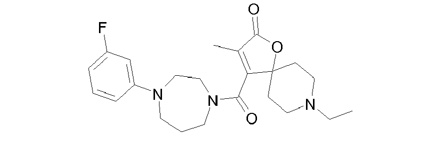 |
| **53** | 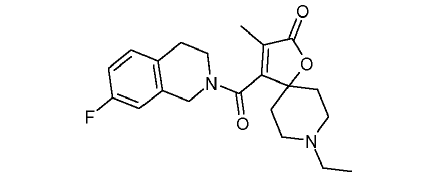 |
| **54** | 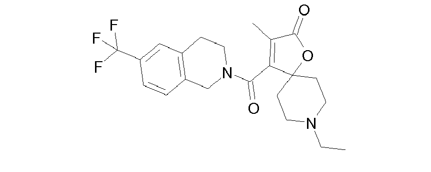 |
| **55** | 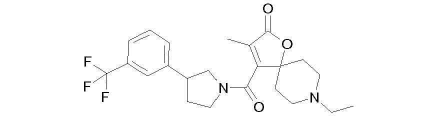 |
| **56** | 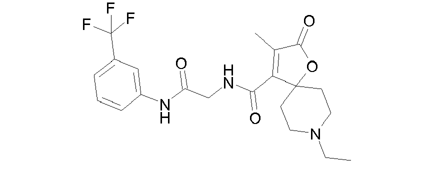 |
| **58** | 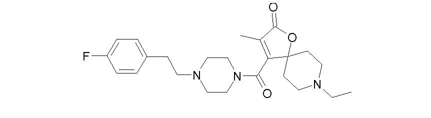 |
| **59** | 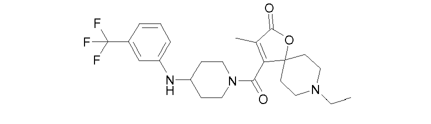 |
| **60** | 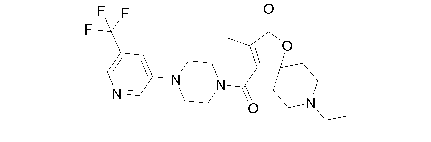 |
| **62** | 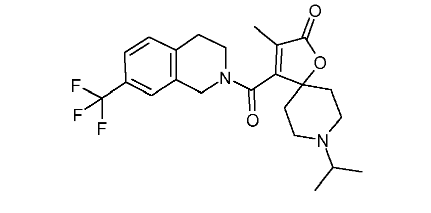 |
| **63** | 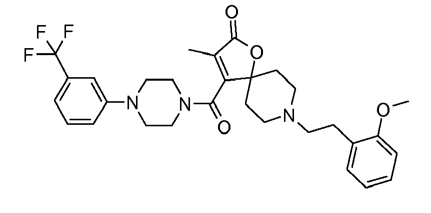 |
| **64** | 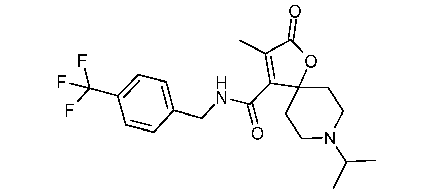 |
| **65** | 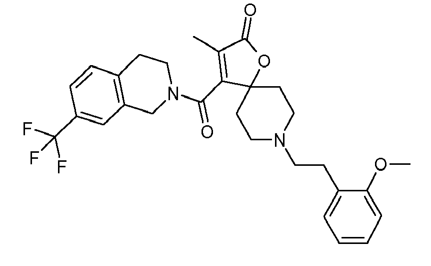 |
| **66** | 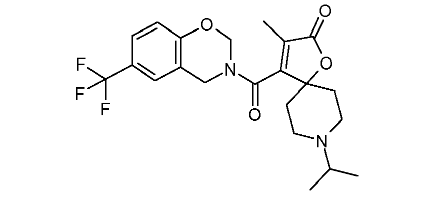 |
| **67** | 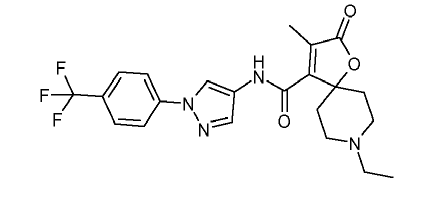 |
| **68** | 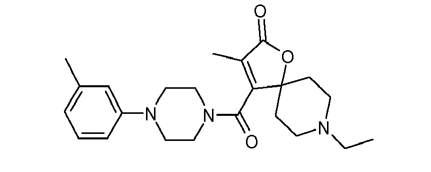 |
| **69** | 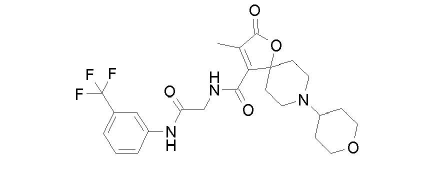 |
| **70** | 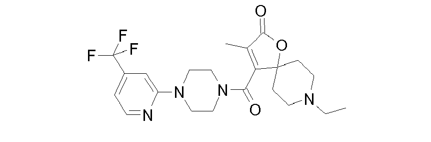 |
| **71** | 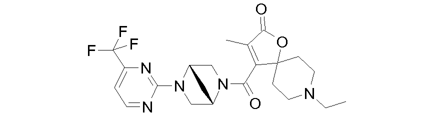 |
| **72** | 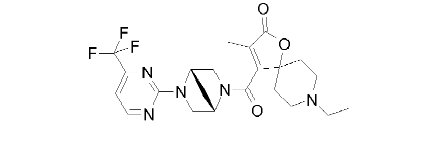 |
| **73** | 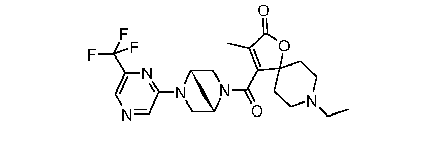 |
| **74** | 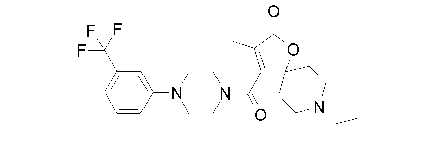 |
| **75** | 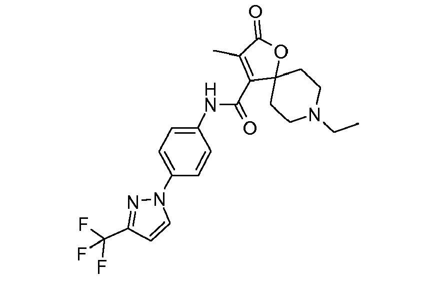 |
| **76** | 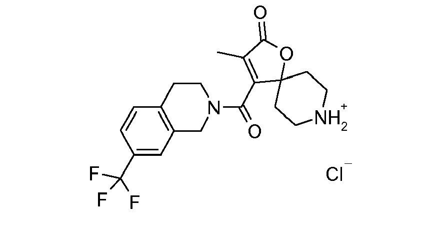 |
| **77** | 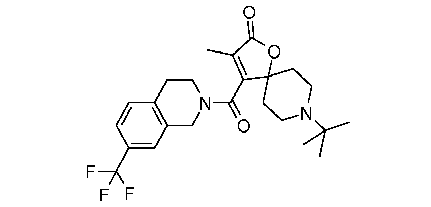 |
| **78** | 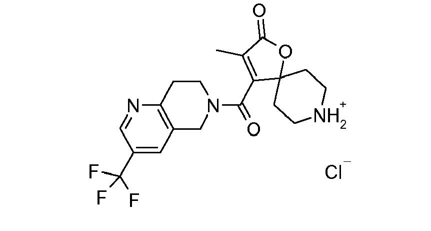 |
| **79** | 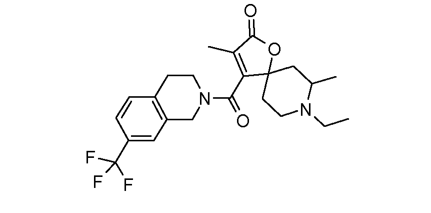 |
| **80** | 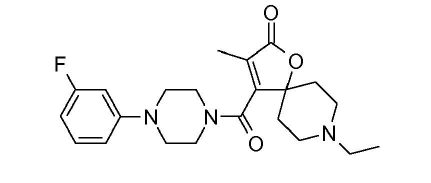 |
| **81** | 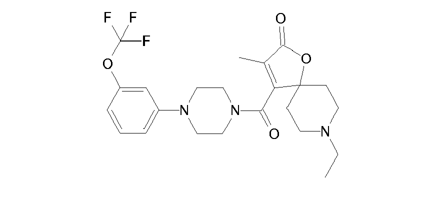 |
| **82** | 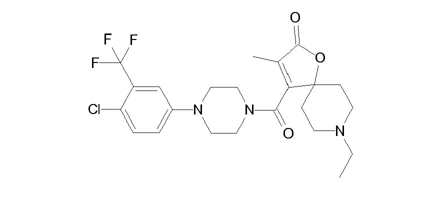 |
| **83** | 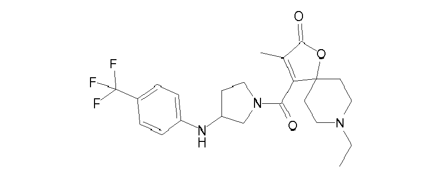 |
| **84** | 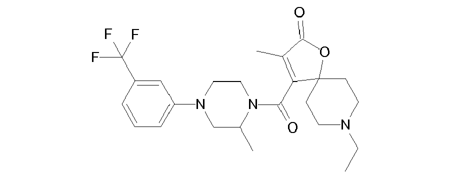 |
| **85** | 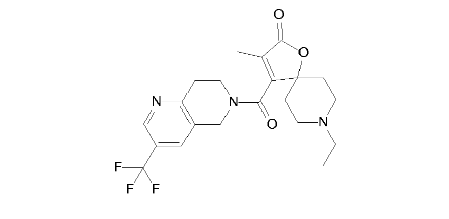 |
| **86** | 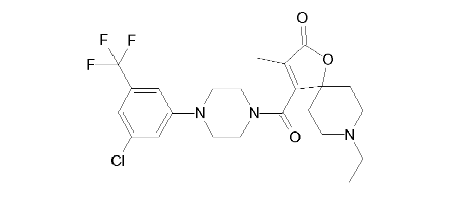 |
| **87** | 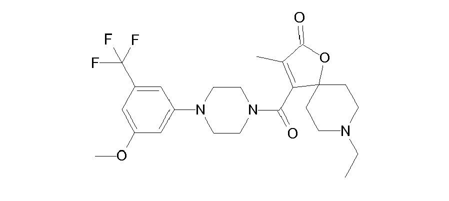 |
| **88** | 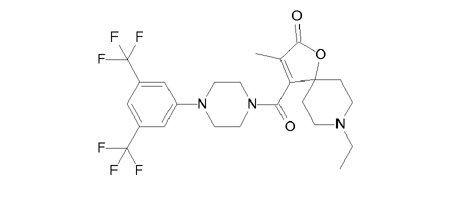 |
| **89** | 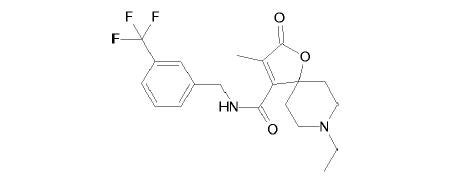 |
| **90** | 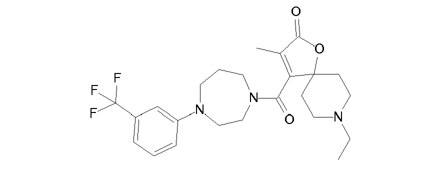 |
| **91** | 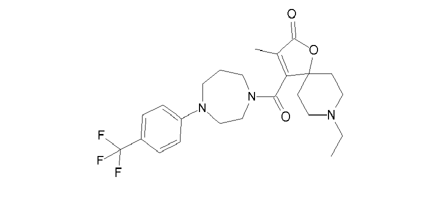 |
| **92** | 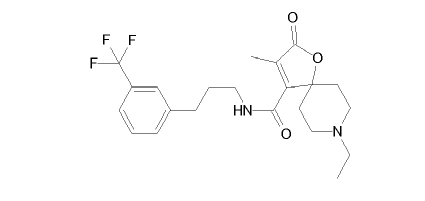 |
| **93** | 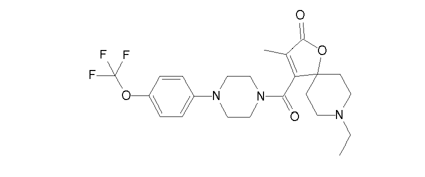 |
| **94** | 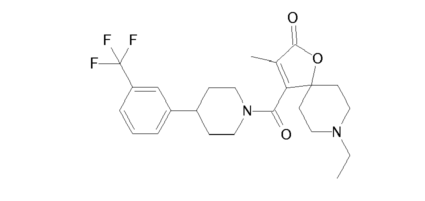 |
| **95** | 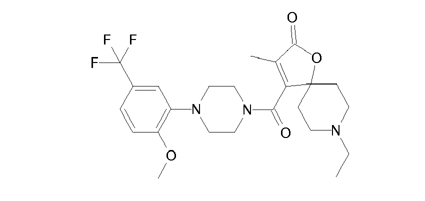 |
| **96** | 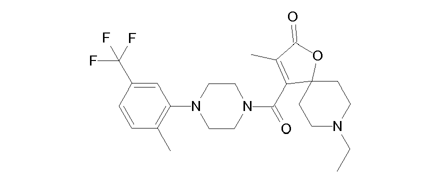 |
| **97** | 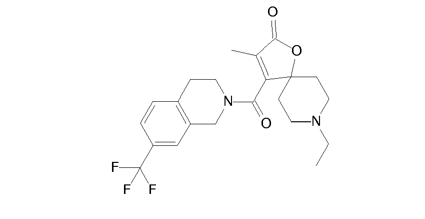 |
| **98** | 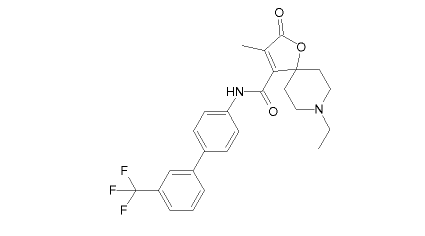 |
| **99** | 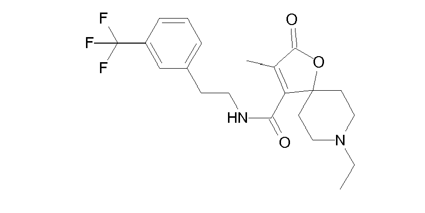 |
| **100** | 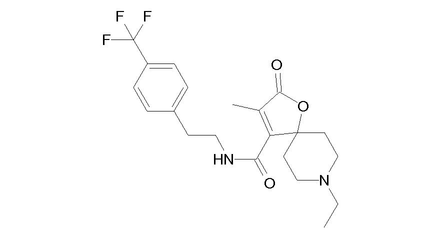 |
| **101** | 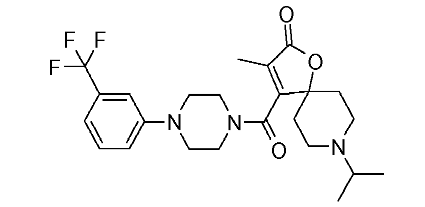 |
| **102** | 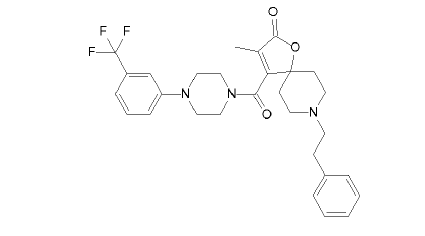 |
| **103** | 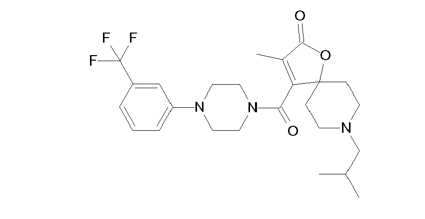 |
| **104** | 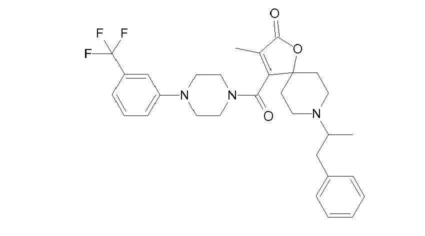 |
| **105** | 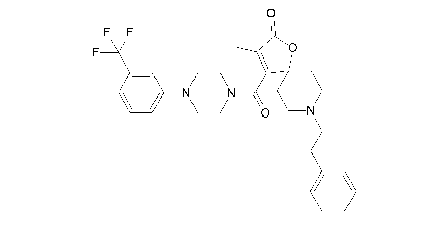 |
| **106** | 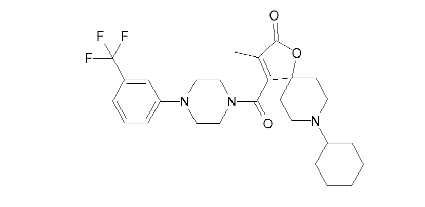 |
| **107** | 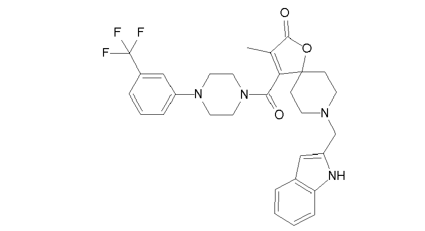 |
| **108** | 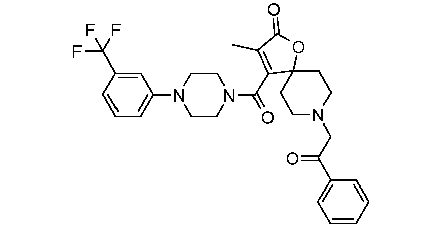 |
| **109** | 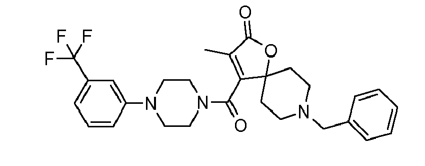 |
| **110** | 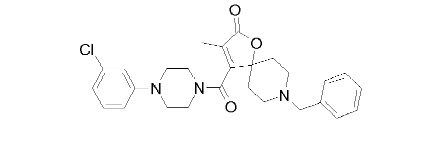 |
| **111** | 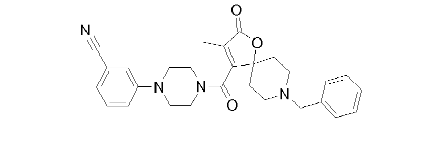 |
| **112** | 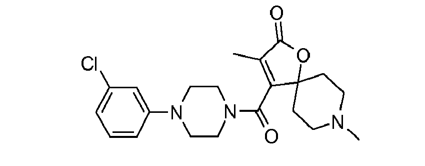 |
| **113** | 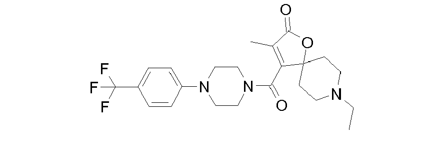 |
| **114** | 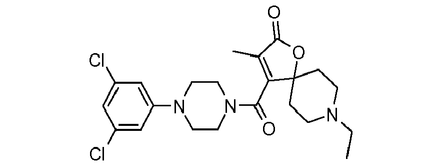 |
| **115** | 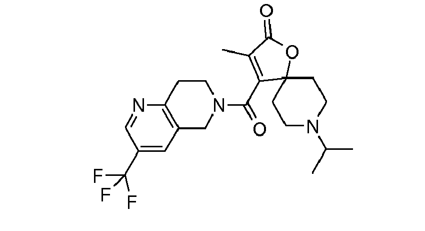 |
| **116** | 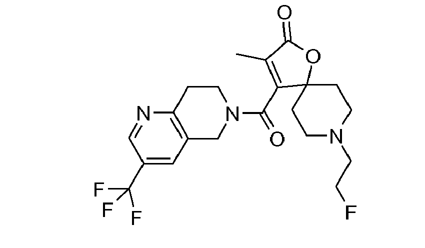 |
| **117** | 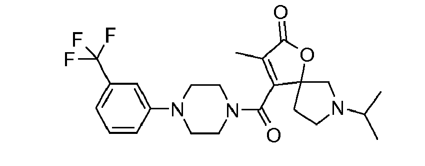 |
| **118** | 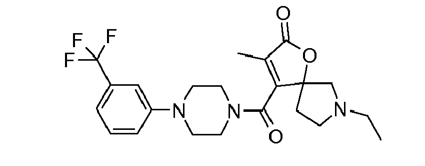 |
| **119** | 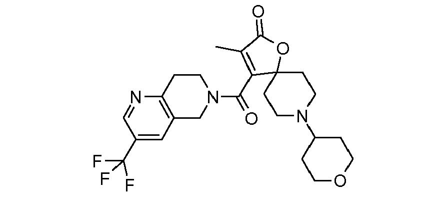 |
| **120** | 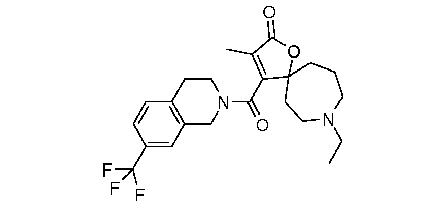 |
| **121** | 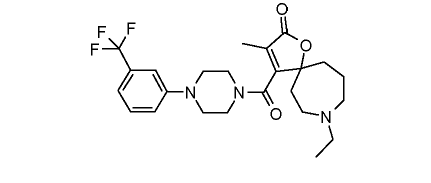 |
| **122** | 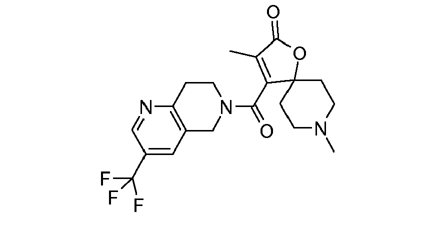 |
| **123** | 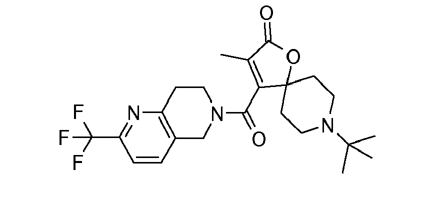 |
| **124** | 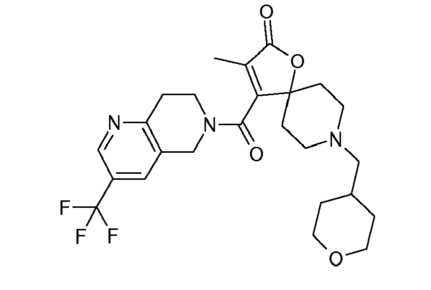 |
| **125** | 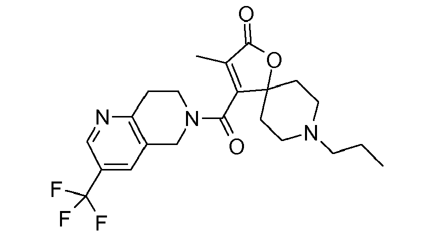 |
| **126** | 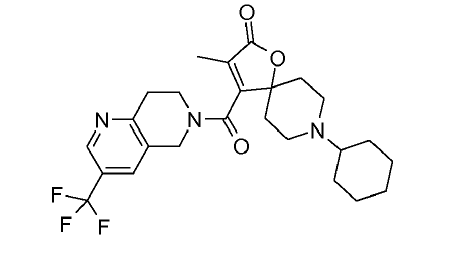 |
| **127** | 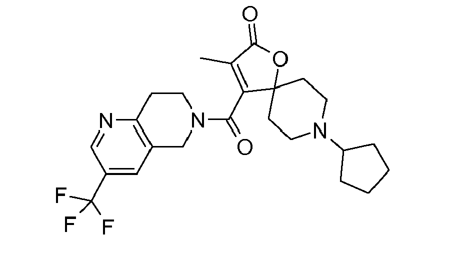 |
| **128** | 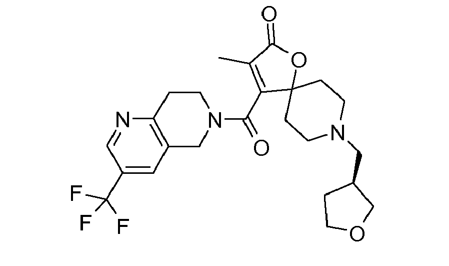 |
| **129** | 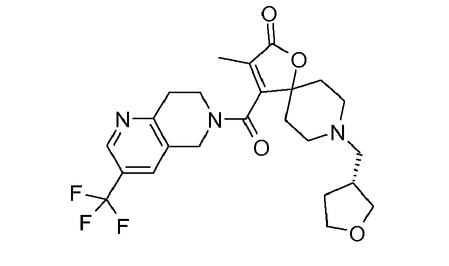 |
| **130** | 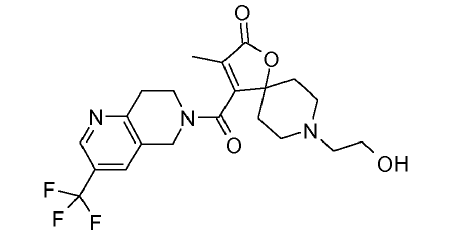 |
| **131** | 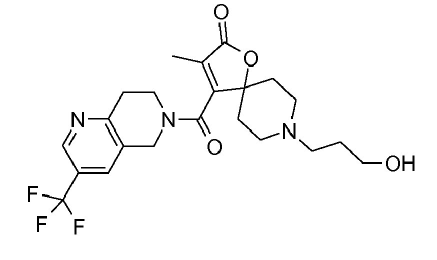 |
| **132** | 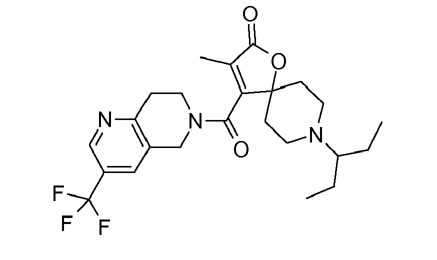 |
| **133** | 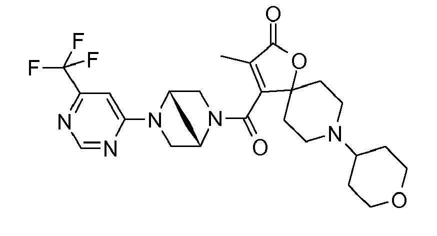 |
| **134** | 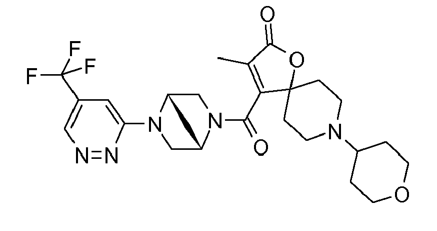 |
| **135** | 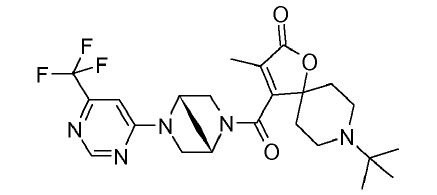 |
| **136** | 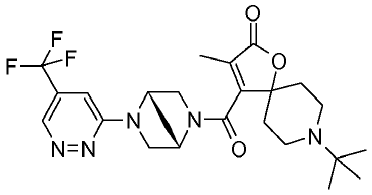 |

The following abbreviations refer to the abbreviations used below:
AcOH (acetic acid), BINAP (2,2'-bis(disphenylphosphino)-1,1'-binaphthalene), dba (dibenzylidene acetone), tBu (tert-Butyl), tBuOK (potassium tert-butoxide), CDI (1,1'-Carbonyldiimidazole), D B U (1,8-dizabicyclo[5.4.0]undec-7-ene), DCM (dichloromethane), DIAD (diisobutylazodicarboxylate), DIEA (di-isopropyl ethylamine), DMA (dimethyl acetamide), DMAP (4-dimethylaminopyridine), DMSO (dimethyl sulfoxide), D M F (N,N-dimethylformamide), EDC.HCl (1-ethyl-3-(3-dimethylaminopropyl)carbodiimide hydrochloride), EtOAc (ethyl acetate), EtOH (ethanol), g (gram), cHex (cyclohexane), HPLC (high performance liquid chromatography), hr (hour), MHz (Megahertz), MeOH (methanol), min (minute), mL (milliliter), mmol (millimole), mM (millimolar), mp (melting point), MS (mass spectrometry), MW (microwave), NMM (N-methyl morpholine), NMR (Nuclear Magnetic Resonance), NBS (N-bromo succinimide), PBS (phosphate buffered saline), PMB (paramethoxybenzyl), RT (room temperature), TBAF (tetra-butylammonium fluoride), TBTU (*N*,*N*,*N'*,*N'*-tetramethyl-*O*-(benzotriazol-1-yl)uronium tetrafluoroborate), T3P (propane phosphonic acid anhydride), TEA (triethyl amine), TFA (trifluoroacetic acid), THF (tetrahydrofuran), PetEther (petroleum ether), TBME (tert-butyl methyl ether), TLC (thin layer chromatography), TMS (trimethylsilyl), TMSI (trimethylsilyl iodide), UV (ultraviolet).

In general, the compounds according to Formula (I) and related formulae of this invention can be prepared from readily available starting materials. If such starting materials are not commercially available, they may be prepared by standard synthetic techniques. In general, the synthesis pathways for any individual compound of Formula (I) and related formulae will depend on the specific substituents of each molecule, such factors being appreciated by those of ordinary skilled in the art. The following general methods and procedures described hereinafter in the examples may be employed to prepare compounds of Formula (I) and related formulae. Reaction conditions depicted in the following schemes, such as temperatures, solvents, or co-reagents, are given as examples only and are not restrictive. It will be appreciated that where typical or preferred experimental conditions (i.e. reaction temperatures, time, moles of reagents, solvents etc.) are given, other experimental conditions can also be used unless otherwise stated. Optimum reaction conditions may vary with the particular reactants or solvents used, but such conditions can be determined by the person skilled in the art, using routine optimisation procedures. For all the protection and deprotection methods, see Philip J. Kocienski, in "Protecting Groups", Georg Thieme Verlag Stuttgart, New York, 1994 and, Theodora W. Greene and Peter G. M. Wuts in "Protective Groups in Organic Synthesis", Wiley Interscience, 3rd Edition 1999.

Depending on the nature of R¹, R², R³, R^{a}, R^{d}, X, Y, Q, ⁻⁻⁻⁻ and n, different synthetic strategies may be selected for the synthesis of compounds of Formula (I). In the process illustrated in the following schemes, R¹, R², R³, R^{a}, R^{d}, X, Y, Q, ⁻⁻⁻⁻ and n, are as above defined in the description unless otherwise mentioned.

Generally, Oxo-oxa-aza-spiroalkene compounds of Formula (I) wherein ⁻⁻⁻⁻ , R¹, R², R³, R^{a}, R^{d}, X, Y, Q and n are defined as above can be prepared following the synthetic pathways described in the general scheme 1.

According to a preferred synthetic pathway, compounds of Formula (I), may be prepared from the corresponding derivatives of Formula (C3), either directly by reaction of compounds of Formula (C3) where R is an alkyl or benzyl group with an amine R^{a}-Q-X-H (E), wherein R^{a}, Q and X are as above defined and wherein H is linked to the nitrogen atom of X, or via the hydrolysis of the ester of Formula (C3) into an acid wherein R is H and subsequent coupling with an amine of Formula (E). Starting from the acid of Formula (C3) wherein R is H, compounds of Formula (I) can be obtained following usual conditions for the formation of an amide starting from a carboxylic acid and an amine R^{a}-Q-X-H by using coupling agents such as DCC, DIC, EDC, HATU or via the formation of an acid chloride or an activated ester. Preferred conditions consist in the treatment of compounds of Formula (C3) wherein R is H with a solution of propane phosphonic acid anhydride (T3P) 50% in EtOAc followed with the addition of the amine R^{a}-Q-X-H derivatives (E), in the presence of a base such as triethylamine in a suitable solvent such as THF at room temperature. The carboxylic acids can be obtained by hydrolysis of the corresponding esters using reagents such as, but not limited to, LiOH, NaOH or KOH in solvents such water, alcohol, THF, dioxane, or mixture thereof. The same amidation procedures can be applied from the intermediates of Formula (C2) to form the Intermediates of Formula (D2).

Alternatively, compounds (I) may be prepared from compounds of Formula (D1) by alkylation reaction with LG-Y-R² (F) wherein Y and R² are defined as above, and LG is a leaving group, preferably a halogen or a sulfonate group. Preferred conditions consist in the treatment of compounds of Formula (D1) with an alkyl halide in the presence of a base such as potassium carbonate, in a suitable solvent such as DMF at a temperature between room temperature and 150°C. The same alkylation procedures can be applied from the intermediates of Formula (C1) to form the Intermediates of Formula (C3).

Alternatively, alkylation of the intermediates (D1) and (C1) may be performed using a reductive amination protocole. Such a reaction may be carried out with a compound of Formula Y'-R², wherein R² is as above defined and Y' denotes a linear or branched alkyl having 1 to 6 carbon atoms or a cyclic alkyl having 3 to 7 carbon atoms, wherin one - CH₂- group, in these linear, branched, or cyclic alkyl groups, is replaced by a -CO-group. In the linear, branched or cyclic alkyl groups of Y', 1 to 5 H atoms, may be replaced by the groups substituting Y. This reaction is particularly suitable for the preparation of the compounds of Formula (I) wherein Y denotes a branched or linear alkylene having 1 to 6 carbon atoms or a cyclic alkylene having 3 to 7 carbon atoms, wherein 1 to 5 H atoms, in these cyclic, linear or branched alkylene, may be replaced by OR⁶, Hal, or CN.

The reductive amination step is typically performed in the presence of a reducing agent such as sodium triacetoxyborohydride, sodium cyanoborohydride, sodium borohydride or hydrogen in the presence of a catalyst such as Pd/C in a suitable solvent such as DCM, DCE, THF, acetic acids (or a mixture thereof). Prefered conditions are the use of sodium triacetoxyborohydride in DCM at room temperature.

Intermediates of Formulae (C1) and (D1) can be obtained from Intermediate (C2) wherein PG is an amino pretecting group such as Boc, Cbz, Benzyl, PMB or Fmoc by a suitable amine deprotection step. Prefered conditions for the deprotection of the Boc protected intermediates of Formula (C2) and (D2) are the treatement under acidic conditions using hydrochloric acid in a suitable solvent such as DCM, THF, dioxane (or a mixture thereof) at room temperature.

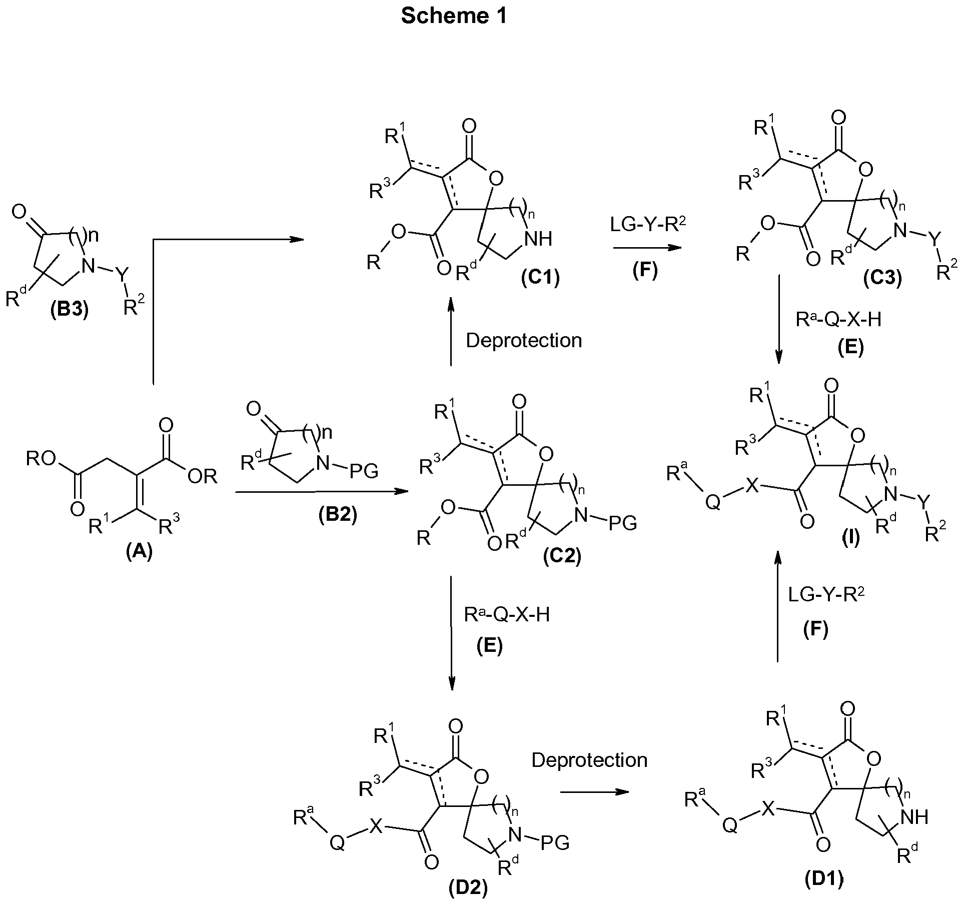

Compounds of Formula (C2) wherein R¹, R³, R^{d ----} and n are defined as above, wherein R is H, a linear or branched alkyl group having 1 to 6 carbon atoms, or a benzyl group, and wherein PG is an amino protecting group, can be prepared by the reaction of the 2-methylene-succinic ester of Formula (A) with a base such as a MeONa, tBuOK, EtONa, LiHDMS, LDA, and a protected amino ketone of Formula (B2) in a suitable solvent. Examples of appropriate solvents are THF or Et₂O. Depending on the reaction conditions or the work-up conditions, esters of Formula (C2), wherein R is a linear or branched alkyl group having 1 to 6 carbon atoms, or a benzyl group, or directly acids of Formula (C2) wherein R is H can be obtained. Prefered conditions for the cyclization steps are the use of sodium methoxide in a suitable solvent such as THF or Et₂O at a temperature between 0°C at 60°C.

A particularly attractive and convergent route for the preparation of Intermediates of Formula (C3) consists in the cyclization of amino ketone of Formula (B3), wherein Y and R² are defined as above, with 2-methylene-succinic ester of Formula (A) following the above process.

Amino ketone of Formulae (B2) or (B3) and the 2-methylene-succinic ester of Formula (A) may be obtained either from commercial sources, from literature procedures or from conventional procedures, well known by one skilled in the art.

Compounds of Formulae (I), (A), (B2), (B3), (C1), (C2), (C3), (D1), (D2), (E) and (F) may be converted to alternative compounds of Formulae (I), (A), (B2), (B3), (C1), (C2), (C3), (D1), (D2), (E) and (F) respectively, using suitable interconversion procedures such as those described hereinafter in the examples, or conventional interconversion procedures, well known by one skilled in the art.

Thus, another object of the present invention is a process for the manufacture of a compound of Formula (I) wherein a compound of Formula (C3), 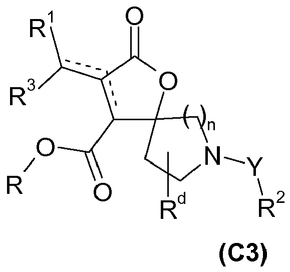 wherein R¹, R³, Y, R^{d}, R² and n are as above defined and R is an alkyl or a benzyl group, is reacted with a compound of Formula R^{a}-Q-X-H (E), wherein R^{a}, Q, and X are as above defined and wherein H is linked to the nitrogen atom of X,
or wherein a compound of Formula (D1) 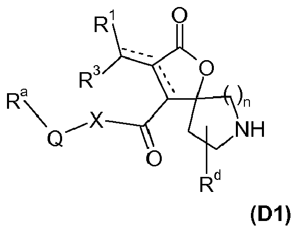 wherein R¹, R², R^{a}, R^{d}, Q, X and n are as above defined, is reacted with a compound of Formula LG-Y-R² (F), wherein Y and R² are as above defined and LG is a living group, or wherein a compound of Formual (D1), wherein R¹, R², R^{a}, R^{d}, Q, X and n are as above defined, is reacted with a compound of Formula Y'-R², wherein R² and Y' are as above defined.

If the above set of general synthetic methods is not applicable to obtain compounds according to Formula (I) and/or necessary intermediates for the synthesis of compounds of Formula (I), suitable methods of preparation known by a person skilled in the art should be used.

In general, the synthesis pathways for any individual compounds of formula (I) will depend on the specific substitutents of each molecule and upon the ready availability of Intermediates necessary; again such factors being appreciated by those of ordinary skill in the art. For all the protection and de-protection methods, see Philip J. Kocienski, in "Protecting Groups", Georg Thieme Verlag Stuttgart, New York, 1994 and, Theodora W. Greene and Peter G. M. Wuts in "Protective Groups in Organic Synthesis", Wiley Interscience, 3rd Edition 1999.

Compounds of this invention can be isolated in association with solvent molecules by crystallization from evaporation of an appropriate solvent. The pharmaceutically acceptable acid addition salts of the compounds of formula (I), which contain a basic center, may be prepared in a conventional manner. For example, a solution of the free base may be treated with a suitable acid, either neat or in a suitable solution, and the resulting salt isolated either by filtration or by evaporation under vacuum of the reaction solvent. Pharmaceutically acceptable base addition salts may be obtained in an analogous manner by treating a solution of compounds of formula (I), which contain an acid center, with a suitable base. Both types of salts may be formed or interconverted using ion-exchange resin techniques.

Depending on the conditions used, the reaction times are generally between a few minutes and 14 days, and the reaction temperature is between about -30°C and 140°C, normally between -10°C and 90°C, in particular between about 0°C and about 70°C.

Compounds of the formula (I) can furthermore be obtained by liberating compounds of the formula (I) from one of their functional derivatives by treatment with a solvolysing or hydrogenolysing agent.

Preferred starting materials for the solvolysis or hydrogenolysis are those which conform to the formula (I), but contain corresponding protected amino and/or hydroxyl groups instead of one or more free amino and/or hydroxyl groups, preferably those which carry an amino-protecting group instead of an H atom bound to an N atom, in particular those which carry an R'-N group, in which R' denotes an amino-protecting group, instead of an HN group, and/or those which carry a hydroxyl-protecting group instead of the H atom of a hydroxyl group, for example those which conform to the formula (I), but carry a - COOR" group, in which R" denotes a hydroxylprotecting group, instead of a -COOH group.

It is also possible for a plurality of - identical or different - protected amino and/or hydroxyl groups to be present in the molecule of the starting material. If the protecting groups present are different from one another, they can in many cases be cleaved off selectively.

The term "amino-protecting group" is known in general terms and relates to groups which are suitable for protecting (blocking) an amino group against chemical reactions, but which are easy to remove after the desired chemical reaction has been carried out elsewhere in the molecule. Typical of such groups are, in particular, unsubstituted or substituted acyl, aryl, aralkoxymethyl or aralkyl groups. Since the amino-protecting groups are removed after the desired reaction (or reaction sequence), their type and size are furthermore not crucial; however, preference is given to those having 1-20, in particular 1-8, carbon atoms. The term "acyl group" is to be understood in the broadest sense in connection with the present process. It includes acyl groups derived from aliphatic, araliphatic, aromatic or heterocyclic carboxylic acids or sulfonic acids, and, in particular, alkoxy-carbonyl, aryloxycarbonyl and especially aralkoxycarbonyl groups. Examples of such acyl groups are alkanoyl, such as acetyl, propionyl and butyryl; aralkanoyl, such as phenylacetyl; aroyl, such as benzoyl and tolyl; aryloxyalkanoyl, such as POA; alkoxycarbonyl, such as methoxy-carbonyl, ethoxycarbonyl, 2,2,2-trichloroethoxycarbonyl, BOC (tert-butoxy-carbonyl) and 2-iodoethoxycarbonyl; aralkoxycarbonyl, such as CBZ ("carbo-benz-oxy"), 4-methoxybenzyloxycarbonyl and FMOC; and aryl-sulfonyl, such as Mtr. Preferred amino-protecting groups are BOC and Mtr, furthermore CBZ, Fmoc, benzyl and acetyl.

The term "hydroxyl-protecting group" is likewise known in general terms and relates to groups which are suitable for protecting a hydroxyl group against chemical reactions, but are easy to remove after the desired chemical reaction has been carried out elsewhere in the molecule. Typical of such groups are the above-mentioned unsubstituted or substituted aryl, aralkyl or acyl groups, furthermore also alkyl groups. The nature and size of the hydroxyl-protecting groups are not crucial since they are removed again after the desired chemical reaction or reaction sequence; preference is given to groups having 1-20, in particular 1-10, carbon atoms. Examples of hydroxyl-protecting groups are, inter alia, benzyl, 4-methoxybenzyl, p-nitro-benzoyl, p-toluenesulfonyl, tert-butyl and acetyl, where benzyl and tert-butyl are particu-larly preferred.

The term "solvates of the compounds" is taken to mean adductions of inert solvent molecules onto the compounds which form owing to their mutual attractive force. Solvates are, for example, mono- or dihydrates or alcoholates.

The compounds of the formula (I) are liberated from their functional derivatives - depending on the protecting group used - for example using strong acids, advantageously using TFA or perchloric acid, but also using other strong inorganic acids, such as hydrochloric acid or sulfuric acid, strong organic carboxylic acids, such as trichloroacetic acid, or sulfonic acids, such as benzene- or p-toluenesulfonic acid. The presence of an additional inert solvent is possible, but is not always necessary. Suitable inert solvents are preferably organic, for example carboxylic acids, such as acetic acid, ethers, such as THF or dioxane, amides, such as DMF, halogenated hydrocarbons, such as DCM, furthermore also alcohols, such as methanol, ethanol or isopropanol, and water. Mixtures of the above-mentioned solvents are furthermore suitable. TFA is preferably used in excess without addition of a further solvent, and perchloric acid is preferably used in the form of a mixture of acetic acid and 70% perchloric acid in the ratio 9:1. The reaction temperatures for the cleavage are advantageously between about 0 and about 50°C, preferably between 15 and 30°C (RT).

The BOC, OBut and Mtr groups can, for example, preferably be cleaved off using TFA in DCM or using approximately 3 to 5N HCI in dioxane at 15-30°C, and the FMOC group can be cleaved off using an approximately 5 to 50% solution of dimethylamine, diethylamine or piperidine in DMF at 15-30°C.

Protecting groups which can be removed hydrogenolytically (for example CBZ, benzyl or the liberation of the amidino group from the oxadiazole derivative thereof) can be cleaved off, for example, by treatment with hydrogen in the presence of a catalyst (for example a noble-metal catalyst, such as palladium, advantageously on a support, such as carbon). Suitable solvents here are those indicated above, in particular, for example, alcohols, such as methanol or ethanol, or amides, such as DMF. The hydrogenolysis is generally carried out at temperatures between about 0 and 100°C and pressures between about 1 and 200 bar, preferably at 20-30°C and 1-10 bar. Hydrogenolysis of the CBZ group succeeds well, for example, on 5 to 10% Pd/C in methanol or using ammonium formate (instead of hydrogen) on Pd/C in methanol/DMF at 20-30°C.

Examples of suitable inert solvents are hydrocarbons, such as hexane, petroleum ether, benzene, toluene or xylene; chlorinated hydrocarbons, such as trichloroethylene, 1,2-dichloroethane, tetrachloromethane, tri-fluoro-methylbenzene, chloroform or DCM; alcohols, such as methanol, ethanol, isopropanol, n-propanol, n-butanol or tert-butanol; ethers, such as diethyl ether, diisopropyl ether, tetrahydrofurane (THF) or dioxane; glycol ethers, such as ethylene glycol monomethyl or monoethyl ether or ethylene glycol dimethyl ether (diglyme); ketones, such as acetone or butanone; amides, such as acetamide, dimethylacetamide, N-methylpyrrolidone (NMP) or dimethyl-formamide (DMF); nitriles, such as acetonitrile; sulfoxides, such as dimethyl sulfoxide (DMSO); carbon disulfide; carboxylic acids, such as formic acid or acetic acid; nitro compounds, such as nitromethane or nitrobenzene; esters, such as EtOAc, or mixtures of the said solvents.

Esters can be saponified, for example, using LiOH, NaOH or KOH in water, water/THF, water/THF/ethanol or water/dioxane, at temperatures between 0 and 100°C. Furthermore, ester can be hydrolysed, for example, using acetic acid, TFA or HCL.

Free amino groups can furthermore be acylated in a conventional manner using an acyl chloride or anhydride or alkylated using an unsubstituted or substituted alkyl halide or reacted with CH₃-C(=NH)-OEt, advantageously in an inert solvent, such as DCM or THF and/or in the presence of a base, such as triethylamine or pyridine, at temperatures between -60°C and +30°C.

Throughout the specification, the term leaving group preferably denotes Cl, Br, I or a reactively modified OH group, such as, for example, an activated ester, an imidazolide or alkylsulfonyloxy having 1-6 carbon atoms (preferably methylsulfonyloxy or trifluoromethylsulfonyloxy) or arylsulfonyloxy having 6-10 carbon atoms (preferably phenyl- or p-tolylsulfonyloxy).
Radicals of this type for activation of the carboxyl group in typical acylation reactions are described in the literature (for example in the standard works, such as Houben-Weyl, Methoden der organischen Chemie [Methods of Organic Chemistry], Georg-Thieme-Verlag, Stuttgart).
Activated esters are advantageously formed in situ, for example through addition of HOBt or N-hydroxysuccinimide.

The term "pharmaceutically usable derivatives" is taken to mean, for example, the salts of the compounds of the formula I and so-called prodrug compounds.
The term "prodrug derivatives" is taken to mean compounds of the formula I which have been modified with, for example, alkyl or acyl groups, sugars or oligopeptides and which are rapidly cleaved in the organism to form the active compounds.
These also include biodegradable polymer derivatives of the compounds according to the invention, as described, for example, in Int. J. Pharm. 115, 61-67 (1995).

### EXPERIMENTAL PART

The microwave chemistry was performed on a single mode microwave reactor Emrys^{™} Optimiser or Initiator^{™} Sixty from Biotage.

The compounds of invention have been named according to the standards used in the program "ACD/Name Batch" from Advanced Chemistry Development Inc., ACD/Labs (7.00 Release). Product version: 7.10, build: 15 Sep 2003

The compounds according to formula (I) can be prepared from readily available starting materials by several synthetic approaches, using both solution-phase and solid-phase chemistry protocols or mixed solution and solid phase protocols. Examples of synthetic pathways are described below in the examples. Unless otherwise stated, compounds of Formula (I) and related formulae obtained as a racemic mixture can be separated to provide an enantiomerically enriched mixture or a pure enantiomer.

The commercially available starting materials used in the following experimental description were purchased from Aldrich, Sigma, ACROS or ABCR unless otherwise reported. SPE cartridges were purchased from IST and used following supplier recommendations.

**¹H NMR analyses** were carried out using BRUKER NMR, model AVANCE II-400 MHz FT-NMR equipped with Ultra-Shield superconducting Magnet system model BC-94/54, 54 mm bore (operational field at 9.397 T) and Bruker high performance Shim System (BOSS1) / Bruker Smart Magnet Control System (BSMS). It also consists of a standard geometry 5 mm Broadband Observe probe (BBO) with z-gradient and a BAC-60 sample-Changer. Residual signal of deuterated solvent was used as internal reference. Chemical shifts (δ) are reported in relative to the residual solvent signal (δ = 2.49 for ¹H NMR in DMSO-d₆). 1 H NMR data are reported as follows: chemical shift (multiplicity, coupling constants, and number of hydrogens). Multiplicity is abbreviated as follows: s (singlet), d (doublet), dd (doublet of doublet), t (triplet), q (quartet), m (multiplet), br (broad).
**LC/MS analyses** of the below examples were carried out using Agilent LC/MSD, model SL mass spectrometer equipped with a single quadrupole mass analyzer, equipped with a Diode array detector and an Agilent 1200 series HPLC system and APCI/APESI multi-ion source.
**The HPLC** data in the examples described below were recorded on Agilent 1100 or 1200 Series equipments, equipped with a Diode array detector.
**The preparative SFC purifications** of the below examples were performed with a Thar SFC-80 equipment.
**The preparative HPLC purifications** of the below examples were performed with a Waters mass directed autopurification Fractionlynx (equipped with a Photodiode Array Detector, a ZQ Mass Detector and MassLynx Software with the FractionLynx Application Manager), following the conditions in the below table.

| **Method Name** | **Type** | **Column** | **Mobile Phase** | **Flow** | **Gradient** | **Detection** |
|---|---|---|---|---|---|---|
| **Method A** | HPLC/ MS | Waters X Bridge C8 (50 x 4.6 mm, 3.5 µm) | A-0.1% TFA in H₂O B - 0.1% TFA in ACN | 2.0 mL/min | 8 min gradient from 5 % B to 100 % B in A | UV (Max. Chrom, 254 nm or 220 nm) |
| **Method B** | HPLC/ MS | Waters X Bridge C8 (50 x 4.6 mm, 3.5 µm) | A-10mM NH₄HCO₃ in H₂O B-ACN | 1.0 mL/min | 8 min gradient from 5% B to 100 % B in A | UV (Max. Chrom, 254 or 220 nm) |
| **Method C** | HPLC/ MS | Waters ATLANTIS d C18 (50 x 4.6 mm, 5 µm) | A-0.1 %HCOOH in H₂O B-ACN | 1.0 mL/min | 5 min gradient from 30 % B to 95 % B in A | UV (Max. Chrom, 254 or 220 nm) |
| **Method E** | HPLC/ MS | Waters X Bridge C8 (50 x 4.6 mm, 3.5 µm) | A-10mM NH₄HCO₃ B-ACN | 1.0 mL/min | 8 min gradient from 5 % B to 100 % B in A | Mass (Positive mode) and UV (Max. Chrom, 254 or 220 nm) |
| **Method D** | HPLC/ MS | Waters X Bridge C8 (50 x 4.6 mm, 3.5 µm) | A-0.1% TFA in H₂O B-0.1% TFA in ACN | 2.0 mL/min | 8 min gradient from 5 % B to 100 % B in A | UV Wave length (nm): 220-400(scan range), 254 or 220 |
| **Method F** | HPLC/ MS | Acquity BEH C-18 (2.1 x 100 mm, 1.7 µm) | A-5mM NH₄CO₃ B-ACN | 0.3 mL/min | 10 min gradient from 10 % B to 90 % B in A | Mass (Positive mode) and UV (Max. Chrom, 254 or 220 nm) |
| **Method G** | UPLC/ MS | Chromolith SpeedRod RP-18e (50 x 4.6 mm) | A-0.1 % TFA in H₂O B - 0.1 % TFA in ACN | 2.4 mL/min | 8 min gradient from 2 % B to 100 % B in A | Mass (Positive mode) and UV (Max. Chrom, 254 or 220 nm) |
| **Method H** | HPLC/ MS | Gemini C18 (50 x 2 mm, 3.5 µm) | A-10mM NH₄CO₃ in H₂O B - 10 mM NH₄CO₃ in ACN/H₂O (95/5) | 0.8 mL/min | 6 min gradient From 2 % B to 98 % B in A | Mass (Positive mode) and UV (Max. Chrom.) |
| **Method I** | HPLC/MS | Waters X Bridge C18 (30 x 2.1 mm, 3.5 µm) | A-10mM NH₄CO₃ in H₂O B-10mM NH₄CO₃ in ACN/H₂O (95/5) | 1.0 mL/min | 6 min gradient from 2 % B to 98 % B in A | Mass (Positive mode) and UV (Max. Chrom.) |
| **Method J** | Preparative HPLC | Waters X Bridge C8 (50 x 19 mm, 5 um) OBD | A - 10mM NH₄CO₃ B - 10mM NH₄CO₃ in ACN | 25 mL/min | 8.5 min gradient from 5 % B to 95%B in A | Mass (Positive mode) and UV |
| **Method K** | Preparative SFC | Daicel Chiralpak IC (250 x 20 mm) | A - CO₂ B - 0.1% ammonia in I PA | 30.0 g/mL | 70% A / 30 % B | UV Wave length (nm) : 220-400 (scan range), 254 or 220 |
| **Method L** | Chiral HPLC | Daicel AD-H (250 x 4.6 mm, 5 µm) | A-0.1% DEA in Hexane B-IPA | 1.0 mL/min | 80% A / 20 %B | UV Wave length (nm) : 220 |

### Preparation of the intermediates

### Intermediate 1: 8-ethyl-3-methyl-2-oxo-1-oxa-8-aza-spiro[4.5]dec-3-ene-4-carboxylic acid, hydrochloride salt

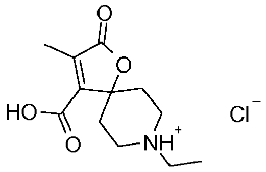

Dimethyl itaconate (5.0 g, 3.9 mmol) and N-ethyl-4-piperidone (6.2 g, 3.9 mmol) were added into a suspension of sodium methoxide (4.2 g, 8.62 mmol) in tetrahydrofuran (50 mL) at 0 °C over 1 hour. The reaction mixture was stirred 2 hours at 0 °C, and then 12 additional hours at RT. The reaction mixture was diluted with water and the organic solvent was removed under reduced pressure. The aqueous layer was acidified with a 1.5N aqueous solution of HCl and concentrated under vacuum. After purification by crystallization from ethanol, the title compound was obtained as a white solid (4.5 g, 63 %). ¹H NMR (DMSO-d₆, 400 MHz): δ 3.35 (m, 2H), 3.05 (m, 2H), 2.66 (m, 4H), 1.85 (s, 3H), 1.40-1.37 (m, 2H), 1.12 (t, *J*=6.6 Hz, 3H). LCMS (Method E): Mass found (M+ 240), Rt (min): 1.34, Area (%): 98.5 (Max. Chrom.), 98.2 (254 nm).

### Intermediate 2: 3-methyl-2-oxo-1-oxa-8-aza-spiro[4,5]dec-3-ene-4,8-dicarboxylic acid 8-tert-butyl ester

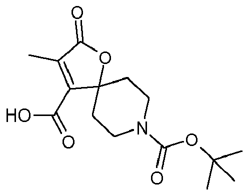

A solution of dimethylitaconate (8.0 g, 5.05 mmol) and tert-butyl 4-oxopiperidine-1-carboxylate (10.0 g, 5.05 mmol) in anhydrous tetrahydrofuran was added into a suspension of sodium methoxide (5.45 g, 10.1 mmol) in anhydrous tetrahydrofuran (75 mL) at 0 °C over a period of 30 minutes. The reaction mixture was stirred 2.5 hours at 0°C, and then 6 hours at room temperature. Water (20 mL) was then added slowly and the solution was stirred for 1 hour. The reaction mixture was concentrated and the residue was taken in ethylacetate (3 x 100 mL), the aqueous layer was acidified with 1 N HCl to pH~2, the solid precipitated was extracted with ethylacetate (2x100 mL). The combined organic layers were washed with water (100 mL), brine solution (100 mL), dried over anhydrous MgSO4 and concentrated to get the product as off white solid that is used without further pourification (5.7 g, 36 %). TLC: chloroform/methanol (9.5/0.5) R_{f}: 0.1. ¹H NMR (400 MHz, DMSO-d₆): δ 13.94 (br s, 1H), 4.00 (m, 2H), 2.94 (m, 2H), 2.21 (m, 2H), 2.05 (s, 3H), 1.43 (m, 11 H). LCMS (Method C): Mass found (M+ 212.2 [M-Boc+2H]⁺), Rt (min): 3.65, Area (%): 91.4.

### Intermediate 3: 8-isopropyl-3-methyl-2-oxo-1-oxa-8-azaspiro[4.5]dec-3-ene-4-carboxylic acid, hydrochloride salt

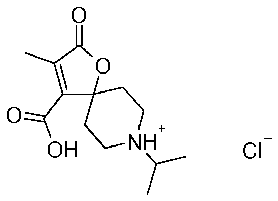

### Step 1: Preparation of 1,1-dimethyl-4-oxopiperidinium iodide

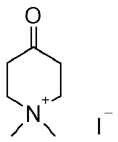

Methyl iodide (1.6 mL, 26.5 mmol) was added into a solution of 1-methyl-piperidin-4-one (2.0 g, 17.6 mmol) in acetone (10 mL) at 0 °C. The reaction mixture was stirred at room temperature for 1 hour. The precipitate was filtered off and washed with acetone to afford the title compound as an off-white solid (4.0 g, 88 %). ¹H NMR (D₂O, 400 MHz): δ 3.38 (t, J=5.8 Hz, 4H), 3.06 (s, 6H), 1.95-2.05 (m, 4H). LCMS (Method D): Mass found (M+ 128.2), Rt (min): 1.19, Area (%): 95.3 (Max. Chrom.)

### Step 2: Preparation of 1-isopropyl-piperidin-4-one

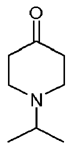

Potassium carbonate (4.2 g, 31.1 mmol) was added into a solution of isopropylamine (2.1 mL, 3.3 mmol) in ethanol (10 mL) and the resulting mixture was heated at 75 °C. A solution of 1,1-dimethyl-4-oxopiperidinium iodide (4.0 g, 15.5 mmol) in water (5 mL) was added over a period of 30 minutes. The reaction mixture was stirred 1 hour at 75 °C, and then 12 hours at room temperature. The reaction mixture was concentrated under reduced pressure. The residue was taken up with water (25 mL) and extracted with dichloromethane (25 mL). The organic layer was dried (Na₂SO₄) and concentrated under vacuum to afford the title compound as a colourless oil (1.5 g, 69 %). ¹H NMR (DMSO-d₆, 400 MHz): δ 2.89-2.83 (m, 1 H), 2.67 (t, *J*=5.9 Hz, 4H), 2.29 (t, *J*=5.9 Hz, 4H), 0.98 (d, *J*=6.5 Hz, 6H). LCMS (Method C): Mass found (M+ 142.2), Rt (min): 0.63, Area (%): 99.4 (Max. Chrom.)

### Step 3: Preparation of 8-isopropyl-3-methyl-2-oxo-1-oxa-8-aza-spiro[4.5]dec-3-ene-4-carboxylicacid, hydrochloride salt

A mixture of dimethylitaconate (1.6 g, 10.6 mmol) and 1-isopropyl-piperidine-4-one (1.5 g, 10.6 mmol) was added into a solution of sodium methoxide (1.1 g, 21.2 mmol) in tetrahydrofuran (50 mL) at 0 °C over 1 hour. The reaction mixture was stirred 2 hours at 0 °C, and then 12 hours at room temperature. The reaction mixture was diluted with water and the organic solvent was removed under reduced pressure. The aqueous layer was acidified with a 1.5N aqueous solution of HCl and concentrated under vacuum. After purification by crystallization from ethanol, the title compound was obtained as a white solid (1.0 g, 32 % yield). ¹H NMR (DMSO-d₆, 400 MHz): δ 3.50-3.45 (m, 2H), 2.98 (m, 2H), 2.89-2.86 (m, 3H), 1.87 (s, 3H), 1.63-1.60 (m, 2H), 1.30 (d, J=6.4 Hz, 6H). LCMS (Method D): Mass found (M+ 254), Rt (min): 1.44, Area (%): 97.6 (Max. Chrom.)

### Intermediate 4: 8-ethyl-3-methyl-2-oxo-1-oxa-8-azaspiro[4.5]dec-3-ene-4-carboxylate, sodium salt

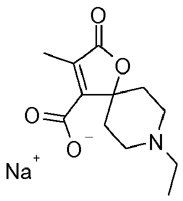

### Step 1: Preparation of methyl 8-ethyl-3-methyl-2-oxo-1-oxa-8-azaspiro[4.5]dec-3-ene-4-carboxylate

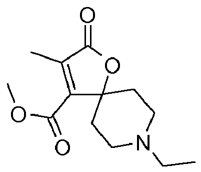

A solution of 1-ethyl-4-piperidone (17.13 mL, 126 mmol) and dimethyl itaconate (8.90 mL, 63.2 mmol) in anhydrous diethyl ether (200 mL) was added into a suspension of sodium methoxide (3.42 g, 63.2 mmol) in anhydrous diethyl ether (100 mL) at 0°C over a period of 2 hours. The reaction mixture was stirred 2 hours at 0 °C, and then at room temperature overnight. The reaction mixture was diluted with water (200 mL) and extracted with diethyl ether (3x100 mL). The combined organic layers were washed with brine, dried (Na₂SO₄) and concentrated under vacuum to give the title compund as a yellow oil (5 g), used without further purification. LCMS (Method I); Area (%) : 82.0.

### Step 2: Preparation of 8-ethyl-3-methyl-2-oxo-1-oxa-8-azaspiro[4.5]dec-3-ene-4-carboxylate, sodium salt

Sodium hydroxide (0.371 g, 9.28 mmol) was added into a solution of methyl 8-ethyl-3-methyl-2-oxo-1-oxa-8-azaspiro[4.5]dec-3-ene-4-carboxylate (2.35 g, 9.28 mmol) in a mixture of water and THF (1:1, 20 mL). The reaction mixture was stirred at room temperature for 3 hours. The solvent was removed under vacuum. The residue was taken up with water (30 mL) and washed with ethyl acetate (2x20 mL). The aqueous layer was lyophilized to give the title compound as a yellow solid (1.75 g), used in the next steps without further purification.

### Intermediate 5: 8-benzyl-3-methyl-2-oxo-1-oxa-8-azaspiro[4.5]dec-3-ene-4-carboxylic acid

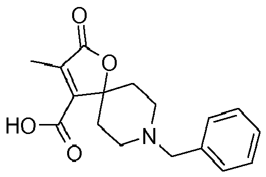

Sodium ethoxide (16.44 mmol) was prepared by the addition of sodium (378 mg, 16.44 mmoles) in anhydrous EtOH (5 mL) at 0-5 °C. 40 mL of Et₂O were then added, followed by 1-benzylpiperidin-4-one (3.1 g, 16.44 mmol) and dimethyl itaconate (1.3 g, 8.22 mmol) dissolved in Et₂O (8 mL). The resulting mixture was stirred 2 hours at 0 °C (under N₂ atmosphere), then 10 hours at RT. The reaction mixture was diluted with diethyl ether (20 mL) and water (10 mL). The aqueous layer was separated and neutralized by the addition of an aquaeous solution of HCl (1 N) to reach pH 7. The aqueous solution was then extracted with AcOEt (3 x 30 mL). The combined organic layers were washed with brine, dried (Na₂SO₄) and concentrated to give the title compound as a yellow solid used without further purifications. HPLC, Rt (min): 1.14, Area (%): 88.6 (Max. Chrom.).

### Intermediate 6: 3,8-dimethyl-2-oxo-1-oxa-8-azaspiro[4.5]dec-3-ene-4-carboxylic acid

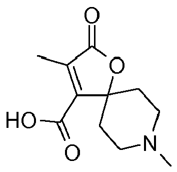

The title Intermediate 6 was obtained following the procedure described for Intermediate 5, but starting 1-methylpiperidin-4-one. The title Intermediate was obtained as a yellow solid used without further purifications. LCMS: Mass found (M- 224.0).

### Intermediate 7: 8-[2-(2-methoxyphenyl)ethyl]-3-methyl-2-oxo-1-oxa-8-azaspiro[4.5]dec-3-ene-4-carboxylic acid, hydrochloride salt

### Step 1: Preparation of 1-[2-(2-methoxy-phenyl)-ethyl]-piperidin-4-one

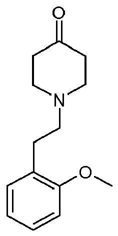

A mixture of 4-piperidone hydrochloride monohydrate (2.0 g, 14.8 mmol), 2-methoxy phenyl ethyl bromide (2.5 g, 11.7 mmol) and anhydrous potassium carbonate (7.2 g, 52.1 mmol) was prepared in DMF (65 mL) and heated at 60 °C for 2 hours. The reaction mixture was concentrated under vacuum. The residue was taken up with ethyl acetate and washed with brine. The organic layer was dried (Na₂SO₄) and concentrated under vacuum. After purification by flash chromatography on silica, the title compound was obtained as a yellow solid (1.1 g, 36 %). TLC: ethylacetate/hexane (5/5): *R_{f}* : 0.6. ¹H NMR (DMSO-d₆, 400 MHz): δ 7.21-7.14 (m, 2H), 6.84-6.75 (m, 2H), 3.83 (s, 3H), 2.84-2.80 (m, 6H), 2.72-2.64 (m, 2H), 2.46-2.43 (m, 4H).

### Step 2: Preparation of 8-[2-(2-methoxyphenyl)ethyl]-3-methyl-2-oxo-1-oxa-8-azaspiro[4.5]dec-3-ene-4-carboxylic acid, hydrochloride salt

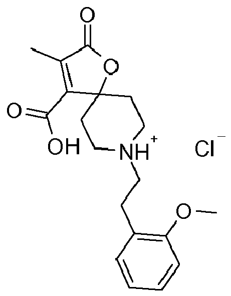

A mixture of dimethyl itaconate (0.75 g, 4.7 mmol) and 1-[2-(2-methoxy-phenyl)-ethyl]-piperidin-4-one (1.1 g, 4.7 mmol) was added into a solution of sodium methoxide (0.51 g, 9.4 mmol) in tetrahydrofuran (50 mL) at 0 °C over 1 hour. The reaction mixture was stirred 2 hours at 0 °C, and then 12 hours at room temperature. The reaction mixture was diluted with water and the organic solvent was removed under vacuum. The aqueous layer was acidified with a 1.5N aqueous solution of HCl and concentrated under reduced pressure. After purification by crystallization from ethanol, the title compound was obtained as a white solid (0.50 g, 31 %). LCMS (Method D): Mass found (M+ 346.3), Rt (min): 2.89, Area (%): 60.9 (Max. Chrom.)

### Intermediate 8: 1-[2-(4-fluorophenyl)ethyl]piperazine, hydrochloride salt

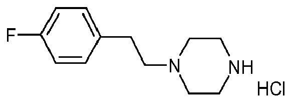

### Step 1: Preparation of 1-(2-bromoethyl)-4-fluorobenzene

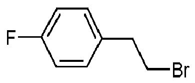

Phosphorus tribromide (19.3 g, 71.3 mmol) was added slowly into a solution of 2-(4-fluorophenyl)ethanol (5.0 g, 35.7 mmol) in hexane at 0 °C. After 12 hours under stirring, the reaction mixture was diluted with a 10% aqueous solution of NaHCO₃ and ethyl acetate. The organic layer was washed with water and brine, dried (Na₂SO₄) and concentrated under vacuum to give the title compound (5.7 g, 78 %). TLC: ethylacetate/ Hexane (3/7): *R_{f}*: 0.85. ¹H NMR (CDCl₃, 400 MHz): δ 7.20-7.16 (m, 2H), 7.03-6.99 (m, 2H), 3.56 (t, J=7.40 Hz, 2H), 3.15 (t, J=7.44 Hz, 2H).

### Step 2: Preparation of tert-butyl 4-[2-(4-fluorophenyl)ethyl]piperazine-1-carboxylate

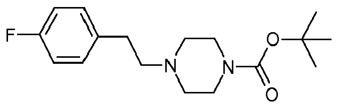

Tert-butyl piperazine-1-carboxylate (5.5 g, 29.6 mmol) was added portionwise into a solution of 1-(2-bromoethyl)-4-fluorobenzene (3.0 g, 14.8 mmol) in dichloromethane at 0 °C. The resulting mixture was stirred for 12 hours. The reaction mixture was diluted with dichloromethane, and then washed with water and brine, dried (Na₂SO₄) and concentrated under vacuum. Purification by flash chromatography (neutral alumina, pet ether 96 / EtOAc 4) gave the title compound (1.99 g, 44 %). TLC: ethylacetate/ hexane (3/7): *R_{f}*: 0.3. LCMS (Method C): Mass found (M+ 309.2), Rt (min): 2.39, Area (%): 74.9 (Max. chrom.).

### Step 3: Preparation of 1-[2-(4-fluorophenyl)ethyl]piperazine, hydrochloride salt

A 4N solution of HCl in 1,4-dioxane (4 mL) was added into a solution of tert-butyl 4-[2-(4-fluorophenyl)ethyl]piperazine-1-carboxylate (0.99 g, 3.21 mmol) in anhydrous 1,4-dioxane (6 mL) and the resulting mixture was stirred 3 hours at room temperature. The reaction mixture was concentrated under vacuum. The residue was triturated with diethyl ether (100 mL), filtered off under nitrogen and dried under vacuum to give the title compound as a white solid (0.66 g, 84 %). LCMS (Method C): Mass found (M+ 209.0), Rt (min): 0.77, Area (%): 97.8 (Max), 97.0 (254 nm).

### Intermediate 9: N-[3-(trifluoromethyl)phenyl]piperidin-4-amine, hydrochloride salt

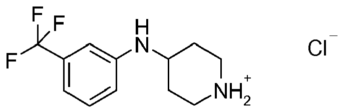

### Step 1: Preparation of tert-butyl 4-{[3-(trifluoromethyl)phenyl]amino}piperidine-1-carboxylate

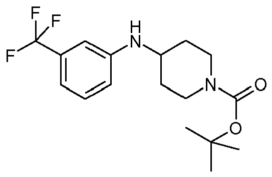

Cesium carbonate (1.4 g, 4.4 mmol), tert-butyl 4-aminopiperidine-1-carboxylate (0.5 g, 2.6 mmol), BINAP (0.11 g, 0.177 mmol) and palladium acetate (0.024 g, 0.111 mmol) were added into a suspension of 3-trifluoromethyl-phenylamine (0.5 g, 2.20 mmol) in 1,4-dioxane (10 mL). The resulting mixture was heated at 90 °C for 16 hours. The reaction mixture was concentrated under vacuum. After purification by flash chromatography (chloroform/methanol), the title compound was obtained as a white solid (0.42 g, 55 %). ¹H NMR (DMSO-d₆, 400 MHz) δ 7.24 (m, 1 H), 6.83 (m, 2H), 6.77 (d, *J* = 7.6 Hz, 1 H), 6.02 (d, *J* = 8.1 Hz, 1 H), 3.86-3.82 (m, 2H), 3.48-3.46 (m, 1 H), 2.92 (m, 2H), 1.87-1.84 (m, 2H), 1.38 (s, 9H), 1.22-1.19 (m, 2H).

### Step 2: Preparation of N-[3-(trifluoromethyl)phenyl]piperidin-4-amine, hydrochloride salt

A 4N solution of HCl in 1,4-dioxane (8 mL) was added into a suspension of tert-butyl 4-{[3-(trifluoromethyl)phenyl]amino}piperidine-1-carboxylate (0.42 g, 1.22 mmol) in 1,4-dioxane (8 mL). The resulting mixture was stirred at room temperature for 4 hours. The reaction mixture was concentrated under vacuum to give the title compound as a white solid (0.27 g, 38 %). ¹H NMR (DMSO-d₆, 400 MHz) δ 9.05 (br s, 2H), 7.28 (m, 1 H), 6.92 (m, 2H), 6.84 (d, *J* = 7.4 Hz, 1 H), 3.62-3.58 (m, 1 H), 3.27-3.24 (m, 2H), 2.98-2.95 (m, 2H), 2.03-1.99 (m, 2H), 1.65-1.57 (m, 2H).

### Intermediate 10: 1-[5-(trifluoromethyl)pyridin-3-yl]piperazine, hydrochloride salt

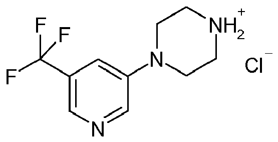

### Step 1: Preparation of tert-butyl 4-[5-(trifluoromethyl)pyridin-3-yl]piperazine-1-carboxylate

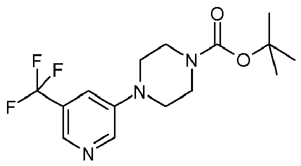

Sodium tert-butoxide (0.31 g, 3.3 mmol), tert-butyl piperazine-1-carboxylate (0.49 g, 2.6 mmol), xanthphos (0.038 g, 0.066 mmol) and Pd(dba)₂ (13 mg, 0.022 mmol) were added into a suspension of 3-bromo-5-trifluoromethyl-pyridine (0.5 g, 2.2 mmol) in toluene (20 mL). The resulting mixture was heated at 90 °C for 16 hours. The reaction mixture was concentrated under vacuum. After purification by flash chromatography (chloroform/methanol), the title compound was obtained as a white solid (0.51 g, 69 %). LCMS (Method C): Mass found (M+ 332.2), Rt (min): 4.19, Area (%): 48 (Max).

### Step 2: Preparation of 1-[5-(trifluoromethyl)pyridin-3-yl]piperazine, hydrochloride salt

A 4N solution of HCl in 1,4-dioxane (10 mL) was added into a suspension tert-butyl 4-[5-(trifluoromethyl)pyridin-3-yl]piperazine-1-carboxylate (0.51 g, 1.54 mmol) in 1,4-dioxane (10 mL). The resulting mixture was stirred at room temperature for 4 hours. The reaction mixture was concentrated under vacuum to give the title compound as a white solid (0.39 g, 59 %). LCMS (Method C): Mass found (M+ 232.2), Rt (min): 1.13, Area (%): 99 (Max).

### Intermediate 11: 1-(3,3,3-trifluoropropyl)piperazine, hydrochloride salt

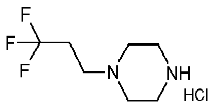

### Step 1: Preparation of tert-butyl 4-(3,3,3-trifluoropropyl)piperazine-1-carboxylate

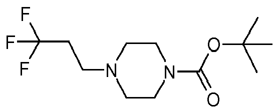

A solution of tert-butyl piperazine-1-carboxylate (3.32 g, 17.8 mmol) in a mixture of tetrahydrofuran (15 mL) and methanol (10 mL) was cooled at 0°C. A solution of 3,3,3-trifluoropropanaldehyde (1.0 g, 8.9 mmol) in tetrahydrofuran (25 mL) was added, followed by acetic acid (0.5 mL, 8.9 mmol) and sodium cyanoborohydride (0.561 g, 8.9 mmol). The resulting mixture was stirred at room temperature for 2 hours. The reaction mixture was diluted with water (10 mL) and an aqueous solution of sodium bicarbonate (15 mL), and then extracted with ethyl acetate. The organic layer was washed with brine, dried (Na₂SO₄) and concentrated under reduced pressure to afford the title compound as a yellow solid (1.8 g, 79 %). TLC: ethylacetate/hexane (3/7): *R_{f}*: 0.4. ¹H NMR (DMSO-d₆, 400 MHz): δ 3.27-3.23 (m, 4H), 2.67 (t, J=5.1 Hz, 1 H), 2.50-2.44 (m, 3H), 2.33 (t, J=5.0 Hz, 4H), 1.37 (s, 9H).

### Step 2: Preparation of 1-(3,3,3-trifluoropropyl)piperazine, hydrochloride salt

A 4N solution of HCl in dioxane (10 mL) was added into a solution of tert-butyl 4-(3,3,3-trifluoropropyl)piperazine-1-carboxylate (1.8 g, 6.3 mmol) in dioxane at 0 °C. The reaction mixture was stirred at room temperature for 2 hours, and then concentrated under reduced pressure to afford the title compound as a white solid (1.0 g, 87 %). TLC: dichloromethane/methanol (9/1): *R_{f}:* 0.2.¹H NMR (DMSO-d₆ 400 MHz): δ 3.60-3.54 (m, 4H), 3.42-3.32 (m, 5H), 3.04 (s, 1 H), 2.93-2.84 (m, 2H). LCMS (Method C): Mass found (M+ 183.2), Rt (min): 0.81, Area (%): 92.9 (Max. chrom.).

### Intermediate 12: 3-(trifluoromethyl)-5,6,7,8-tetrahydro-1,6-naphthyridine, hydrochloride salt

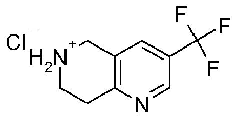

### Step 1: Preparation of N-[(2)-3-(dimethylamino)-2-(trifluoromethyl)prop-2-en-1-ylidene]-N-methylmethanaminium hexafluorophosphate

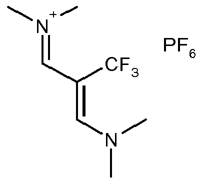

POCl₃ (14.6 mL, 0.156 mol) was added dropwise into a solution of 3,3,3-trifluoropropanoic acid (10.0 g, 78.9 mmol) in anhydrous DMF (40 mL) at 65 °C. The resulting mixture was stirred at 70 °C for 3 hours, and then cooled at RT. In a separated flask, a mixture of hexafluoro phosphoric acid (26.6 mL, 113.2 mmol, 62% in water) and water (50 mL) was prepared to 0 °C, and then a solution of KOH (6.3 g, 113.2 mmol) in water (25 mL) was added over a period of 15 minutes, maintaining the temperature between -5 °C and 0°C. To this slurry was added the Vilsmeier reagent solution dropwise over a period of 1 hour, maintaining the reaction temperature between -5°C and 0°C. The reaction mixture was stirred for 15 minutes, and then the precipitate was filtered off, washed with cold water and dried to afford the title intermediate as a yellow solid (20.5 g, 77%). TLC-chloroform/methanol: (9/1): R*_{f}* = 0.1. LCMS (Method D): Mass found (M+ 195.2). Rt (min): 1.59, Area % 93.0.

### Step 2: Preparation of tert-butyl 3-(trifluoromethyl)-7,8-dihydro-1,6-naphthyridine-6(5H)-carboxylate

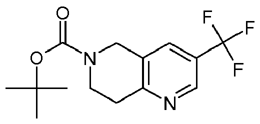

A solution of tert-butyl 4-oxopiperidine-1-carboxylate (12.0 g, 60.2 mmol) in anhydrous tetrahydrofuran (75 mL) was added dropwise into a 1 M solution of LiHMDS in tetrahydrofuran (60.2 mL, 60.2 mmol, 1.0 M solution in THF) diluted with anhydrous tetrahydrofuran (150 mL) at -20°C. The reaction mixture was stirred at 0°C for 45 minutes, and then warming up to room temperature. This mixture was added dropwise into a slurry of *N*-[(2)-3-(dimethylamino)-2-(trifluoromethyl)prop-2-en-1-ylidene]-*N-*methylmethanaminium hexafluorophosphate (20.5 g, 60.2 mmol) in tetrahydrofuran (150 mL) at -20°C. The resulting mixture was stirred at -20°C for 2 hours, then acetic acid (5.2 mL) was added and the reaction mixture was allowed to warm up slowly to room temperature and stirred for 30 additional minutes. Ammonium acetate (13.3 g, 172.7 mmol) was added and the mixture was heated at 65°C for 2 hours. The reaction mixture was cooled, diluted with water and extracted with diethylether. The organic layer was washed with brine, dried and concentrated under vacuum. After purification by flash chromatography (silica, pet ether/ethyl acetate), the title compound was obtained as pale yellow oil (9.2 g, 51 %). TLC-chloroform/methanol (9/1): R_{f}= 0.4. ¹H NMR (DMSO-d₆, 400 MHz) δ 8.73 (d, J = 1.1 Hz, 1 H), 8.09 (d, J = 1.3 Hz, 1 H), 4.63 (2H, s), 3.68 (t, J = 6.0 Hz, 2H), 2.95 (t, J = 5.8 Hz, 2H),1.42 (9H, s).

### Step 3: Preparation of 3-(trifluoromethyl)-5,6,7,8-tetrahydro-1,6-naphthyridine, hydrochloride salt

A 4N solution of HCl in dioxane (100 mL) was added dropwise into a solution of tert-butyl 3-(trifluoromethyl)-7,8-dihydro-1,6-naphthyridine-6(5H)-carboxylate (6.0 g) in dioxane (100 mL) at 0°C. The resulting mixture was stirred at room temperature for 12 hours. The reaction mixture was concentrated under vacuum to afford the title compound as a yellow solid (3.8 g, 80 %). TLC-chloroform/methanol (9/1): Rf = 0.2. LCMS (Method D): Mass found (M+ 203.0), Rt (min): 1.53, Area % 92.7. ¹H NMR (DMSO-d₆, 400MHz) δ 10.05 (bs, 2H), 8.84 (s, 1 H), 8.17 (s, 1 H), 4.37-4.35 (t, 2H), 3.48-3.46 (m, 2H), 3.22-3.18 (t, 2H).

### Intermediate 13: 8-tert-butyl-3-methyl-2-oxo-1-oxa-8-azaspiro[4.5]dec-3-ene-4-carboxylic acid, hydrochloride salt

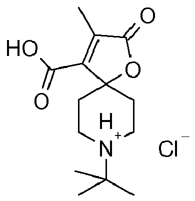

### Step 1: Preparation of 1-ethyl-1-methyl-piperidin-4-one iodide

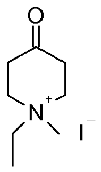

Methyl iodide (18.4 g, 0.118 mol) was added into a solution of 1-ethylpiperidin-4-one (10.0 g, 0.078 mol) in acetone (200 mL) at 0°C. The reaction mixture was stirred at room temperature for 6 hours. The reaction mixture was concentrated under vacuum to afford the title compound as a yellow solid (20 g, 95 %). 1 H NMR (DMSO-d6, 400 MHz) δ 3.72 (t, J = 6.52 Hz, 4H), 3.58 (q, J = 7.28, 7.28 Hz, 2H), 3.17 (s, 3H), 2.77-2.60 (m, 4H), 1.29 (t, J = 7.20 Hz, 3H).

### Step 2: Preparation of 1-tert-butylpiperidin-4-one

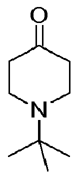

Sodium bicarbonate (2.0 g, 0.037 mol) was added into a solution of 1-ethyl-1-methyl-piperidin-4-one iodide (10.0 g, 0.037 mol) in a mixture of toluene (100 ml) and water (10 mL) and cooled at 0°C. Tert-butylamine (13.6 g, 0.186 mol) was added dropwise. The resulting mixture was heated at 80°C for 5 hours. The reaction mixture was concentrated under vaccum. After purification by flash chromatography (silica, chloroform/methanol), the title compound was obtained as a yellow liquid (2.1 g, 37%). ¹H NMR (CDCl₃, 400 MHz): δ 2.86 (t, *J* = 6.0 Hz, 4H), 2.45 (t, *J* = 6.0 Hz, 4H), 1.14 (s, 9H).

### Step 2: Preparation of 8-tert-butyl-3-methyl-2-oxo-1-oxa-8-azaspiro[4.5]dec-3-ene-4-carboxylic acid, hydrochloride salt

Dimethylitaconate (1.32 g, 8.33 mmol) and 1-tert-butylpiperidin-4-one (1.3 g, 8.33 mmol) were added into a solution of sodium methoxide (0.94 g, 17.4 mmol) in tetrahydrofuran (50 mL) at 0°C over 30 minutes. The resulting mixture was stirred 2 hours at 0°C, and then 12 hours at room temperature. The reaction mixture was diluted with water and the organic solvent was removed under reduced pressure. The aqueous layer was acidified with a 1.5N aqueous solution of HCl and concentrated under reduced pressure. After purification by crystallization from ethanol, the title compound was obtained as a white solid (0.7 g, 31%). ¹H NMR (DMSO-d6, 400 MHz): δ 3.55-3.66 (m, 2H), 2.82-2.99 (m, 4H), 1.84 (s, 3H), 1.66-1.63 (m, 2H), 1.41 (s, 9H). LCMS (Method D) Mass found (M+ 268.3) Rt (min): 1.65; Area (%) 75.8.

### Intermediate 14: (1S,4S)-2-[4-(trifluoromethyl)pyridin-2-yl]-2,5-diazabicyclo[2.2.1]heptane, dihydrochloride salt

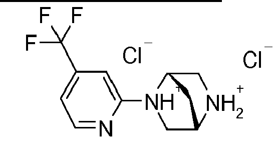

### Step : 1 Preparation of tert-butyl (1S,4S)-5-(4-(trifluoromethyl)pyridin-2-yl]-2,5-diazabicyclo[2.2.1]heptane-2-carboxylate

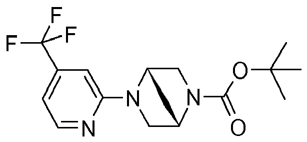

2-bromo-4-(trifluoromethyl)pyridine (0.5 g, 2.21 mmol), cesium fluoride (0.33 g, 2.21 mmol) and tert-butyl (1S,4S)-2,5-diazabicyclo[2.2.1]heptane-2-carboxylate (1.3 g, 6.63 mmol) were added in DMSO (5 mL). The reaction was then heated to 120°C for 18 hours. The reaction mass was diluted with water and extracted with DCM (3 X 100 mL). The organic layer was washed with water, brine solution, dried over anhydrous MgSO₄ and concentrated to get the crude product which was dissolved in DCM. The title intermediate was precipitated by the addition of n-hexane as an off white solid (0.4 g, 66 %). ¹HNMR (DMSO-d6, 400 MHz): δ 8.27 (s, 1 H), 6.82-6.83 (m, 2H), 4.91 (s, 1 H), 4.42-4.52 (m, 1 H), 3.84 (s, 1 H), 3.36-3.59 (m, 1 H), 3.34-3.34 (m, 1 H), 3.14-3.16 (m, 1 H), 1.89 (s, 2H), 1.36 (s, 9H). LCMS (Method D): Mass found (M+ 344.0), Rt (min): 3.58, Area (%) 97.2 (Max), 98.9 (254 nm).

### Step 2: Preparation of 2-(4-(trifluoromethyl)pyridin-2-yl)-2,5-diazabicyclo[2.2.1]heptane, hydrochloride salt

HCl in 1,4 dioxane (4 N, 10 mL) was added to a solution of tert-butyl (1S,4S)-5-[4-(trifluoromethyl)pyridin-2-yl]-2,5-diazabicyclo[2.2.1]heptane-2-carboxylate (0.4 g in 200 mL of anhydrous 1,4 dioxane). The resulting mixture was stirred for 3 hours at room temperature, then concentrated completely. The residue was triturated with diethyl ether (100 mL), filtered under N₂ atm, and dried over pump to get the title product as a white solid (0.2 g, 71 %). LCMS (Method D): Mass found (M+ 244.0), Rt (min): 1.90, Area (%) 99.9 (Max), 99.8 (254 nm).

### Intermediate 15: (1S,4S)-2-[4-(trifluoromethyl)pyrimidin-2-yl]-2,5-diazabicyclo[2.2.1]heptane, dihydrochloride salt

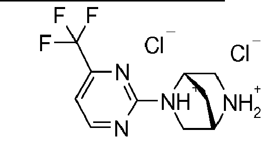

### Step 1: Preparation of tert-butyl (1S,4S)-5-(4-(trifluoromethyl)pyrimidin-2-yl]-2,5-diazabicyclo[2.2.1]heptane-2-carboxylate

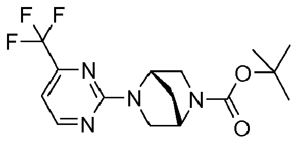

The intermediate was obtained following the procedure as described for intermediate 14 but starting from 2-chloro-4-(trifluoromethyl)pyrimidine. The tiltle intermediate was isolated as a white solid (0.4 g, 42 %). ¹HNMR (DMSO-d6, 400MHz) : δ 8.66 (d, *J* = 4.84 Hz, 1 H), 7.04 (d, *J* = 4.88 Hz, 1 H), 4.84-4.93 (m, 1 H), 4.44-4.50 (m, 1 H), 3.54-3.55 (m, 1H), 3.32-3.43 (m, 2H), 3.16-3.18 (m, 1H), 1.92-1.95 (m, 2H), 1.39 (s, 9H). LCMS (Method D): Mass found ([M-tBu+2H]⁺, 289), Rt (min):5.19 Area (%) 99.33(Max),

### Step 2: Preparation of (1S,4S)-2-(4-(trifluoromethyl)pyrimidin-2-yl)-2,5-diazabicyclo[2.2.1]heptane, hydrochloride salt

The intermediate was obtained following the procedure as described for intermediate 14 but starting from tert-butyl (1 S,4S)-5-[4-(trifluoromethyl)pyrimidin-2-yl]-2,5-diazabicyclo[2.2.1]heptane-2-carboxylate. The title intermediate was isolated as a white solid (0.2 g, 71 %). ¹HNMR (DMSO-d6, 400 MHz) : δ 9.73 (br s, 1 H), 9.21 (br s, 1 H), 8.73 (d, *J* = 4.84 Hz, 1 H), 7.14 (d, *J* = 4.88 Hz, 1 H), 4.94-5.00 (m, 1 H), 4.48-4.49 (m, 1 H), 3.70-3.73 (m, 1 H), 3.56-3.61 (m, 1 H), 3.30-3.33 (m, 1H), 3.19-3.21 (m, 1H), 2.14-2.17 (m, 1 H), 1.92-1.94 (m, 1 H). LCMS (Method D): Mass found (M+ 245.0), Rt (min): 2.31, Area (%) 99.6 (Max), 99.4 (254 nm).

### Intermediate 16: Preparation of (1S,4S)-2-[6-(trifluoromethyl)pyrazin-2-yl]-2,5-diazabicyclo[2.2.1]heptane, dihydrochloride salt

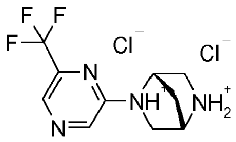

### Step 1: Preparation of tert-butyl (1S,4S)-5-[6-(trifluoromethyl)pyrazin-2-yl]-2,5-diazabicyclo[2.2.1]heptane-2-carboxylate

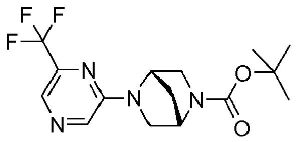

The intermediate was obtained following the procedure as described for intermediate 14 but starting from 2-chloro-6-(trifluoromethyl)pyrazine. The title intermediate was isolated as a white solid (0.3 g, 31 %). ¹HNMR (DMSO-d6, 400 MHz) : δ 8.38 (s, 1 H), 8.19 (s, 1 H), 4.93 (s, 1 H), 4.48-4.53 (m, 1 H), 3.55-3.58 (m, 1 H), 3.37-3.41 (m, 2H), 3.18-3.21 (m, 1 H), 1.92-1.99 (m, 2H), 1.39 (s, 9H). LCMS (Method D): LCMS: Mass found (M+ 345.0), Rt (min):4.95, Area (%) 97.6 (Max), 96.8 (254 nm).

### Step 2: Preparation of (1S,4S)-2-(6-(trifluoromethyl)pyrazin-2-yl)-2,5-diazabicyclo[2.2.1]heptane, hydrochloride salt

The intermediate was obtained following the procedure as described for intermediate 14 **but starting from** tert-butyl (1 S,4S)-5-[6-(trifluoromethyl)pyrazin-2-yl]-2,5-diazabicyclo[2.2.1]heptane-2-carboxylate. The title intermediate was isolated as a white solid (0.2 g, 94 %). LCMS (Method D): LCMS: Mass found (M+ 245.0), Rt (min): 2.27 Area (%), 97.3 (Max), 96.0 (254 nm).

### Intermediate 17: Preparation of 3-methyl-4-{[7-(trifluoromethyl)-3,4-dihydroisoquinolin-2(1 1H)-yl]carbonyl}-1-oxa-7-azaspiro[4.4]non-3-en-2-one. hydrochloride salt

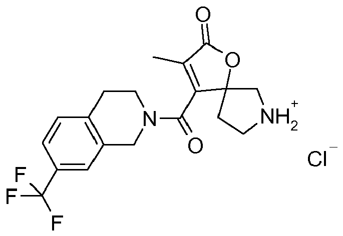

### Step 1: Preparation of 7-tert-butyl 4-methyl 3-methyl-2-oxo-1-oxa-7-azaspiro[4.4]non-3-ene-4,7-dicarboxylate

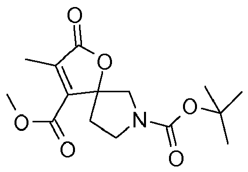

A solution of 3-oxo-pyrrolidine-1-carboxylic acid tert-butyl ester (4.0 g, 21. 6 mmol) and 2-methylene-succinic acid dimethyl ester (3.04 mL, 21.61 mmol) in anhydrous THF (80 mL) was added dropwise to a suspension of sodium methanolate (2.570 g, 47.57 mmol) in 320 mL dry THF at 0°C under N2 atmosphere (over 1 hour). The reaction was stirred at 0°C for 2 hours and at RT overnight. Water was then added and the THF was evaporated under vacuum. The aqueous residue was extracted with EtOAc. The organic phase was washed with brine, dried over Na₂SO₄, filtered and evaporated . The residue was purified by silica gel flash chromatography (eluent DCM to DCM:MeOH 99:1) affording the 7-tert-butyl 4-methyl 3-methyl-2-oxo-1-oxa-7-azaspiro[4.4]non-3-ene-4,7-dicarboxylate (250 mg, 0.803 mmol, 4 %). The aqueous phase was acidified with an aqueous solution of HCl (6 N) and extracted with AcOEt. The organic layer was dried over Na₂SO₄ and evaporated in vacuo to give 7-(tert-butoxycarbonyl)-3-methyl-2-oxo-1-oxa-7-azaspiro[4.4]non-3-ene-4-carboxylic acid (878 mg, 2.95 mmol, 14 %). The crude 7-tert-butyl 4-methyl 3-methyl-2-oxo-1-oxa-7-azaspiro[4.4]non-3-ene-4,7-dicarboxylate was combined with the 7-(tert-butoxycarbonyl)-3-methyl-2-oxo-1-oxa-7-azaspiro[4.4]non-3-ene-4-carboxylic acid and used in the next steps without further purification.

### Step 2: Preparation of 7-(tert-butoxycarbonyl)-3-methyl-2-oxo-1-oxa-7-azaspiro[4.4]non-3-ene-4-carboxylic acid

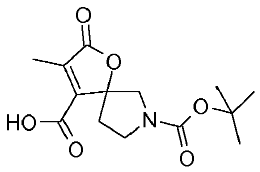

7-tert-butyl 4-methyl 3-methyl-2-oxo-1-oxa-7-azaspiro[4.4]non-3-ene-4,7-dicarboxylate (0,258 g; 0,829 mmol) was dissolved in THF (5 mL). An aqueous solution of NaOH (2 N) was added and the resulting mixture was stirred at 0°C for 6 hours. The mixture was partitioned between water and AcOEt and the aqueous phase was acidified with an aqueous solution of HCl (37%) and extracted with DCM. The organic layer was dried over Na₂SO₄ and evaporated to give the title intermediate (0.230 g, 93.4%)

### Step 3: Preparation of tert-butyl 3-methyl-2-oxo-4-{[7-(trifluoromethyl)-3,4-dihydroisoquinolin-2(1H)-yl]carbonyl}-1-oxa-7-azaspiro[4.4]non-3-ene-7-carboxylate

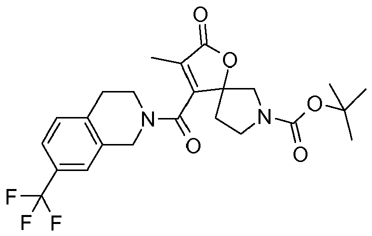

7-Trifluoromethyl-1,2,3,4-tetrahydro-isoquinoline hydrochloride (710 mg, 3.0 mmol) was added to a stirred mixture of 7-(tert-butoxycarbonyl)-3-methyl-2-oxo-1-oxa-7-azaspiro[4.4]non-3-ene-4-carboxylic acid (887 mg, 2.98 mmol), 4-methyl-morpholine (617 mg, 5.98 mmol) and HATU (1249 mg, 3.29 mmol) in DMF. The resulting mixture was stirred overnight at RT. The solvent was removed in vacuo and the residue was partitioned between water and AcOEt. The organic layer was dried over Na₂SO₄ and evaporated to dryness. The crude was purified by column chromatography to give the title intermediate (835 mg, 58.2 %).

### Step 4: Preparation of 3-methyl-4-{[7-(trifluoromethyl)-3,4-dihydroisoquinolin-2(1H)-yl]carbonyl}-1-oxa-7-azaspiro[4.4]non-3-en-2-one, hydrochloride salt

A mixture of tert-butyl 3-methyl-2-oxo-4-{[7-(trifluoromethyl)-3,4-dihydroisoquinolin-2(1H)-yl]carbonyl}-1-oxa-7-azaspiro[4.4]non-3-ene-7-carboxylate (835.0 mg, 1.74 mmol) and HCl in dioxane (1.74 mL, 6.95 mmol) in dioxane (10.0 mL) was stirred at 0°C for 8 hours. The solvent was removed in vacuo to give the title intermediate (720 mg, 99.4%) used in the next steps without any purification.

### Intermediate 18: Preparation of 3-methyl-4-({4-[3-(trifluoromethyl)phenyl]piperazin-1-yl}carbonyl)-1-oxa-7-azaspiro[4.4]non-3-en-2-one, dihydrochloride salt

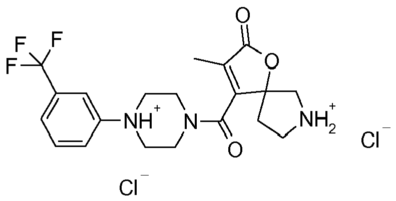

### Step 1: Preparation of tert-butyl 3-methyl-2-oxo-4-({4-[3-(trifluoromethyl)phenyl]piperazin-1-yl}carbonyl)-1-oxa-7-azaspiro[4.4]non-3-ene-7-carboxylate

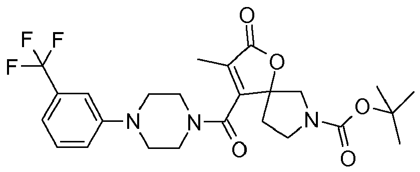

***7-(tert-butoxycarbonyl)-3-methyl-2-oxo-1-oxo-7-azaspiro[4.4]non-3-ene-4-carboxylic acid*** (Intermediate 17, step 2, 0.230 g, 0.774 mmol) was dissolved in anhydrous DMF (9 mL) under N₂ atmosphere. HATU (324 mg, 0.85 mmol) and N-methyl morpholine (0.19 mL, 1.68 mmol) were added in one portion at 0°C. After 15 min 1-(3-trifluoromethyl-phenyl)-piperazine (0.178 g, 0.774 mmol) dissolved in dry DMF (1 mL), was added at 0 °C. The mixture was stirred at RT for 19 hours. The solvent was evaporated and the residue was redissolved in DCM and washed with a saturated solution of Na₂CO₃. The organic phase was washed with brine, dried over Na₂SO₄, filtered and evaporated. The residue was purified by silica gel flash chromatography (eluent DCM:MeOH:NH₃ 97:3:0.2) affording the title intermediate (390 mg, 98.9 %), used in the next steps without further purification.

### Step 2: Preparation of 3-methyl-4-({4-[3-(trifluoromethyl)phenyl]piperazin-1-yl}carbonyl)-1-oxa-7-azaspiro[4.4]non-3-en-2-one, dihydrochloride salt

A mixture of tert-butyl 3-methyl-2-oxo-4-({4-[3-(trifluoromethyl)phenyl]piperazin-1-yl}carbonyl)-1-oxa-7-azaspiro[4.4]non-3-ene-7-carboxylate (390.0 mg, 0,765 mmol) and HCl in Dioxane (4.0 mL, 16.0 mmol) in dioxane (4.0 mL) was stirred at 0°C for 6 hours and then left overnight at 4°C.The solvent was removed in vacuo to give the title intermediate (340 mg, 99.6 %), used in the next steps without further purification.

### Intermediate 19: 3-methyl-4-({4-[3-(trifluoromethyl)phenyl]piperazin-1-yl}carbonyl)-1-oxa-8-azaspiro[4.6]undec-3-en-2-one, dihydrochloride salt

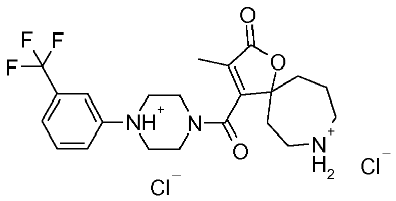

### Step1: Preparation of 8-tert-butyl 4-methyl 3-methyl-2-oxo-1-oxa-8-azaspiro[4.6]undec-3-ene-4,8-dicarboxylate

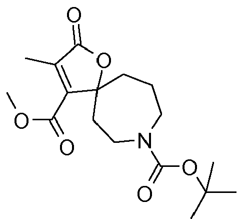

To a suspension of sodium methanolate (558 mg, 10.32 mmol) in dry THF (80 mL), a mixture of 2-methylene-succinic acid dimethyl ester (742 mg, 4.69 mmol) and 4-oxo-azepane-1-carboxylic acid tert-butyl ester (1.0 g, 4.69 mmol) dissolved in anhydrous THF (20 mL) was added at 0°C during 1 hour. The mixture was stirred at 0°C for 2 hours under nitrogen. The reaction was allowed to reach room temperature and was then stirred for additional 18 hours. Water (20 mL) was added and the organic solvent was evaporated under vacuum. The aqueous residue was extracted with EtOAc (50 mL). The organic phase, was dried over sodium sulfate, filtered and concentrated under vacuum. The crude was purified by silica gel flash chromatography (eluent petroleum ether/EtOAC 8/2) to afford the title compound (455 mg, 29%) that was used in the next step without any purification. The aqueous layer was then acidified by addition of an aqueous HCl (6 N, 1.72 mL, 10.32 mmol) at 0°C, and extracted with EtOAc (2 x 50 mL). The combined organic layers were dried over sodium sulfate, filtered and concentrated to afford the title Intermediate (721 mg, 2.22 mmol, 47%) as a pale yellow solid.

### Step 2: Preparation of 8-(tert-butoxycarbonyl)-3-methyl-2-oxo-1-oxa-8-azaspiro[4.6]undec-3-ene-4-carboxylic acid

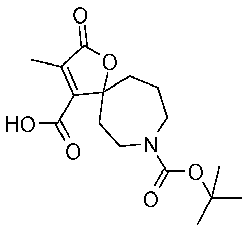

A mixture of 8-tert-butyl 4-methyl 3-methyl-2-oxo-1-oxa-8-azaspiro[4.6]undec-3-ene-4,8-dicarboxylate (455 mg, 1.34 mmol) and an aqueous solution of NaOH (3.35 mL, 6.70 mmol) in THF (5.0 mL) was stirred at RT for 4 hours. Water was added and the aqueous layer was extracted with EtOAc. The combined organic layers were dried over Na₂SO₄ and evaporated in vacuo to afford the title Intgermediate (150 mg, 34 %), used in the next steps without any further purification.

### Step 3: Prepration of tert-butyl 3-methyl-2-oxo-4-({4-[3-(trifluoromethyl)phenyl]piperazin-1-yl}carbonyl)-1-oxa-8-azaspiro[4.6]undec-3-ene-8-carboxylate

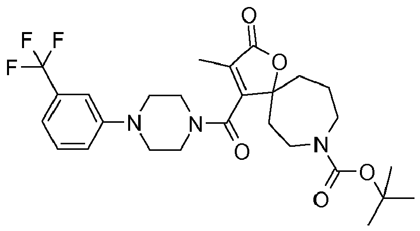

A solution of 8-(tert-butoxycarbonyl)-3-methyl-2-oxo-1-oxa-8-azaspiro[4.6]undec-3-ene-4-carboxylic acid (234 mg, 0.72 mmol), NMM (0.16 mL, 1.44 mmol) and HATU (301 mg, 0.78 mmol) in dry DMF (4 mL) was stirred 30 min at room temperature. 1-(3-(trifluoromethyl)phanyl)piperazine (199 mg, 0.86 mmol) was added and the mixture was stirred overnigt at room temperature. 1.1 eq of HATU were further added and the mixture stirred at room temperature for another 24 hours. Water was added and the mixture extracted with Et₂O. The organic layer was dried over Na₂SO₄ and the solvent was evaporated. The crude was purified by flash chromatography (petroleum ether/EtOAc 1:1 to 4:6) to afford the title Intermediate as a white foam (152 mg, 39.3 %)

### Step 4: Preparation of 3-methyl-4-({4-[3-(trifluoromethyl)phenyl]piperazin-1-yl}carbonyl)-1-oxa-8-azaspiro[4.6]undec-3-en-2-one, dihydrochloride salt.

A solution of tert-butyl 3-methyl-2-oxo-4-({4-[3-(trifluoromethyl)phenyl]piperazin-1-yl}carbonyl)-1-oxa-8-azaspiro[4.6]undec-3-ene-8-carboxylate in Et₂O (2.8 mL) and a solution of HCl in dioxane (4 M, 1.41 mL) in dioxane was stirred overnight at 0-4°C. The solvent was evaporated to afford the title Intermediate as a white solid (149 mg, quantitative), used in the next steps without further purification.

### Example 1: Preparation of 3-methyl-4-({4-[3-(trifluoromethyl)phenyl]piperazin-1-yl}carbonyl)-1-oxa-8-azaspiro[4.5]dec-3-en-2-one, dihydrochloride salt

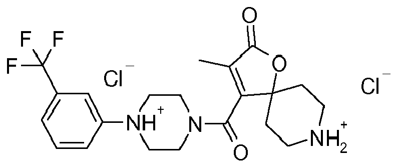

### Step 1: Preparation of 3-methyl-2-oxo-4-[4-(3-trifluoromethyl-phenyl)-piperazine-1-carbonyl]-1-oxa-aza-spiro[4,5]dec-3-ene-8-carboxylic acid tert-butyl ester

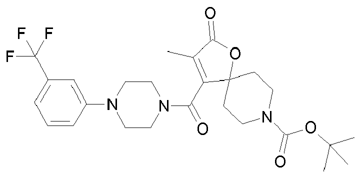

Diisopropylethylamine (18.6 g, 144.2 mmol) and 1-(3-trifluoromethylphenyl)piperazine dihydrochloride salt (5.76 g, 21.5 mmol) were added into a solution of 3-methyl-2-oxo-1-oxa-8-aza-spiro[4,5]dec-3-ene-4,8-dicarboxylic acid 8-tert-butyl ester (Intermediate 2, 5.6 g, 18.0 mmol) in anhydrous dichloromethane (100 mL) and stirred for 5 minutes, then 1-propane phosphonic acid cyclic anhydride (22.9 g, 72.0 mmol) was added dropwise. The resulting mixture was stirred for 16 hours. The reaction mixture was diluted with cold water (100 mL). The organic layer was washed with brine (100 mL), dried (MgSO₄) and concentrated under vacuum. After purification by flash chromatography (silica), the title compound was obtained as an off-white solid (4.9 g, 52%). TLC: chloroform/methanol (9/1) R_{f}: 0.4. ¹H NMR (400 MHz, CDCl₃): δ 7.43 (m, 1 H), 7.18 (d, J = 7.7 Hz, 1 H), 7.14 (s, 1H), 7.11 (d, J = 8.2 Hz, 1H), 4.13 (m, 2H), 3.95-3.90 (m, 2H), 3.76-3.57 (m, 2H), 3.31-3.30 (m, 2H), 3.21-3.14 (m, 4H), 2.33 (m, 1 H), 1.92 (s, 3H), 1.89 (m, 1 H), 1.57 (m, 2H), 1.46 (s, 9H). LCMS (Method C): Mass found (M+ 424.2 [M-Boc+2H]⁺), Rt (min): 4.36, Area (%): 93.9.

### Step 2: Preparation of 3-methyl-4-({4-[3-(trifluoromethyl)phenyl]piperazin-1-yl}carbonyl)-1-oxa-8-azaspiro[4.5]dec-3-en-2-one, dihydrochloride salt

A 4N solution of HCl in 1,4-dioxane (25 mL) was added into a solution of 3-methyl-2-oxo-4-[4-(3-trifluoromethyl-phenyl)-piperazine-1-carbonyl]-1-oxa-aza-spiro[4,5]dec-3-ene-8-carboxylic acid tert-butyl ester (4.9 g, 0.93 mmol) in anhydrous 1,4-dioxane (50 mL). The resulting mixture was stirred 3 hours at room temperature. The reaction mixture was concentrated under vacuum. The residue was triturated with diethyl ether (100 mL), filtered under nitrogen and dried under vacuum to give the title compound as a white solid (3.0 g, 76 %). ¹H NMR (DMSO-d₆, 400 MHz): δ 9.23-9.21 (m, 1H), 8.73-8.75 (m, 1 H), 7.47-7.43 (m, 1H), 7.27 (d, J = 7.8 Hz, 1H), 7.21 (s, 1 H), 7.13 (d, *J* = 7.3 Hz, 1H), 3.89-3.87 (m, 1H), 3.72-3.60 (m, 3H), 3.44-3.36 (m, 4H), 3.06-2.97 (m, 4H), 2.52-2.50 (m, 1 H), 2.09-2.00 (m, 1H), 1.95-1.89 (m, 1H), 1.78 (s, 4H). LCMS (Method D): Mass found (M+ 424.0), Rt (min): 3.69, Area (%): 97.8 (Max), 97.03 (254 nm). HPLC (Method A): Rt (min): 3.77, Area (%): 97.9 (Max), 96.8 (254 nm). Elemental analysis: C₂₁H₂₄F₃N₃O₃.2HCl.1.5H₂O, calculated: C 48.19%, H 5.58%, N 8.03%, found: C 48.03%, H 5.37%, N 7.72%.

### Example 2: Preparation of 8-(3-methoxypropyl)-3-methyl-4-({4-[3-(trifluoromethyl)phenyl]piperazin-1-yl}carbonyl)-1-oxa-8-azaspiro[4.5]dec-3-en-2-one

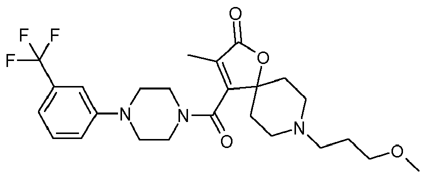

A mixture of 3-methyl-4-({4-[3-(trifluoromethyl)phenyl]piperazin-1-yl}carbonyl)-1-oxa-8-azaspiro[4.5]dec-3-en-2-one dihydrochloride (Example 1, 300 mg, 0.61 mmol), anhydrous potassium carbonate (180 mg, 1.3 mmol) and 1-bromo-3-methoxypropane (120 mg, 1.63 mmol) in anhydrous N,N-dimethylformamide (3 mL) was heated at 150°C for 2 hours under microwave irradiation. The reaction mixture was concentrated under vacuum. The residue was taken up in dichloromethane (20 mL), washed with water (20 mL) and brine (20 mL), dried (MgSO₄) and concentrated under vacuum. After purification by flash chromatography (silica), the title compound was obtained as an off-white solid (104 mg, 32%). ¹H NMR (400 MHz, CDCl₃): δ 7.40 (m, 1 H), 7.19 (d, *J* = 7.7 Hz, 1H), 7.12 (s, 1 H), 7.10 (d, *J* = 8.2 Hz, 1 H), 3.95 (m, 1 H), 3.87 (m, 1 H), 3.56 (m, 2H), 3.43 (t, *J* = 6.1 Hz, 2H), 3.33 (s, 3H), 3.29 (m, 2H), 3.22 (m, 2H), 3.06 (m, 2H), 2.62 (m, 5H), 2.26 (m, 1 H), 1.90 (s, 3H), 1.87 (m, 2H), 1.74 (m, 2H). LCMS (Method D): Mass found (M+ 496.3), Rt (min): 3.94, Area (%): 96.0 (Max), 95.7 (254 nm). HPLC (Method A): Rt (min): 4.08, Area (%): 96.8 (Max), 96.7 (254 nm).

### Example 3: Preparation of 8-[2-(4-fluorophenyl)ethyl]-3-methyl-4-({4-[3-(trifluoromethyl)phenyl]piperazin-1-yl}carbonyl)-1-oxa-8-azaspiro[4.5]dec-3-en-2-one

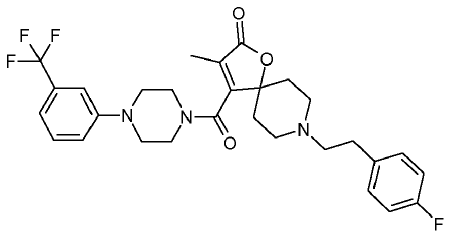

A mixture of 3-methyl-4-({4-[3-(trifluoromethyl)phenyl]piperazin-1-yl}carbonyl)-1-oxa-8-azaspiro[4.5]dec-3-en-2-one dihydrochloride (Example 1, 300 mg, 0.61 mmol), anhydrous potassium carbonate (180 mg, 1.3 mmol) and 4-fluorophenethylbromide (160 mg, 0.78 mmol) in anhydrous N,N-dimethylformamide (3 mL) was heated at 80°C for 45 minutes under microwave irradiation. The reaction mixture was concentrated under vacuum. The residue was taken up in dichloromethane (20 mL), washed with water (20 mL) and brine (20 mL), dried (MgSO₄) and concentrated under vacuum. After purification by flash chromatography (silica), the title compound was obtained as a white solid (190 mg, 54%). ¹H NMR (DMSO-d₆, 400 MHz): δ 7.41 (m, 1 H), 7.15 (m, 5H), 6.99 (m, 2H), 3.94 (m, 1 H), 3.87 (m, 1 H), 3.56 (m, 2H), 3.26 (m, 4H), 2.96 (m, 2H), 2.80 (m, 2H), 2.63 (m, 2H), 2.49 (m, 3H), 2.03 (m, 1 H), 2.03 (s, 3H), 1.74 (m, 2H). LCMS (Method D): Mass found (M+ 546.3), Rt (min): 4.47, Area (%): 95.5 (Max), 96.0 (254 nm). HPLC (Method A): Rt (min): 4.67, Area (%): 96.8 (Max), 96.4 (254 nm).

### Example 4: Preparation of 8-(2-methoxyethyl)-3-methyl-4-({4-[3-(trifluoromethyl)phenyl]piperazin-1-yl}carbonyl)-1-oxa-8-azaspiro[4.5]dec-3-en-2-one

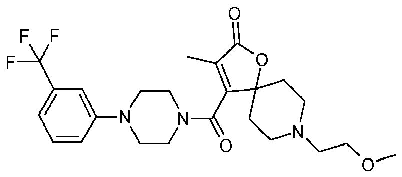

A mixture of 3-methyl-4-({4-[3-(trifluoromethyl)phenyl]piperazin-1-yl}carbonyl)-1-oxa-8-azaspiro[4.5]dec-3-en-2-one dihydrochloride (Example 1, 300 mg, 0.61 mmol), anhydrous potassium carbonate (180 mg, 1.3 mmol) and 2-chloro-ethylmethylether (0.28 g, 2.61 mmol) in anhydrous N,N-dimethylformamide (3 mL) was heated at 150°C for 2 hours under microwave irradiation. The reaction mixture was concentrated under vacuum. The residue was taken up in dichloromethane (20 mL), washed with water (20 mL) and brine (20 mL), dried (MgSO₄) and concentrated under vacuum. After purification by flash chromatography (silica), the title compound was obtained as an off-white solid (111 mg, 35%). ¹H NMR (CDCl₃, 400 MHz): δ 7.40 (m, 1 H), 7.19 (d, *J* = 7.8 Hz, 1 H), 7.13 (s, 1 H), 7.10 (d, *J* = 8.3 Hz, 1 H), 3.93 (m, 1H), 3.84 (m, 1H), 3.55 (m, 2H), 3.51 (t, *J* = 5.4 Hz, 2H), 3.36 (s, 3H), 3.24 (m, 4H), 2.96 (m, 2H), 2.63 (t, *J* = 5.4 Hz, 2H), 2.48 (m, 3H), 2.11 (m, 1 H), 1.89 (s, 3H), 1.67 (m, 2H). LCMS (Method D): Mass found (M+ 482.3), Rt (min): 3.81, Area (%): 98.2 (Max), 98.1 (254 nm). HPLC (Method A): Rt (min): 3.97, Area (%): 98.7 (Max), 98.9 (254 nm).

### Example 5: Preparation of 3-methyl-8-(3-phenylpropyl)-4-({4-[3-(trifluoromethyl)phenyl]piperazin-1-yl}carbonyl)-1-oxa-8-azaspiro[4.5]dec-3-en-2-one

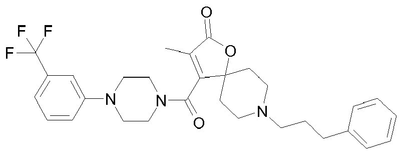

A mixture of 3-methyl-4-({4-[3-(trifluoromethyl)phenyl]piperazin-1-yl}carbonyl)-1-oxa-8-azaspiro[4.5]dec-3-en-2-one dihydrochloride (Example 1, 300 mg, 0.61 mmol), anhydrous potassium carbonate (271 mg, 1.96 mmol) and 1-bromo-3-phenyl propane (195 mg, 0.98 mmol) in anhydrous N,N-dimethylformamide (50 mL) was heated at 90°C for 15 hours. The reaction mixture was filtered through a Celite pad and concentrated under vacuum. The residue was taken up in dichloromethane (20 mL), washed with water (20 mL) and brine (20 mL), dried (Na₂SO₄) and concentrated under vacuum. After purification by flash chromatography (neural alumina, chloroform/methanol), the title compound was obtained as a white solid (31 mg, 9%). TLC: dichloromethane/methanol (9/1): R_{f}: 0.6. ¹H NMR (400 MHz, CDCl₃): δ 7.42-7.38 (m, 1 H), 7.30 (s, 1 H), 7.27 (s, 1 H), 7.19-7.17 (m, 4H), 7.12 (s, 1H), 7.09 (d, *J*= 8.1 Hz, 1 H), 3.90 (d, *J*= 9.5 Hz, 2H), 3.55 (s, 2H), 3.28-3.18 (m, 4H), 2.90 (m, 2H), 2.65 (t, *J* = 7.5 Hz, 2H), 2.43 (m, 5H), 2.05 (m, 1 H), 1.89 (s, 3H), 1.85 (m, 2H), 1.70 (br s, 1H), 1.56 (s, 1H). LCMS (Method D): Mass found (M+ 542.0), Rt (min): 4.68, Area (%): 97.5 (Max), 97.4 (254 nm). HPLC (Method A): Rt (min) 4.85 : Area (%): 96.5 (Max), 96.8 (254 nm).

### Example 6: Preparation of 8-[2-(4-methoxyphenyl)ethyl]-3-methyl-4-({4-[3-(trifluoromethyl)phenyl]piperazin-1-yl]carbonyl)-1-oxa-8-azaspiro[4.5]dec-3-en-2-one, dihydrochloride salt

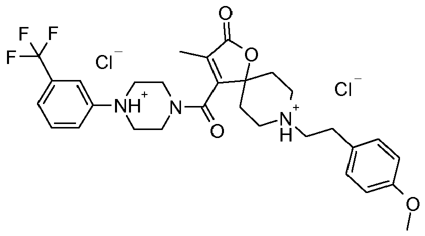

A mixture of 3-methyl-4-({4-[3-(trifluoromethyl)phenyl]piperazin-1-yl}carbonyl)-1-oxa-8-azaspiro[4.5]dec-3-en-2-one dihydrochloride (Example 1 , 300 mg, 0.61 mmol), anhydrous potassium carbonate (180 mg, 1.3 mmol) and 4-methoxyphenethylbromide (0.17 g, 0.78 mmol) in anhydrous N,N-dimethylformamide (3 mL) was heated at 120°C for 1 hour under microwave irradiation. The reaction mixture was concentrated under vacuum. The residue was taken up in dichloromethane (20 mL), washed with water (20 mL) and brine (20 mL), dried (MgSO₄) and concentrated under vacuum. Purification by flash chromatography (silica) gave the parent compound as a white solid. This solid was taken up in diethylether (5 mL) and a 1 N solution of HCl in diethylether (5 mL) was added. The precipitate was filtered off and dried under vacuum to give the title compound (50 mg, 13%). ¹H NMR (CDCl₃, 400 MHz): δ 12.96 (br s, 1 H), 7.42 (s, 1 H), 7.22 (m, 3H), 7.18 (m, 2H), 6.87 (m, 2H), 3.97 (m, 2H), 3.79 (s, 3H), 3.62 (m, 4H), 3.30 (m, 4H), 3.20 (m, 8H), 1.93 (m, 5H). LCMS (Method D): Mass found (M+ 558.3), Rt (min): 4.57, Area (%): 97.7 (Max), 97.8 (254 nm). HPLC (Method A): Rt (min): 4.55, Area (%): 96.9 (Max), 97.2 (254 nm). Elemental analysis: C₃₀H₃₄F₃N₃O₄.2HCl.1.5H₂O, calculated: C 54.80%, H 5.98%, N 6.39%, found C 54.68%, H 5.59%, N 6.37%.

### Example 7: Preparation of 8-(2-methoxy-2-phenylethyl)-3-methyl-4-({4-[3-(trifluoromethyl)phenyl]piperazin-1-yl}carbonyl)-1-oxa-8-azaspiro[4.5]dec-3-en-2-one

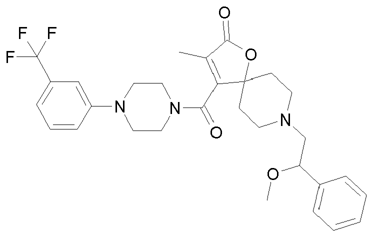

### Step 1: Preparation of 2-methoxy-2-phenylethyl-methanesulfonate

Diisopropylethylamine (2.54 g, 19.7 mmol) was added into a solution of 2-methoxy-2-phenylethanol (1.0 g, 6.57 mmol) in anhydrous dichloromethane (25 mL) cooled at 10°C, then methane sulfonylchloride (1.5 g, 13.1 mmol) was added dropwise. The resulting mixture was stirred for 1 hour. The reaction mixture was diluted with water (10 mL). The organic layer was washed with a 10% aqueous solution of NaHCO₃ (20 mL), water (20 mL) and brine (20 mL), dried (MgSO₄) and concentrated under vacuum to give the title compund as brown oil, used without further purification in the next step.

### Step 2: Preparation of of 8-(2-methoxy-2-phenylethyl)-3-methyl-4-({4-[3-(trifluoromethyl)phenyl]piperazin-1-yl}carbonyl)-1-oxa-8-azaspiro[4.5]dec-3-en-2-one

A mixture of 3-methyl-4-({4-[3-(trifluoromethyl)phenyl]piperazin-1-yl}carbonyl)-1-oxa-8-azaspiro[4.5]dec-3-en-2-one dihydrochloride (Example 1, 300 mg, 0.61 mmol), anhydrous potassium carbonate (270 mg, 1.96 mmol) and 2-methoxy-2-phenylethyl-methanesulfonate (0.18 g, 0.78 mmol) in anhydrous N,N-dimethylformamide (3 mL) was heated at 120°C for 12 hours under microwave irradiation. The reaction mixture was concentrated under vacuum. The residue was taken up in dichloromethane (20 mL), washed with water (20 mL) and brine (20 mL), dried (MgSO₄) and concentrated under vacuum. After purification by flash chromatography (silica), the title compound was obtained as an orange solid (13 mg, 4%). ¹H-NMR (400 MHz, CDCl₃): δ 7.37-7.43 (m, 5H), 7.27 (s, 1 H), 7.18-7.20 (m, 3H), 5.21-5.20 (m, 1 H), 4.20 (d, J = 10.5 Hz, 1 H), 3.75-4.05 (m, 3H), 3.58-3.62 (m, 2H), 3.47-3.52 (m, 2H), 3.30-3.47 (m, 8H), 3.12-3.21 (m, 3H), 1.94 (s, 3H), 1.69-1.93 (m, 2H). LCMS (Method D): Mass found (M+ 558.3), Rt (min): 4.52, Area (%): 95.3 (Max), 94.1 (254 nm). HPLC (Method A): Rt (min): 4.61, Area (%): 93.4 (Max), 93.8 (254 nm).

### Example 8: Preparation of 8-[2-(3-methoxyphenyl)ethyl]-3-methyl-4-({4-[3-(trifluoromethyl)pheny)]piperazin-1-yl}carbonyl)-1-oxa-8-azaspiro[4.5]dec-3-en-2-one, dihydrochloride salt

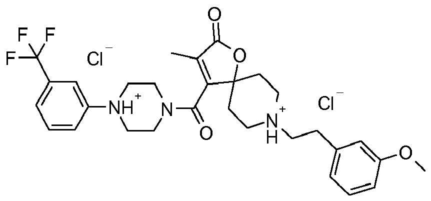

A mixture of 3-methyl-4-({4-[3-(trifiuoromethyl)phenyl]piperazin-1-yl}carbonyl)-1-oxa-8-azaspiro[4.5]dec-3-en-2-one dihydrochloride (Example 1, 300 mg, 0.61 mmol), anhydrous potassium carbonate (180 mg, 1.3 mmol) and 3-methoxyphenethylbromide (0.17 g, 0.78 mmol) in anhydrous N,N-dimethylformamide (3 mL) was heated at 120°C for 1 hours under microwave irradiation. The reaction mixture was concentrated under vacuum. The residue was taken up in dichloromethane (20 mL), washed with water (20 mL) and brine (20 mL), dried (MgSO₄) and concentrated under vacuum. Purification by flash chromatography (silica) gave the parent compound as an off-white solid. This solid was taken up in diethylether (5 mL) and a 1 N solution of HCl in diethylether (5 mL) was added. The precipitate was filtered off and dried under vacuum to give the title compound as a white solid (130 mg, 34%). ¹H NMR (400 MHz, CDCl₃): δ 12.90 (br s, 1H), 7.46 (m, 2H), 7.29-7.31 (m, 2H), 7.24-7.28 (m, 2H), 6.80-6.84 (m, 2H), 4.21-4.39 (m, 1 H), 3.96-4.02 (m, 1H), 3.31 (s, 3H), 3.64-3.71 (m, 3H), 3.38-3.49 (m, 3H), 3.20-3.25 (m, 6H), 1.94 (s, 3H), 1.53-1.90 (m, 6H). LCMS (Method D): Mass found (M+ 558.3), Rt (min): 4.57, Area (%): 95.8 (Max), 96.7 (254 nm). HPLC (Method A): Rt (min): 4.59, Area (%): 93.6 (Max), 95.2 (254 nm). Elemental analysis: C₃₀H₃₄F₃N₃O₄.2HCl.H₂O, calculated: C 55.56%, H 5.91%, N 6.48%, found: C 55.54%, H 5.71%, N 6.15%.

### Example 9: Preparation of [3-methyl-2-oxo-4-({4-[3-(trifluoromethyl)phenyl]piperazin-1-yl}carbonyl)-1-oxa-8-azaspiro[4.5]dec-3-en-8-yl]acetic acid

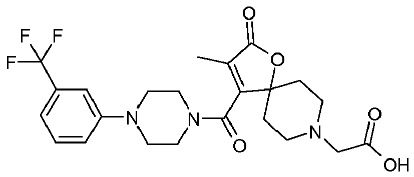

### Step 1: Preparation of methyl [3-methyl-2-oxo-4-({4-[3-(trifluoromethyl)phenyl]piperazin-1-yl}carbonyl)-1-oxa-8-azaspiro[4.5]dec-3-en-8-yl]acetate

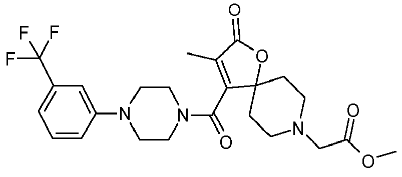

A mixture of 3-methyl-4-({4-[3-(trifluoromethyl)phenyl]piperazin-1-yl}carbonyl)-1-oxa-8-azaspiro[4.5]dec-3-en-2-one dihydrochloride (Example 1, 250 mg, 0.51 mmol), anhydrous potassium carbonate (225 mg, 1.63 mmol) and methyl bromoacetate (0.06 mL, 0.65 mmol) in anhydrous N,N-dimethylformamide (3 mL) was heated at 90°C for 1.5 hours under microwave irradiation. The reaction mixture was concentrated under vacuum. The residue was taken up in dichloromethane (20 mL), washed with water (20 mL) and brine (20 mL), dried (MgSO₄) and concentrated under vacuum. After purification by flash chromatography (silica), the title compound was obtained as a white solid (130 mg, 51 %). ¹H NMR (DMSO-d₆, 400 MHz): δ 7.46-7.42 (m, 1H), 7.24 (d, *J* = 8.4 Hz, 1H), 7.20 (s, 1H), 7.11 (d, *J* = 7.5 Hz, 1H), 3.80-3.79 (m, 1H), 3.70 (s, 1H), 3.65 (s, 3H), 3.54 (m, 2H), 3.34-3.33 (m, 1H), 3.26 (s, 4H), 3.16 (s, 1H), 2.81-2.78 (m, 2H), 2.45-2.42 (m, 2H), 2.24 (t, *J* = 9.6 Hz, 1H), 1.92-1.86 (m, 1H), 1.86 (s, 3H), 1.76-1.75 (m, 1H), 1.70-1.69 (m, 1H). LCMS (Method D): Mass found (M+ 496), Rt (min): 3.87, Area (%): 97.1 (Max), 97.7 (254 nm). HPLC (Method A): Rt (min): 3.96, Area (%): 97.9 (Max), 98.0 (254 nm).

### Step 2: Preparation of [3-methyl-2-oxo-4-({4-[3-(trifluoromethyl)phenyl]piperazin-1-yl}carbonyl)-1-oxa-8-azaspiro[4.5]dec-3-en-8-yl]acetic acid

Lithium hydroxide monohydrate (33 mg, 0.78 mmol) was added into a solution of methyl [3-methyl-2-oxo-4-({4-[3-(trifluoromethyl)phenyl]piperazin-1-yl}carbonyl)-1-oxa-8-azaspiro[4.5]dec-3-en-8-yl]acetate (130 mg, 0.26 mmol) in a mixture of tetrahydrofuran (8 mL) and methanol (2 mL). The resulting mixture was stirred 2 hours at room temperature. The reaction mixture was concentrated under vacuum. The residue was taken up with dichloromethane (20 mL) and washed with a saturated solution of citric acid (pH 4), water (20 mL) and brine (20 mL). The organic layer was dried (MgSO₄) and concentrated under vacuum to give the title compound as a white solid (96 mg, 77 %). ¹H NMR (DMSO-d₆, 400 MHz): δ 7.46-7.42 (m, 1 H), 7.25 (d, *J* = 8.3 Hz, 1 H), 7.20 (s, 1 H), 7.11 (d, *J* = 7.5 Hz, 1 H), 3.82-3.81 (m, 1 H), 3.69 (s, 1 H), 3.56 (t, *J* = 4.8 Hz, 2H), 3.36-3.35 (m, 3H), 3.16 (s, 4H), 2.89 (d, *J* = 10.2 Hz, 2H), 2.54 (d, *J* = 5.1 Hz, 1H), 2.32 (d, *J* = 2.0 Hz, 1 H), 1.93 (d, *J* = 6.0 Hz, 1 H), 1.80 (s, 3H), 1.69 (s, 1 H), 1.52 (s, 1 H). LCMS (Method D): Mass found (M+ 482), Rt (min): 3.66, Area (%): 95.3 (Max), 96.6 (254 nm). HPLC (Method A): Rt (min): 3.73, Area (%): 96.0 (Max), 97.1 (254 nm).

### Example 10: Preparation of 3-[3-methyl-2-oxo-4-({4-[3-(trifluoromethyl)phenyl]piperazin-1-yl}carbonyl)-1-oxa-8-azaspiro[4.5]dec-3-en-8-yl]propanoic acid

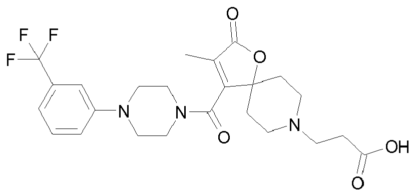

### Step 1: Preparation of 3-{3-methyl-2-oxo-4-[4-(3-trifluoromethyl-phenyl)-piperazine-1-carbonyl]-1-oxa-8-aza-spiro[4.5]dec-3-en-8-yl]-propionic acid ethyl ester

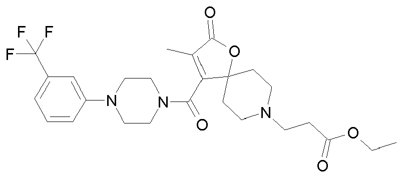

A mixture of 3-methyl-4-({4-[3-(trifluoromethy)phenyl]piperazin-1-yl}carbonyl)-1-oxa-8-azaspiro[4.5]dec-3-en-2-one dihydrochloride (Example 1, 250 mg, 0.51 mmol), anhydrous potassium carbonate (225 mg, 1.63 mmol) and ethyl 3-bromopropanoate (116 mg, 0.64 mmol) in anhydrous N,N-dimethylformamide (3 mL) was heated at 90°C for 1.5 hours under microwave irradiation. The reaction mixture was concentrated under vacuum. The residue was taken up in dichloromethane (20 mL), washed with water (20 mL) and brine (20 mL), dried (MgSO₄) and concentrated under vacuum. After purification by flash chromatography (silica), the title compound was obtained as a white solid (110 mg, 41%). ¹H NMR (DMSO-d₆, 400 MHz): δ 7.46-7.42 (m, 1 H), 7.25 (d, *J* = 8.4 Hz, 1 H), 7.20 (s, 1 H), 7.11 (d, *J* = 7.5 Hz, 1 H), 4.06-4.01 (m, 2H), 3.79-3.78 (m, 1 H), 3.68-3.67 (m, 1 H), 3.55 (s, 2H), 3.26-3.25 (m, 1 H), 3.14-3.13 (m, 3H), 2.80 (d, *J* = 6.4 Hz, 2H), 2.59 (t, *J* = 6.7 Hz, 2H), 2.43 (t, *J* = 6.8 Hz, 2H), 2.20 (s, 3H), 1.82 (s, 1 H), 1.78 (s, 3H), 1.70-1.69 (m, 1 H), 1.47-1.46 (m, 1 H), 1.16 (t, J = 7.1 Hz, 3H). LCMS (Method D): Mass found (M+ 524.3), Rt (min): 4.03, Area (%): 90.0 (Max), 91.3 (254 nm). HPLC (Method A): Rt (min): 4.11, Area (%): 92.1 (Max), 92.5 (254 nm).

### Step 2: Preparation of 3-[3-methyl-2-oxo-4-({4-[3-(trifluoromethyl)phenyl]piperazin-1-yl}carbonyl)-1-oxa-8-azaspiro[4.5]dec-3-en-8-yl]propanoic acid

Lithium hydroxide monohydrate (24 mg, 0.57 mmol) was added into a solution of 3-{3-methyl-2-oxo-4-[4-(3-trifluoromethyl-phenyl)-piperazine-1-carbonyl]-1-oxa-8-aza-spiro[4.5]dec-3-en-8-yl}-propionic acid ethyl ester (100 mg, 0.19 mmol) in a mixture of tetrahydrofuran (8 mL) and methanol (2 mL). The resulting mixture was stirred 2 hours at room temperature. The reaction mixture was concentrated under vacuum. The residue was taken up with dichloromethane (20 mL) and washed with a saturated aqueous solution of citiric acid (pH 4), water (20 mL) and brine (20 mL). The organic layer was dried (MgSO₄) and concentrated to give the title compound as a white solid (46 mg, 49%). ¹H NMR (DMSO-d₆, 400 MHz): δ 7.46-7.42 (m, 1 H), 7.24 (d, *J* = 8.4 Hz, 1 H), 7.20 (s, 1 H), 7.11 (d, *J* = 7.6 Hz, 1H), 3.79-3.78 (m, 1 H), 3.70-3.69 (m, 1H), 3.55 (d, *J* = 4.9 Hz, 2H), 3.16-3.15 (m, 4H), 2.83 (d, *J* = 7.3 Hz, 2H), 2.58 (t, *J* = 5.8 Hz, 2H), 2.36 (t, *J* = 7.0 Hz, 2H), 2.19-2.17 (m, 3H), 1.81-1.80 (m, 1 H), 1.76 (s, 3H), 1.52 (s, 1H), 1.60-1.59 (m, 1H). LCMS (Method D): Mass found (M+ 496), Rt (min): 3.76, Area (%): 95.4 (Max), 95.5 (254 nm). HPLC (Method A): Rt (min): 3.77, Area (%): 95.8 (Max), 96.0 (254 nm).

### Example 11: Preparation of 4-[3-methyl-2-oxo-4-({4-[3_

### (trifluoromethyl)phenyl]piperazin-1-yl}carbonyl)-1-oxa-8-azaspiro[4.5]dec-3-en-8-yl]butanoic acid

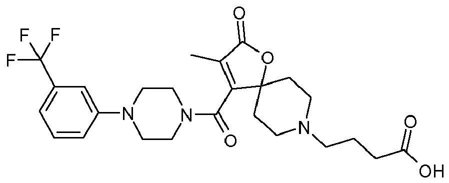

### Step 1: Preparation of methyl 4-[3-methyl-2-oxo-4-({4-(3-(trifluoromethyl)phenyl]piperazin-1-yl}carbonyl)-1-oxa-8-azaspiro[4.5]dec-3-en-8-yl]butanoate

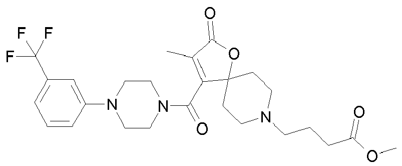

A mixture of 3-methyl-4-({4-[3-(trifluoromethyl)phenyl]piperazin-1-yl}carbonyl)-1-oxa-8-azaspiro[4.5]dec-3-en-2-one dihydrochloride (Example 1, 250 mg, 0.51 mmol), anhydrous potassium carbonate (225 mg, 1.63 mmol) and methyl 4-bromobutanoate (116 mg, 0.64 mmol) in anhydrous N,N-dimethylformamide (3 mL) was heated at 90°C for 1.5 hours under microwave irradiation. The reaction mixture was concentrated under vacuum. The residue was taken up in dichloromethane (20 mL), washed with water (20 mL) and brine (20 mL), dried (MgSO₄) and concentrated under vacuum. After purification by flash chromatography (silica), the title compound was obtained as a white solid (100 mg, 37%). ¹H NMR (DMSO-d₆, 400 MHz): δ 7.46-7.42 (m, 1 H), 7.25 (d, *J* = 8.3 Hz, 1 H), 7.20 (s, 1 H), 7.11 (d, *J* = 7.5 Hz, 1 H), 3.57-3.56 (m, 1 H), 3.55-3.54 (m, 1 H), 3.36 (s, 5H), 3.34 (d, *J* = 1.8 Hz, 2H), 3.26-3.25 (m, 2H), 2.78 (d, *J* = 9.5 Hz, 2H), 2.30-2.27 (m, 4H), 2.13 (s, 3H), 1.76-1.75 (m, 1 H), 1.68 (s, 3H), 1.66 (m, 3H), 1.54-1.53 (m, 1 H). LCMS (Method D): Mass found (M+ 524), Rt (min): 3.99, Area (%): 96.4 (Max), 97.0 (254 nm). HPLC (Method A): Rt (min): 3.99, Area (%): 97.5 (Max), 97.5 (254 nm).

### Step 2: Preparation of 4-[3-methyl-2-oxo-4-({4-[3-(trifluoromethyl)phenyl]piperazin-1-yl}carbonyl)-1-oxa-8-azaspiro[4.5]dec-3-en-8-yl]butanoic acid

Lithium hydroxide monohydrate (24 mg, 0.57 mmol) was added into a solution of methyl 4-[3-methyl-2-oxo-4-({4-[3-(trifluoromethyl)phenyl]piperazin-1-yl}carbonyl)-1-oxa-8-azaspiro[4.5]dec-3-en-8-yl]butanoate (0.100 g, 0.19 mmol) in a mixture of tetrahydrofuran (8 mL) and methanol (2 mL). The resulting mixture was stirred 2 hours at room temperature. The reaction mixture was concentrated under vacuum. The residue was taken up with dichloromethane (20 mL) and washed with a saturated aqueous solution of citiric acid (pH 4), water (20 mL) and brine (20 mL). The organic layer was dried (MgSO₄) and concentrated to give the title compound as a white solid (42 mg, 43%). ¹H NMR (DMSO-d₆, 400 MHz): δ 7.46-7.42 (m, 1H), 7.25 (d, *J* = 8.5 Hz, 1H), 7.20 (s, 1H), 7.11 (d, *J* = 7.7 Hz, 1H), 3.80-3.79 (m, 1H), 3.71-3.70 (m, 1H), 3.56 (s, 2H), 3.31-3.30 (m, 4H), 2.83 (d, *J* = 8.3 Hz, 2H), 2.33 (t, *J* = 6.6 Hz, 2H), 2.23-2.22 (m, 5H), 1.90 (s, 1H), 1.86 (s, 3H), 1.76 (s, 3H), 1.62-1.61 (m, 1H). LCMS (Method D): Mass found (M+ 510), Rt (min): 3.76, Area (%): 98.5 (Max), 98.7 (254 nm). HPLC (Method A): Rt (min): 3.79, Area (%): 98.5 (Max), 98.7 (254 nm).

### Example 12: Preparation of 3-methyl-8-(2-pyridin-4-ylethyl)-4-({4-[3-(trifluoromethyl)phenyl]piperazin-1-yl}carbonyl)-1-oxa-8-azaspiro[4.5]dec-3-en-2-one

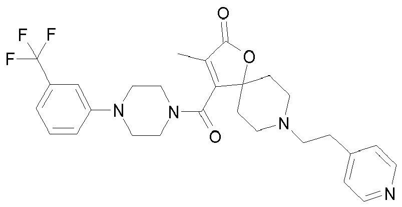

A solution of 3-methyl-4-({4-[3-(trifluoromethyl)phenyl]piperazin-1-yl}carbonyl)-1-oxa-8-azaspiro[4.5]dec-3-en-2-one dihydrochloride (Example 1, 200 mg, 0.40 mmol), diisopropylethylamine (0.15 mL, 1.3 mmol) and 4-vinyl-pyridine (0.05 mL, 0.52 mmol) was prepared in anhydrous N,N-dimethylformamide (3 mL) and heated at 90°C for 12 hours. The reaction mixture was concentrated under vacuum. The residue was taken up in dichloromethane (20 mL) and washed with water (20 mL) and brine (20 mL). The organic layer was dried (MgSO₄ and concentrated under vacuum. After purification by flash chromatography (silica), the title compound was obtained as a brown solid (83 mg, 39%). ¹H NMR (DMSO-d₆, 400 MHz): δ 8.43-8.41 (m, 2H), 7.46-7.42 (m, 1H), 7.25-7.19 (m, 4H), 7.11 (d, *J* = 7.6 Hz, 1 H), 3.78-3.77 (m, 1H), 3.69-3.68 (m, 1H), 3.55 (d, *J* = 4.6 Hz, 2H), 3.22-3.21 (m, 1H), 3.16-3.15 (m, 3H), 2.92 (d, *J* = 8.7Hz, 2H), 2.76-2.72 (m, 2H), 2.59 (d, *J* = 7.2 Hz, 2H), 2.22-2.21 (m, 3H), 1.85-1.84 (m, 1H), 1.76 (s, 3H), 1.67-1.66 (m, 1H), 1.51-1.50 (m, 1H). LCMS (Method D): Mass found (M+ 529.3), Rt (min): 3.47, Area (%): 97.0 (Max), 96.9 (254 nm). HPLC (Method A): Rt (min): 3.45, Area (%): 93.6 (Max), 95.8 (254 nm).

### Example 13: Preparation of 8-[2-(4-chlorophenyl)ethyl]-3-methyl-4-({4-[3-(trifluoromethyl)phenyl]piperazin-1-yl}carbonyl)-1-oxa-8-azaspiro[4.5]dec-3-en-2-one

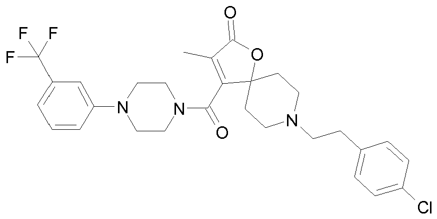

A mixture of 3-methyl-4-({4-[3-(trifluoromethyl)phenyl]piperazin-1-yl}carbonyl)-1-oxa-8-azaspiro[4.5]dec-3-en-2-one dihydrochloride (Example 1, 200 mg, 0.40 mmol), anhydrous potassium carbonate (180 mg, 1.30 mmol) and 1-(2-bromo-ethyl)-4-chlorobenzene (0.07 mL, 0.22 mmol) in anhydrous N,N-dimethylformamide (3 mL) was heated at 90°C for 1.5 hours under microwave irradiation. The reaction mixture was concentrated under vacuum. The residue was taken up in dichloromethane (20 mL), washed with water (20 mL) and brine (20 mL), dried (MgSO₄) and concentrated under vacuum. After purification by flash chromatography (silica), the title compound was obtained as a white solid (46 mg, 20%). ¹H NMR (DMSO-d₆, 400 MHz): δ 7.43 (m, 1H), 7.30 (d, *J* = 8.3 Hz, 2H), 7.24-7.19 (m, 4H), 7.11 (d, *J* = 7.6 Hz, 1H), 3.78-3.77 (m, 1H), 3.69-3.68 (m, 1H), 3.55 (s, 2H), 3.23 (s, 1H), 3.15-3.14 (m, 3H), 2.88 (d, *J* = 7.7 Hz, 2H), 2.71 (t, *J* = 6.8 Hz, 2H), 2.66 (d, *J* = 1.4 Hz, 2H), 2.22-2.20 (m, 3H), 1.84-1.83 (m, 1 H), 1.75 (s, 3H), 1.68-1.67 (m, 1 H), 1.50 (d, *J* = 7.3 Hz, 1 H). LCMS (Method D): Mass found (M+ 562.3), Rt (min): 4.74, Area (%): 98.6 (Max), 98.2 (254 nm). HPLC (Method A): Rt (min): 4.73, Area (%): 99.2 (Max), 98.8 (254 nm).

### Example 14: Preparation of 8-[2-(2-chlorophenyl)ethyl]-3-methyl-4-({4-[3-(trifluoromethyl)phenyl]piperazin-1-yl}carbonyl)-1-oxa-8-azaspiro[4.5]dec-3-en-2-one

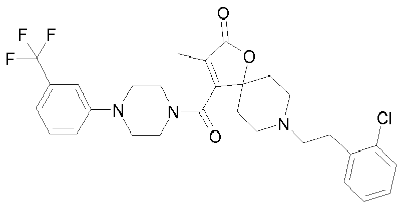

A mixture of 3-methyl-4-({4-[3-(trifluoromethyl)phenyl]piperazin-1-yl}carbonyl)-1-oxa-8-azaspiro[4.5]dec-3-en-2-one dihydrochloride (Example 1 , 200 mg, 0.40 mmol), anhydrous potassium carbonate (180 mg, 1.30 mmol) and 1-(2-bromo-ethyl)-2-chlorobenzene (0.07 mL, 0.22 mmol) in anhydrous N,N-dimethylformamide (3 mL) was heated at 90°C for 1.5 hours under microwave irradiation. The reaction mixture was concentrated under vacuum. The residue was taken up in dichloromethane (20 mL), washed with water (20 mL) and brine (20 mL), dried (MgSO₄) and concentrated under vacuum. After purification by flash chromatography (silica), the title compound was obtained as a white solid (76 mg, 34%). ¹H NMR (DMSO-d₆, 400 MHz): δ 7.46-7.34 (m, 3H), 7.26-7.23 (m, 4H), 7.11 (d, *J* = 7.5 Hz, 1 H), 3.80-3.79 (m, 1H), 3.68-3.69 (m, 1H), 3.56 (s, 2H), 3.27 (s, 1H), 3.16-3.15 (m, 2H), 2.93-2.92 (m, 1H), 2.87-2.83 (m, 4H), 2.53 (s, 2H), 2.25-2.24 (m, 3H), 1.88-1.87 (m, 1H), 1.76 (s, 3H), 1.72 (s, 1H), 1.52 (s, 1H). LCMS (Method D): Mass found (M+ 562), Rt (min): 4.64, Area (%): 99.5 (Max), 99.6 (254 nm). HPLC (Method A): Rt (min): 4.61, Area (%): 98.5 (Max), 99.3 (254 nm).

### Example 15: Preparation of 8-[2-(3-chlorophenyl)ethyl]-3-methyl-4-({4-[3-(trifluoromethyl)phenyl]piperazin-1-yl}carbonyl)-1-oxa-8-azaspiro[4.5]dec-3-en-2-one

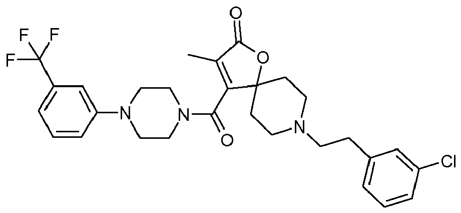

A mixture of 3-methyl-4-({4-[3-(trifluoromethyl)phenyl]piperazin-1-yl}carbonyl)-1-oxa-8-azaspiro[4.5]dec-3-en-2-one dihydrochloride (Example 1, 200 mg, 0.40 mmol), anhydrous potassium carbonate (180 mg, 1.30 mmol) and 1-(2-bromo-ethyl)-3-chlorobenzene (0.07 mL, 0.22 mmol) in anhydrous N,N-dimethylformamide (3 mL) was heated at 90°C for 1.5 hours under microwave irradiation. The reaction mixture was concentrated under vacuum. The residue was taken up in dichloromethane (20 mL), washed with water (20 mL) and brine (20 mL), dried (MgSO₄) and concentrated under vacuum. After purification by flash chromatography (silica), the title compound was obtained as a white solid (44 mg, 20%). ¹H NMR (DMSO-d₆, 400 MHz): δ 7.46-7.42 (m, 1 H), 7.30-7.17 (m, 6H), 7.11 (d, *J* = 7.5 Hz, 1H), 3.79-3.78 (m, 1H), 3.68-3.67 (m, 1H), 3.55 (s, 3H), 3.55 (d, *J* = 4.9 Hz, 2H), 2.89-2.88 (m, 3H), 2.73-2.71 (m, 2H), 2.49-2.48 (m, 2H), 2.23-2.21 (m, 3H), 1.82-1.81 (m, 1H), 1.75 (s, 3H), 1.67-1.66 (m, 1H), 1.51-1.49 (d, *J* = 7.3 Hz, 1H). LCMS (Method D): Mass found (M+ 562), Rt (min): 4.75, Area (%): 99.6 (Max), 99.5 (254 nm). HPLC (Method A): Rt (min): 4.72, Area (%): 99.3 (Max), 99.2 (254 nm).

The following Examples 16 to 20 were obtained following procedures described for Examples 1 to 15, but starting from 3-methyl-4-{[7-(trifluoromethyl)-3,4-dihydroisoquinolin-2(1H)-yl]carbonyl}-1-oxa-8-azaspiro[4.5]dec-3-en-2-one hydrochloride (Example 25, step 2).

| **Ex.** | **Structure** | **Characterization** |
|---|---|---|
| 16 | 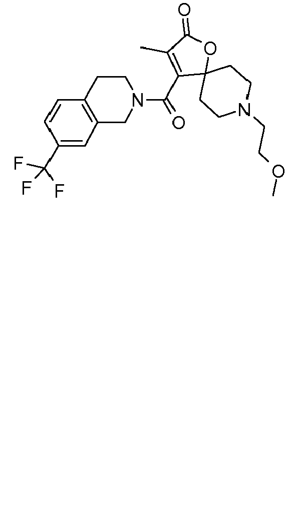 | **8-(2-methoxyethyl)-3-methyl-4-{[7-(trifluoromethyl)-3,4-dihydroisoquinolin-2(1H)-yl]carbonyl}-1-oxa-8-azaspiro[4.5]dec-3-en-2-one** |
| | | After purification by flash chromatography, the compound was obtained as a colorless gum (41 mg, 41 %). ¹H NMR (400 MHz, CDCl₃): δ 7.48 (m, 2H), 7.34 (d, J = 8.0 Hz, 1H), 4.87 (d, J = 6.6 Hz, 1H), 4.56 (d, J= 6.3 Hz, 1H), 4.05-3.91 (m, 1H), 3.66-3.65 (m, 1H), 3.51-3.46 (m, 2H), 3.34 (s, 3H), 3.01 (s, 1H), 2.93-2.96 (m, 3H), 2.63-2.58 (m, 2H), 2.48-2.42 (m, 2H), 2.05 (s, 1H), 1.89 (s, 3H), 1.79-1.66 (m, 3H). LCMS (Method D): Mass found (M+ 453.3), Rt (min) 4.36, Area (%): 97.0 (max), 96.7 (220 nm); HPLC (Method A) Rt (min): 3.7, Area (%): 96.8 (max), 94.5 (254 nm). |
| 17 | 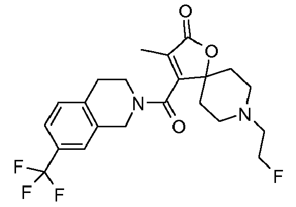 | **8-(2-fluoroethyl)-3-methyl-4-{[7-(trifluoromethyl)-3,4-dihydroisoquinolin-2(1H)-yl]carbonyl}-1-oxa-8-azaspiro[4.5]dec-3-en-2-one** |
| | | After purification by preparative HPLC, the compound was obtained as a white solid (19 mg, 19%). ¹H NMR (CDCl₃, 400 MHz): δ 7.47-7.49 (m, 2H), 7.27-7.34 (m, 1 H), 4.88 (s, 1H), 4.47-4.63 (m, 3H), 3.85-4.02 (m, 1 H), 3.55-3.67 (m, 1H), 2.79-3.02 (m, 4H), 2.75-2.80 (m, 1 H), 2.68-2.73 (m, 1H), 2.49-2.55 (m, 3H), 2.08-2.11 (m, 1 H), 1.80-1.90 (m, 3H),1.58-1.66 (m, 2H). LCMS (Method D): Mass found (M+ 441), Rt (min) 3.62, Area (%): 95.9 (max), 96.8 (220 nm); HPLC (Method D) Rt (min): 3.70, Area (%): 98.6 (max), 98.2 (220 nm). |
| 18 | 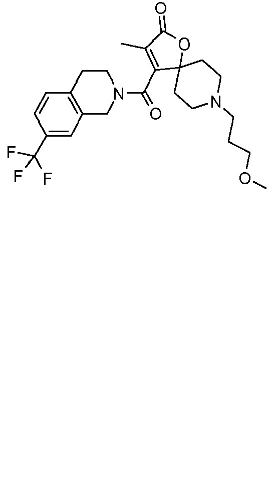 | **8-(3-methoxypropyl)-3-methyl-4-{[7-(trifluoromethyl)-3,4-dihydroisoquinolin-2(1H)-yl]carbonyl}-1-oxa-8-azaspiro[4.5]dec-3-en-2-one** |
| | | After purification by flash chromatography, the compound was obtained as an off-white solid (29 mg, 28%). ¹H NMR (DMSO-d₆, 400 MHz): δ 7.70 (s, 1 H), 7.53 (d, *J* = 7.8 Hz, 1 H), 7.41 (d, *J* = 7.8 Hz, 1 H), 4.72-4.88 (m, 2H), 3.86 (s, 1 H), 3.70 (s, 1H), 3.32 (s, 2H), 3.32 (s, 3H), 2.88-2.95 (m, 2H), 2.67-2.79 (m, 2H), 2.32 (s, 2H), 2.12 (m, 3H), 1.76 (s, 3H), 1.08-1.62 (m, 5H). LCMS (Method D): Mass found (M+ 467.3), Rt (min) 3.79, Area(%) 98.1 (max), 97.7 (220 nm); HPLC (Method D) Rt (min): 3.8, Area (%): 97.7 (max), 97.6 (220 nm). |
| 19 | 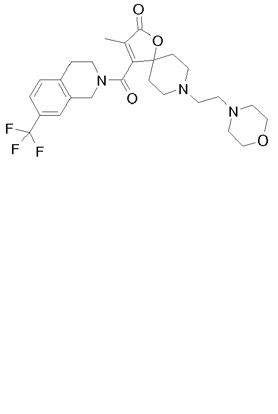 | **3-methyl-8-(2-morpholin-4-ylethyl)-4-{[7-(trifluoromethyl)-3,4-dihydroisoquinolin-2(1H)-yl]carbonyl}-1-oxa-8-azaspiro[4.5]dec-3-en-2-one** |
| | | After purification by flash chromatography, the compound was obtained as a yellow solid 23 mg (19%). ¹H NMR (400 MHz, CDCl₃): δ 7.49 (m, 2H), 7.28 (m, 1H), 4.89 (s, 1 H), 4.60 (m, 1H), 3.73-3.70 (m, 1H), 3.69-3.66 (m, 6H), 3.01-2.93 (m, 4H), 2.59-2.47 (s, 13H), 1.89 (s, 3H). LCMS (Method D): Mass found (M+ 508.3), Rt (min) 3.35, Area (%), 95.9 (max), 95.6 (220 nm); HPLC (Method D) Rt (min): 3.39, Area (%): 98.9 (max), 98.5 (220 nm). |
| 20 | 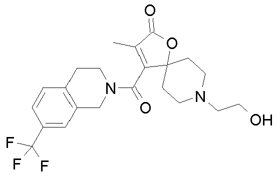 | **8-(2-hydroxyethyl)-3-methyl-4-{[7-(trifluoromethyl)-3,4-dihydroisoquinolin-2(1H)-yl]carbonyl}-1-oxa-8-azaspiro[4.5]dec-3-en-2-one** |
| | | After purification by flash chromatography, the compound was obtained as a white solid 70 mg (68%). ¹H NMR (400 MHz, CDCl₃): δ 7.47 (m, 2H), 7.29-7.28 (m, 1H), 4.90-4.96 (m, 1H), 4.56-4.60 (m, 1H), 4.05 (m, 1H), 3.66-3.60 (m, 3H), 3.04-2.89 (m, 4H), 2.61-2.50 (m, 5H), 1.99-2.12 (m, 1H), 1.91 (m, 3H), 1.80 (s, 2H). LCMS (Method D): Mass found (M+ 439.3), Rt (min) 3.48, Area (%) 93.4 (max), 93.2 (220 nm); HPLC (Method D) Rt (min): 3.55, Area (%): 97.6 (max), 96.2 (220 nm). |

### Example 21: 3-methyl-8-(tetrahydro-2H-pyran-4-yl)-4-({4-[3-(trifluoromethyl)phenyl]piperazin-1-yl}carbonyl)-1-oxa-8-azaspiro[4.5]dec-3-en-2-one

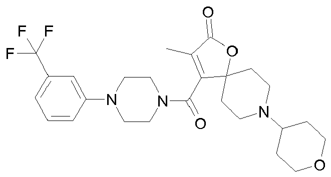

Triethylamine (0.08 mL, 0.65 mmol) and tetrahydropyran-4-one (108 mg, 1.08 mmol) were added into a solution of 3-methyl-4-({4-[3-(trifluoromethyl)phenyl]piperazin-1-yl}carbonyl)-1-oxa-8-azaspiro[4.5]dec-3-en-2-one dihydrochloride (Example 1, 100 mg, 0.20 mmol) in anhydrous dichloroethane (5 mL) cooled at 0°C. Sodium triacetoxyborohydride (137 mg, 0.65 mmol) was added and the resulting mixture was stirred at room temperature for 12 hours. The reaction mixture was diluted with dichloromethane (20 mL) and washed with water (20 mL) and brine (20 mL). The organic layer was dried (MgSO₄) and concentrated under vacuum. After purification by flash chromatography (silica), the title compound was obtained as a white solid. ¹H NMR (DMSO-d₆, 400 MHz): δ 7.46-7.42 (m, 1 H), 7.25 (d, J = 8.8 Hz, 1H), 7.20 (s, 1H), 7.11 (d, J = 7.7 Hz, 1H), 3.86 (d, J = 7.9 Hz, 2H), 3.79 (s, 1 H), 3.69 (s, 1 H), 3.55 (s, 2H), 3.12 (s, 4H), 2.84 (d, J = 9.6 Hz, 2H), 2.37-2.32 (m, 3H), 2.22-2.15 (m, 2H), 1.80-1.79 (m, 2H), 1.76 (s, 3H), 1.67-1.63 (m, 3H), 1.43-1.40 (m, 3H). LCMS (Method D): Mass found (M+ 508), Rt (min): 3.85, Area (%): 99.0 (Max. Chrom.), 99.0 (254 nm). HPLC (Method A): Rt (min): 3.82, Area (%): 96.3 (Max. Chrom.), 98.5 (254 nm).

### Example 22: 3-methyl-8-(tetrahydrofuran-3-ylmethyl)-4-({4-[3-(trifluoromethyl)phenyl]piperazin-1-yl}carbonyl)-1-oxa-8-azaspiro[4.5]dec-3-en-2-one

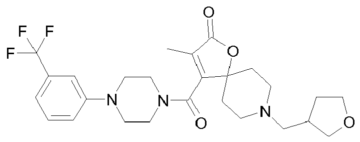

Triethylamine (0.08 mL, 0.65 mmol) and a 50% aqueous solution of tetrahydro-furan-3-carboxaldehyde (108 mg, 1.08 mmol) were added into a solution of 3-methyl-4-({4-[3-(trifluoromethyl)phenyl]piperazin-1-yl}carbonyl)-1-oxa-8-azaspiro[4.5]dec-3-en-2-one dihydrochloride (Example 1, 100 mg, 0.20 mmol) in anhydrous dichloroethane (5 mL) cooled at 0°C. Sodium triacetoxyborohydride (137 mg, 0.65 mmol) and trimethyl orthoformate (120 mg, 1.08 mmol) were added and the resulting mixture was stirred for 12 hours. The reaction mixture was diluted with dichloromethane (20 mL) and washed with water (20 mL) and brine (20 mL). The organic layer was dried (MgSO₄) and concentrated under vacuum. After purification by flash chromatography (silica), the title compound was obtained as a white solid. ¹H NMR (DMSO-d₆, 400 MHz): δ 7.46-7.42 (m, 1H), 7.25 (d, *J* = 8.4 Hz, 1 H), 7.20 (s, 1H), 7.11 (d, *J* = 7.7 Hz, 1 H), 3.79-3.78 (m, 1H), 3.71-3.65 (m, 3H), 3.60-3.56 (m, 3H), 3.35-3.34 (m, 2H), 3.23-3.17 (m, 2H), 2.88-2.81 (m, 2H), 2.66-2.65 (m, 1H), 2.42-2.37 (m, 1H), 2.32-2.26 (m, 2H), 2.19-2.18 (m, 3H), 1.93-1.86 (m, 2H), 1.76 (s, 3H), 1.69-1.68 (m, 1 H), 1.49 (t, *J* = 5.5 Hz, 2H). LCMS (Method D): Mass found (M+ 508.3), Rt (min): 3.86, Area (%): 98.8 (Max. Chrom.), 99.6 (254 nm). HPLC (Method A): Rt (min): 3.82, Area (%): 96.3 (Max. Chrom.), 98.5 (254 nm).

### Example 23: 3-methyl-8-(tetrahydro-2H-pyran-3-yl)-4-({4-r3-(trifluoromethyl)phenyl]piperazin-1-yl}carbonyl)-1-oxa-8-azaspiro[4.5]dec-3-en-2-one

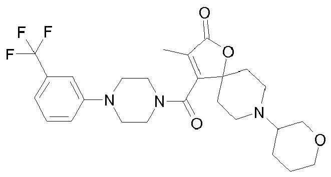

Triethylamine (0.08 mL, 0.65 mmol) and dihydro-pyran-3-one (108 mg 1.08 mmol) were added into a solution of 3-methyl-4-({4-[3-(trifluoromethyl)phenyl]piperazin-1-yl}carbonyl)-1-oxa-8-azaspiro[4.5]dec-3-en-2-one dihydrochloride (Example 1, 100 mg, 0.20 mmol) in anhydrous dichloroethane (5 mL) cooled at 0°C. Sodium triacetoxyborohydride (137 mg, 0.65 mmol) and trimethylorthoformate (120 mg, 1.08 mmol) were added and the resulting mixture was stirred at room temperature for 12 hours. The reaction mixture was diluted with dichloromethane (20 mL) and washed with water (20 mL) and brine (20 mL). The organic layer was dried (MgSO₄) and concentrated under vacuum. After purification by flash chromatography (silica), the title compound was obtained as a white solid. ¹H NMR (CDCl₃, 400 MHz): δ 7.45-7.42 (m, 1 H), 7.24 (d, J = 9.0 Hz, 1 H), 7.20 (s, 1 H), 7.11 (d, J = 7.6 Hz, 1 H), 3.81-3.80 (m, 2H), 3.71-3.69 (m, 2H), 3.55 (s, 2H), 3.15 (s, 4H), 2.81-2.80 (m, 2H), 2.32-2.31 (m, 4H), 2.17 (s, 2H), 1.89-1.88 (m, 2H), 1.75 (s, 3H), 1.69-1.61 (m, 2H), 1.48-1.38 (m, 3H). LCMS (Method D): Mass found (M+ 508.3), Rt (min): 3.88, Area (%): 93.6 (Max. Chrom.), 94.5 (254 nm). HPLC (Method A): Rt (min): 3.89, Area (%): 93.6 (Max. Chrom.), 94.4 (254 nm).

### Example 24: 4-[3-methyl-2-oxo-4-({4-[3-(trifluoromethyl)phenyl]piperazin-1-yl}carbonyl)-1-oxa-8-azaspiro[4.5]dec-3-en-8-yl]cyclohexanecarboxylic acid

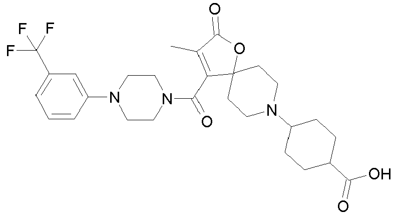

### Step 1 : Preparation of ethyl 4-[3-methyl-2-oxo-4-({4-[3-(trifluoromethyl)phenyl]piperazin-1-yl}carbonyl)-1-oxa-8-azaspiro[4.5]dec-3-en-8-yl]cyclohexanecarboxylate

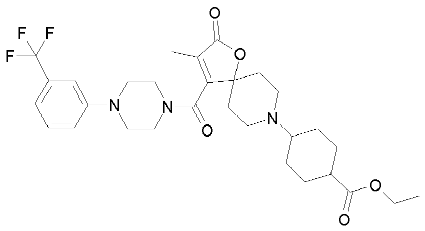

Triethylamine (0.8 mL, 5.4 mmol) and 4-oxo-cyclohexanecarboxylic acid ethyl ester (185 mg, 1.08 mmol) were added into a solution 3-methyl-4-({4-[3-(trifluoromethyl)phenyl]piperazin-1-yl}carbonyl)-1-oxa-8-azaspiro[4.5]dec-3-en-2-one dihydrochloride (Example 1, 500 mg, 1.01 mmol)) in anhydrous dichloroethane (10 mL) cooled at 0°C. Sodium triacetoxyborohydride (461 mg, 2.1 mmol) was added and the resulting mixture was stirred at room temperature for 12 hours. The reaction mixture was diluted with dichloromethane (20 mL) and washed with water (20 mL) and brine (20 mL). The organic layer was dried (MgSO₄) and concentrated under vacuum. After purification by preparative HPLC, the first eluting isomer was isolated as a white solid (100 mg). ¹H NMR (DMSO-d₆, 400 MHz): δ 7.46-7.42 (m, 1H), 7.24 (d, J = 8.4 Hz, 1H), 7.19 (s, 1H), 7.11 (d, J = 7.4 Hz, 1 H), 4.08-4.04 (m, 3H), 3.79 (m, 1H), 3.71 (m, 2H), 3.56 (s, 2H), 3.17 (m, 3H), 2.72 (m, 3H), 2.26 (m, 3H), 2.06 (d, *J* = 8.5 Hz, 2H), 1.68 (s, 4H), 1.46 (s, 8H), 1.32 (t, *J* = 4.6 Hz, 3H). LCMS (Method D): Mass found (M+ 578.3), Rt (min): 4.25, Area (%): 95.1 (Max. Chrom.), 95.9 (254 nm).

### Step 2: Preparation of 4-[3-methyl-2-oxo-4-({4-[3-(trifluoromethyl)phenyl]piperazin-1-yl}carbonyl)-1-oxa-8-azaspiro[4.5]dec-3-en-8-yl]cyc/ohexanecarboxylic acid

Lithium hydroxide monohydrate (21 mg, 0.51 mmol) was added into a solution of ethyl 4-[3-methyl-2-oxo-4-({4-[3-(trifluoromethyl)phenyl]piperazin-1-yl}carbonyl)-1-oxa-8-azaspiro[4.5]dec-3-en-8-yl]cyclohexanecarboxylate (100 mg, 0.17 mmol) in a mixture of tetrahydrofuran (8 mL) and methanol (2 mL) was added. The resulting mixture was stirred at room temperature for 2 hours. The reaction mixture was concentrated under vacuum. The residue was taken up with dichloromethane (20 mL) and washed with a saturated aqueous solution of citric acid (20 mL), water (20 mL) and brine (20 mL). The organic layer was dried (MgSO₄) and concentrated under vacuum to give the title compound as an off-white solid. ¹H NMR (CDCl₃, 400 MHz): δ 7.41-7.37 (m, 1 H), 7.17 (d, *J* = 7.4 Hz, 1H), 7.11-7.08 (m, 2H), 3.94-3.84 (m, 3H), 3.65 (s, 2H), 3.54 (s, 2H), 3.49-3.29 (m, 3H), 2.93 (s, 2H), 2.55-2.22 (m, 3H), 2.14-2.05 (m, 5H), 1.90 (s, 3H), 1.63-1.60 (m, 3H), 1.45-1.33 (m, 3H). LCMS (Method D): Mass found (M+ 550.3), Rt (min): 3.86, Area (%): 94.8 (Max. Chrom.), 95.0 (254 nm). HPLC (Method A): Rt (min): 3.89, Area (%): 88.7 (Max. Chrom.), 93.0 (254 nm).

### Example 25: 8-[4-hydroxy-4-(6-methoxypyridin-3-yl)cyclohexyl]-3-methyl-4-{[7-(trifluoromethyl)-3,4-dihydroisoquinolin-2(1H)-yl]carbonyl}-1-oxa-8-azaspiro[4.5]dec-3-en-2-one

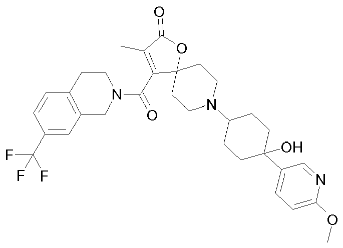

### Step 1: Preparation of tert-butyl 3-methyl-2-oxo-4-{[7-(trifluoromethyl)-3,4-dihydroisoquinolin-2(1H)-yl]carbonyl}-1-oxa-8-azaspiro[4.5]dec-3-ene-8-carboxylate

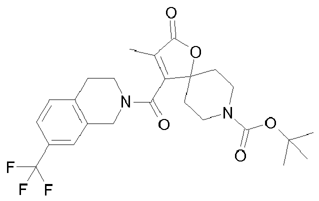

Diisopropylethylamine (8.3 g, 40.1 mmol) and 7-trifluoromethyl-1,2,3,4-tetrahydro-isoquinoline hydrochloride (2.2 g, 9.6 mmol) were added into a solution of 3-methyl-2-oxo-1-oxa-8-aza-spiro[4,5]dec-3-ene-4,8-dicarboxylic acid 8-tert-butyl ester (Intermediate 2, 2.5 g, 8.0 mmol) in anhydrous dichloromethane (50 mL) and stirred for 5 minutes, then 1-propane phosphoric acid cyclic anhydride (10.2 g, 32.1 mmol) was added dropwise. The resulting mixture was stirred at room temperature for 16 hours. The reaction mixture was diluted with cold water (100 mL). The organic layer was washed with brine (100 mL), dried (MgSO₄) and concentrated under vacuum. After purification by flash chromatography (silica), the title compound was obtained as an off-white solid. TLC: chloroform/methanol (9/1) R*_{f}*: 0.4. ¹H NMR (CDCl₃, 400 MHz): δ 7.50-7.47 (m, 1 H), 7.30-7.27 (m, 2H), 4.92-4.91 (m, 1 H), 4.75-4.74 (m, 1 H), 3.99-3.98 (m, 2H), 3.67 (s, 1 H), 3.58 (s, 1 H), 3.02-2.95 (m, 4H), 2.32-2.31 (m, 1 H), 1.92 (s, 2H), 1.80 (s, 4H), 1.45 (s, 9H). LCMS (Method D): Mass found (M+ 395.0 [M-Boc+2H]⁺), Rt (min): 5.35, Area (%): 97.1 (Max. Chrom.), 97.6 (254 nm). HPLC (Method A): Rt (min): 5.42, Area (%): 98.9 (Max. Chrom.), 98.8 (220 nm).

### Step 2: Preparation of 3-methyl-4-{[7-(trifluoromethyl)-3,4-dihydroisoquinolin2(1H)-yl]carbonyl}-1-oxa-8-azaspiro[4.5]dec-3-en-2-one, hydrochloride salt

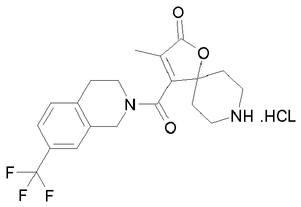

A 4N solution of HCl in 1,4-dioxane (15 mL) was added into a solution of tert-butyl 3-methyl-2-oxo-4-{[7-(trifluoromethyl)-3,4-dihydroisoquinolin-2(1H)-yl]carbonyl}-1-oxa-8-azaspiro[4.5]dec-3-ene-8-carboxylate (1.2 g, 2.4 mmol) in 1,4-dioxane (15 mL). The resulting mixture was stirred at room temperature for 3 hours. The reaction mixture was concentrated under vacuum. The residue was triturated in diethyl ether (100 mL), filtered off and dried under vacuum to give the title compound as a white solid (1.0 g, 97%). LCMS (Method D): Mass found (M+ 395.0), Rt (min): 3.49, Area (%): 98.4 (Max. Chrom.), 98.3 (254 nm). HPLC (Method A): Rt (min): 3.77, Area (%): 97.9 (Max. Chrom.), 96.8 (254 nm).

### Step 3: Preparation of 8-[4-hydroxy-4-(6-methoxy-pyridin-3-yl)-cyclohexyl]-3-methyl-4-(7-trifluoromethyl-3,4-dihydro-1H-isoquinoline-2-carbonyl)-1-oxa-8-azaspiro[4.5]dec-3-en-2-one

Triethylamine (0.1 mL, 0.69 mmol) and 4-hydroxy-4-(6-methoxy-pyridin-3-yl)-cyclohexanone (222 mg, 1.08 mmol) were added into a solution of 3-methyl-4-{[7-(trifluoromethyl)-3,4-dihydroisoquinolin-2(1H)-yl]carbonyl}-1-oxa-8-azaspiro[4.5]dec-3-en-2-one hydrochloride (100 mg, 0.23 mmol) in anhydrous dichloroethane (5 mL) cooled at 0°C. Sodium triacetoxyborohydride (146 mg, 0.69 mmol) was added and the resulting mixture was stirred at room temperature for 12 hours. The reaction mixture was diluted with dichloromethane (20 mL) and washed with water (20 mL) and brine (20 mL). The organic layer was dried (MgSO₄) and concentrated under vacuum. After purification by flash chromatography (silica), the title compound was obtained as a white solid (109 mg, 42%). ¹H NMR (CDCl₃, 400 MHz): δ 8.43-8.41 (m, 1 H), 7.76-7.68 (m, 1 H), 7.49-7.46 (m, 1 H), 7.34-7.32 (m, 1 H), 7.28-7.27 (m, 1 H), 6.76-6.71 (m, 1 H), 4.90 (s, 1 H), 4.56 (s, 1 H), 3.99 (m, 1 H), 3.94 (s, 3H), 3.67-3.64 (m, 1 H), 3.02 (m, 1 H), 2.95 (d, *J* = 5.8 Hz, 2H), 2.89 (m, 1 H), 2.49-2.26 (m, 4H), 1.95-1.92 (m, 1 H), 1.80 (s, 3H), 1.68 (s, 2H), 1.65-1.56 (m, 8H). LCMS (Method D): Mass found (M+ 600.3), Rt (min): 5.74, Area (%): 93.4 (Max. Chrom.), 92.6 (220 nm). HPLC (Method A): Rt (min): 3.56, Area (%): 97.7 (Max. Chrom.), 97.4 (220 nm).

### Example 26: 3-methyl-8-(tetrahydro-2H-pyran-4-yl)-4-{[7-(trifluoromethyl)-3,4-dihydroisoquinolin-2(1H)-yl]carbony}-1-oxa-8-azaspiro[4.5]dec-3-en-2-one

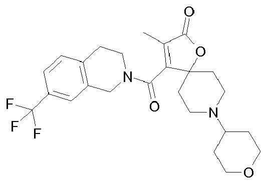

Triethylamine (0.1 mL, 0.69 mmol) and tetrahydro-pyran-4-one (108 mg, 1.08 mmol) were added into a solution of 3-methyl-4-{[7-(trifluoromethyl)-3,4-dihydroisoquinolin-2(1H)-yl]carbonyl}-1-oxa-8-azaspiro[4.5]dec-3-en-2-one hydrochloride (Example 25 (step 2), 100 mg, 0.23 mmol) in anhydrous dichloroethane (5 mL) cooled to 0°C. Sodium triacetoxyborohydride (146 mg, 0.69 mmol) was added and the resulting mixture was stirred at room temperature for 12 hours. The reaction mixture was diluted with dichloromethane (20 mL) and washed with water (20 mL) and brine (20 mL). The organic layer was dried (MgSO₄) and concentrated under vacuum. After purification by flash chromatography (silica), the title compound was obtained as a white solid. ¹H NMR (CDCl₃, 400 MHz): δ 7.50-7.46 (m, 2H), 7.34-7.32 (m, 1 H), 4.89 (s, 1 H), 4.57 (s, 1 H), 4.04-4.01 (m, 3H), 3.66 (s, 1 H), 3.40-3.37 (m, 2H), 3.02-2.95 (m, 4H), 2.65-2.57 (m, 4H), 2.05-2.01 (m, 2H), 1.80 (s, 2H), 1.74-1.62 (m, 6H). LCMS (Method D): Mass found (M+ 479.3), Rt (min): 3.57 area (%): 97.9 (Max. Chrom.), 97.6 (220 nm). HPLC (Method A): Rt (min): 3.62, Area (%): 97.5 (Max. Chrom.), 97.3 (220 nm).

The following Examples 27-42 were obtained following the procedures described for Examples 25 and 26. Example 42 was obtained from Intermediate 17. The absolute and relative stereochemistry of Examples 40 and 41 were assigned arbitrarily.

| **Ex.** | **Structure** | **Characterization** |
|---|---|---|
| 27 | 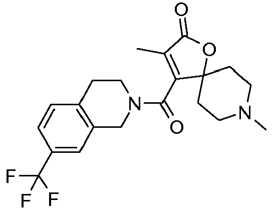 | **3,8-dimethyl-4-{[7-(trifluoromethyl)-3,4-dihydroisoquinolin-2(1H)-yl]carbonyl}-1-oxa-8-azaspiro[4.5]dec-3-en-2-one** |
| | | After purification by flash chromatography, the compound was obtained as an off-white solid (16 mg). ¹H NMR (CDCl₃, 400 MHz): δ 7.46-7.50 (m, 2H), 7.27-7.33 (m, 1H), 4.87 (s, 1 H), 4.57 (d, *J* = 5.2 Hz, 1 H), 3.85-4.09 (m, 1H), 3.67 (d, *J* = 5.7 Hz, 1 H), 2.93-3.00 (m, 2H), 2.72-2.83 (m, 2H), 2.38-2.41 (m, 3H), 2.34 (s, 3H), 1.98.-2.02 (m, 1H), 1.82-1.90 (m, 3H), 1.63-1.70 (m, 2H). LCMS (Method D): Mass found (M+ 409.3), Rt (min): 3.58, Area (%): 97.1 (Max. Chrom.), 96.8 (220 nm). HPLC (Method A): Rt (min): 3.59, Area (%): 99.1 (Max. Chrom.), 99.12 (220 nm). |
| 28 | 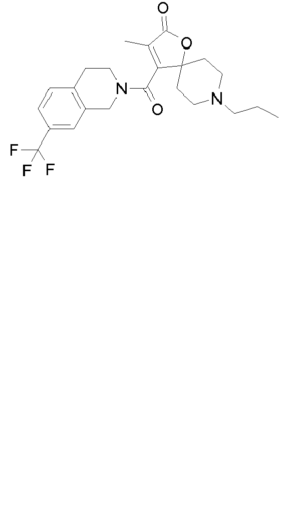 | **3-methyl-8-propyl-4-{[7-(trifluoromethyl)-3,4-dihydroisoquinolin-2(1H)-yl]carbonyl}-1-oxa-8-azaspiro[4.5]dec-3-en-2-one** |
| | | After purification by flash chromatography, the compound was obtained as an off-white solid (63 mg, 63%). ¹H NMR (CDCl₃, 400 MHz): δ 7.46-7.48 (m, 2H), 7.27-7.33 (m, 1H), 4.88 (s, 1H), 4.57 (d, *J* = 5.4 Hz, 1 H), 3.85-4.09 (m, 1H), 3.67 (d, *J* = 5.7 Hz, 1H), 2.89-3.01 (m, 4H), 2.31-2.40 (m, 4H), 1.92-2.02 (m, 3H), 1.86 (d, 3H), 1.69-1.79 (m, 1H), 1.41-1.55 (m, 2H), 0.86-0.91 (m, 3H). LCMS (Method D): Mass found (M+ 437.3); Rt (min): 3.79, Area (%): 90.1 (Max. Chrom.), 92.1 (220 nm); HPLC (Method A), Rt (min): 3.86, Area (%): 95.7 (Max. Chrom.), 94.0 (220 nm). |
| 29 | 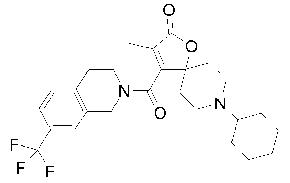 | **8-cyclohexyl-3-methyl-4-{[7-(trifluoromethyl)-3,4-dihydroisoquinolin-2(1H)-yl]carbonyl}-1-oxa-8-azaspiro[4.5]dec-3-en-2-one** |
| | | After purification by flash chromatography, the compound was obtained as a white solid (32 mg). ¹H NMR (CDCl₃, 400 MHz): δ 7.46-7.50 (m, 2H), 7.27-7.33 (m, 1H), 4.88 (s, 1H), 4.56 (d, *J* = 5.4 Hz, 1 H), 3.96-3.97 (m, 1H), 3.58-3.68 (m, 1H), 3.02-3.03 (m, 1H), 2.93-2.96 (m, 1H), 2.85-2.86 (m, 1H), 2.64-2.76 (m, 2H), 2.33-2.50 (m, 2H), 2.10-2.12 (m, 1H), 1.80-1.92 (m, 6H), 1.62-1.70 (m, 5H), 1.10-1.26 (m, 4H), 1.07-1.09 (m, 1 H). LCMS (Method D): Mass found (M+ 477.2), Rt (min) 4.11, Area (%): 92.2 (Max. Chrom.), 92.2 (220 nm). HPLC (Method A), Rt (min): 4.16, Area (%): 95.4 (Max. Chrom.), 95.7 (220 nm). |
| 30 | 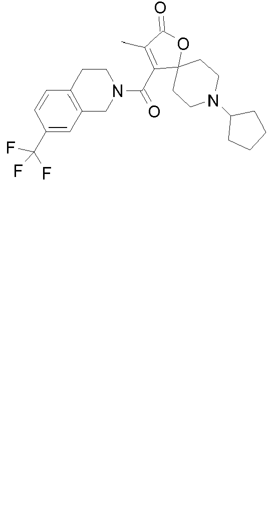 | **8-cyclopentyl-3-methyl-4-{[7-(trifluoromethyl)-3,4-dihydroisoquinolin-2(1H)-yl]carbonyl}-1-oxa-8-azaspiro[4.5]dec-3-en-2-one** |
| | | After purification by flash chromatography, the compound was obtained as a white solid (23 mg). ¹H NMR (CDCl₃, 400 MHz): δ 7.46-7.53 (m, 2H), 7.25-7.33 (m, 1 H), 4.87 (s, 1 H), 4.56 (d, *J* = 5.4 Hz, 1 H), 3.91-4.02 (m, 1 H), 3.54-3.65 (m, 2H), 2.98-3.03 (m, 3H), 2.95 (t, *J*= 4.0 Hz, 2H), 2.50-2.64 (m, 1 H), 2.46-2.48 (m, 4H), 2.11-2.13 (m, 1 H), 1.84-1.92 (m, 2H), 1.80-1.81 (m, 2H), 1.64-1.70 (m, 2H). 1.57-1.61 (m, 2H), 1.40-1.42 (m, 2H). LCMS (Method D): Mass found (M+ 463.3), Rt (min): 3.97, Area (%): 93.6 (Max. Chrom.), 93.9 (220 nm); HPLC (Method A); Rt (min): 3.99 Area (%): 96.9 (Max. Chrom.), 97.0 (220 nm). |
| 31 | 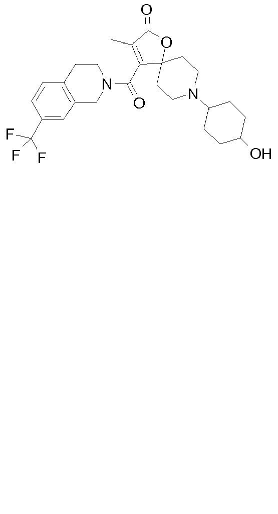 | **8-(4-hydroxycyclohexyl)-3-methyl-4-{[7-(trifluoromethyl)-3,4-dihydroisoquinolin-2(1H)-yl]carbonyl}-1-oxa-8-azaspiro[4.5]dec-3-en-2-one** |
| | | After purification by flash chromatography, the compound was obtained as a white solid (95 mg, 83%). ¹H NMR (DMSO-d₆, 400 MHz): δ 7.70 - 7.63 (m, 1 H), 7.53 (d, *J* = 8.1 Hz, 1 H), 7.41 (d, *J* = 7.9 Hz, 1 H), 4.71-4.87 (m, 2H), 4.25-4.48 (m, 1 H), 3.69-3.85 (m, 3H), 3.32 (s, 1 H), 2.88-2.95 (m, 2H), 2.77 (s, 2H), 2.39 (s, 2H), 2.22 (s, 2H), 1.69-1.75 (m, 4H), 1.61 (s, 4H), 1.49 (s, 1 H), 1.37 (s, 2H), 1.12-1.47 (m, 1 H). LCMS: (Method D): Mass found (M+ 493.2), Rt (min): 3.62, Area (%): 96.3 (Max. Chrom.), 94.6 (220 nm). HPLC (Method A): Rt (min): 3.62, Area (%): 98.8 (Max. Chrom.), 98.4 (220 nm). |
| 32 | 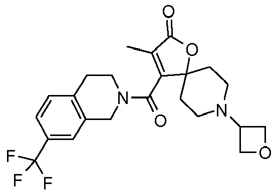 | **3-methyl-8-oxetan-3-yl-4-{[7-(trifluoromethyl)-3,4-dihydroisoquinolin-2(1H)-yl]carbonyl}-1-oxa-8-azaspiro[4.5]dec-3-en-2-one** |
| | | After purification by flash chromatography, the compound was obtained as a white solid (101 mg, 64%). ¹H NMR (CDCl₃, 400 MHz): δ 7.47-7.51 (m, 2H), 7.26-7.35 (m, 1H), 4.91 (s, 1H), 4.67-4.69 (m, 2H), 4.54-4.58 (m, 2H), 3.85-4.02 (m, 1H), 3.67 (t, *J* = 8.0 Hz, 1H), 3.50-3.56 (m, 1 H), 2.97-3.03 (m, 1 H), 2.96-2.97 (m, 1H), 2.71-2.74 (m,2H), 2.49-2.50 (m, 1H), 2.25-2.30 (m, 2H), 2.10-2.16 (m, 1H), 1.81-1.9 (m, 3H), 1.66-1.70 (m, 2H), 1.54-1.61 (m, 1H). LCMS: (Method D): Mass found (M+ 451.2), Rt (min): 3.50, Area (%): 96.7 (Max. Chrom.), 95.5 (220 nm). HPLC (Method A) Rt (min): 3.55, Area (%): 96.8 (Max. Chrom.), 96.3 (220 nm). |
| 33 | 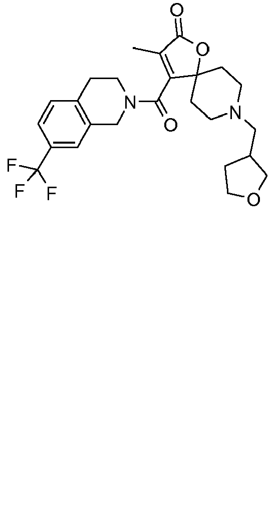 | **3-methyl-8-(tetrahydrofuran-3-ylmethyl)-4-{[7-(trifluoromethyl)-3,4-dihydroisoquinolin-2(1H)-yl]carbonyl}-1-oxa-8-azaspiro[4.5]dec-3-en-2-one** |
| | | After purification by flash chromatography, the compound was obtained as a white solid (44 mg). ¹H NMR (DMSO-d₆, 400 MHz): δ 7.69 (s, 1H), 7.53 (d, *J* = 7.6 Hz, 1H), 7.41 (d, *J* = 8.1 Hz, 1H), 4.90 (s, 1H), 4.70-4.81 (m, 1H), 3.85-3.87 (m, 1 H), 3.64-3.71 (m, 3H), 3.54-3.59 (m, 1H), 2.96 (t, *J* = 6.0 Hz, 1 H), 2.78-2.88 (m, 3H), 2.36-2.39 (m, 1 H), 2.11-2.32 (m, 5H), 1.86-1.90 (m, 2H), 1.76 (s, 3H), 1.61 (s, 2H), 1.47 (s, 2H). LCMS: (Method D): Mass found (M+ 479.3), Rt (min): 3.62, Area (%): 92.8 (Max. Chrom.), 92.2 (220 nm). HPLC (Method A), Rt (min): 3.68, Area (%): 98.6 (Max. Chrom.), 95.8 (220 nm). |
| 34 | 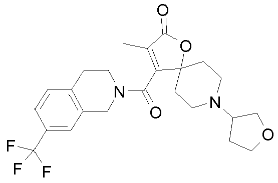 | **3-methyl-8-(tetrahydrofuran-3-yl)-4-{[7-(trifluoromethyl)-3,4-dihydroisoquinolin-2(H)-yl]carbonyl}-1-oxa-8-azaspiro[4.5]dec-3-en-2-one** |
| | | After purification by flash chromatography, the compound was obtained as a white solid (102 mg, 63%). ¹H NMR (CDCl₃, 400 MHz): δ 7.47-7.51 (m, 2H), 7.26-7.34 (m, 1 H), 4.88 (s, 1 H), 4.56 (d, *J* = 7.7 Hz, 1 H), 3.95-4.02 (m, 1 H), 3.86-3.94 (m, 2H), 3.74-3.84(m, 1 H), 3.58-3.67 (m, 2H), 2.95-3.05 (m, 2H), 2.94-2.94 (m, 2H), 2.72-2.75 (m, 1H), 2.48-2.54 (m, 3H), 2.04-2.07 (m, 1H), 1.81-1.91 (m, 4H), 1.69-1.72 (m, 2H), 1.59-1.61 (m, 1H). LCMS: (Method D): Mass found (M+ 465.3), Rt (min): 3.58, Area (%): 96.1 (Max. Chrom.), 97.1 (220 nm). HPLC (Method A), Rt (min): 3.62, Area (%): 97.3 (Max. Chrom.), 97.0 (254 nm). |
| 35 | 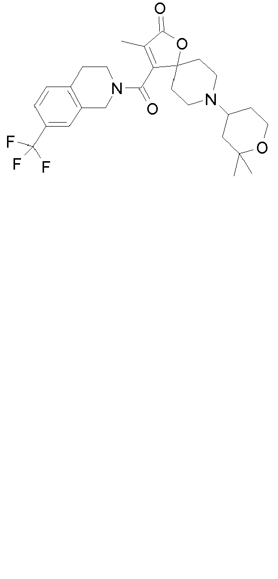 | **8-(2,2-dimethyltetrahydro-2H-pyran-4-yl)-3-methyl-4-{[7-(trifluoromethyl)-3,4-dihydroisoquinolin-2(1H)-yl]carbonyl}-1-oxa-8-azaspiro[4.5]dec-3-en-2-one** |
| | | After purification by flash chromatography, the compound was obtained as a white solid (16 mg). ¹H NMR (DMSO-d₆, 400 MHz): δ 7.54-7.63 (m, 1 H), 7.53 (d, *J* = 8.0 Hz, 1H), 7.41 (d, *J* = 8.1 Hz, 1H), 4.82-4.87 (m, 1H), 4.71 (s, 1 H), 3.84-3.86 (m, 1H), 3.66-3.70 (m, 1 H), 3.61-3.64 (m, 1 H), 3.48-3.52 (m, 1H), 2.95 (t, *J* = 6.0 Hz, 1H), 2.87-2.88 (m, 1H), 2.77-2.79 (m, 1H), 2.59-2.67 (m, 2H), 2.31-2.36 (m, 2H), 2.15-2.16 (m, 1H), 1.74-1.75 (m, 3H), 1.43-1.62 (m, 5H), 1.21-1.27 (m, 2H), 1.08-1.11 (m, 6H). LCMS (Method D): Mass found (M+ 507.3), Rt (min): 3.86, Area (%): 90.6 (Max. Chrom.), 90.8 (220 nm). HPLC (Method A) Rt (min): 3.91, Area (%): 95.1 (Max. Chrom.), 92.3 (254 nm). |
| 36 | 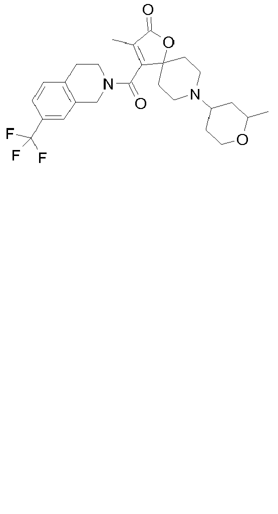 | **3-methyl-8-(2-methyltetrahydro-2H-pyran-4-yl)-4-{[7-(trifluoromethyl)-3,4-dihydroisoquinolin-2(1H)-yl]carbonyl}-1-oxa-8-azaspiro[4.5]dec-3-en-2-one** |
| | | After purification by preparative HPLC, the compound was obtained as a white solid (29 mg). ¹H NMR (CDCl₃, 400 MHz): δ 7.46-7.52 (m, 2H), 7.27-7.34 (m, 1H), 4.89 (d, *J* = 3.1Hz, 1H), 4.57 (d, *J* = 6.8 Hz, 1H), 3.90-4.04 (m, 2H), 3.67 (t, *J* = 11.6 Hz, 1H), 3.38-3.44 (m, 2H), 2.89-3.02 (m, 4H), 2.54-2.62 (m, 3H), 2.35-2.42 (m, 1H), 2.01-2.08 (m, 1H), 1.77-1.90 (m, 4H), 1.69-1.77 (m, 3H), 1.49-1.52 (m, 2H), 1.16 (d, *J* = 2.0 Hz, 3H). LCMS (Method D): Mass found (M+ 493.2), Rt (min): 3.79, Area (%): 97.0 (Max. Chrom.), 96.4 (220 nm). HPLC (Method A): Rt (min): 3.82, Area (%): 95.3 (Max. Chrom.), 95.2 (220 nm). |
| 37 | 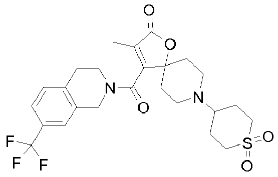 | **8-(1,1-dioxidotetrahydro-2H-thiopyran-4-yl)-3-methyl-4-{[7-(trifluoromethyl)-3,4-dihydroisoquinolin-2(1H)-yl]carbonyl}-1-oxa-8-azaspiro[4.5]dec-3-en-2-one** |
| | | After purification by flash chromatography, the compound was obtained as a white solid (73 mg). ¹H NMR (DMSO-d₆, 400 MHz,) δ 7.64-7.69 (m, 1 H), 7.53 (d, *J* = 7.7 Hz, 1 H), 7.41 (d, *J* = 8.0 Hz, 1 H), 4.70-4.89 (m, 2H), 3.87 (t, *J*= 7.9 Hz, 1 H), 3.69 (t, *J* = 11.6 Hz, 1 H), 3.06 (m, 4H), 2.95 (t, *J* = 5.4 Hz, 1 H), 2.88 (m, 1 H), 2.65-2.76 (m, 3H), 2.37-2.42 (m, 2H), 2.12-2.31 (m, 1 H), 1.96-2.06 (m, 4H), 1.77-1.78 (m, 3H), 1.61-1.62 (m, 2H), 1.48-1.57 (m, 1H). LCMS (Method D): Mass found (M+ 527.3), Rt (min): 3.58, Area (%): 90.0 (Max. Chrom.), 90.6 (220 nm). HPLC (Method A): Rt (min): 3.67, Area (%): 90.6 (Max. Chrom.), 90.3 (220 nm). |
| 38 | 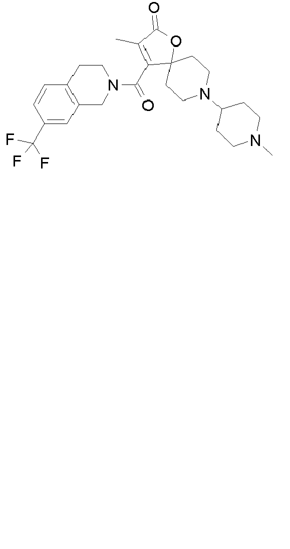 | **3-methyl-8-(1-methylpiperidin-4-yl)-4-{[7-(trifluoromethyl)-3,4-dihydroisoquinolin-2(1H)-yl]carbonyl}-1-oxa-8-azaspiro[4.5]dec-3-en-2-one** |
| | | After purification by flash chromatography, the compound was obtained as an off-white solid (33 mg). ¹H NMR (DMSO-d₆, 400 MHz) δ 7.64-7.69 (m, 1 H), 7.53 (d, *J* = 8.0 Hz, 1H), 7.41 (d, *J* = 8.1 Hz, 1H), 4.82-4.87 (m, 1H), 4.71 (d, *J* = 6.7 Hz, 1 H), 3.85 (t, *J* = 6.1 Hz, 1 H), 3.69 (t, *J* = 5.8 Hz, 1H), 2.95 (t, *J* = 5.8 Hz, 1H), 2.82-2.88 (m, 1H), 2.74-2.77 (m, 4H), 2.32-2.38 (m, 2H), 2.10-2.19 (m, 5H), 1.75-1.82 (m, 5H), 1.61 (s, 4H), 1.32-1.51 (m, 3H). LCMS (Method D): Mass found (M+ 492.3), Rt (min): 3.29, Area (%): 95.5 (Max. Chrom.), 95.8 (220 nm). HPLC (Method A): Rt (min): 3.34, Area (%): 95.8 (Max. Chrom.), 94.9 (220 nm). |
| 39 | 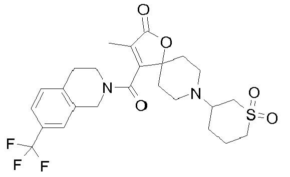 | **8-(1,1-dioxidotetrahydro-2H-thiopyran-3-yl)-3-methyl-4-{[7-(trifluoromethyl)-3,4-dihydroisoquinolin-2(1H)-yl]carbonyl}-1-oxa-8-azaspiro[4.5]dec-3-en-2-one** |
| | | After purification by flash chromatography, the compound was obtained as an off-white solid (30 mg). ¹H NMR (DMSO-d₆, 400 MHz) δ 7.64-7.69 (m, 1 H), 7.53 (d, *J* = 7.5 Hz, 1H), 7.41 (d, *J* = 8.1 Hz, 1 H), 4.70-4.88 (m, 2H), 3.84 (t, *J* = 8.0 Hz, 1 H), 3.69 (t, *J* = 5.8 Hz, 1 H), 3.16-3.20 (m, 1 H), 3.09-3.10 (m, 1 H), 2.88-2.96 (m, 5H), 2.78-2.79 (m, 1H), 2.67-2.68 (m, 1H), 2.61-2.62 (m, 1H), 2.47-2.48 (m, 1H), 2.17-2.17 (m, 1H), 1.99-2.02 (m, 1H), 1.76-1.78 (m, 4H), 1.61-1.64 (m, 3H), 1.39-1.52 (m, 2H). LCMS (Method D): Mass found (M+ 527.0), Rt (min): 3.66, Area (%): 90.9 (Max. Chrom.), 91.6 (220 nm). HPLC (Method A): Rt (min): 3.70, Area (%): 90.3 (Max. Chrom.), 90.8 (220 nm). |
| 40 | 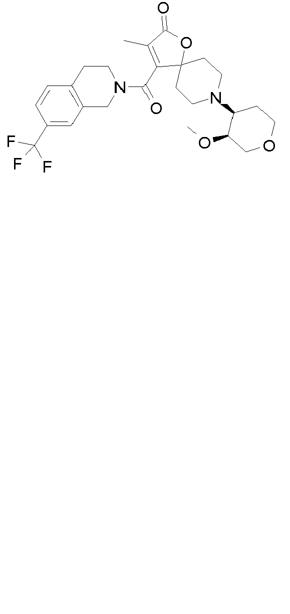 | **(-)-1,5-anhydro-2,3-dideoxy-4-O-methyl-3-(3-methyl-2-oxo-4-{[7-(trifluoromethyl)-3,4-dihydroisoquinolin-2(1H)-yl]carbonyl}-1-oxa-8-azaspiro[4.5]dec-3-en-8-yl) -pentitol (second eluting isomer)** |
| | | After purification by SFC - chiral chromatography (Method K), the compound was obtained as a white solid (49 mg). ¹H NMR (CDCl₃, 400 MHz): δ 7.31-7.34 (m, 2H), 7.27-7.28 (m, 1 H), 4.88 (d, *J* = 8.0 Hz, 1 H), 4.56 (d, *J* = 5.8 Hz, 1 H), 4.18-4.22 (m, 1H), 3.90-4.06 (m, 2H), 3.65-3.67 (m, 1 H), 3.42-3.47 (m, 5H), 3.24-3.25 (m, 1 H), 3.93-4.03 (m, 4H), 2.37-2.55 (m, 4H), 2.02-2.05 (m, 2H), 1.81-1.90 (m, 3H), 1.65-1.74 (m, 3H). LCMS (Method D): Mass found (M+ 509.3), Rt (min): 3.63, Area (%): 95.8 (Max. Chrom.), 96.0 (220 nm). HPLC (Method A): Rt (min): 3.67, Area (%): 95.9 (Max. Chrom.), 96.1 (220 nm). CHIRAL HPLC (Method L): Rt (min): 13.36, Area (%): 98.8. [α]^{D}20= -25.3 ± 0.4 (c=1.07, MeOH) |
| 41 | 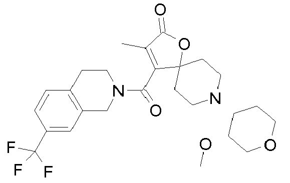 | **(+)-1,5-anhydro-3,4-dideoxy-2-O-methyl-3-(3-methyl-2-oxo-4-{[7-(trifluoromethyl)-3,4-dihydroisoquinolin-2(1H)-yl]carbonyl}-1-oxa-8-azaspiro[4.5]dec-3-en-8-yl)- pentitol (first eluting isomer)** |
| | | After purification by SFC - chiral chromatography (Method K), the compound was obtained as a white solid (10 mg). ¹H NMR (CDCl₃, 400 MHz): δ 7.31-7.34 (m, 2H), 7.27-7.28 (m, 1 H), 4.88 (d, *J* = 8.0 Hz, 1 H), 4.56 (d, *J* = 5.8 Hz, 1 H), 4.19-4.22 (m, 1H), 3.90-4.06 (m, 2H), 3.65-3.67 (m, 1 H), 3.42-3.47 (m, 5H), 3.24-3.25 (m, 1H), 2.93-3.04 (m, 4H), 2.37-2.55 (m, 4H), 2.02-2.05 (m, 2H), 1.86-1.97 (m, 3H), 1.65-1.74 (m, 3H). LCMS (Method D): Mass found (M+ 509.3), Rt (min): 3.62, Area (%): 94.5 (Max. Chrom.), 94.5 (220 nm). HPLC (Method A): Rt (min): 3.67, Area (%) 96.6 (Max. Chrom.), 96.4 (220 nm). CHIRAL HPLC (Method L): Rt (min): 11.60, Area (%): 100. |
| 42 | 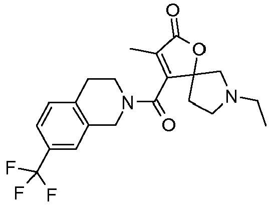 | **7-ethyl-3-methyl-4-{[7-(trifluoromethyl)-3,4-dihydroisoquinolin-2(1H)-yl]carbonyl}-1-oxa-7-azaspiro[4.4]non-3-en-2-one** |
| | | After purification by flash chromatography, the compound was obtained as a brown gummy solid (40 mg). ¹H NMR (CDCl₃, 400 MHz): δ 7.47-7.49 (m, 2H), 7.28-7.32 (m, 1 H), 4.92 (d, J = 5.2 Hz, 1H), 4.58 (s, 1 H), 3.65-3.69 (m, 3H), 2.80-3.04 (m, 5H), 2.02-2.88 (m, 4H), 1.87 (m, 3H), 0.90-1.09 (m, 3H). LCMS (Method D): Mass found (M+ 409.3), Rt (min): 3.64, Area (%): 92.0 (Max. Chrom.), 94.7 (254 nm). HPLC (Method A): Rt (min): 3.70, Area (%): 96.4 (Max. Chrom.), 97.0 (220 nm). |

The following Examples 44-48 were obtained following the procedures described for Examples 25 and 26, but starting from 3-(trifluoromethyl)-5,6,7,8-tetrahydro-1,6-naphthyridine hydrochloride (Intermediate 12). The absolute and relative stereochemistry of Examples 44 and 48 were assigned arbitrarily.

| | | |
|---|---|---|
| 44 | 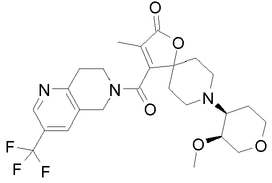 | **(-)-1,5-anhydro-2,3-dideoxy-4-O-methyl-3-(3-methyl-2-oxo-4-{[3-(trifluoromethyl)-7,8-dihydro-1,6-naphthyridin-6(5H)-yl]carbonyl}-1-oxa-8-azaspiro[4.5]dec-3-en-8-yl)-pentitol (second eluting isomer)** |
| | | After purification by SFC - chiral chromatography (Method J), the compound was obtained as a white solid (114 mg). ¹H NMR (CDCl₃, 400 MHz): δ 8.75-8.78 (m, 1H), 7.68 (d, J = 6.4 Hz, 1H), 4.96-4.97 (m, 1H), 4.60-4.61 (m, 1H), 4.40-4.45 (m, 1H), 4.27-4.30 (m, 1H), 4.16-4.19 (m, 1H), 3.79-3.90 (m, 3H), 3.42-3.53 (m, 6H), 3.22-3.29 (m, 3H), 3.02-3.07 (m, 4H), 2.35-2.37 (m, 1H), 1.92-1.94 (m, 3H), 1.77 (s, 1H), 1.64-1.66 (m, 2H). LCMS (Method D): Mass found (M+ 510.3), Rt (min): 2.97, Area (%): 97.1 (Max. Chrom.), 94.6 (254 nm). HPLC (Method A): Rt (min): 2.97, Area (%): 97.7 (Max. Chrom.), 95.0 (254 nm). CHIRAL HPLC (Method L, but using EtOH instead of IPA): Rt (min): 3.45, Area (%): 98.5. [α]^{D}20= -28.7 ± 0.4 (c=1.10, MeOH) |
| 45 | 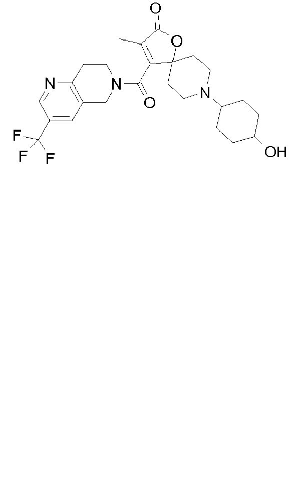 | **8-(4-hydroxycyclohexyl)-3-methyl-4-{[3-(trifluoromethyl)-7,8-dihydro-1,6-naphthyridin-6(5H)-yl]carbonyl}-1-oxa-8-azaspiro[4.5]dec-3-en-2-one** |
| | | After purification by flash chromatography, the compound was obtained as a pale pink solid (33 mg). ¹H NMR (CDCl₃, 400 MHz): δ 8.76 (d, *J* = 10.0 Hz, 1H), 7.62-7.67 (m, 1H), 4.61-4.95 (m, 2H), 3.98-4.11 (m, 1H), 3.57-3.80 (m, 2H), 3.14-3.20 (m, 2H), 2.85-2.90 (m, 2H), 2.55-2.63 (m, 2H), 2.30-2.42 (m, 2H), 1.90-2.04 (m, 2H), 1.92-1.53 (m, 10H), 1.21-1.35 (m, 3H). LCMS (Method D): Mass found (M+ 494.3), Rt (min): 2.78, Area (%): 98.6 (Max. Chrom.), 98.7 (254 nm). HPLC (Method A): Rt (min): 2.76, Area (%): 97.4 (Max. Chrom.), 98.0 (254 nm). |
| 46 | 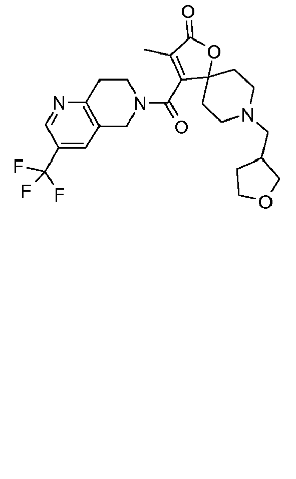 | **3-methyl-8-(tetrahydrofuran-3-ylmethyl)-4-{[3-(trifluoromethyl)-7,8-dihydro-1,6-naphthyridin-6(5H)-yl]carbonyl}-1-oxa-8-azaspiro[4.5]dec-3-en-2-one** |
| | | After purification by flash chromatography, the compound was obtained as a white solid (54 mg). ¹H NMR (CDCl₃, 400 MHz): δ 8.75-8.78 (m, 1H), 7.63-7.77 (m, 1H), 4.55-5.01 (m, 2H), 4.10-4.12 (m, 1H), 3.70-3.86 (m, 5H), 3.48-3.51 (m, 1H), 3.14-3.21 (m, 2H), 2.84-2.90 (m, 2H), 2.33-2.46 (m, 6H), 1.99-2.02 (m, 1H), 1.91-1.96 (m, 4H), 1.59-1.61 (m, 1 H), 1.56-1.58 (m, 1 H). LCMS (Method D): Mass found (M+ 480.3), Rt (min): 2.78, Area (%): 95.2 (Max. Chrom.), 94.6 (220 nm). HPLC (Method A): Rt (min): 2.82, Area (%): 94.4 (Max. Chrom.), 93.3 (254 nm). |
| 47 | 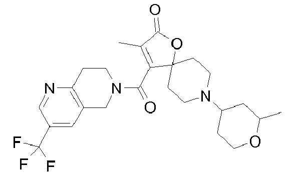 | **3-methyl-8-(2-methyltetrahydro-2H-pyran-4-yl)-4-{[3-(trifluoromethyl)-7,8-dihydro-1,6-naphthyridin-6(5H)-yl]carbonyl}-1-oxa-8-azaspiro[4.5]dec-3-en-2-one** |
| | | After purification by flash chromatography, the compound was obtained as a white solid (13 mg). ¹H NMR (CDCl₃, 400 MHz): δ 8.76 (d, J = 10.1 Hz, 1H), 7.62-7.76 (m, 1 H), 4.91-4.96 (m, 1.5H), 4.60-4.62 (m, 0.5H), 4.03-4.06 (m, 1.5H), 3.78-3.81 (m, 1.5H), 3.39-3.45 (m, 2H), 3.14-3.22 (m, 2H), 2.90-2.92 (m, 2H), 2.39-2.65 (m, 4H), 1.78-2.01 (m, 8H), 1.40-1.50 (m, 2H), 1.72 (d, J = 4.3 Hz, 3H). LCMS (Method D): Mass found (M+ 494.3), Rt (min):2.98, Area (%): 95.8 (Max. Chrom.), 96.3 (220 nm). HPLC (Method A): Rt (min): 2.94, Area (%): 94.6 (Max. Chrom.), 96.8 (254 nm). |
| 48 | 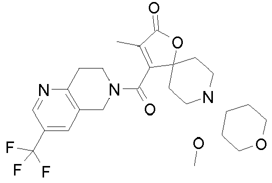 | **(+)-1,5-anhydro-3,4-dideoxy-2-O-methyl-3-(3-methyl-2-oxo-4-{[3-(trifluoromethyl)-7,8-dihydro-1,6-naphthyridin-6(5H)-yl]carbonyl}-1-oxa-8-azaspiro[4.5]dec-3-en-8-yl)-pentitol (first eluting isomer)** |
| | | After purification by SFC - chiral chromatography (Method K), the compound was obtained as a white solid (28 mg). ¹H NMR (CDCl₃, 400 MHz): δ 8.75-8.78 (m, 1H), 8.13 (d, J = 7.6 Hz, 1 H), 5.75-5,95 (m, 2H), 4.60 (s, 1H), 3.80-4.26 (m, 5H), 3.55-3.64 (m, 1H), 3.00-3.33 (m, 10H), 2.66-3.80 (m, 2H), 1.95-2.05 (m, 2H), 1.51-1.79 (m, 5H). LCMS (Method D): Mass found (M+ 510.3), Rt (min): 2.98, Area (%): 93.5 (Max. Chrom.), 91.7 (254 nm). HPLC (Method A): Rt (min): 2.98, Area (%): 93.0 (Max. Chrom.), 92.0 (254 nm). CHIRAL HPLC (Method L, but using EtOH instead of IPA): Rt (min): 2.12, Area (%): 93.0. [α]^{D}20= +30.6 ± 0.4 (c=1.03, MeOH) |

### Example 49: 2-[(8-ethyl-3-methyl-2-oxo-1-oxa-8-azaspiro[4.5]dec-3-en-4-yl)carbonyl]-1,2,3,4-tetrahydroisoquinoline-7-carbonitrile

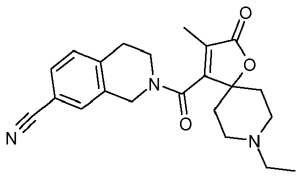

A solution of 8-ethyl-3-methyl-2-oxo-1-oxa-8-aza-spiro[4.5]dec-3-ene-4-carboxylic acid hydrochloride (Intermediate 1, 300 mg, 1.09 mmol) and triethylamine (0.8 mL, 5.78 mmol) was prepared in dichloromethane (15 mL) and cooled at 0°C. 1-Hydroxy-benzotriazole (294 mg, 2.18 mmol) and EDC.HCl (418 mg, 2.18 mmol) were added, followed by 1,2,3,4-tetrahydro-isoquinoline-7-carbonitrile hydrochloride (172 mg, 1.09 mmol). The resulting mixture was stirred at room temperature for 12 hours. The reaction mixture was diluted with a 10% aqueous solution of sodium bicarbonate (15 mL) and extracted with dichloromethane (25 mL). The organic layer was separated, dried (Na₂SO₄) and concentrated under vacuum. After purification by flash chromatography (neutral alumina), the title compound was obtained as a white solid (40 mg). TLC: dichloromethane/methanol (9/1): *R_{f}*: 0.35. ¹H NMR (DMSO-d₆, 400 MHz): δ 7.80 (s, 1 H), 7.68-7.63 (m, 1 H), 7.40-7.38 (m, 1 H), 4.82-4.78 (m, 1 H), 4.66 (s, 1 H), 3.88-3.81 (m, 1 H), 3.67 (t, *J*=5.4 Hz, 1 H), 2.94 (t, *J*=5.9 Hz, 1 H), 2.87 (s, 1 H), 2.82-2.80 (m, 2H), 2.33-2.27 (m, 2H), 2.24 (s, 1 H), 2.19-2.06 (m, 2H), 1.68 (s, 3H), 1.63-1.50 (m, 1 H), 1.47 (m, 2H), 0.96-0.92 (t, *J*=7.1 Hz, 3H). LCMS (Method D): Mass found (M+ 380), Rt (min): 2.68, Area (%): 99.4 (Max. Chrom.), 98.9 (220 nm). HPLC (Method A): Rt (min): 2.72, Area (%): 99.5 (Max. Chrom.), 99.5 (220 nm).

The following Examples 50-60 were obtained following the procedures described for Example 49, but starting from 7-chloro-1,2,3,4-tetrahydro-isoquinoline hydrochloride, 8-trifluoromethyl-1,2,3,4-tetrahydroisoquinoline hydrochloride, 1-(3-fluoro-phenyl)-[1,4]diazepane hydrochloride, 7-fluoro-1,2,3,4-tetrahydro-isoquinoline hydrochloride, 6-trifluoromethyl-1,2,3,4-tetrahydro-isoquinoline hydrochloride, 3-(3-trifluoromethyl-phenyl)-pyrrolidine, amino-N-(3-trifluoromethyl-phenyl)-acetamide hydrochloride, 1-[2-(4-fluorophenyl)-ethyl]-piperazine hydrochloride (Intermediate 8), N-[3-(trifluoromethyl)phenyl]piperidin-4-amine hydrochloride (Intermediate 9), 1-[5-(trifluoromethyl)pyridin-3-yl]piperazine (Intermediate 10), respectively.

| **Ex.** | **Structure** | **Characterization** |
|---|---|---|
| 50 | 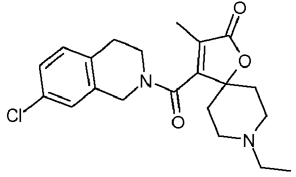 | **4-[(7-chloro-3,4-dihydroisoquinolin-2(1H)-yl)carbonyl]-8-ethyl-3-methyl-1-oxa-8-azaspiro[4.5]dec-3-en-2-one** |
| | | Isolated as a white solid. TLC: dichloromethane/methanol (9/1): *R_{f}*: 0.4. ¹H NMR (DMSO-d₆, 400 MHz): 7.38-7.31 (m, 1H), 7.24-7.19 (m, 2H), 4.77-4.73 (m, 1H), 4.61 (s, 1 H), 3.84-3.79 (m, 1 H), 3.65 (t, *J*=5.7 Hz, 1 H), 2.85-2.77 (m, 4H), 2.33-2.19 (m, 2H), 2.19-2.06 (m, 3H), 1.74 (s, 3H), 1.70 (s, 2H), 1.63-1.47 (m, 1H), 0.95 (t, *J*=7.0 Hz, 3H). LCMS (Method D): Mass found (M+ 389), Rt (min): 3.32, Area (%): 98.4 (Max. Chrom.), 98.4 (220 nm). HPLC (Method A): Rt (min): 3.38, Area (%): 97.9 (Max. Chrom.), 97.7 (220 nm). |
| 51 | 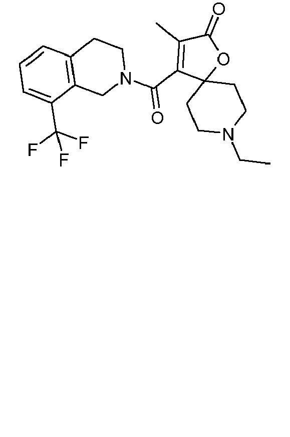 | **8-ethyl-3-methyl-4-{[8-(trifluoromethyl)-3,4-dihydroisoquinolin-2 (1H)-yl]carbonyl}-1-oxa-8-azaspiro[4.5]dec-3-en-2-one** |
| | | Isolated as a bown solid. TLC: dichloromethane/methanol (9/1): *R_{f}*: 0.4. ¹H NMR (DMSO-d₆, 400 MHz) 7.63-7.40 (m, 3H), 5.03-4.66 (m, 2H), 3.98-3.70 (m, 2H), 3.01-2.91 (m, 2H), 2.82-2.79 (m, 2H), 2.33-2.31 (d, *J*=6.6 Hz, 2H), 2.18-2.08 (m, 4H), 1.81 (s, 3H), 1.56 (s, 2H), 0.94 (t, *J*=7.1 Hz, 3H). LCMS (Method D): Mass found (M+ 423), Rt (min): 3.46, Area (%): 96.2 (Max. chrom.), 96.0 (220 nm). HPLC (Method A): Rt (min): 3.48, Area (%): 95.9 (Max. Chrom.), 94.1 (254 nm). |
| 52 | 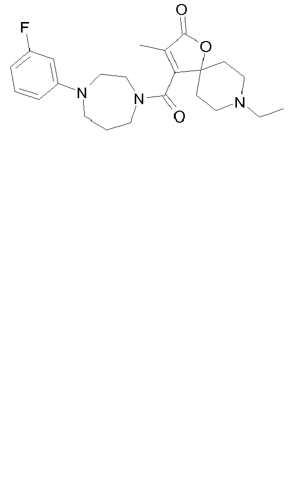 | **8-ethyl-4-{[4-(3-fluorophenyl)-1,4-diazepan-1-yl]carbonyl}-3-methyl-1-oxa-8-azaspiro[4.5]dec-3-en-2-one** |
| | | Isolated as a bown solid. TLC: dichloromethane/methanol (9/1): *R_{f}*: 0.35. ¹H NMR (DMSO-d₆, 400 MHz): δ 7.14-7.08 (m, 1H), 6.54-6.51 (m, 2H), 6.35-6.31 (m, 1H), 3.77 (m, 2H), 3.66 (m, 4H), 3.59-3.57 (m, 3H), 2.71 (m, 1 H), 2.28 (d, *J*=7.4 Hz, 2H), 2.11-2.10 (m, 3H), 1.78 (s, 2H), 1.60 (s, 3H), 1.35-1.25 (m, 3H), 0.94 (t, *J*=7.0 Hz, 3H). LCMS (Method D): Mass found (M+ 416.3), Rt (min): 3.15, Area (%): 98.2 (Max. chrom.) 98.6 (254 nm). HPLC (Method A): Rt (min): 3.22, Area (%): 99.1 (Max. Chrom.), 99.1 (254 nm). |
| 53 | 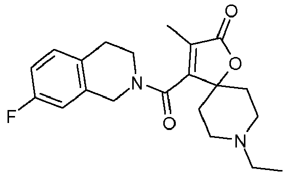 | **8-ethyl-4-[(7-fluoro-3,4-dihydroisoquinolin-2(1H)-yl)carbonyl]-3-methyl-1-oxa-8-azaspiro[4.5]dec-3-en-2-one** |
| | | Isolated as an off-white solid. TLC: dichloromethane/ methanol (9/1): *R_{f}*: 0.35. ¹H NMR (DMSO-d₆, 400 MHz): δ 7.22-7.00 (m, 3H), 4.77-4.73 (m, 1H), 4.60-4.54 (m, 1H), 3.85-3.78 (m, 1H), 3.66-3.61 (m, 1H), 284-2.77 (m, 4H), 2.33-2.28 (m, 2H), 2.14-2.06 (m, 3H), 1.74 (s, 3H), 1.63 (s, 2H), 1.50 (m, 1 H), 0.94 (t, *J*=7.4 Hz, 3H). LCMS (Method D): Mass found (M+ 373), Rt (min): 2.99, Area (%): 95.8 (Max. chrom.) 95.3 (220 nm). HPLC (Method A): Rt (min): 3.03, Area (%): 95.1 (Max. Chrom.), 96.6 (254 nm). |
| 54 | 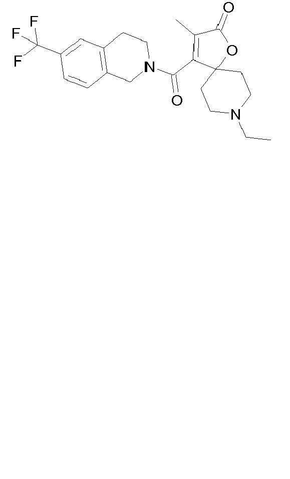 | **8-ethyl-3-methyl-4-{[6-(trifluoromethyl)-3,4-dihydroisoquinolin-2 (1H)-yl]carbonyl}-1-oxa-8-azaspiro[4.5]dec-3-en-2-one** |
| | | Isolated as an off-white solid. TLC: dichloromethane/ methanol (9/1): *R_{f}*: 0.45. ¹H NMR (DMSO-d₆, 400 MHz): δ 7.57-7.41 (m, 3H), 4.86-4.82 (m, 1H), 4.70 (s, 1H), 3.86 (d, *J*=2.7 Hz, 1 H), 3.69 (t, *J*=5.6 Hz, 1 H), 2.97-2.89 (m, 2H), 2.82-2.80 (m, 2H), 2.35-2.27 (m, 2H), 2.19-2.06 (m, 3H), 1.63 (s, 3H), 1.63-1.47 (m, 3H), 0.94 (t, *J*=7.6 Hz, 3H). LCMS (Method D): Mass found (M+ 423), Rt (min): 3.61, Area (%): 96.0 (Max. chrom.) 96.8 (220 nm). HPLC (Method A): Rt (min): 3.67, Area (%): 97.3 (Max. Chrom.), 97.2 (220 nm). |
| 55 | 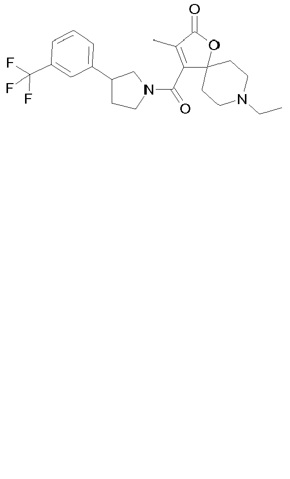 | **8-ethyl-3-methyl-4-({3-[3-(trifluoromethyl)phenyl]pyrrolidin-1-yl}carbonyl)-1-oxa-8-azaspiro[4.5]dec-3-en-2-one** |
| | | Isolated as an off-white solid. TLC: dichloromethane/ methanol (9/1): *R_{f}*: 0.4. ¹H NMR (DMSO-d₆, 400 MHz): δ 7.69-7.55 (m, 4H), 3.53 (d, *J*=5.1 Hz, 1 H), 3.50-3.46 (m, 1 H), 3.45 (d, *J*=3.8 Hz, 2H), 3.41-3.29 (m, 1 H), 2.82 (d, *J*=5.3 Hz, 2H), 2.53 (s, 1H), 2.37-2.33 (m, 3H), 2.31-2.06 (s, 4H), 1.79 (s, 3H), 1.47-1.44 (m, 2H), 0.94 (t, *J*=7.0 Hz, 3H). LCMS (Method D): Mass found (M+ 437.3), Rt (min): 3.69, Area (%): 98.0 (Max. chrom.) 97.7 (220 nm). HPLC (Method A): Rt (min): 3.75, Area (%): 97.8 (Max. Chrom.), 94.4 (254 nm). |
| 56 | 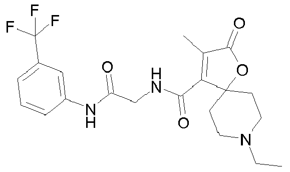 | **8-ethyl-3-methyl-2-oxo-N-(2-oxo-2-{[3-(trifluoromethyl)phenyl]amino}ethyl)-1-oxa-8-azaspiro[4.5]dec-3-ene-4-carboxamide** |
| | | Isolated as an off-white solid. TLC: dichloromethane methanol (9/1): *R_{f}*: 0.4. ¹H NMR (DMSO-d₆, 400 MHz) δ 10.48 (s, 1H), 8.86 (t, *J*=5.8 Hz, 1 H), 8.04 (s, 1 H), 7.77 (d, *J*=8.8 Hz, 1H), 7.58-7.54 (m, 1 H), 7.41 (d, *J*=7.6 Hz, 1 H), 4.05 (d, *J*=6.0 Hz, 2H), 2.83-2.81 (m, 2H), 2.33 (t, *J*=7.2 Hz, 2H), 2.19-2.10 (m, 4H), 1.91 (s, 3H), 1.47-1.44 (m, 2H), 0.99 (t, *J*=7.0 Hz, 3H). LCMS (Method D): Mass found (M+ 440), Rt (min): 3.31, Area (%): 97.7 (Max. chrom.), 97.1 (254 nm). HPLC (Method A): Rt (min): 3.36, Area (%): 98.3 (Max. Chrom.), 97.6 (254 nm). |
| 58 | 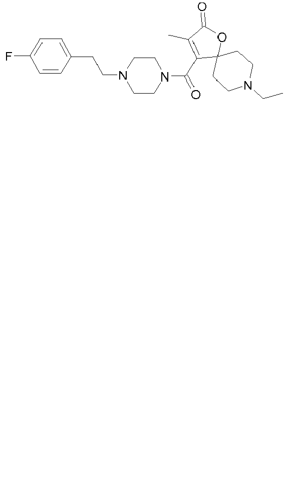 | **8-ethyl-4-({4-[2-(4-fluorophenyl)ethyl]piperazin-1-yl}carbonyl)-3-methyl-1-oxa-8-azaspiro[4.5]dec-3-en-2-one** |
| | | Isolated as a white solid. TLC: dichloromethane/methanol (9.5/0.5): *R_{f}*: 0.4. ¹H NMR (DMSO-d₆, 400 MHz): δ 7.25-7.23 (m, 2H), 7.09-7.07 (m, 2H), 3.65 (m, 1 H), 3.50 (m, 1 H), 3.39-3.37 (m, 2H), 2.80 (m, 2H), 2.73-2.69 (m, 2H), 2.54-2.52 (m, 3H), 2.48-2.33 (m, 5H), 2.19-2.11 (m, 3H), 1.87 (m, 1H), 1.78 (s, 3H), 1.71 (m, 1H), 1.51 (m, 1H), 0.99-0.95 (m, 3H). LCMS (Method D): Mass found (M+ 430.3), Rt (min): 2.31, Area (%): 97.1 (Max. Chrom.), 97.5 (220 nm). HPLC (Method A): Rt (min): 2.36, Area (%): 98.7 (Max. Chrom.), 98.6 (220 nm). |
| 59 | 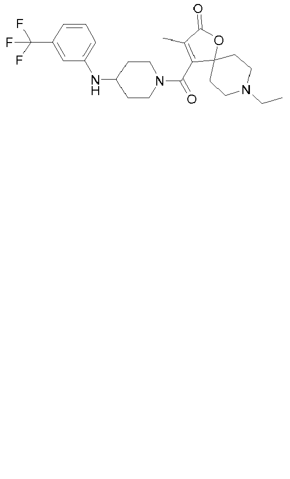 | **8-ethyl-3-methyl-4-[(4-{[3-(trifluoromethyl)phenyl]amino}piperidin-1-yl)carbonyl]-1-oxa-8-azaspiro[4.5]dec-3-en-2-one** |
| | | Isolated as a white solid. TLC: dichloromethane/methanol (9.5/0.5): *R_{f}*: 0.7. ¹H NMR (CDCl3 400 MHz): δ 7.27 (s, 1 H), 6.97 (d, J=7.5 Hz, 1 H), 6.78-6.73 (m, 2H), 4.55-4.70 (m, 1 H), 3.71-3.61 (m, 3H), 3.3 (m, 1 H), 3.02-2.95 (m, 3H), 2.50-2.37 (m, 5H), 2.18-2.03 (m, 3H), 1.88-1.86 (m, 3H), 1.71 (m, 1H), 1.63 (m, 3H), 1.12 (t, J=6.9 Hz, 3H). LCMS (Method D): Mass found (M+ 466.0), Rt (min): 3.83, Area (%): 98.8 (Max. Chrom.), 98.5 (254 nm). HPLC (Method A): Rt (min): 3.90, Area (%): 98.8 (Max. Chrom.), 97.7 (254 nm). |
| 60 | 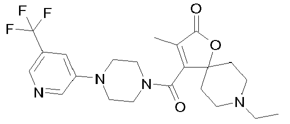 | **8-ethyl-3-methyl-4-({4-[5-(trifluoromethyl)pyridin-3-yl]piperazin-1-yl}carbonyl)-1-oxa-8-azaspiro[4.5]dec-3-en-2-one** |
| | | Isolated as an off-white solid. ¹H NMR (CDCl₃, 400 MHz): δ 8.49 (s, 1H), 8.43 (s, 1 H), 7.35 (s, 1H), 3.97-3.87 (m, 2H), 3.59 (m, 2H), 3.36-3.23 (m, 4H), 2.96-2.94 (m, 2H), 2.53-2.44 (m, 4H), 1.90 (s, 3H), 1.74-1.63 (m, 4H), 1.13 (t, *J* = 7.2 Hz, 3H). LCMS (Method D): Mass found (M+453.0), Rt (min): 2.51, Area (%): 99.7 (Max. Chrom.), 99.6 (254 nm). HPLC (Method A): Rt (min): 2.54, Area (%): 98.6 (Max. Chrom.), 98.7 (254 nm). |

### Example 62: 8-isopropyl-3-methyl-4-{[7-(trifluoromethyl)-3,4-dihydroisoquinolin-2 (1H)-yl]carbonyl}-1-oxa-8-azaspiro[4.5]dec-3-en-2-one

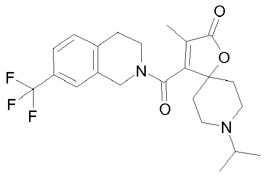

A solution of 8-isopropyl-3-methyl-2-oxo-1-oxa-8-azaspiro[4.5]dec-3-ene-4-carboxylic acid hydrochloride (Intermediate 3, 200 mg, 0.69 mmol) and triethylamine (0.5 mL, 3.61 mmol) was prepared in dichloromethane (15 mL) and cooled at 0°C. 1-Hydroxy benzotriazole (186 mg, 1.38 mmol) and EDC.HCl (265 mg, 1.38 mmol) were added, followed by 7-trifluoromethyl-1,2,3,4-tetrahydro-isoquinoline hydrochloride (164 mg, 0.69 mmol). The resulting mixture was stirred at room temperature for 12 hours. The reaction mixture was diluted with a 10% aqueous solution of sodium bicarbonate (15 mL) and extracted with dichloromethane (25 mL). The organic layer was separated, dried (Na₂SO₄) and concentrated under vacuum. After purification by flash chromatography, the title compound was obtained as a white solid (69 mg, 23%). TLC: dichloromethane/ methanol (9/1): R_{f}: 0.45. ¹H NMR (CDCl₃, 400 MHz): δ 7.44-7.42 (m, 2H), 7.27 (d, J=7.2 Hz, 1 H), 4.85 (s, 1 H), 4.56-4.55 (m, 1 H), 3.92-3.91 (m, 1 H), 3.64 (s, 1 H), 2.83-2.82 (m, 5H), 2.61 (s, 2H), 2.43 (s, 1 H), 2.17-2.16 (m, 1 H), 2.05 (s, 2H), 1.86 (s, 3H), 1.04 (s, 6H). LCMS (Method D): Mass found (M+ 437), Rt (min): 3.71, Area (%): 96.4 (Max. chrom.), 96.4 (220 nm). HPLC (Method A): Rt (min): 3.63, Area (%): 97.7 (Max. Chrom.), 97.6 (220 nm).

### Example 63: 8-[2-(2-methoxyphenyl)ethyl]-3-methyl-4-({4-[3-(trifluoromethyl)phenyl]piperazin-1-yl}carbonyl)-1-oxa-8-azaspiro[4.5]dec-3-en-2-one

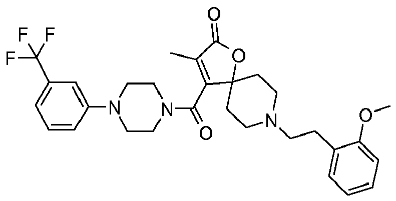

A solution of 8-[2-(2-methoxyphenyl)ethyl]-3-methyl-2-oxo-1-oxa-8-azaspiro[4.5]dec-3-ene-4-carboxylic acid hydrochloride (Intermediate 7, 300 mg, 0.79 mmol) and triethylamine (0.8 mL, 5.78 mmol) was prepared in dichloromethane (15 mL) and cooled at 0°C. 1-Hydroxy-benzotriazole (235 mg, 1.74 mmol) and EDC.HCl (333 mg, 1.74 mmol) were added, followed by 1-(3-trifluoromethylphenyl)piperazine hydrochloride (0.463 g, 1.74 mmol). The resulting mixture was stirred at room temperature for 12 hours. The reaction mixture was diluted with a 10% aqueous solution of sodium bicarbonate (15 mL) and extracted with dichloromethane (25 mL). The organic layer was separated, dried (Na₂SO₄) and concentrated under vacuum. After purification by flash chromatography, the title compound was obtained as a white solid. TLC: dichloromethane/methanol (9/1): R_{f}: 0.35. ¹H NMR (DMSO-d₆, 400 MHz): δ 7.43-7.39 (m, 1 H), 7.27-7.08 (m, 5H), 6.90-6.84 (m, 2H), 3.94 (m, 1 H), 3.82 (m, 4H), 3.57 (m, 2H), 3.25 (m, 4H), 3.19 (m, 2H), 2.83 (m, 2H), 2.63 (m, 2H), 2.51 (m, 3H), 2.01 (s, 1 H), 1.97 (s, 3H), 1.74 (m, 1 H), 1.27 (d, J=8.6 Hz, 1 H). LCMS (Method D): Mass found (M+ 558.3), Rt (min): 4.64, Area (%): 97.2 (Max. Chrom.), 97.4 (254 nm). HPLC (Method A): Rt (min): 4.67, Area (%): 97.5 (Max. Chrom.), 97.3 (254 nm).

### Example 64: 8-isopropyl-3-methyl-2-oxo-N-[4-(trifluoromethyl)benzyl]-1-oxa-8-azaspiro[4.5]dec-3-ene-4-carboxamide

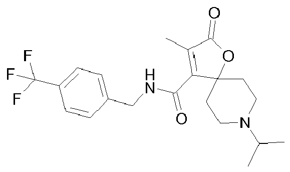

A solution of 8-isopropyl-3-methyl-2-oxo-1-oxa-8-azaspiro[4.5]dec-3-ene-4-carboxylic acid hydrochloride (Intermediate 3, 200 mg, 0.69 mmol) and triethylamine (0.5 mL, 3.61 mmol) was prepared in dichloromethane (15 mL) and cooled at 0°C. 1-Hydroxy benzotriazole (186 mg, 1.38 mmol) and EDC.HCl (265 mg, 1.38 mmol) were added, followed by 4-trifluoromethyl benzyl amine (121 mg, 0.69 mmol). The resulting mixture was stirred at room temperature for 12 hours. The reaction mixture was diluted with a 10% aqueous solution of sodium bicarbonate (15 mL) and extracted with dichloromethane (25 mL). The organic layer was separated, dried (Na₂SO₄) and concentrated under vacuum. After purification by flash chromatography (neutral alumina), the title compound was obtained as a white solid. TLC: dichloromethane/methanol (9/1): *R_{f}* = 0.45. ¹H NMR (DMSO-d₆, 400 MHz): δ 9.11 (s, 1 H), 7.73 (d, *J*=8.1 Hz, 2H), 7.51 (d, *J*=8.1 Hz, 2H), 4.50 (d, *J*=5.8 Hz, 2H), 2.70 (s, 3H), 2.38-2.32 (m, 2H), 2.11 (d, *J*=9.0 Hz, 2H), 1.83 (s, 3H), 1.47-1.44 (m, 2H), 0.95 (d, *J*=5.8 Hz, 6H). LCMS (Method D): Mass found (M+ 411), Rt (min): 3.49, Area (%): 97.2 (Max. chrom.), 96.0 (254 nm). HPLC (Method A): Rt (min): 3.63, Area (%): 97.7 (Max. Chrom.), 97.6 (220 nm).

### Example 65: 8-[2-(2-methoxyphenyl)ethyl]-3-methyl-4-{[7-(trifluoromethyl)-3,4-dihydroisoquinolin-2(1H)-yl]carbonyl}-1-oxa-8-azaspiro[4.5]dec-3-en-2-one

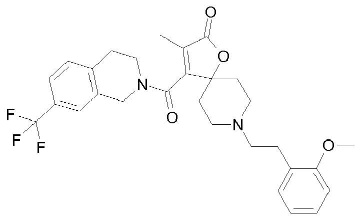

A solution of 8-[2-(2-methoxyphenyl)ethyl]-3-methyl-2-oxo-1-oxa-8-azaspiro[4.5]dec-3-ene-4-carboxylic acid hydrochloride (Intermediate 7, 200 mg, 0.53 mmol) and triethylamine (0.36 mL, 2.60 mmol) was prepared in dichloromethane (10 mL) and cooled at 0°C. 1-Hydroxy-benzotriazole (197 mg, 1.46 mmol) and EDC.HCl (278 mg, 1.46 mmol) were added, followed by 7-trifluoromethyl-1,2,3,4-tetrahydro-isoquinoline (158 mg, 0.67 mmol). The resulting mixture was stirred at room temperature for 12 hours. The reaction mixture was diluted with a 10% aqueous solution of sodium bicarbonate (15 mL) and extracted with dichloromethane (25 mL). The organic layer was separated, dried (Na₂SO₄) and concentrated under vacuum. After purification by flash chromatography (neutral alumina), the title compound was obtained as a white solid. TLC: dichloromethane/methanol (9/1): *R_{f}* : 0.4. ¹H NMR (DMSO-d₆, 400 MHz): δ 7.69-7.64 (m, 1 H), 7.54 (d, *J*=7.8 Hz, 1 H), 7.41 (d, *J*=8.0 Hz, 1 H), 7.16-7.09 (m, 2H), 6.87 (d, *J*=8.2 Hz, 1 H), 6.82-6.80 (m, 1 H), 4.87-4.82 (m, 1 H), 4.72 (s, 1 H), 3.88-3.83 (m, 1 H), 3.77-3.68 (m, 4H), 2.95 (t, *J*=5.7 Hz, 1 H), 2.89 (d, *J*=4.1 Hz, 2H), 2.73-2.70 (m, 3H), 2.49-2.44 (m, 2H), 2.21-2.19 (m, 3H), 1.76 (s, 3H), 1.63 (s, 2H), 1.51-1.50 (m, 1 H). LCMS (Method D): Mass found (M+ 529), Rt (min): 4.46, Area (%): 92.4 (Max. chrom.), 92.3 (220 nm). HPLC (Method A): Rt (min): 4.42, Area (%): 93.4 (Max. Chrom.), 93.6 (254 nm).

### Example 66: 8-isopropyl-3-methyl-4-{[6-(trifluoromethyl)-2H-1,3-benzoxazin-3(4H)-yl]carbonyl}-1-oxa-8-azaspiro[4.5]dec-3-en-2-one

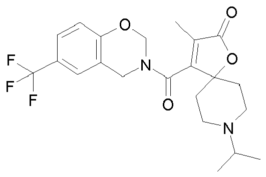

### Step 1: Preparation of 2-tert-butoxy-5-trifluoromethyl-benzonitrile

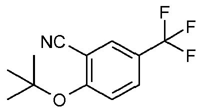

A suspension of potassium tert-butoxide (2.9 g, 26.4 mmol) in tetrahydrofuran (15 mL) was added dropwise into a solution of 2-fluoro-5-trifluoromethylbenzonitrile (2.5 g, 13.2 mmol) in tetrahydrofuran (75 mL) cooled at 0°C. The resulting mixture was stirred at room temperature for 4 hours. The reaction mixture was concentrated under vacuum. The residue was taken up with diethylether (50 mL) and washed with a 1 N aqueous solution of HCI and a saturated aqueous solution of sodium bicarbonate. The organic layer was dried (MgSO₄) and concentrated under vacuum. After purification by flash chromatography (silica), the title compound was obtained as a colorless liquid (2.6 g, 81%). TLC: ethyl acetate/hexane (5/5) R_{f} : 0.4. ¹H NMR (DMSO-d₆, 400 MHz): δ 8.22 (d, *J*=2.0 Hz, 1 H), 7.92 (dd, *J*=6.6, 2.8 Hz, 1 H), 7.55 (d, *J*=8.8 Hz, 1 H), 1.48 (s, 9H).

### Step 2: Preparation of 2-tert-butoxy-5-trifluoromethyl-benzylamine

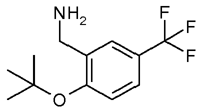

A mixture of 2-tert-butoxy-5-trifluoromethyl-benzonitrile (2.6 g, 10.6 mmol), aqueous ammonium hydroxide solution (28-30%, ~15 mL) and Raney nickel (slurry in water,∼2 g) in ethanol (50 mL) was stirred under hydrogen atmosphere (50 PSI) for 24 hours. The reaction mixture was filtered through a Celite pad, which was rinsed with ethanol (100 mL) and water (50 mL). The filtrate was concentrated under vacuum. After purification by flash chromatography (silica), the title compound was obtained as a colorless liquid (1.2 g, 46%). ¹H NMR (DMSO-d₆, 400 MHz): δ 7.73 (s, 1 H), 7.45 (dd, *J*=6.5, 2.1 Hz, 1 H), 7.21 (d, *J*=8.5 Hz, 1 H), 3.68 (s, 2H), 1.88 (s, 2H), 1.40 (s, 9H).

### Step 3: Preparation of 8-isopropyl-3-methyl-2-oxo-1-oxa-8-aza-spiro[4.5]dec-3-ene-4-carboxylic acid 2-tert-butoxy-5-trifluoromethyl-benzylamide

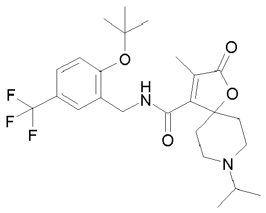

A solution of 8-isopropyl-3-methyl-2-oxo-1-oxa-8-azaspiro[4.5]dec-3-ene-4-carboxylic acid hydrochloride (Intermediate 3, 400 mg, 1.38 mmol) and triethylamine (0.96 mL, 6.94 mmol) was prepared in dichloromethane (15 mL) and cooled at 0°C. 1-Hydroxy benzotriazole (375 mg, 2.78 mmol) and EDC.HCl (530 mg, 2.78 mmol) were added, followed by 2-tert-butoxy-5-trifluoromethyl-benzylamine (342 mg, 1.38 mmol). The resulting mixture was stirred at room temperature for 12 hours. The reaction mixture was diluted with a 10% aqueous solution of sodium bicarbonate (15 mL) and extracted with dichloromethane (25 mL). The organic layer was separated, dried (Na₂SO₄) and concentrated under vacuum. After purification by flash chromatography, the title compound was obtained as an off-white solid (170 mg, 26%). ¹H NMR (DMSO-d₆, 400 MHz): δ 8.94 (t, *J*=5.6 Hz, 1 H), 7.55-7.51 (m, 2H), 7.32 (d, *J*=8.4 Hz, 1 H), 4.42 (d, *J*=5.8 Hz, 2H), 2.71-2.70 (m, 3H), 2.38-2.32 (m, 3H), 2.14-2.11 (m, 2H), 1.83 (s, 3H), 1.45 (s, 10H), 1.39 (d, *J*=4.0 Hz, 6H). LCMS (Method D): Mass found (M+ 483.3), Rt (min): 4.32, Area (%): 97.1 (Max. Chrom.). HPLC (Method A): Rt (min): 4.27, Area (%): 92.3 (Max. Chrom.), 92.3 (220 nm).

### Step 4: Preparation of 8-isopropyl-3-methyl-2-oxo-1-oxa-8-aza-spiro[4.5]dec-3-ene-4-carboxylic acid 2-hydroxy-5-trifluoromethyl-benzylamide

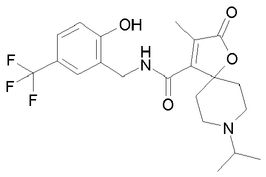

A 4N solution of HCI in 1,4-dioxane (15 mL) was added into a solution of 8-isopropyl-3-methyl-2-oxo-1-oxa-8-aza-spiro[4.5]dec-3-ene-4-carboxylic acid 2-tert-butoxy-5-trifluoromethyl-benzylamide (170 mg, 0.35 mmol) in 1,4-dioxane (10 mL). The resulting mixture was stirred at room temperature for 3 hours. The reaction mixture was concentrated under vacuum. The residue was triturated with diethyl ether (10 mL), filtered under N₂ atm and dried under vacuum to give the title compound as an off-white solid (100 mg, 67%). ¹H NMR (DMSO-d₆, 400 MHz): δ 10.68 (s, 1 H), 9.63 (s, 1 H), 7.47-7.46 (m, 2H), 7.03 (d, *J*=9.0 Hz, 1 H), 4.41 (d, *J*=5.6 Hz, 2H), 3.37-3.33 (m, 3H), 3.10-3.07 (m, 2H), 2.59 (d, *J*=3.8 Hz, 2H), 1.90 (s, 3H), 1.85 (t, *J*=8.6 Hz, 2H), 1.39 (d, *J*=4.0 Hz, 6H). LCMS (Method D): Mass found (M+ 427.2), Rt (min): 3.27, Area (%): 92.4 (Max. Chrom.), 93.0 (220 nm).

### Step 5: Preparation of 8-isopropyl-3-methyl-4-{[6-(trifluoromethyl)-2H-1,3-benzoxazin-3 (4H)-yl]carbonyl}-1-oxa-8-azaspiro[4.5]dec-3-en-2-one

A solution of 8-isopropyl-3-methyl-2-oxo-1-oxa-8-aza-spiro[4.5]dec-3-ene-4-carboxylic acid 2-hydroxy-5-trifluoromethyl-benzylamide (100 mg, 0.23 mmol), para-formaldehyde (75 mg, 2.5 mmol) and p-toluenesulphonic acid (22 mg, 0.13 mmol) in anhydrous toluene (10 mL) was heated at reflux for 4 hours in a flask equipped with a Dean-Stark apparatus. The reaction mixture was concentrated under vacuum. The residue was taken up with dichloromethane (20 mL) and washed with water (20 mL) and brine (20 mL). The organic layer was dried (MgSO₄) and concentrated under vacuum. After purification by flash chromatography (silica), the title compound was obtained as a white solid. ¹H NMR (CDCl₃, 400 MHz): δ 7.47-7.42 (m, 2H), 6.98 (d, *J*=8.6 Hz, 1H), 5.72-5.55 (m, 1 H), 5.24 (s, 2H), 4.96 (s, 2H), 4.80-4.59 (m, 1 H), 2.90-2.89 (m, 3H), 2.68-2.67 (m, 2H), 1.92 (s, 3H), 1.69-1.68 (m, 2H), 1.10 (d, *J*=5.9 Hz, 6H). LCMS (Method D): Mass found (M+ 439.3), Rt (min): 3.27, Area (%): 94.7 (Max. Chrom.), 94.5 (220 nm). HPLC (Method A): Rt (min): 3.75, Area (%): 96.3 (Max. Chrom.), 98.9 (220 nm).

### Example 67: 8-ethyl-3-methyl-2-oxo-N-{1-[4-(trifluoromethyl)phenyl]-1H-pyrazol-4-yl}-1-oxa-8-azaspiro[4.5]dec-3-ene-4-carboxamide

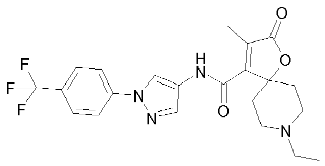

### Step 1: Preparation of 4-Nitro-1-(4-trifluoromethyl-phenyl)-1H-pyrazole

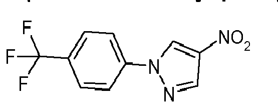

A mixture of 4-bromo-benzotrifluoride (2.00 g, 8.88 mmol), 4-nitro-1H-pyrazole (2.01 g, 17.8 mmol), potassium carbonate (3.68 g, 26.6 mmol) and cuprous iodide (338 mg, 1.78 mmol) in DMF (50 mL) was refluxed overnight. The reaction mixture was filtered through the Celite pad and the filtrate was concentrated under high vacuum. The residue was taken up with dichloromethane and washed with brine. The organic layer was dried (Na₂SO₄) and concentrated under vacuum. After purification by flash chromatography (silica), the title compound was obtained as a dark yellow solid. TLC: chloroform/methanol (9/1): *R_{f}* : 0.7. ¹H NMR (DMSO-d₆, 400 MHz): δ 9.80 (s, 1 H), 8.62 (s, 1 H), 8.20 (d, J= 8.7 Hz, 2H), 7.96 (d, J= 8.7 Hz, 2H).

### Step 2: Preparation of 1-(4-trifluoromethyl-phenyl)-1H-pyrazol-4-ylamine

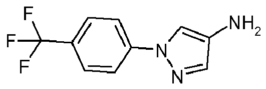

A mixture of 4-nitro-1-(4-trifluoromethyl-phenyl)-1H-pyrazole (550 mg, 2.14 mmol) and Raney Nickel (1 g) in ethyl acetate and methanol (60 mL, 1:1) was stirred at room temperature for 4 hours under an hydrogen atmosphere (3 Kg/atm). The reaction mixture was filtered through the Celite pad. The filtrate was concentrated under vacuum. After purification by trituration in hexane and diethyl ether, the title compound was obtained as an off-white solid. TLC: chloroform/methanol (9/1): R_{f} : 0.4. LCMS (Method D): Mass found (M+ 228.0), Rt (min): 2.86, Area (%): 95.7 (Max. Chrom.). ¹H NMR (DMSO-d₆, 400 MHz): δ 7.87 (d, J= 8.6 Hz, 2H), 7.77 (s, 1 H), 7.75 (d, J= 8.6 Hz, 2H), 7.33 (s, 1 H), 4.31 (s, 2H).

### Step 3: Preparation of 8-ethyl-3-methyl-2-oxo-N-{1-[4-(trifluoromethyl)phenyl]-1H-pyrazol-4-yl}-1-oxa-8-azaspiro[4.5]dec-3-ene-4-carboxamide

1-(4-Trifluoromethyl-phenyl)-1H-pyrazol-4-ylamine (290 mg, 1.28 mmol), EDC.HCI (490 mg, 2.55 mmol) and 1-hydroxy benzotriazole (391 mg, 2.56 mmol) were added into a solution of 8-ethyl-3-methyl-2-oxo-1-oxa-8-aza-spiro[4.5]dec-3-ene-4-carboxylic acid hydrochloride (Intermediate 1, 350 mg, 1.28 mmol) and triethyl amine (0.88 mL, 6.38 mmol) in anhydrous DMF (20 mL) cooled at 0°C. The resulting mixture was stirred at room temperature for 48 hours. The reaction mixture was concentrated under high vacuum. After purification by flash chromatography (neutral alumina), the title compound was obtained as a pale yellow solid. TLC: dichloromethane/methanol (9/1): *R_{f}* : 0.3. ¹H NMR (DMSO-d₆, 400 MHz): δ 10.83 (s, 1 H), 8.80 (s, 1 H), 8.07 (d, J= 8.6 Hz, 2H), 7.91 (s, 1 H), 7.85 (d, J=8.6 Hz, 2H), 2.84 (d, *J*=9.5 Hz, 2H), 2.37-2.32 (m, 2H), 2.24-2.10 (m, 4H), 1.90 (s, 3H), 1.54-1.51 (m, 2H), 1.00 (m, 3H). LCMS (Method D): Mass found (M+ 449.0), Rt (min): 3.74, Area (%): 95.8 (Max. Chrom.), 95.4 (254 nm). HPLC (Method A): Rt (min) 3.82 : Area (%): 95.1 (Max. Chrom.), 94.6 (254 nm).

### Example 68: 8-ethyl-3-methyl-4-{[4-(3-methylphenyl)piperazin-1-yl]carbonyl}-1-oxa-8-azaspiro[4.5]dec-3-en-2-one

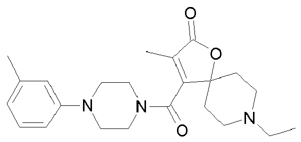

A solution of 8-ethyl-3-methyl-2-oxo-1-oxa-8-aza-spiro[4.5]dec-3-ene-4-carboxylic acid hydrochloride (Intermediate 1, 312 mg, 1.13 mmol) and triethylamine (0.79 mL, 5.71 mmol) was prepared in anhydrous dimethylformamide (50 mL) and cooled at 0°C. 1-Hydroxy-benzotriazole (347 mg, 2.27 mmol) and EDC.HCI (435 mg, 2.27 mmol) were added, followed by 1-(3-methylphenyl)piperazine (200 mg, 1.13 mmol). The resulting mixture was stirred at room temperature for 36 hours. The reaction mixture was concentrated under vacuum. The residue was diluted with dichloromethane and washed with an aqueous solution of sodium bicarbonate and brine. The organic layer was dried (Na₂SO₄) and concentrated under vacuum. After purification by flash chromatography (neutral alumina), the title compound was obtained as a pale yellow solid. TLC: dichloromethane/methanol (9/1): R_{f}: 0.4. ¹H NMR (DMSO-d₆, 400 MHz): δ 7.12-7.08 (m, 1 H), 6.77-6.73 (m, 2H), 6.64 (d, *J*=7.2 Hz, 1 H), 3.78 (m, 1 H), 3.67 (m, 1 H), 3.53 (t, *J*=4.8 Hz, 2H), 3.20 (m, 3H), 3.12 (m, 1 H), 2.82 (d, *J*=10.0 Hz, 2H), 2.36-2.31 (m, 2H), 2.24 (s, 3H), 2.14-2.08 (m, 2H), 1.84 (m, 2H), 1.75 (s, 3H), 1.70 (m, 1 H), 1.50 (m, 1 H), 0.99 (m, 3H). LCMS (Method D): Mass found (M+ 398.3), Rt (min): 3.07, Area (%): 99.6 (Max. Chrom.), 99.5 (254 nm). HPLC (Method A): Rt (min) 3.19, Area (%): 97.7 (Max. Chrom.), 98.5 (254 nm).

### Example 69: 3-methyl-2-oxo-N-(2-oxo-2-{[3-(trifluoromethyl)phenyl]amino}ethyl)-8-(tetrahydro-2H-pyran-4-yl)-1-oxa-8-azaspiro[4.5]dec-3-ene-4-carboxamide

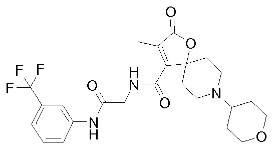

### Step 1: Preparation of [(3-trifluoromethyl-phenylcarbamoyl)-methyl]-carbamic acid tert-butyl ester

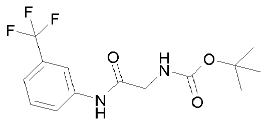

A solution of N-(tert-butoxycarbonyl)glycine (1.0 g, 5.7 mmol) and triethylamine (4 mL, 28.5 mmol) was prepared in dichloromethane (25 mL) and cooled at 0°C. 1-Hydroxy-benzotriazole (1.70 g, 11.4 mmol) and EDC.HCI (2.19 g, 11.4 mmol) were added, followed by 3-trifluoromethyl aniline (0.92 g, 5.7 mmol). The resulting mixture was stirred at room temperature for 12 hours. The reaction mixture was diluted with dichloromethane (25 mL) and washed with a 10% aqueous solution of sodium bicarbonate (15 mL). The organic layer was dried (Na₂SO₄) and concentrated under vacuum. After purification by flash chromatography (silica), the title compound was obtained as a white solid. ¹H NMR (DMSO-d₆, 400 MHz): δ 11.28 (s, 1 H), 8.07 (s, 1 H), 7.75 (d, *J*=8.1 Hz, 1 H), 7.56-7.52 (m, 1 H), 7.38 (d, *J*=7.7 Hz, 1 H), 7.11 (t, *J*=6.0 Hz, 1 H), 3.72 (d, *J*=6.0 Hz, 2H), 1.38 (s, 9H). LCMS (Method D): Mass Found (M+ 219 [M-Boc+2H]⁺), Rt (min): 4.56, Area (%): 99.0 (Max. Chrom.).

### Step 2: Preparation of 2-amino-N-(3-trifluoromethyl-phenyl)-acetamide, hydrochloride salt

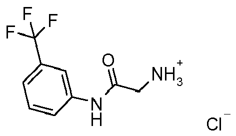

A 4N solution of HCI in dioxane (5 mL) was added into a solution of [(3-trifluoromethyl-phenylcarbamoyl)-methyl]-carbamic acid tert-butyl ester (600 mg, 1.88 mmol) in dioxane cooled at 0°C. The resulting mixture was stirred at room temperature for 2 hours. The reaction mixture was concentrated under vacuum to give the title compound as a white solid (400 mg, 84%). ¹H NMR (DMSO-d₆, 400 MHz): δ 11.28 (s, 1 H), 8.29 (s, 3H), 8.09 (s,1 H), 7.84 (d, *J*=8.1 Hz, 1 H), 7.60-7.56 (m, 1 H), 7.44 (d, *J*=7.7 Hz, 1 H), 3.83 (s, 2H).

### Step 3: Preparation of tert-butyl 3-methyl-2-oxo-4-{[(2-oxo-2-{[3-(trifluoromethyl)phenyl]amino}ethyl)amino]carbonyl}-1-oxa-8-azaspiro[4.5]dec-3-ene-8-carboxylate

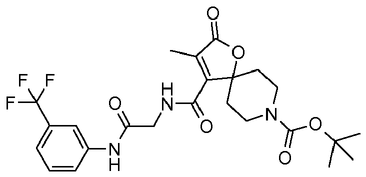

A solution of 8-(tert-butoxycarbonyl)-3-methyl-2-oxo-1-oxa-8-azaspiro[4.5]dec-3-ene-4-carboxylic acid (Intermediate 2, 800 mg, 2.57 mmol) and triethylamine (1.78 mL, 12.9 mmol) was prepared in dichloromethane (25 mL) and cooled at 0°C. 1-Hydroxy-benzotriazole (695 mg, 5.14 mmol) and EDC.HCI (986 g, 5.14 mmol) were added, followed by 2-amino-N-(3-trifluoromethyl-phenyl)-acetamide hydrochloride (655 mg, 2.57 mmol). The resulting mixture was stirred at room temperature for 12 hours. The reaction mixture was diluted with dichloromethane (25 mL) and washed with a 10% aqueous solution of sodium bicarbonate (15 mL). The organic layer was dried (Na₂SO₄) and concentrated under vacuum. After purification by flash chromatography (silica), the title compound was obtained as a white solid. ¹H NMR (DMSO-d₆, 400 MHz): δ 10.74 (s, 1 H), 8.88 (t, *J*=6.0 Hz, 1 H), 8.06 (s, 1 H), 7.74 (d, *J*=8.6 Hz, 1 H), 7.57-7.53 (m, 1 H), 7.40 (d, *J*=7.6 Hz, 1 H), 4.05-3.97 (m, 4H), 2.97-2.98 (m, 2H), 2.10-2.02 (m, 2H), 1.92 (s, 3H), 1.51-1.48 (m, 2H), 1.37 (s, 9H). LCMS (Method D): Mass Found (M+ 412 [M-Boc+2H]⁺), Rt (min): 4.92, Area (%): 92.0 (Max. Chrom.).

### Step 4: Preparation of 3-methyl-2-oxo-N-(2-oxo-2-{[3-(trifluoromethyl)phenyl]amino}ethyl)-1-oxa-8-azaspiro[4.5]dec-3-ene-4-carboxamide, hydrochloride salt

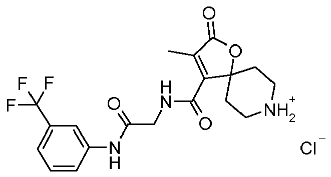

A 4N solution of HCl in dioxane (5 mL) was added into a solution of tert-butyl 3-methyl-2-oxo-4-{[(2-oxo-2-{[3-(trifluoromethyl)phenyl]amino}ethyl)amino]carbonyl}-1-oxa-8-azaspiro[4.5]dec-3-ene-8-carboxylate (400 mg, 0.78 mmol) in dioxane cooled at 0°C. The reaction mixture was stirred at room temperature for 2 hours. The reaction mixture was concentrated under vacuum to give the title compound as a white solid (300 mg, 86 %). ¹H NMR (DMSO-d₆, 400 MHz): δ 10.65 (s, 1 H), 9.00-8.95 (m, 2H), 8.51-8.48 (m, 1 H), 8.09 (s, 1 H), 7.78 (d, *J*=8.7 Hz, 1 H), 7.58-7.54 (m, 1 H), 7.41 (d, *J*=7.8 Hz, 1 H), 4.10 (d, *J*=6.0 Hz, 2H), 3.38-3.39 (m, 2H), 3.07-2.98 (m, 2H), 2.33-2.22 (d, *J*=1.5 Hz, 2H), 1.95 (s, 3H), 1.91 (m, 2H). LCMS (Method D): Mass Found (M+ 412.3), Rt (min): 3.22, Area (%): 91.0 (Max. Chrom.).

### Step 5: Preparation of 3-methyl-2-oxo-N-(2-oxo-2-{[3-(trifluoromethyl)phenyl]amino}ethyl)-8-(tetrahydro-2H-pyran-4-yl)-1-oxa-8-azaspiro[4.5]dec-3-ene-4-carboxamide

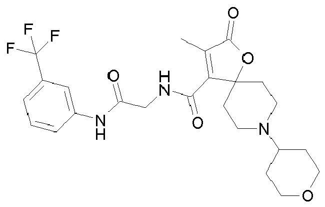

The title compound was prepared following the protocol described for Example 21, but starting from 3-methyl-2-oxo-N-(2-oxo-2-{[3-(trifluoromethyl)phenyl]amino}ethyl)-1-oxa-8-azaspiro[4.5]dec-3-ene-4-carboxamide hydrochloride. After purification by flash chromatography, the title compound was obtained as an off-white solid. ¹H NMR (DMSO-d₆, 400 MHz): δ 10.51 (s, 1 H), 8.87 (s, 1 H), 8.05 (s, 1 H), 7.77 (d, *J* = 7.4 Hz, 1 H), 7.56 (m, 1 H), 7.41 (d, *J* = 7.2 Hz, 1 H), 4.07 (s, 2H), 3.87 (d, *J* = 9.8 Hz, 2H), 3.33 (s, 2H), 2.83 (s, 2H), 2.49 (s, 2H), 2.22 (s, 2H), 1.90-1.96 (m, 5H), 1.65 (s, 2H), 1.46-1.49 (m, 3H). LCMS: (Method D): Mass found (M+ 496.3), Rt (min): 3.29, Area (%): 97.5 (Max. Chrom.), 97.7 (254 nm). HPLC (Method A): Rt (min): 3.36, Area (%): 98.7 (Max. Chrom.), 98.9 (254 nm).

The following Examples 70-73 were obtained following the procedures described for Example 49.

| **Ex.** | **Structure** | **Characterization** |
|---|---|---|
| 70 | 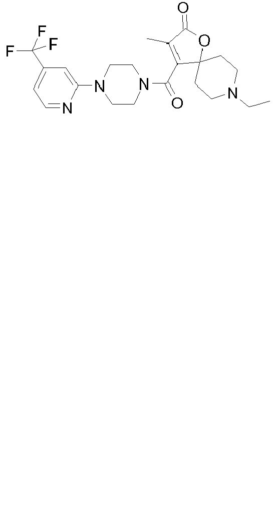 | **8-ethyl-3-methyl-4-({4-[4-(trifluoromethyl)pyridin-2-yl]piperazin-1-yl}carbonyl)-1-oxa-8-azaspiro[4.5]dec-3-en-2-one** |
| | | Purification following column chromatography. The compound was obtained as a white solid (164.9 mg). ¹H NMR: 400 MHz, DMSO-d6: δ 8.35 (d, *J* = 5.1 Hz, 1 H), 7.13 (s, 1 H), 6.93 (d, *J* = 5.1 Hz, 1 H), 3.61-3.76 (m, 5H), 3.52 (s, 3H), 2.82 (d, *J* = 9.2 Hz, 2H), 2.34 (d, *J* = 6.92 Hz, 2H), 2.12-2.23 (m, 3H), 1.871.91 (m, 1H), 1.75 (s, 3H), 1.68 (s, 1H), 1.48-1.51 (m, 1H), 0.98 (t, *J* = 7.1 Hz, 3H). LCMS: (Method D): Mass found (M+ 453.3); Rt (min): 3.61 Area (%) 99.1 (Max. Chrom.), 99.2 (254 nm). HPLC (Method A), Rt (min): 3.02 Area % 98.5 (Max. Chrom.), 99.1 (254 nm). |
| 71 | 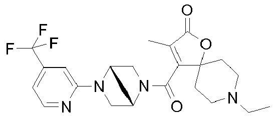 | **8-ethyl-3-methyl-4-({(1S,4S)-5-[4-(trifluoromethyl)pyridin-2-yl]-2,5-diazabicyclo[2.2.1]hept-2-yl}carbonyl)-1-oxa-8-azaspiro[4.5]dec-3-en-2-one** |
| | | Purification following column chromatography. The compound was obtained as a white solid (35.1 mg). ¹H NMR: 400 MHz, DMSO-d6: δ 8.29 (d, J = 5.1 Hz, 1H), 6.86 (t, J = 5.2 Hz, 2H), 5.04 (s, 1 H), 4.80-4.90 (m, 1 H), 4.66 (s, 1 H), 3.62 (d, J = 9.6 Hz, 1 H), 3.47-3.56 (m, 2H), 2.74-2.83 (m, 2H), 2.49-2.50 (m, 1 H), 2.27-2.35 (m, 2H), 2.04-2.12 (m, 3H), 1.91-1.97 (m, 2H), 1.80 (s, 2H), 1.44-1.58 (m, 3H), 0.92-1.00 (m, 3H). LCMS: (Method D): Mass found (M+ 465.3); Rt (min): 2.52, Area (%) : 95.6 (Max. Chrom.), 88.4 (254 nm). HPLC (Method A) Rt (min): 2.45 Area % 98.6 (Max. Chrom.), 98.0 (254 nm). |
| 72 | 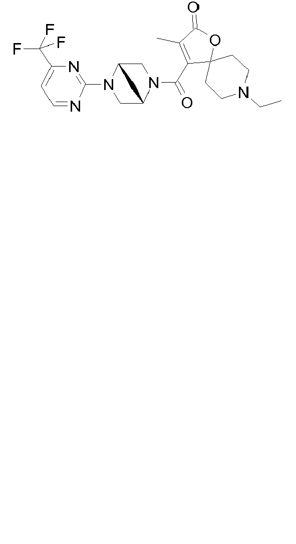 | **8-ethyl-3-methyl-4-({(1S,4S)-5-[4-(trifluoromethyl)pyrimidin-2-yl]-2.5-diazabicyclo[2.2.1]hept-2-yl}carbonyl)-1-oxa-8-azaspiro[4.5]dec-3-en-2-one** |
| | | Purification following column chromatography. The compound was obtained as a white solid (184.5 mg). ¹H NMR: 400 MHz, DMSO-d6: δ 8.69-8.70 (m, 1H), 7.08-7.09 (m, 1 H), 4.66-5.07 (m, 2H), 3.65 (d, *J* = 10.7 Hz, 1H), 3.55 (d, *J* = 7.6 Hz, 1H), 3.50 (s, 1H), 3.23 (s, 1H), 2.83 (s, 2H), 2.31-2.32 (m, 2H), 1.99-2.11 (m, 5H), 1.80 (s, 2H), 1.60 (s, 3H), 1.21 (s, 1 H), 0.98 (s, 3H). LCMS: (Method D): Mass found (M+ 466.3); Rt (min): 3.14; Area (%): 99.6 (Max. Chrom.), 99.7 (254 nm). HPLC (Method A): Rt (min): 3.21 Area % 99.4 (Max. Chrom.), 99.7 (254 nm). |
| 73 | 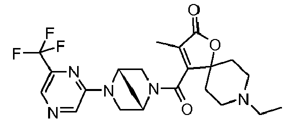 | **8-ethyl-3-methyl-4-({(1S,4S)-5-[6-(trifluoromethyl)pyrazin-2-yl]-2,5-diazabicyclo[2.2.1]hept-2-yl}carbonyl)-1-oxa-8-azaspiro[4.5]dec-3-en-2-one** |
| | | Purification following column chromatography. The compound was obtained as a white solid (4 mg). ¹H NMR: 400 MHz, DMSO-d6: δ 8.42 (s, 1H), 8.21-8.23 (m, 1 H), 4.94-5.04 (m, 2H), 4.69 (s, 1 H), 3.58-3.62 (m, 3H), 3.48-3.49 (m, 2H), 2.84-2.86 (m, 2H), 2.34-2.35 (m, 3H), 2.00-2.13 (m, 4H), 1.81 (s, 3H), 1.39-1.42 (m, 1H), 0.90-1.12 (m, 3H). LCMS: (Method D): Mass found (M+ 466.3); Rt (min): 3.13; Area (%): 99.1 (Max. Chrom.), 98.7 (254 nm). HPLC (Method A) Rt (min): 3.09 Area % 99.3 (Max. Chrom.), 99.1 (254 nm). |

### Example 74: 8-ethyl-3-methyl-4-({4-[3-(trifluoromethyl)phenyl]piperazin-1-yl}carbonyl)-1-oxa-8-azaspiro[4.5]dec-3-en-2-one

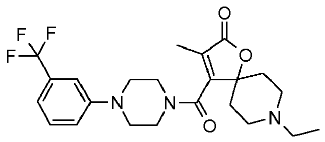

A mixture of of 3-methyl-4-({4-[3-(trifluoromethyl)phenyl]piperazin-1-yl}carbonyl)-1-oxa-8-azaspiro[4.5]dec-3-en-2-one, hydrochloride salt (Example 1, 0.4 g, 1.67 mmol), triethylamine (0.50 g, 5.02 mmol), 1-(3-trifluoromethyl)piperazine monohydrochloride (0.53 g, 2.00 mmol) and 1-propane phosphonic cyclic anhydride (1.5 g, 5.02 mmol) in DCM (15 mL) was stirred at room temperature for 12 hours. The reaction mixture was poured into water and the organic layer was separated, washed with water (2 X 20 mL), dried over anhydrous sodium sulphate, filtered and concentrated. After purification by flash chromatography (silica), the title compound was isolated as a white solid. ¹H NMR (CDCl₃, 400 MHz) δ 7.42 (t, *J* = 7.9 Hz, 1 H), 7.19 (d, *J* = 7.8 Hz, 1 H), 7.12 (s, 1 H), 7.09 (d, *J* = 8.32 Hz, 1 H), 3.93-3.84 (m, 2H), 3.56 (s, 2H), 3.29-3.18 (m, 4H), 2.95 (m, 2H), 2.52-2.41 (m, 5H), 2.08 (m, 1 H), 1.89 (s, 3H), 1.73-1.62 (m, 2H), 1.13 (t, *J* = 7.2 Hz, 3H). LCMS (Method D): Mass found (M+ 452), Rt (min): 3.83, Area (%): 99.8 (Max. Chrom.), 99.9 (254 nm). HPLC (Method A): Rt (min): 3.84, Area (%): 99.7 (Max. Chrom.), 99.4 (254 nm).

### Example 75: 8-ethyl-3-methyl-2-oxo-N-{4-[3-(trifluoromethyl)-1H-pyrazol-1-yl]phenyl}-1-oxa-8-azaspiro[4.5]dec-3-ene-4-carboxamide

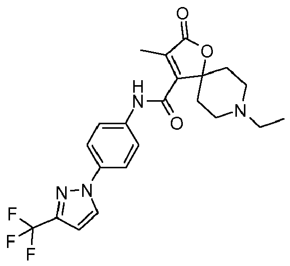

### Step 1: Preparation of 1-(4-nitro-phenyl)-3-trifluoromethyl-1H-pyrazole

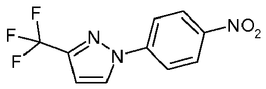

1-fluoro-4-nitro benzene (4.14 g, 29.4 mmol) was added slowly to a solution of 3-trifluoromethylpyrazole (2 g, 14.7 mmol) and anhydrous potassium carbonate (6 g, 44.1 mmol) in DMF at room temperature.The reaction mixture was then heated at 110°C overnight then cooled to RT. The reaction mixture was diluted with water then extracted with ethyl acetate (2 x). The combined organic layers were washed with water and brine, then dried over sodium sulfate, filtered and concentrated to give the title compound as a beige solid (3.3 g, 87.4%). TLC: Ethylacetate/Hexane (3/7): *R_{f}* : 0.85. ¹H NMR (CDCl₃; 400 MHz): δ 8.38 (dd, J = 9.1 Hz, 2H), 8.10 (d, J = 2.4 Hz, 1 H), 7.94 (dd, J = 9.1 Hz, 2H), 6.83 (d, J = 2.5 Hz, 1 H).

### Step 2: Preparation of 4-(3-trifluoromethyl-pyrazole-1-yl)-phenylamine

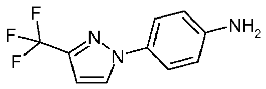

A solution of 1-(4-nitro-phenyl)-3-trifluoromethyl-1H-pyrazole (2 g, 7.78 mmol) in methanol was hydrogenated at room temperature in presence of palladium on carbon (0.2 g, 10% wt) overnight. The reaction mixture was filtered through a celite pad. The pad was rinced with ethyl acetate and methanol. The combined organic layers were concentrated under reduced pressure to give the title compound as a beige solid (1.3 g, 73.5 %). TLC: Dichloromethane: Methanol (9.5/0.5): *R_{f}* : 0.5. ¹H NMR (DMSO-d₆; 400 MHz): δ 8.41 (d, J = 2.3 Hz, 1 H), 7.44 (d, J = 8.7 Hz, 2H), 6.90 (d, J = 2.1 Hz, 1 H), 6.64 (d, J = 8.8 Hz, 2H), 5.38 (s, 2H).

### Step 3: Preparation of 8-ethyl-3-methyl-2-oxo-N-{4-[3-(trifluoromethyl)-1H-pyrazol-1-yl]phenyl}-1-oxa-8-azaspiro[4.5]dec-3-ene-4-carboxamide

A solution of 8-ethyl-3-methyl-2-oxo-1-oxa-8-aza-spiro[4.5]dec-3-ene-4-carboxylic acid hydrochloride (Intermediate 1, 200 mg, 0.73 mmol), triethylamine (0.4 mL, 2.9 mmol) and propane phosphoric acid cyclic anhydride in dichloromethane (10 mL) was stirred at 0°C for 15 minutes. 4-(3-trifluoromethyl-pyrazole-1-yl)-phenylamine (245 mg, 1.09 mmol) was added and the reaction mixture was warmed to room temperature. After stirring for 12 hours, the reaction mixture was poured into water. The organic layer was washed with water, brine, dried over Na₂SO₄, filtered and concentrated. After purification by flash chromatography (neutral alumina), the title compound was isolated as a white solid. ¹H NMR (DMSO-d₆; 400 MHz): δ 10.66 (s, 1 H), 8.69 (d, J = 0.8 Hz, 1 H), 7.89 (d, J = 9.04Hz, 2H), 7.82(d, *J* = 9.1 Hz, 2H), 7.04 (d, J = 2.3 Hz, 1 H), 2.84 (m, 2H), 2.35 (q, J = 7.0 Hz 2H), 2.20-2.14 (m, 4H), 1.91 (s, 3H), 1.57-1.54 (m, 2H), 0.95 (t, J = 7.1 Hz, 3H). LCMS (Method D): Mass found (M+ 449.0), Rt (min): 3.81, Area (%): 99.2 (Max. Chrom.), 99.64 (254 nm). HPLC (Method A): Rt (min): 3.9, Area (%): 99.3 (Max. Chrom.), 99.6 (254 nm).

The following Examples 76-78 were obtained following the procedures described for Example 74. For Examples 76 and 78, the compounds were obtained in 2 steps comprising in the procedures described for Example 74 but from starting Intermediate 2 followed by the Boc deprotection with HCI. Example 77 was prepared from Intermediate 13.

| **Ex.** | **Structure** | **Characterization** |
|---|---|---|
| 76 | 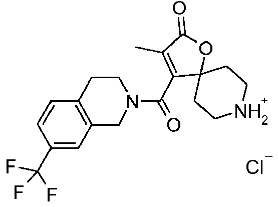 | **3-methyl-4-{[7-(trifluoromethyl)-3,4-dihydroisoquinolin-2(1H)-yl]carbonyl}-1-oxa-8-azaspiro[4.5]dec-3-en-2-one, hydrocloride salt** |
| | | Purification following column chromatography. The compound was obtained as a white solid (58.9 mg, 64 %) ¹H NMR (DMSO-d6; 400MHz) δ.90-9.10 (m, 1H), 8.43-8.53 (m, 1 H), 7.59-7.72 (m, 1 H), 7.54-7.56 (d, *J* = 8.0 Hz, 1H), 7.42-7.44 (d, *J* = 8.0 Hz, 1H), 3.78-3.99 (m, 3H), 3.33-3.35 (m, 1H), 3.21-3.32 (m, 1H), 2.80-3.10 (m, 5H), 1.96-1.98 (m, 2H), 1.74-1.80 (m, 3H), 1.69-1.74 (m, 2H). LCMS: (Method D): Mass found (M+ 395.3), Rt (min): 3.54; Area (%): 94.23 (Max. Chrom.), 94.2 (220 nm). HPLC (Method A): Rt (min): 3.56; Area (%): 99.3 (Max. Chrom.), 98.2 (254 nm) |
| 77 | 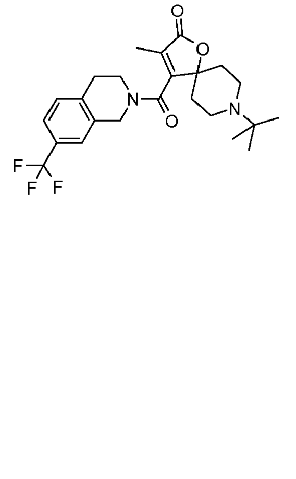 | **8-tert-butyl-3-methyl-4-{[7-(trifluoromethyl)-3,4-dihydroisoquinolin-2(1H)-yl]carbonyl}-1-oxa-8-azaspiro[4.5]dec-3-en-2-one** |
| | | Purification following column chromatography. The compound was obtained as a white solid (141.4 mg). ¹H NMR (CDCl₃, 400 MHz): δ 7.46-7.50 (m, 2H), 7.26-7.35 (m, 1H), 4.92 (d, J = 13.4 Hz, 1H), 4.57 (s, 1H), 4.10-4.14 (m, 1 H), 3.97-3.92 (m, 1 H), 2.94-3.26 (m, 3H), 2.70-3.90 (m, 3H), 1.55-1.91 (m, 7H), 1.27 (s, 9H). LCMS: (Method D): Mass found (M+ 451.2); Rt (min): 3.85, Area (%): 92.6 (Max. Chrom.), 92.1 (220 nm). HPLC (Method A) Rt (min): 3.93 Area (%): 93.8 (Max. Chrom.), 93.7 (220 nm). |
| 78 | 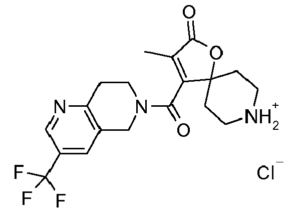 | **3-methyl-4-{[3-(trifluoromethyl)-7,8-dihydro-1,6-naphthyridin-6(5H)-yl]carbonyl}-1-oxa-8-azaspiro[4.5]dec-3-en-2-one, hydrocloride salt** |
| | | Purification following recrystallization.The compound was obtained as a white solid (45 mg, 93 %). ¹H NMR (DMSO-d6, 400 MHz): δ 8.75-8.78 (m, 1 H), 8.00-8.25 (m, 1 H), 4.82-5.00 (m, 2H), 3.84-4.16 (m, 2H), 3.25-3.37 (m, 2H), 2.96-3.11 (m, 4H), 2.50-2.52 (m, 1H), 2.04-2.06 (m, 1 H), 1.81-1.86 (m, 1H), 1.77-1.81 (m, 2H), 1.70-1.73 (m, 2H). LCMS: (Method D): Mass found (M+ 396.0); Rt (min): 2.68, Area (%): 94.8 (Max. Chrom.), 94.8 (220 nm). HPLC (Method A): Rt (min): 2.67; Area (%) 95.0 (Max. Chrom.), 92.1 (254 nm). |

### Example 79: 8-ethyl-3,7-dimethyl-4-{[7-(trifluoromethyl)-3,4-dihydroisoquinolin-2(1H)-yl]carbonyl}-1-oxa-8-azaspiro[4.5]dec-3-en-2-one

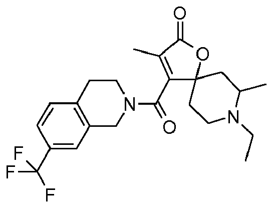

### Step 1: Preparation of 8-(tert-butoxycarbonyl)-3,7-dimethyl-2-oxo-1-oxa-8-azaspiro[4.5]dec-3-ene-4-carboxylic acid

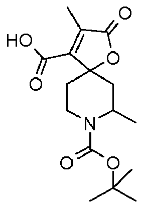

Dimethyl itaconate (1.11 g, 7.04 mmol) and 1-Boc-2-methyl-piperidin-4-one (1.5 g, 7.04 mmols) were added over 20 min to a solution of sodium methoxide (0.83 g, 15.49 mmol) in tetrahydrofuran (100 mL) maintained at 0°C. The reaction mixture was stirred at 0°C for 2 hours then 16 hours at RT under nitrogen. Wate was then added. The THF was removed under reduced pressure and the aqueous layer was acidified by addition of an aqueous solution of HCI (1.5 N). The aqueous layer was then extracted (EtOAc) and the combined organic layers were evaporated under reduced pressure. After purification by flash chromatography (silica), the title compound was obtained as an off white solid. ¹H NMR (CDCl₃, 400 MHz) δ 5.0 (s, 2H), 4.57-4.48 (m, 1 H), 4.15-4.02 (m, 1 H), 3.79-3.68 (m, 1 H), 3.31-2.78 (m, 1 H), 2.77-2.63 (m, 1 H), 2.68-2.56 (m, 1 H), 2.40-2.30 (m, 1 H), 2.23 (s, 1 H), 1.49 (s, 9H), 1.36-1.26 (m, 3H). LCMS (Method D) Mass found (M+ 226.2) Rt (min): 4.06; Area (%) 84.5 (Max. Chrom.).

### Step 2: Preparation of tert-butyl 3,7-dimethyl-2-oxo-4-{[7-(trifluoromethyl)-3,4-dihydroisoquinolin-2(1H)-yl]carbonyl}-1-oxa-8-azaspiro[4.5]dec-3-ene-8-carboxylate

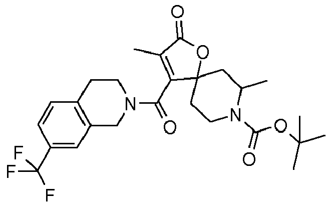

A solution of 8-(tert-butoxycarbonyl)-3,7-dimethyl-2-oxo-1-oxa-8-azaspiro[4.5]dec-3-ene-4-carboxylic acid (0.9 g, 2.76 mmol), diisopropylethylamine (81.07 g, 8.30 mmol), 7-trifluoromethyl-1, 2, 3, 4-tetrahydro-isoquinoline hydrochloride (0.73 g, 3.06 mmol), EDC.HCI (0.63 g, 3.34 mmol) and 1-hydroxy benzotriazole (0.41 g, 3.06 mmol) in dry dichloromethane (50 mL) was stirred at room temperature for 16 hours. The reaction mixture was diluted with dichloromethane (100 mL), washed with water (100 mL) and brine (100 mL), dried over anhydrous sodium sulphate, filtered and concentrated. After purifiaction by flash chromatography (silica), the title compound was isolated as a white solid. LCMS (Method D) Mass found (M+ 409.3, Boc cleaved); Rt (min): 5.57; Area (%) 76.7 (Max. Chrom.).

### Step 3: Preparation of 3,7-dimethyl-4-{[7-(trifluoromethyl)-3,4-dihydroisoquinolin-2(1H)-yl]carbonyl}-1-oxa-8-azaspiro[4.5]dec-3-en-2-one, hydrochloride salt

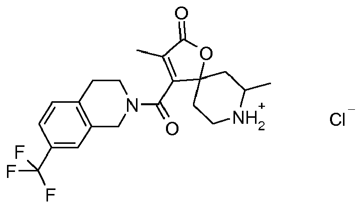

A solution of HCI (4 N in dioxane, 15 mL) was added to a solution of tert-butyl 3,7-dimethyl-2-oxo-4-{[7-(trifluoromethyl)-3,4-dihydroisoquinolin-2(1H)-yl]carbonyl}-1-oxa-8-azaspiro[4.5]dec-3-ene-8-carboxylate (0.31 g, 0.61 mmol) in dry dioxane (15 mL). The reaction mixture was stirred at RT for 3 hours. THF was removed under reduced pressure and the residue was triturated in Et₂O, filtered under N₂ atm, and dried to give the title intermediate as a white solid (0.26 g, 80.8 %). LCMS (Method D) Mass found (M+ 409.3) Rt (min): 3.59; Area (%): 73.1 (Max. Chrom.)

### Step 4: Preparation of 8-ethyl-3,7-dimethyl-4-{[7-(trifluoromethyl)-3,4-dihydroisoquinolin-2(1H)-yl]carbonyl}-1-oxa-8-azaspiro[4.5]dec-3-en-2-one

A mixture of 3 , 7-dimethyl-4-{[7-(trifluoromethyl)-3,4-dihydroisoquinolin-2(1H)-yl]carbonyl}-1-oxa-8-azaspiro[4.5]dec-3-en-2-one, hydrochloride salt (0.25 g, 0.346 mmol), triethylamine (0.14 g, 1.40 mmol), acetaldehyde (0.071 g, 1.83 mmol) and sodium triacetoxyborohydride (0.26 g, 1.22 mmol) in DCM (20 mL) was stirred for 16 hours at room temperature. The reaction mixture was quenched with water and the organic layer was washed with water (25 mL x 2), dried over anhydrous sodium sulphate, filtered and concentrated. Purification by fash chromatography (silica) afforded the titled compound as a pale yellow. ¹H NMR (CDCl₃, 400 MHz): δ 7.46-7.52 (m, 2H), 7.26-7.34 (m, 1 H), 4.89 (d, J = 4.2 Hz, 1 H), 4.57 (d, J = 6.8 Hz, 1 H), 3.90-4.04 (m, 2H), 3.64-3.67 (m, 1 H), 3.25-3.03 (m, 1 H), 2.80-3.02 (m, 4H), 2.52-2.66 (m, 4H), 1.84-1.85 (m, 3H), 1.63-1.70 (m, 2H), 1.01-1.07 (m, 5H). LCMS: (Method D): Mass found (M+ 437.3); Rt (min): 3.70 Area (%): 99.0 (Max. Chrom.), 99.1 (220 nm). HPLC (Method A): Rt (min): 3.70 Area (%): 99.1 (Max. Chrom.), 99.3 (254 nm).

### Example 80: 8-ethyl-4-{[4-(3-fluorophenyl)piperazin-1-yl]carbonyl}-3-methyl-1-oxa-8-azaspiro[4.5]dec-3-en-2-one

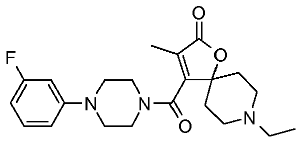

HATU (1.1 equiv, 96 mg, 0.25 mmol) and N,N-diisopropylethylamine (1.2 equiv, 115 mg, 0.27 mmol) were added to a solution of the sodium salt of 8-ethyl-3-methyl-2-oxo-1-oxa-8-azaspiro[4.5]dec-3-ene-4-carboxylate (Intermediate 4, 100 mg, 0.23 mmol) in dry N,N-dimethylformamide (1 mL). The reaction mixture was stirred at room temperature for 30 min before the addition of 3-fluorophenyl-4-piperazine (1.5 equiv, 62 mg, 0.35 mmol). The reaction mixture was then stirred at room temperature for 16 hours. After removal of DMF under reduced pressure, the crude compound was purified by preparative LCMS (Method J). The title compound was obtained as a yellow solid. LCMS (Method D) Mass found (M+ 402.2) Rt (min): 3.25; Area (%): 95.5 (Max. Chrom.)

The following Examples 81-100 were obtained following the same procedure described for Example 80, but starting from different amines.

| **Ex.** | **Structure** | **Characterization** |
|---|---|---|
| 81 | 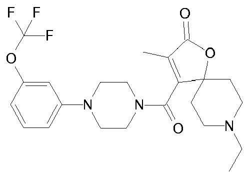 | **8-ethyl-3-methyl-4-({4-[3-(trifluoromethoxy)phenyl]piperazin-1-yl}carbonyl)-1-oxa-8-azaspiro[4.5]dec-3-en-2-one** |
| | | Purification following the Method J. The compound was obtained as a white solid (99.7 mg). LCMS (Method Method I): Mass found (M+ 468.2); Rt (min): 3.57, Area (%): 99.9. |
| 82 | 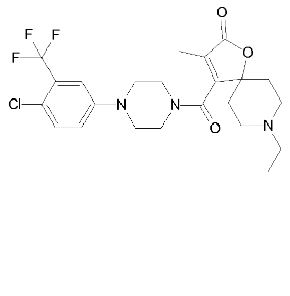 | **4-({4-[4-chloro-3-(trifluoromethyl)phenyl]piperazin-1-yl}carbonyl)-8-ethyl-3-methyl-1-oxa-8-azaspiro[4.5]dec-3-en-2-one** |
| | | Purification following the Method J. The compound was obtained as a white solid (36.9 mg). LCMS (Method Method I): Mass found (M+ 487.2); Rt (min): 3.73, Area (%): 99.6. |
| 83 | 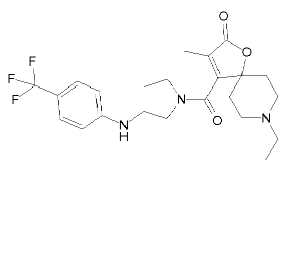 | **8-ethyl-3-methyl-4-[(3-{[4-(trifluoromethyl)phenyl]amino}pyrrolidin-1-yl)carbonyl]-1-oxa-8-azaspiro[4.5]dec-3-en-2-one** |
| | | Purification following the Method J. The compound was obtained as an off-white solid (31.3 mg). LCMS (Method Method I): Mass found (M+ 452.2); Rt (min): 3.38, Area (%): 97.8. |
| 84 | 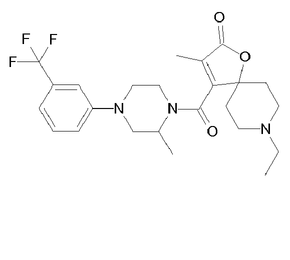 | **8-ethyl-3-methyl-4-({2-methyl-4-[3-(trifluoromethyl)phenyl]piperazin-1-yl}carbonyl)-1-oxa-8-azaspiro[4.5]dec-3-en-2-one** |
| | | Purification following the Method J. The compound was obtained as a light brown solid (37.9 mg). LCMS (Method Method I): Mass found (M+ 466.2); Rt (min): 3.64, Area (%): 99.9. |
| 85 | 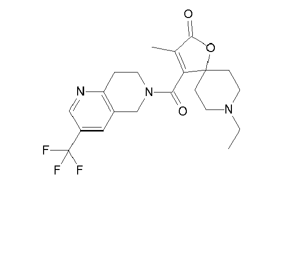 | **8-ethyl-3-methyl-4-{[3-(trifluoromethyl)-7,8-dihydro-1,6-naphthyridin-6(5H)-yl]carbonyl}-1-oxa-8-azaspiro[4.5]dec-3-en-2-one** |
| | | Purification following the Method J. The compound was obtained as a brown solid (28.8 mg). LCMS (Method Method I): Mass found (M+ 424.2); Rt (min): 3.03, Area (%): 96.1. |
| 86 | 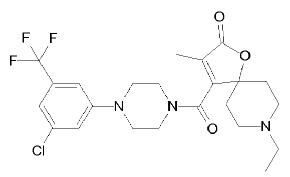 | **4-({4-[3-chloro-5-(trifluoromethyl)phenyl]piperazin-1-yl}carbonyl)-8-ethyl-3-methyl-1-oxa-8-azaspiro[4.5]dec-3-en-2-one** |
| | | Purification following the Method J. The compound was obtained as a white solid (48.5 mg).LCMS (Method Method I): Mass found (M+ 486.2); Rt (min): 3.74, Area (%): 98.4. |
| 87 | 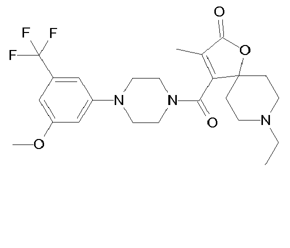 | **8-ethyl-4-({4-[3-methoxy-5-(trifluoromethyl)phenyl]piperazin-1-yl}carbonyl)-3-methyl-1-oxa-8-azaspiro[4.5]dec-3-en-2-one** |
| | | Purification following the Method J. The compound was obtained as a white solid (71.3 mg). LCMS (Method Method I): Mass found (M+ 482.2); Rt (min): 3.53, Area (%): 99.6. |
| 88 | 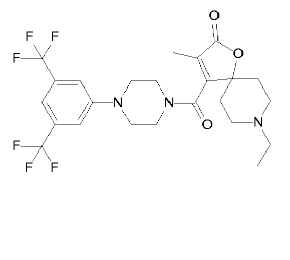 | **4-({4-[3,5-bis(trifluoromethyl)phenyl]piperazin-1-yl}carbonyl)-8-ethyl-3-methyl-1-oxa-8-azaspiro[4.5]dec-3-en-2-one** |
| | | Purification following the Method J. The compound was obtained as a white solid (67.3 mg). LCMS (Method Method I): Mass found (M+ 520.2); Rt (min): 3.80, Area (%): 99.4. |
| 89 | 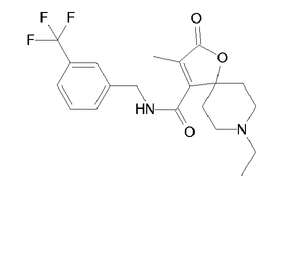 | **8-ethyl-3-methyl-2-oxo-N-[3-(trifluoromethyl)benzyl]-1-oxa-8-azaspiror[4.5]dec-3-ene-4-carboxamide** |
| | | Purification following the Method J. The compound was obtained asa yellow solid (26.7 mg). LCMS (Method Method I): Mass found (M+ 397.2); HPLC (MethodMethod I) Rt (min): 3.28, Area (%): 97.5. |
| 90 | 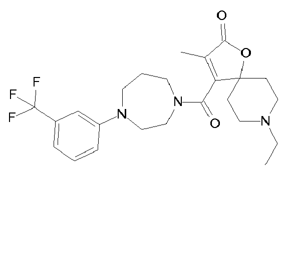 | **8-ethyl-3-methyl-4-({4-[3-(trifluoromethyl)phenyl]-1,4-diazepan-1-yl}carbonyl)-1-oxa-8-azaspiro[4.5]dec-3-en-2-one** |
| | | Purification following the Method J. The compound was obtained as a white solid (35.9 mg). LCMS (Method Method I): Mass found (M+ 466.2); HPLC (MethodMethod I) Rt (min): 3.39, Area (%): 99.0. |
| 91 | 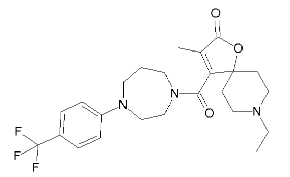 | **8-ethyl-3-methyl-4-({4-[4-(trifluoromethyl)phenyl]-1,4-diazepan-1-yl}carbonyl)-1-oxa-8-azaspiro[4.5]dec-3-en-2-one** |
| | | Purification following the Method J. The compound was obtained as a white solid (47.2 mg). LCMS (Method Method I): Mass found (M+ 466.2); HPLC (MethodMethod I) Rt (min): 3.49, Area (%): 98.7. |
| 92 | 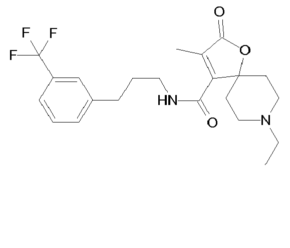 | **8-ethyl-3-methyl-2-oxo-N-{3-[3-(trifluoromethyl)phenyl]propyl}-1-oxa-8-azaspiro[4.5]dec-3-ene-4-carboxamide** |
| | | Purification following the Method J. The compound was obtained as a brown oil (68.4 mg). LCMS (Method Method I): Mass found (M+ 425.2); HPLC (MethodMethod I) Rt (min): 3.48, Area (%): 97.8. |
| 93 | 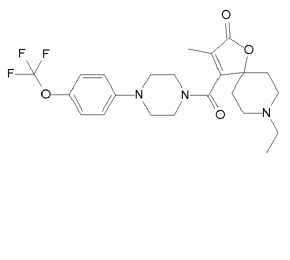 | **8-ethyl-3-methyl-4-({4-[4-(trifluoromethoxy)phenyl]piperazin-1-yl}carbonyl)-1-oxa-8-azaspiro[4.5]1dec-3-en-2-one** |
| | | Purification following the Method J. The compound was obtained as a white solid (31.5 mg). LCMS (Method Method I): Mass found (M+ 468.2); HPLC (MethodMethod I) Rt (min): 3.55, Area (%): 99.9. |
| 94 | 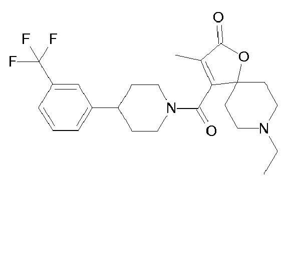 | **8-ethyl-3-methyl-4-({4-[3-(trifluoromethyl)phenyl]piperidin-1-yl}carbonyl)-1-oxa-8-azaspiro[4.5]dec-3-en-2-one** |
| | | Purification following the Method J. The compound was obtained as a brown solid (48.0 mg). LCMS (Method Method I): Mass found (M+ 451.2); HPLC (MethodMethod I) Rt (min): 3.66, Area (%): 95.9. |
| 95 | 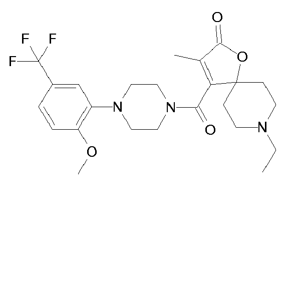 | **8-ethyl-4-({4-[2-methoxy-5-(trifluoromethyl)phenyl]piperazin-1-yl}carbonyl)-3-methyl-1-oxa-8-azaspiro[4.5]dec-3-en-2-one** |
| | | Purification following the Method J. The compound was obtained as as an off-white solid (27.1 mg). LCMS (Method Method I): Mass found (M+ 482.2); HPLC (MethodMethod I) Rt (min): 3.46, Area (%): 95.1. |
| 96 | 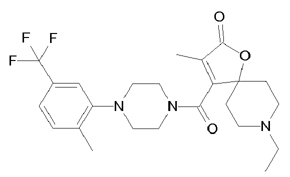 | **8-ethyl-3-methyl-4-({4-[2-methyl-5-(trifluoromethyl)phenyl]piperazin-1-yl}carbonyl)-1-oxa-8-azaspiro[4.5]dec-3-en-2-one** |
| | | Purification following the Method J. The compound was obtained as a white solid (93.5 mg). LCMS (Method Method I): Mass found (M+ 466.2); HPLC (MethodMethod I) Rt (min): 3.69, Area (%): 99.9. |
| 97 | 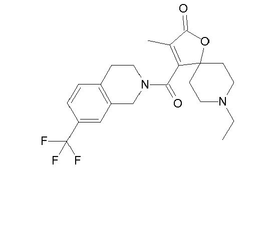 | **8-ethyl-3-methyl-4-{[7-(trifluoromethyl)-3,4-dihydroisoquinolin-2(1H)-yl]carbonyl}-1-oxa-8-azaspiro[4.5]dec-3-en-2-one** |
| | | Purification following the Method J. The compound was obtained as a white solid (34.4 mg). LCMS (Method Method I): Mass found (M+ 423.2); HPLC (MethodMethod I) Rt (min): 3.53, Area (%): 98.8. |
| 98 | 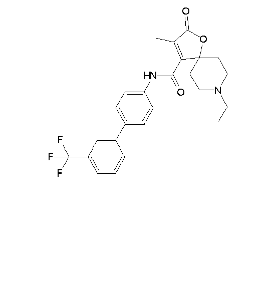 | **8-ethyl-3-methyl-2-oxo-N-[3'-(trifluoromethyl)biphenyl-4-yl]-1-oxa-8-azaspiro[4.5]dec-3-ene-4-carboxamide** |
| | | Purification following the Method J. The compound was obtained as as an off-white solid (37.1 mg). LCMS (Method Method I): Mass found (M+ 459.2); HPLC (MethodMethod I) Rt (min): 3.90, Area (%): 99. |
| 99 | 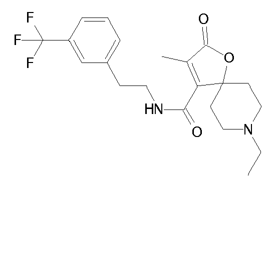 | **8-ethyl-3-methyl-2-oxo-N-{2-[3-(trifluoromethyl)phenyl]ethyl}-1-oxa-8-azaspiro[4.5]dec-3-ene-4-carboxamide** |
| | | Purification following the Method J. The compound was obtained as as a yellow solid (38.9 mg). LCMS (Method Method I): Mass found (M+ 411.2); Rt (min): 3.34, Area (%): 95.1. |
| 100 | 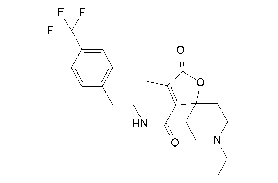 | **8-ethyl-3-methyl-2-oxo-N-{2-[4-(trifluoromethyl)phenyl]ethyl}-1-oxa-8-azaspiro[4.5]dec-3-ene-4-carboxamide** |
| | | Purification following the Method J. The compound was obtained as as a white solid (37.4 mg). LCMS (Method Method I): Mass found (M+ 411.2); Rt (min): 3.36, Area (%): 99.8. |

### Example 101: 8-isopropyl-3-methyl-4-({4-[3-(trifluoromethyl)phenyl]piperazin-1-yl}carbonyl)-1-oxa-8-azaspiro[4.5]dec-3-en-2-one

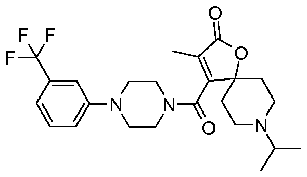

3-methyl-4-({4-[3-(trifluoromethyl)phenyl]piperazin-1-yl}carbonyl)-1-oxa-8-azaspiro[4.5]dec-3-en-2-one, hydrochloride salt (Example 1, 150 mg, 0.30 mmol) and acetone (20 mg, 0.36 mmol) were suspended in dichloromehtane. N,N-diisopropylethylamine (77 µL, 0.45 mmol) was added. After 5 minutes stirring, sodium triacetoxyborohydride (90 mg, 0.45 mmol) was added portionwise. The reaction mixture was stirred at RT for 4 hours, then quenched with an aqueous solution of sodium hydroxide (1 N) until the aqueous layer was basic. The mixture was extracted with dichloromethane. The combined organic phases were dried over MgSO₄, filtered and concentrated. After purification by flash chromatography (silica; heptane/EtOAc, gradient from 100:0 to 30:70), the title compound was obtained as a white solid (26 mg, 18 %). LCMS (Method Method I) Mass found (M+ 466.2) Rt (min): 3.66; Area (%): 95.7 (Max. Chrom.). The ¹H NMR analysis was performed on the hydrochloride salt of the title compound. The latter was preparedas follow: an excess of HCI in diethylether (5 mL, 1 M) was added to the title compound dissolved in DCM, the solid precipitated was filtered and dried to give the hydrochloride salt of the title compound. ¹H NMR (DMSO-d₆, 400 MHz) δ 9.81 (s, 1 H), 7.4 (t, *J* = 7.8 Hz, 1 H), 7.28 (d, *J* = 9.2 Hz, 1 H), 7.21 (s, 1 H), 7.14 (d, *J* = 7.8 Hz, 1 H), 3.91 (m, 1 H), 3.62 (m, 3H), 3.49-3.46 (m, 4H), 3.37 (m, 1 H), 3.12-3.04 (m, 4H), 2.72 (m, 1 H), 2.27 (m, 1 H), 2.11 (m, 2H), 1.79 (s, 3H), 1.27 (t, *J* = 6.6 Hz, 6H). LCMS (Method D): Mass found (M+ 466.0), Rt (min): 3.93, Area (%): 99.2 (Max. Chrom.), 98.9 (254 nm). HPLC (Method A): Rt (min) 4.05: Area (%): 98.1 (Max. Chrom.), 98.9 (254 nm). CHN (C₂₄H₃₀F₃N₃O₃ . 2 HCl . 0.5 H₂O; Calculated C Measured C 52.89, H 5.77, N 7.59).

The following Examples 102-106 were obtained following the same procedure described for Example 101, but starting from different aldehydes or ketones.

| **Ex.** | **Structure** | **Characterization** |
|---|---|---|
| 102 | 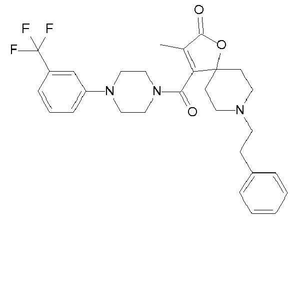 | **3-methyl-8-(2-phenylethyl)-4-({4-[3-(trifluoromethyl)phenyl]piperazin-1-yl}carbonyl)-1-oxa-8-azaspiro[4.5]dec-3-en-2-one** |
| | | Purification by flash chromatography (100% heptane to 30% heptane in ethyl acetate for 20 minutes). The compound was obtained as a white solid (51.9 mg). LCMS (MethodMethod I): Mass found (M+ 528.2); Rt (min): 4.00, Area (%): 98.4 (Max. Chrom.). |
| 103 | 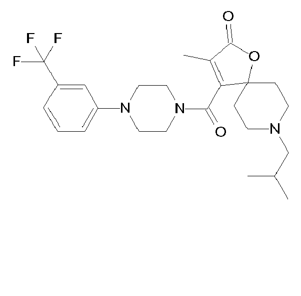 | **8-isobutyl-3-methyl-4-({4-[3-(trifluoromethyl)phenyl]piperazin-1-yl}carbonyl)-1-oxa-8-azaspiro[4.5]dec-3-en-2-one** |
| | | Purification by flash chromatography (100% heptane to 30% heptane in ethyl acetate for 20 minutes). The compound was obtained as a white solid (53.4 mg). LCMS (MethodMethod I): Mass found (M+ 480.2); Rt (min): 4.10, Area (%): 99.6 (Max. Chrom.). |
| 104 | 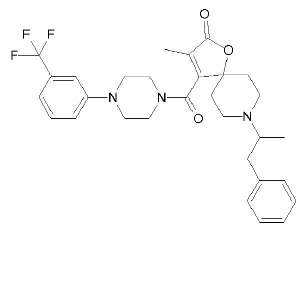 | **3-methyl-8-(1-methyl-2-phenylethyl)-4-({4-[3-(trifluoromethyl)phenyl]piperazin-1-yl}carbonyl)-1-oxa-8-azaspiro[4.5]dec-3-en-2-one** |
| | | Purification by flash chromatography (100% heptane to 30% heptane in ethyl acetate for 20 minutes). The compound was obtained as a white solid (29.7 mg). LCMS (MethodMethod I): Mass found (M+ 542.2); Rt (min): 4.16, Area (%): 97.7 (Max. Chrom.). |
| 105 | 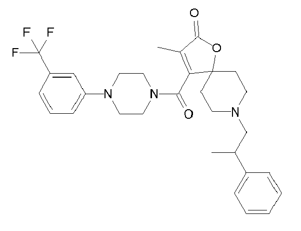 | **3-methyl-8-(2-phenylpropyl)-4-({4-[3-(trifluoromethyl)phenyl]piperazin-1-yl}carbonyl)-1-oxa-8-azaspiro[4.5]dec-3-en-2-one** |
| | | Purification by flash chromatography (100% heptane to 30% heptane in ethyl acetate for 20 minutes). The compound was obtained as a white solid (44.8 mg). LCMS (MethodMethod I): Mass found (M+ 542.2); Rt (min): 4.28, Area (%): 98.8 (Max. Chrom.). |
| 106 | 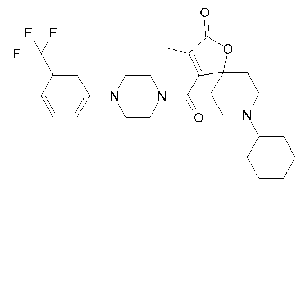 | **8-cyclohexyl-3-methyl-4-({4-[3-(trifluoromethyl)phenyl]piperazin-1-yl}carbonyl)-1-oxa-8-azaspiro[4.5]dec-3-en-2-one** |
| | | Purification by flash chromatography (100% heptane to 30% heptane in ethyl acetate for 20 minutes). The compound was obtained as a white solid (51.1 mg). LCMS (MethodMethod I): Mass found (M+ 506.2); Rt (min): 4.03, Area (%): 99.7 (Max. Chrom.). |
| 107 | 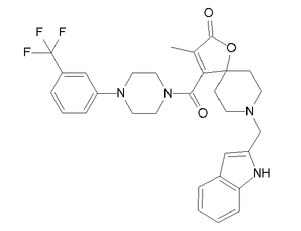 | **8-(1H-indol-2-ylmethyl)-3-methyl-4-({4-[3-(trifluoromethyl)phenyl]piperazin-1-yl}carbonyl)-1-oxa-8-azaspiro[4.5]dec-3-en-2-one** |
| | | Purification by flash chromatography (100% heptane to 30% heptane in ethyl acetate for 20 minutes). The compound was obtained as a white solid (41.4 mg). LCMS (MethodMethod I): Mass found (M+ 553.2); Rt (min): 3.99, Area (%): 99.8 (Max. Chrom.). |

### Example 108: 3-methyl-8-(2-oxo-2-phenylethyl)-4-({4-[3-(trifluoromethyl)phenyl]piperazin-1-yl}carbonyl)-1-oxa-8-azaspiro[4.5]dec-3-en-2-one

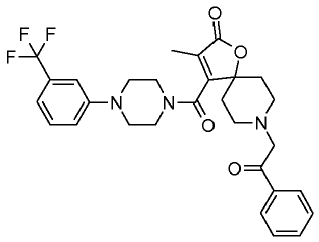

Sodium hydrogen carbonate (101 mg, 1.0 mmol) was added to a suspension of 3-methyl-4-({4-[3-(trifluoromethyl)phenyl]piperazin-1-yl}carbonyl)-1-oxa-8-azaspiro[4.5]dec-3-en-2-one, dihydrochloride salt (Example 1, 250 mg, 0.5 mmol) and 2-bromo-1-phenylethanone (100 mg, 0.5 mmol) in dichloromethane (3.0 mL). The reaction mixture was stirred at RT for 16 hours. It was then partitioned between dichloromethane and saturated aqueous sodium bicarbonate. The organic layer was separated and the aqueous layer was extracted with dichloromethane. The combined organic layers were dried over MgSO₄, filtered and concentrated. After purification by flash chromatography (silica; heptane/EtOAc gradient from 100:0 to 70:30), the title compound was obtained as a white solid. HPLC purity (Method H): Area: 96.9 % (Max. Chrom.), Rt: 3.86, (M+ 542.2).

### Example 109: 8-benzyl-3-methyl-4-({4-[3-(trifluoromethyl)phenyl]piperazin-1-yl}carbonyl)-1-oxa-8-azaspiro[4.5]dec-3-en-2-one

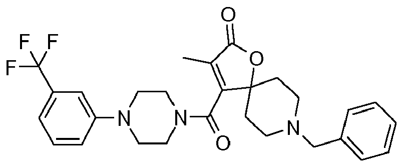

EDC.HCI (190 mg, 0.99 mmol) and HOBt (135 mg, 0.99 mmol) were added to a cold (0°C) solution of 1-(3-trifluoromethyl-phenyl)-piperazine (250 mg, 0.83 mmol) and 8-benzyl-3-methyl-2-oxo-1-oxa-8-azaspiro[4.5]dec-3-ene-4-carboxylic acid (intermediate 5, 192 mg, 0.83 mmol) in DMF (10 mL), followed by dropwise addition of trietylamine (335 mg, 3.32 mmol). The mixture was then stirred ar RT for 12 hours. The solvents were removed by evaporation. Water was added (10 mL) and the mixture was extracted with EtOAc (3 x). The combined organic layers were dried over MgSO₄, filtered and concentrated. After purification by flash chromatography (silica; DCM/MeOH 96:4), the title compound was obtained as a white powde . LCMS (Method JU): Mass found (M+ 514.4); Rt (min): 6.84, Area (%): 99.6. HPLC (Method G): Rt (min): 3.8, Area (%): 99.7.

The following Examples 110-111 were obtained following the same procedure as describe for Example 109, but starting from 1-(3-chloro-phenyl)-piperazine or 3-piperazin-1-yl-benzonitrile, respectively.

| **Ex.** | **Structure** | **Characterization** |
|---|---|---|
| 110 | 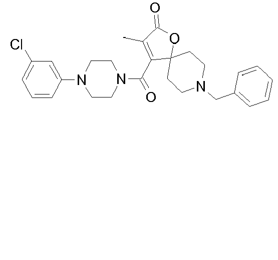 | **8-benzyl-4-{[4-(3-chlorophenyl)piperazin-1-yl]carbonyl}-3-methyl-1-oxa-8-azaspiro[4.5]dec-3-en-2-one** |
| | | The compound was obtained as a white powder (41 mg). LCMS (Method JU): Mass found (M+ 480.3); Rt (min): 6.75, Area (%): 99.3. HPLC (Method G): Rt (min): 3.59, Area (%): 95.6 |
| 111 | 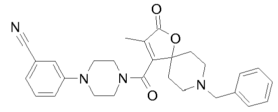 | **3-{4-[(8-benzyl-3-methyl-2-oxo-1-oxa-8-azaspiro[4.5]dec-3-en-4-yl)carbonyl]piperazin-1-yl}benzonitrile** |
| | | The compound was obtained as a white powder (40 mg). LCMS (Method JU): Mass found (M+ 471.3); Rt (min): 6.25, Area (%): 99.7. HPLC (Method G): Rt (min): 3.06, Area (%): 99.0. |

### Example 112: 4-{[4-(3-chlorophenyl)piperazin-1-yl]carbonyl}-3,8-dimethyl-1-oxa-8-azaspiro[4.5]dec-3-en-2-one

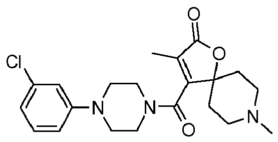

EDC.HCI (813 mg, 4.26 mmol) and HOBt (575 mg, 4.26 mmol) were added to a cold (0°C) solution of 1-(3-chloro-phenyl)-piperazine (476 mg, 1.7 mmol) and 3,8-dimethyl-2-oxo-1-oxa-8-azaspiro[4.5]dec-3-ene-4-carboxylic acid (Intermediate 6, 800 mg, 3.55 mmol) in DMF (20 mL), followed by dropwise addition of trietylamine (1.9 mL). The reacton mixture was then stirred ar RT for 12 hours. The solvents were removed by evaporation. Water was added (10 mL) and the mixture was extracted with EtOAc (3 x). The combined organic layers were dried over MgSO₄, filtered and concentrated. After purification by flash chromatography (silica; DCM/MeOH 97:3), the title compound was obtained as a white foam. The crude mixture was purified by Mass directed purification system to yield the title compound as a white powder. LCMS (Method JU): Mass found (M+ 404.3); Rt (min): 5.30, Area (%): 95.5. HPLC (Method G): Rt (min): 2.99, Area (%): 95.0.

The following Examples 113-114 were obtained following the same procedure as described for Example 112, but starting from 1-(4-trifluoromethyl-phenyl)-piperazine or 1-(3,5-dichloro-phenyl)-piperazine, respectively and Intermediate 1.

| **Ex.** | **Structure** | **Characterization** |
|---|---|---|
| 113 | 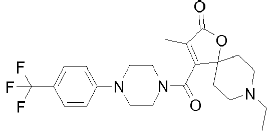 | **8-ethyl-3-methyl-4-({4-[4-(trifluoromethyl)phenyl]piperazin-1-yl}carbonyl)-1-oxa-8-azaspiro[4.5]dec-3-en-2-one** |
| | | The compound was obtained as a white powder (16 mg). LCMS (Method JU): Mass found (M+ 452.4); Rt (min): 5.66, Area (%): 96.1. HPLC (Method G): Rt (min): 3.44, Area (%): 96.9. |
| 114 | 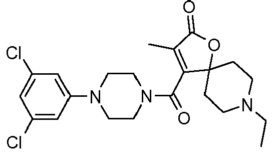 | **4-{[4-(3,5-dichlorophenyl)piperazin-1-yl]carbonyl}-8-ethyl-3-methyl-1-oxa-8-azaspiro[4.5]dec-3-en-2-one** |
| | | Purification by chromatography (5% MeOH in DCM). The compound was obtained as a yellow powder (20 mg). LCMS (Method JU): Mass found (M+ 452.3); Rt (min): 6.13, Area (%): 94.8. HPLC (Method G): Rt (min): 3.8, Area (%): 95.4. |

### Example 115: 8-isopropyl-3-methyl-4-{[3-(trifluoromethyl)-7,8-dihydro-1,6-naphthyridin-6(5H)-yl]carbonyl}-1-oxa-8-azaspiro[4.5]dec-3-en-2-one

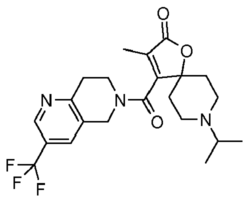

A suspension of 3-methyl-4-{[3-(trifluoromethyl)-7,8-dihydro-1,6-naphthyridin-6(5H)-yl]carbonyl}-1-oxa-8-azaspiro[4.5]dec-3-en-2-one hydrochloride (Example 78, 300 mg, 0.68 mmol), K₂CO₃ (354 mg, 2.56 mmol) and 2-iodopropane (131 mg, 0.77 mmol) in CH₃CN (6.4 mL) was stirred overnight at room temperature. Additional 1.2 eq of 2-iodopropane (131 mg, 0.77 mmol) were added and the mixture heated in MW at 100°C for 1 hour. The solvent was evaporated and the residue partitioned between EtOAc and water. The organic layer was dried over Na₂SO₄, filtered and concentrated. After purification by flash chromatography (silica; DCM/MeOH 97:3), the title compound was obtained as a white foam (195 mg, 70 %). ¹H-NMR (DMSO-d₆, 300 MHz) δ 8.75 (s, 1H), 8.09 (s, 1 H), 4.84 (brs, 2H), 3.93 (brs, 2H), 3.08 (t, 2H), 2.62-2.83 (m, 3H), 2.43 (td, 2H), 2.01 (brs, 2H), 1.74 (brs, 3H), 1.51-1.70 (m, 2H), 0.98 (d, 6H). LCMS (Method D): Mass found (M+ 438.3); Rt (min): 5.9, Area (%): 98.0.

### Example 116: 8-(2-fluoroethyl)-3-methyl-4-{[3-(trifluoromethyl)-7,8-dihydro-1,6-naphthyridin-6(5H)-yl]carbonyl}-1-oxa-8-azaspiro[4.5]dec-3-en-2-one

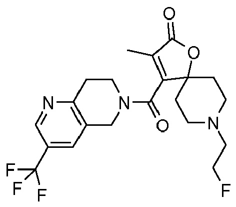

A suspension of 3-methyl-4-{[3-(trifluoromethyl)-7,8-dihydro-1,6-naphthyridin-6(5H)-yl]carbonyl}-1-oxa-8-azaspiro[4.5]dec-3-en-2-one hydrochloride (Example 78, 187 mg, 0.4 mmol), 1-bromo-2-fluoro-ethane (61 mg, 0.48 mmol) and potassium carbonate (221 mg, 1.6 mmol) in acetonitrile (4.0 mL) was heated in MW at 80°C for 1 hour. Additional 1-bromo-2-fluoro-ethane (61 mg, 0.48 mmol) was added and the mixture was heated in MW at 100°C for another 1 hour. Additional 1-bromo-2-fluoroethane (122 mg, 0.94 mmol) were added and the mixture was heated in MW at 100°C for futher 3 hours. The solvent was evaporated and the residue was partitioned between ethyl acetate and water. The organic layer was dried over Na₂SO₄, filtered and concentrated. After purification by flash chromatography (silica; DCM/MeOH 97:3), the title compound was obtained as a white foam. LCMS ¹H-NMR (DMSO-d₆, 300 MHz) δ 8.78 (brs, 1 H), 8.07 (s, 1 H), 4.92 (d, 1 H), 4.79 (s, 1 H), 4.49-4.65 (m, 1 H), 4.33-4.49 (m, 1 H), 3.88-4.16 (m, 1 H), 3.82 (t, 1 H), 3.04 (dt, 2H), 2.87 (d, 2H), 2.64-2.72 (m, 1 H), 2.55-2.64 (m, 1 H), 2.16-2.39 (m, 3H), 1.72-1.97 (m, 2H), 1.66 (s, 3H), 1.39-1.60 (m, 1 H). (Method D): Mass found (M+ 442.2); Rt (min): 8.0, Area (%): 95.1.

### Example 117: 7-isopropyl-3-methyl-4-({4-[3-(trifluoromethyl)phenyl]piperazin-1-yl}carbonyl)-1-oxa-7-azaspiro[4.4]non-3-en-2-one

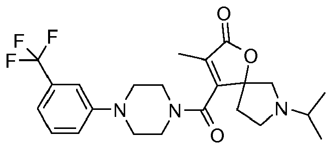

A mixture of 3-methyl-4-({4-[3-(trifluoromethyl)phenyl]piperazin-1-yl}carbonyl)-1-oxa-7-azaspiro[4.4]non-3-en-2-one, dihydrochloride salt (Intermediate 18, 170 mg, 0.35 mmol), K₂CO₃ (195 mg, 1.4 mmol) and 2-iodo propane (120 mg, 0.71 mmol) in CH₃CN, was heated in MW at 100°C for 1 hour. The resulting slurry was partitioned between water and EtOAc. The organic layer was dried over Na₂SO₄, filtered and concentrated. After purification by flash chromatography (silica; DCM/MeOH/NH4OH 98:2:0.2), the title compound was obtained as a pale yellow foam. ¹H-N MR (DMSO-d₆, 300 MHz) δ 7.46 (t, 1 H), 7.24-7.30 (m, 1 H), 7.20-7.23 (m, 1 H), 7.13 (d, 1 H), 3.76 (t, 2H), 3.52-3.64 (m, 2H), 3.30-3.44 (m, H), 3.10-3.24 (m, 2H), 2.78-2.93 (m, 2H), 2.56-2.63 (m, 3H), 2.35-2.46 (m, 1 H), 1.89-2.11 (m, 1 H), 1.79 (s, 3H), 0.99 (6, 6H). LCMS (Method D): Mass found (M+ 452.2); Rt (min): 8.3, Area (%): 96.7.

### Example 118: 7-ethyl-3-methyl-4-({4-[3-(trifluoromethyl)phenyl]piperazin-1-yl}carbonyl)-1-oxa-7-azaspiro[4.4]non-3-en-2-one

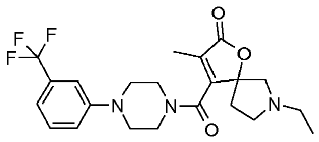

A solution of sodium cyanoborohydride (33 mg, 0.52 mmol), 3-methyl-4-({4-[3-(trifluoromethyl)phenyl]piperazin-1-yl}carbonyl)-1-oxa-7-azaspiro[4.4]non-3-en-2-one, dihydrochloride salt (Intermediate 18, 154 mg, 0.35 mmol), acetaldehyde (30.4 mg, 0.69 mmol) and acetic acid (0.30 mL; 5.2 mmol) was stirred for 8 hours at 0°C, then at 4°C overnight. The solvent was evaporated and the crude was taken up with DCM. Organiclayer was washed with water, dried over Na₂SO₄, filtered and concentrated. and evaporated to dryness. After purification by flash chromatography (silica; DCM/MeOH/NH4OH 96:4:0.2), the title compound was obtained as a white solid. ¹H-NMR (DMSO-d₆, 300 MHz) δ 7.37-7.53 (m, 1 H), 7.21-7.29 (m, 1 H), 7.17-7.21 (m, 1 H), 7.07-7.16 (m, 1 H), 3.68 (brs, 4H), 3.23-3.42 (m, 4H), 2.77-2.89 (m, 2H), 2.54-2.67 (m, 2H), 2.35 -2.47 (m, 3H), 1.95-2.13 (m, 1 H), 1.81 (s, 3H), 1.02 (t, 3H). LCMS (Method D): Mass found (M+ 438.3); Rt (min): 6.59, Area (%): 98.0.

### Example 119: 3-methyl-8-(tetrahydro-2H-pyran-4-yl)-4-{[3-(trifluoromethyl)-7,8-dihydro-1,6-naphthyridin-6(5H)-yl]carbonyl}-1-oxa-8-azaspiro[4.5]dec-3-en-2-one

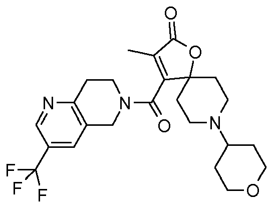

A mixture 3-methyl-4-{[3-(trifluoromethyl)-7,8-dihydro-1,6-naphthyridin-6(5H)-yl]carbonyl}-1-oxa-8-azaspiro[4.5]dec-3-en-2-one hydrochloride (Example 78, 299 mg, 0.64 mmol), dihydro-2H-pyran-4(3H)-one (96 mg, 0.96 mmol) and NaBH(OAc)₃ (271 mg, 1.28 mmol) in dry THF (6.4 mL) in presence of a catalytic amount of AcOH was stirred 24 hours at room temperature. Additional 0.5 eq of dihydro-2H-pyran-4(3H)-one (29 µL, 0.5 eq) and NaBH(OAc)₃ (135 mg, 1 eq) were added and the reaction mixture was heated to 40°C for 6 hours. It was then quenched with water and basified by addition of saturated NaHCO₃. The aqueous layer was extracted twice with EtOAc and the combined organic layers were dried over Na₂SO₄, filtrated and concentrated. After purification by flash chromatography (silica; DCM/MeOH; 94:6), the title compound was obtained as a white solid. ¹H-NMR (DMSO-d₆, 300 MHz,) δ 8.78 (brs, 1 H), 8.07 and 8.20 (s, 1 H), 4.67-5.00 (m, 2H), 3.82 and 4.00 (t, 2H), 3.76-3.92 (m, 2H), 3.16-3.26 (m, 3H), 2.94-3.13 (m, 2H), 2.69-2.93 (m, 2H), 2.03-2.46 (m, 4H), 1.64 and 1.79 (s, 3H), 1.29-1.70 (m, 6H). LCMS (Method D): Mass found (M+ 480.2); Rt (min): 6.4, Area (%): 96.9. ¹H NMR (300 MHz, DMSO-d6) δ 8.79 (s, 1 H), 8.14 (d, *J* = 45.3 Hz, 1 H), 4.92 (d, *J* = 15.9 Hz, 1 H), 4.79 (s, 1 H), 4.11 - 3.94 (m, 1 H), 3.93 - 3.67 (m, 3H), 3.25 (t, *J* = 12.2 Hz, 2H), 3.07 (t, *J* = 5.7 Hz, 1 H), 3.00 (t, *J* = 5.6 Hz, 1 H), 2.84 (s, 2H), 2.43 - 2.08 (m, 3H), 1.79 (s, 2H), 1.73 - 1.27 (m, 8H).

### Example 120: 8-ethyl-3-methyl-4-{[7-(trifluoromethyl)-3,4-dihydroisoquinolin-2(1H)-yl]carbonyl}-1-oxa-8-azaspiro[4.6]undec-3-en-2-one

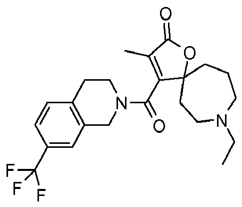

3-methyl-4-({4-[3-(trifluoromethyl)phenyl]piperazin-1-yl}carbonyl)-1-oxa-8-azaspiro[4.6]undec-3-en-2-one, dihydrochloride salt (Intermediate 19, 185 mg, 0.42 mmol), acetaldehyde (37 mg, 0.83 mmol) and sodium triacetoxyborohydride (177 mg, 0.84 mmol) in DCM (3.0 mL) and acetic acid (0.100 mL) was stirred for 1 hour ar 0°C. The reaction mixture was then quenched with water and basified by addition of saturated NaHCO₃. The aqueous layer was extracted twice with DCM and the combined organic layers were dried over Na₂SO₄, filtrated and concentrated. After purification by flash chromatography (silica; DCM/MeOH; gradient from 94:6 to 92:8), the title compound was obtained as a white foam (148 mg, 78 %). ¹H-NMR (DMSO-d₆, 300 MHz) δ 7.64-7.78 (m, 1 H), 7.49-7.61 (m, 1 H), 7.35-7.49 (m, 1 H), 4.63-5.10 (m, 2H), 3.61-4.06 (m, 2H), 2.82-3.07 (m, 2H), 2.54-2.74 (m, 3H), 2.15-2.46 (m, 3H), 1.79-2.04 (m, 6H), 1.62 and 1.75 (s, 3H), 0.95 (t, 3H). LCMS (Method D): Mass found (M+ 437.2); Rt (min): 7.9, Area (%): 95.5.

### Example 121: 8-ethyl-3-methyl-4-({4-[3-(trifluoromethyl)phenyl]piperazin-1-yl}carbonyl)-1-oxa-8-azaspiro[4.6]undec-3-en-2-one

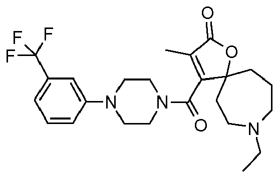

A mixture of 3-methyl-4-({4-[3-(trifluoromethyl)phenyl]piperazin-1-yl}carbonyl)-1-oxa-8-azaspiro[4.6]undec-3-en-2-one, dihydrochloride salt (Intermediate 19, 144 mg, 0.28 mmol), acetaldehyde (32 µL, 0.56 mmol) and NaBH(OAc)₃ (117 mg, 0.56 mmole) in DCM (3.0 mL) in presence of a catalytic amount of AcOH was stirred 1 hour at 0°C. The reaction mixture was then quenched with water and basified by addition of saturated NaHCO₃. The aqueous layer was extracted twice with DCM and the combined organic layers were dried over Na₂SO₄, filtrated and concentrated. After purification by flash chromatography (silica; DCM/MeOH; gradient from 94:6 to 92:8), the title compound was obtained as a white foam. 1 H-NMR (DMSO-d₆, 300 MHz) δ 7.46 (t, 1 H), 7.23-7.30 (m, 1 H), 7.21 (t, 1 H), 7.06-7.17 (m, 1 H), 3.77 (br. s., 2H), 3.58 (t, 2H), 3.31-3.42 (m, 2H), 3.10-3.24 (m, 2H), 2.55-2.71 (m, 4H), 2.46 (q, 2H), 1.77-2.05 (m, 4H), 1.75 (s, 3H), 1.42-1.70 (m, 2H), 0.96 (t, 3H). LCMS (Method D): Mass found (M+ 466.2); Rt (min): 8.2, Area (%): 98.7.

### Example 122: 3,8-dimethyl-4-{[3-(trifluoromethyl)-7,8-dihydro-1,6-naphthyridin-6(5H)-yl]carbonyl}-1-oxa-8-azaspiro[4.5]dec-3-en-2-one

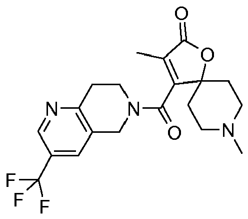

A suspension of 3-methyl-4-{[3-(trifluoromethyl)-7,8-dihydro-1,6-naphthyridin-6(5H)-yl]carbonyl}-1-oxa-8-azaspiro[4.5]dec-3-en-2-one hydrochloride (Example 78, 190 mg, 0.41 mmol), formaldehyde 37% in water (66 mg, 0.81 mmol), sodium triacetoxyborohydride (172 mg, 0.81 mmol) in DCM (4.0 mL) and acetic acid (0.100 mL) were stirred at 0°C. After 1.5 hours, additional sodium triacetoxyborohydride (172 mg, 0.81 mmol) and formaldehyde 37% in water (65.9 mg, 0.81 mmol) were added and the reaction mixture was stirred at 0°C for an additional 2 hours. The reaction mixture was then quenched with water and basified by addition of a saturated solution of NaHCO₃ The aqueous layer was extracted twice with DCM and combined organic layers were dried over Na₂SO₄, filtrated and concentrated.. After purification by flash chromatography (silica; DCM/MeOH; 93:7), the title compound was obtained as a white foam (122 mg, 73 %). ¹H-NMR (DMSO-d₆, 300 MHz) δ 8.79 (s, 1 H), 8.06 and 8.20 (d, 1 H), 4.71-5.03 (m, 2H), 3.67-4.17 (m, 2H), 3.01 and 3.07 (t, 2H), 2.56-2.80 (m, 2H), 2.16 and 2.19 (s, 3H), 2.00-2.15 (m, 4H), 1.66 and 1.79 (s, 3H), 1.36-1.65 (m, 2H). LCMS (Method D): Mass found (M+ 410.2); Rt (min): 6.9, Area (%): 97.0.

Example CD1 and CD2 were purchased from ChemDiv, Inc. - 6605 Nancy Ridge Dr San Diego, CA, 92121, USA - (cat. number E616-0377 and E616-0492, respectively).

| **Ex.** | **Structure** | **Name** |
|---|---|---|
| CD1 | 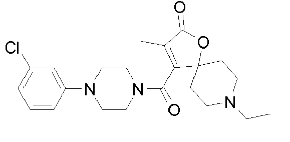 | 4-{[4-(3-chlorophenyl)piperazin-1-yl]carbonyl}-8-ethyl-3-methyl-1-oxa-8-azaspiro[4.5]dec-3-en-2-one |
| CD2 | 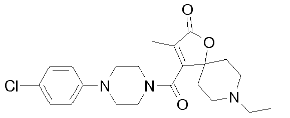 | 4-{[4-(4-chlorophenyl)piperazin-1-yl]carbonyl}-8-ethyl-3-methyl-1-oxa-8-azaspiro[4.5]dec-3-en-2-one |

**The following Examples can be made following the above procedures described for the Examples 1-122**

| **Ex.** | **Structure** | **Name** |
|---|---|---|
| 123 | 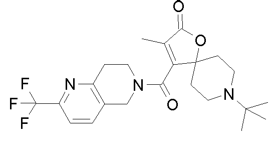 | 8-tert-butyl-3-methyl-4-{[2-(trifluoromethyl)-7,8-dihydro-1,6-naphthyridin-6(5H)-yl]carbonyl}-1-oxa-8-azaspiro[4.5]dec-3-en-2-one |
| 124 | 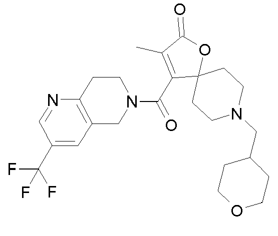 | 3-methyl-8-(tetrahydro-2H-pyran-4-ylmethyl)-4-{[3-(trifluoromethyl)-7,8-dihydro-1,6-naphthyridin-6(5H)-yl]carbonyl}-1-oxa-8-azaspiro[4.5]dec-3-en-2-one |
| 125 | 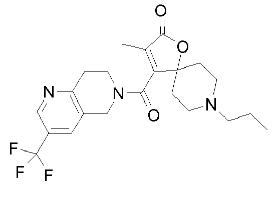 | 3-methyl-8-propyl-4-{[3-(trifluoromethyl)-7,8-dihydro-1,6-naphthyridin-6(5H)-yl]carbonyl}-1-oxa-8-azaspiro[4.5]dec-3-en-2-one |
| 126 | 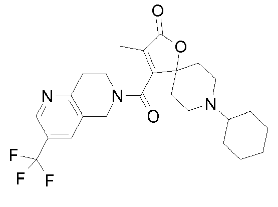 | 8-cyclohexyl-3-methyl-4-{[3-(trifluoromethyl)-7,8-dihydro-1,6-naphthyridin-6(5H)-yl]carbonyl}-1-oxa-8-azaspiro[4.5]dec-3-en-2-one |
| 127 | 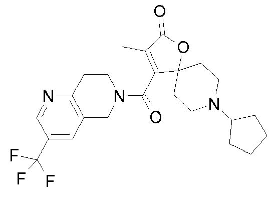 | 8-cyclopentyl-3-methyl-4-{[3-(trifluoromethyl)-7,8-dihydro-1,6-naphthyridin-6(5H)-yl]carbonyl}-1-oxa-8-azaspiro[4.5]dec-3-en-2-one |
| 128 | 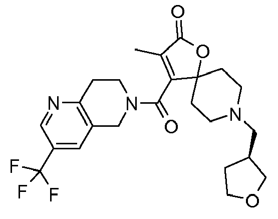 | 3-methyl-8-[(3S)-tetrahydrofuran-3-ylmethyl]-4-{[3-(trifluoromethyl)-7,8-dihydro-1,6-naphthyridin-6(5H)-yl]carbonyl}-1-oxa-8-azaspiro[4.5]dec-3-en-2-one |
| 129 | 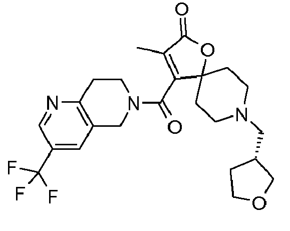 | 3-methyl-8-[(3 R)-tetrahydrofuran-3-yl methyl]-4-{[3-(trifluoromethyl)-7,8-dihydro-1,6-naphthyridin-6(5H)-yl]carbonyl}-1-oxa-8-azaspiro[4.5]dec-3-en-2-one |
| 130 | 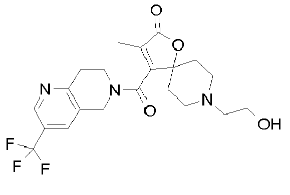 | 8-(2-hydroxyethyl)-3-methyl-4-{[3-(trifluoromethyl)-7,8-dihydro-1,6-naphthyridin-6(5H)-yl]carbonyl}-1-oxa-8-azaspiro[4.5]dec-3-en-2-one |
| 131 | 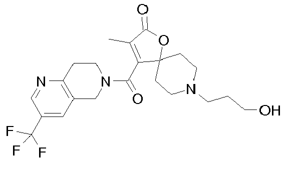 | 8-(3-hydroxypropyl)-3-methyl-4-{[3-(trifluoromethyl)-7,8-dihydro-1,6-naphthyridin-6(5H)-yl]carbonyl}-1-oxa-8-azaspiro[4.5]dec-3-en-2-one |
| 132 | 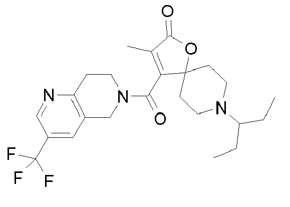 | 8-(1-ethylpropyl)-3-methyl-4-{[3-(trifluoromethyl)-7,8-dihydro-1,6-naphthyridin-6(5H)-yl]carbonyl}-1-oxa-8-azaspiro[4.5]dec-3-en-2-one |
| 133 | 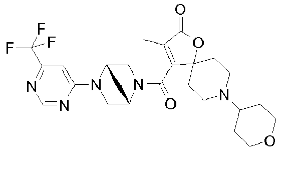 | 3-methyl-8-(tetrahydro-2H-pyran-4-yl)-4-({(1S,4S)-5-[6-(trifluoromethyl)pyrimidin-4-yl]-2,5-diazabicyclo[2.2.1]hept-2-yl}carbonyl)-1-oxa-8-azaspiro[4.5]dec-3-en-2-one |
| 134 | 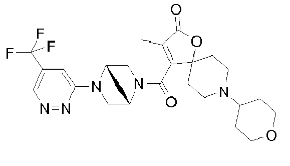 | 3-methyl-8-(tetrahydro-2H-pyran-4-yl)-4-({(1S,4S)-5-[5-(trifluoromethyl)pyridazin-3-yl]-2,5-diazabicyclo[2.2.1]hept-2-yl}carbonyl)-1-oxa-8-azaspiro[4.5]dec-3-en-2-one |
| 135 | 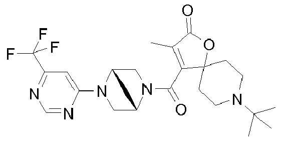 | 8-tert-butyl-3-methyl-4-({(1S,4S)-5-[6-(trifluoromethyl)pyrimidin-4-yl]-2,5-diazabicyclo[2.2.1]hept-2-yl}carbonyl)-1-oxa-8-azaspiro[4.5]dec-3-en-2-one |
| 136 | 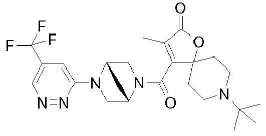 | 8-tert-butyl-3-methyl-4-({(1S,4S)-5-[5-(trifluoromethyl)pyridazin-3-yl]-2,5-diazabicyclo[2.2.1]hept-2-yl}carbonyl)-1-oxa-8-azaspiro[4.5]dec-3-en-2-one |

### Example 137: In vitro assays

### A. Binding assay:

Membranes were prepared from CHO cells expressing mCCR2b or hCCR2b for use in ligand binding studies. Cells were suspended in 50mM Tris-HCL, pH 7.4, 2 mM EDTA, 250 mM Sucrose and 1x Complete EDTA-free protease inhibitor cocktail (Roche) (Buffer A), and disrupted at 4°C by N₂ decompression using a cell disruption bomb (Parr Instrument). Following centrifugation at 200 RCF for 10 min at 4°C, the supernatant was suspended in buffer A and centrifuged again at 19000 RPM (RCSC rotor SS34 code 05 Sorvall Instruments DUPONT) for 90 min at 4°C. The pellet was then suspended in 10 mM HEPES, pH 7.4, 1 mM EDTA, 250 mM Sucrose, and 1x Complete EDTA-free protease inhibitor cocktail (Buffer B) and homogenized using a potter. Membranes were flash frozen in liquid N₂ and stored at -80°C.
[125I] Monocyte Chemoattractant Protein -1 (MCP-1) (25µCi; Perkin Elmer) was added to test compounds in DMSO (1% final). Membranes and WGA SPA beads (GE Healthcare) were added to give a final volume of 100 µl in 96-well plates with assay concentrations of 50 pM or 100 pM [125I]Monocyte Chemoattractant Protein -1 (respectively for hCCR2b or mCCR2b), 50 mM HEPES, pH 7.4, 5 mM MgCl₂, 1 mM CaCl₂, 0.1% BSA, 1-5 µg/well of membranes and 100 µg/well of WGA SPA beads. Binding was performed for 90 min at RT with strong agitation of speed 5 (Heidolph Titramax 100 SCITEC) and bound radioactivity was measured on a PerkinElmer 1450 MicroBeta counter. Specific binding was calculated by subtracting remaining radioactivity in the presence of 1000-fold excess of unlabeled compound.
Binding data were analyzed using the Genedata Screener® software.

### B. Chemotaxis:

L1.2 cells, a murine pre-LB cellline, were transfected with the human CCR2b receptor for use in the transmigration of L1.2 hCCR2b on the chemotaxis system: ChemotX® provided by Neuroprobes (8µm pore size). Monocyte Chemoattractant Protein-1 (MCP-1) stimulation leads to activate the CCR2 receptor and inititates a cascade of events including chemotaxis. MCP-1 ligand will be replaced by MCP-1 P8A protein with genetic modifications to prevent the oligomerization of the protein.
L1.2-hCCR2b are incubated in white RPMI 1640, 5% v/v Heat inactivated FCS, 1% v/v penicillin-streptomycin supplemented with 5 mM Butyrate overnight to enhance receptor expression level at the cell surface.
L1.2-hCCR2b cells and compounds in DMSO (1% final) are preincubated 15 min in chemotaxis medium and placed in the Upper Chamber of the chemotaxis system. The MCP-1 P8A initially present at its EC80 (concentration of MCP-1 P8A which allows 80% of cell migration) in the Lower Chamber (with compounds to avoid any compound gradient) diffuses through the pores of the membrane and interacts with the CCR2 receptor of cells present in the upper chamber. The tested compounds can also potentially interact with CCR2 receptor and therefore inhibit the migration of the cells from the Upper Chamber to the Lower Chamber. After a 4-hours incubation time at 37°C, 5% CO₂, a quantification of the cells which have migrated is performed using the Cyquant (Invitrogen) and a fluorescent reader as a Fluostar (exc: 485nm; emm: 520 nm). Specific migration is assessed by substracting the basal migration of cells without any stimulation. Chemotaxis data were analyzed using the Genedata Screener® software.

### C. Whole blood assay:

### Preparation of Buffer:

FACS buffer: PBS/ 0.1% BSA/ 0.01 % azide.
Ice cold fixative: 0.25% formaldehyde qsp PBS 1X.
Lysis solution: 0.15 M NH₄Cl, 10 mM sodium bicarbonate, and 1 mM EDTA.

### Preparation of dose response (DR):

Dilute CCR2 antagonists (stock 10mM in 100% DMSO) 1/10 in 100% DMSO in well A1 and perform a 1/4 serial dilution in 20µl DMSO 100% in wells A2 to A8.

Further dilute the dose response 1/40 in PBS 1X.

### Preparation of whole blood assay:

50 µL of blood was transferred per well in a 96-well 2ml plate and 2 µL of the antagonist dose response was added to the blood (in 0.1% DMSO final). An incubation for 30 minutes at room temperature was performed, followed by an incubation for 10 minutes at 37°C with addition of 2 µL of 250 nM Alexa647-MCP1 (ALMAC GROUP LTD, Almac House, 20 Seagoe Industrial Estate, Craigavon, BT63 5QD, 10 nM final) per well. The samples were then placed on ice and the cells were fixed with 125 µL of ice cold light fixative (0.25% formaldehyde qsp PBS 1X) for 1 min. Red blood cells were lysed by addition of 500 µL of ice cold lysis solution (0.15 M NH₄Cl, 10 mM sodium bicarbonate, and 1 mM EDTA) followed by an incubation on ice for 20 minutes. After complete lysis of red blood cells, the plate was centrifuged at 470 x g for 5 min, the supernatant was discarded and the pellet was resuspended in 500 µL of PBS. This step was repeated twice and the cells were resuspended in 100 µL of FACS buffer (PBS/ 0.1 % BSA/ 0.01 % azide) and run though a Flow cytometer to quantify the percentage of monocytes that were Alexa647-MCP1+.

Results are summarized in table 1 below:

| **EX** | **Structure** | **Binding hCCR2 (Ki)** | **Chemotaxis (Ki)** |
|---|---|---|---|
| **1** | 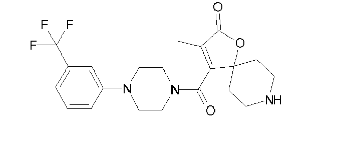 | ++ | n.d. |
| **2** | 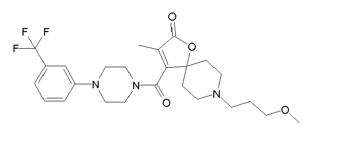 | ++++ | n.d. |
| **3** | 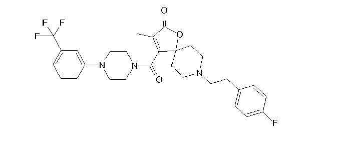 | ++++ | n.d. |
| **4** | 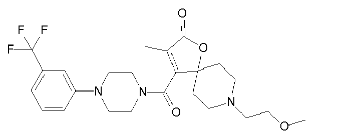 | ++++ | n.d. |
| **5** | 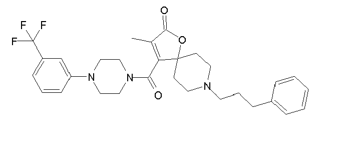 | ++++ | n.d. |
| **6** | 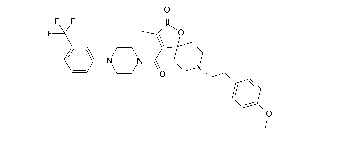 | ++++ | n.d. |
| **7** | 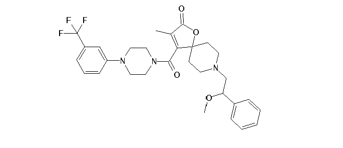 | +++ | n.d. |
| **8** | 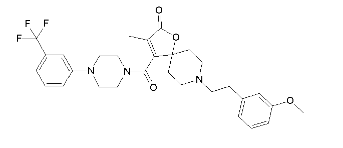 | ++++ | n.d. |
| **9** | 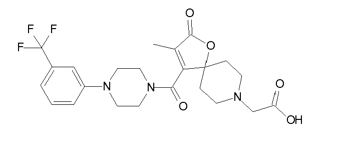 | ++ | n.d. |
| **10** | 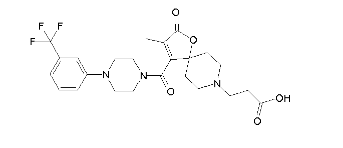 | ++ | n.d. |
| **11** | 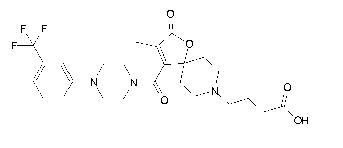 | ++ | n.d. |
| **12** | 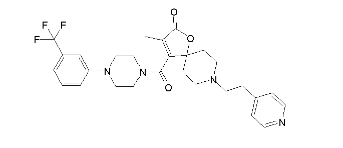 | +++ | n.d. |
| **13** | 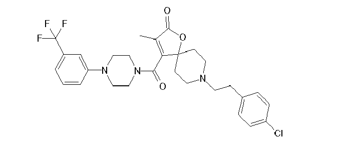 | ++++ | n.d. |
| **14** | 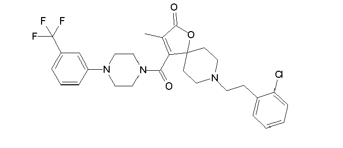 | ++ | n.d. |
| **15** | 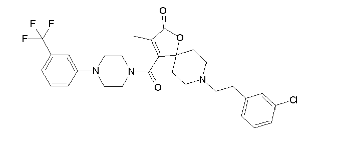 | ++++ | n.d. |
| **16** | 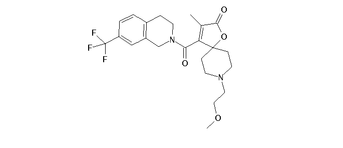 | ++++ | n.d. |
| **17** | 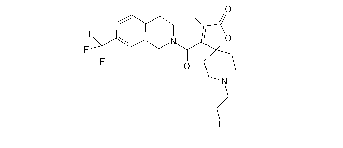 | ++++ | n.d. |
| **18** | 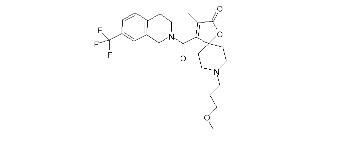 | ++++ | n.d. |
| **19** | 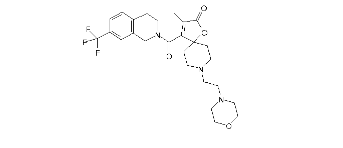 | ++++ | n.d. |
| **20** | 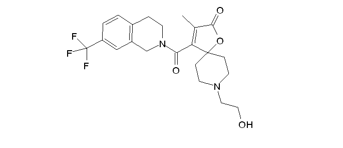 | ++++ | n.d. |
| **21** | 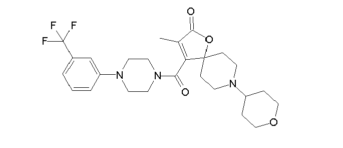 | ++++ | n.d. |
| **22** | 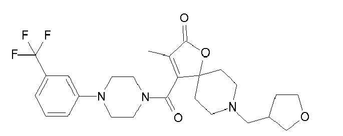 | ++++ | n.d. |
| **23** | 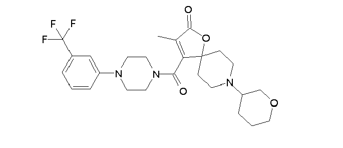 | ++++ | n.d. |
| **24** | 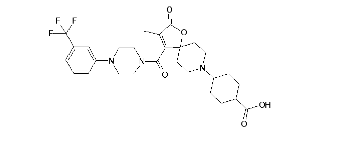 | +++ | n.d. |
| **25** | 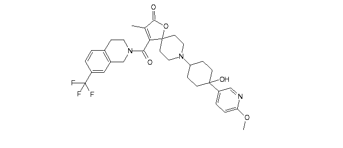 | ++++ | n.d. |
| **26** | 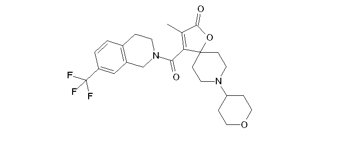 | ++++ | ++++ |
| **27** | 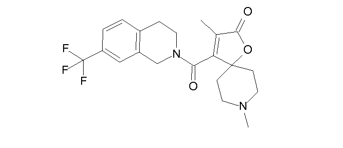 | ++++ | n.d. |
| **28** | 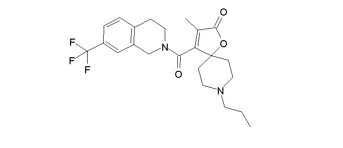 | ++++ | n.d. |
| **29** | 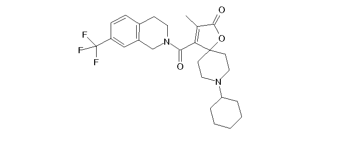 | ++++ | n.d. |
| **30** | 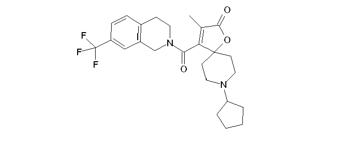 | ++++ | n.d. |
| **31** | 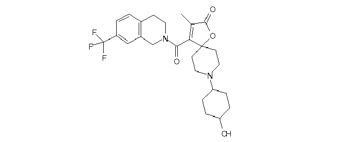 | ++++ | n.d. |
| **32** | 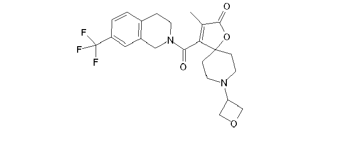 | +++ | n.d. |
| **33** | 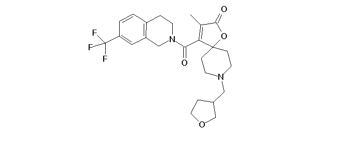 | ++++ | n.d. |
| **34** | 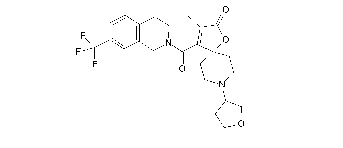 | ++++ | n.d. |
| **35** | 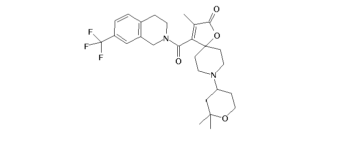 | ++++ | n.d. |
| **36** | 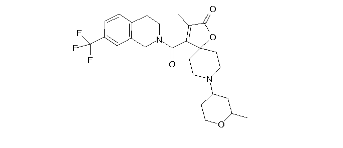 | ++++ | n.d. |
| **37** | 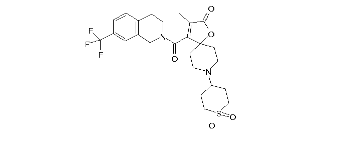 | +++ | n.d. |
| **38** | 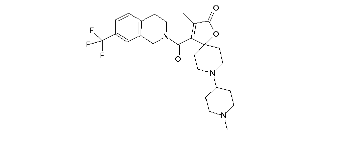 | ++ | n.d. |
| **39** | 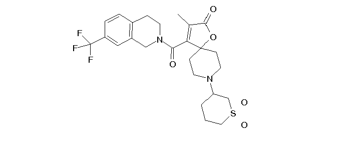 | +++ | n.d. |
| **40** | 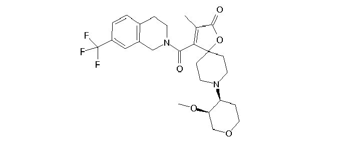 | ++++ | n.d. |
| **41** | 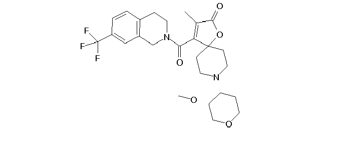 | ++++ | n.d. |
| **42** | 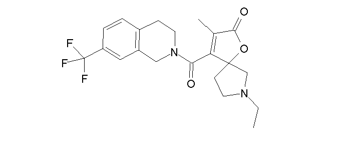 | ++++ | n.d. |
| **44** | 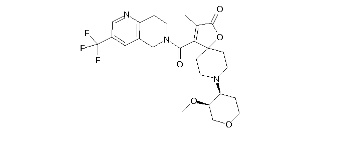 | ++ | n.d. |
| **45** | 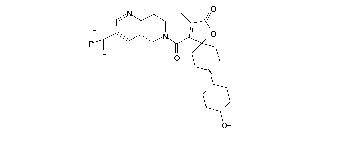 | ++++ | n.d. |
| **46** | 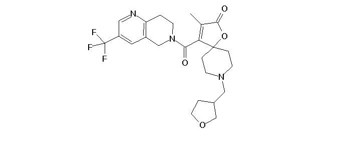 | ++++ | n.d. |
| **47** | 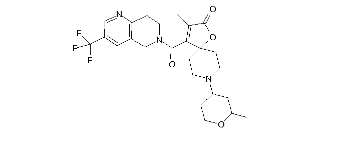 | ++++ | n.d. |
| **48** | 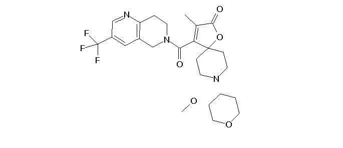 | ++ | n.d. |
| **49** | 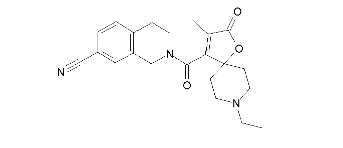 | ++ | n.d. |
| **50** | 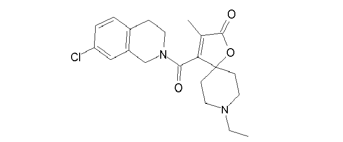 | ++++ | n.d. |
| **51** | 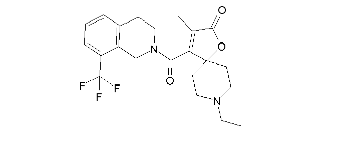 | + | n.d. |
| **52** | 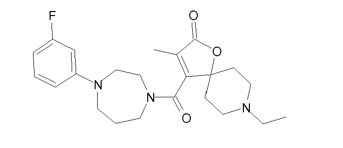 | + | n.d. |
| **53** | 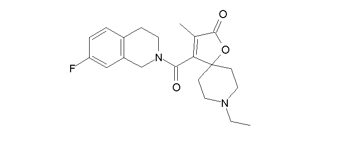 | ++ | n.d. |
| **54** | 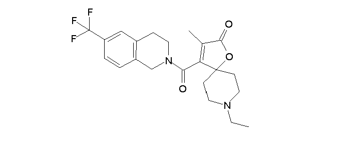 | ++++ | n.d. |
| **55** | 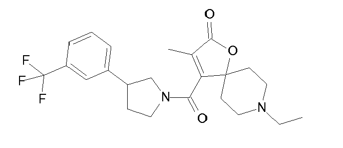 | ++ | n.d. |
| **56** | 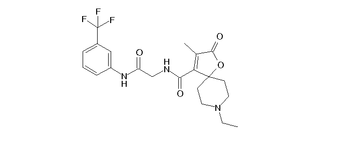 | ++++ | n.d. |
| **58** | 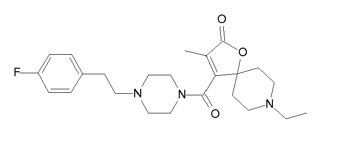 | ++ | n.d. |
| **59** | 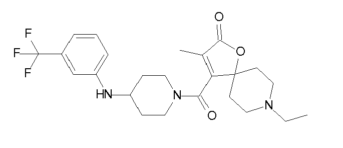 | + | n.d. |
| **60** | 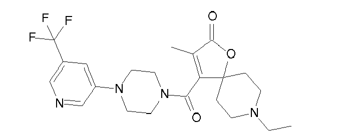 | +++ | n.d. |
| **62** | 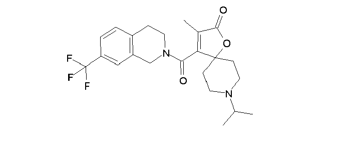 | ++++ | ++++ |
| **63** | 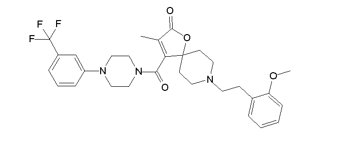 | ++++ | n.d. |
| **64** | 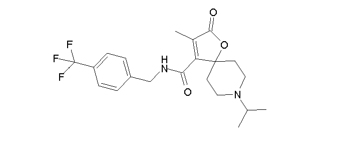 | + | n.d. |
| **65** | 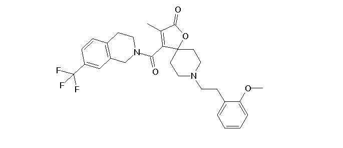 | ++++ | n.d. |
| **66** | 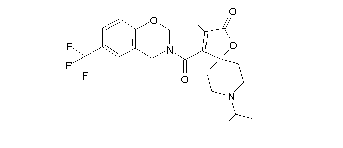 | ++++ | ++++ |
| **67** | 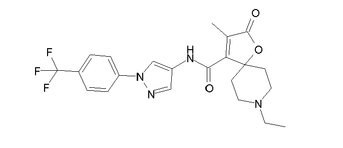 | + | n.d. |
| **68** | 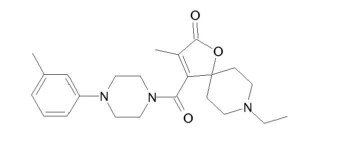 | + | n.d. |
| **69** | 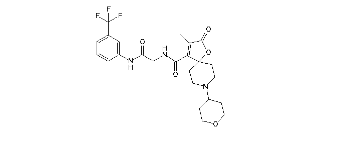 | ++++ | n.d. |
| **70** | 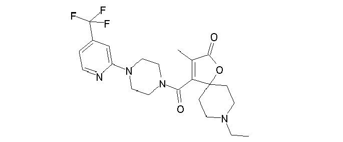 | ++++ | n.d. |
| **71** | 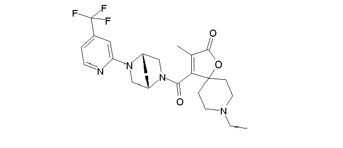 | ++++ | n.d. |
| **72** | 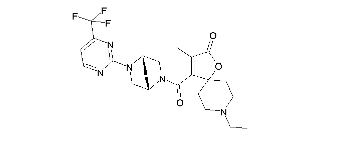 | ++++ | n.d. |
| **73** | 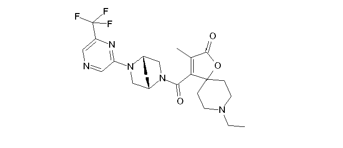 | n.d. | n.d. |
| **74** | 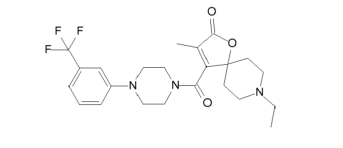 | ++++ | ++++ |
| **75** | 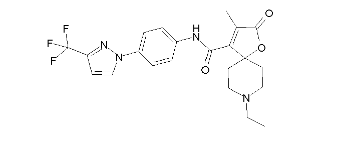 | + | n.d. |
| **76** | 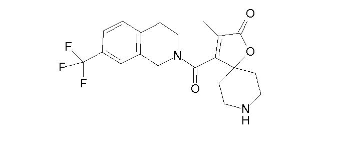 | ++++ | n.d. |
| **77** | 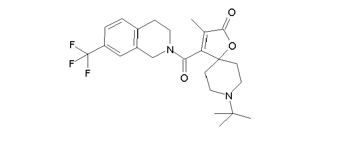 | ++++ | n.d. |
| **78** | 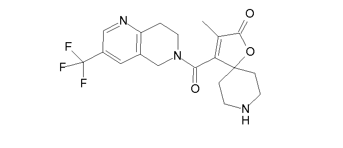 | ++ | n.d. |
| **79** | 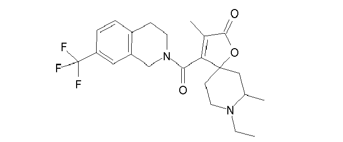 | ++++ | n.d. |
| **80** | 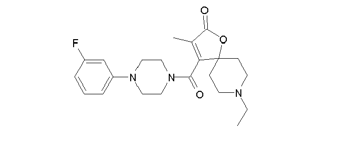 | + | n.d. |
| **81** | 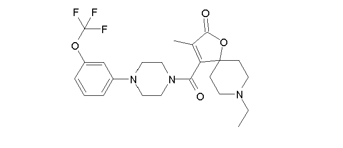 | ++++ | n.d. |
| **82** | 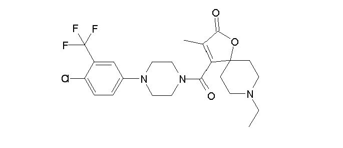 | ++++ | n.d. |
| **83** | 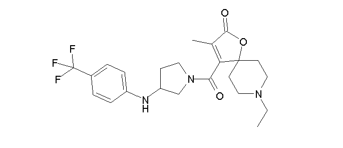 | ++++ | n.d. |
| **84** | 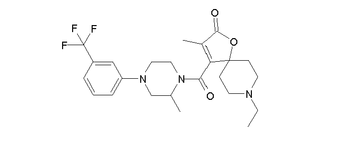 | ++++ | n.d. |
| **85** | 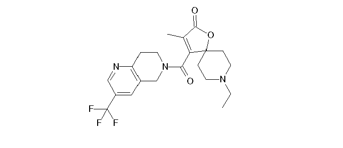 | ++++ | n.d. |
| **86** | 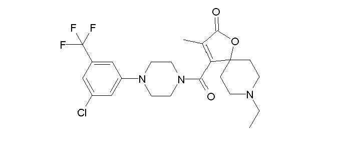 | ++++ | n.d. |
| **87** | 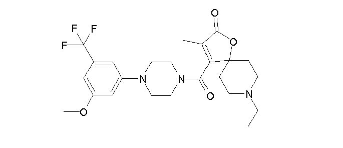 | ++++ | n.d. |
| **88** | 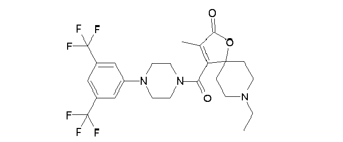 | ++++ | n.d. |
| **89** | 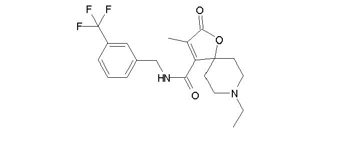 | +++ | n.d. |
| **90** | 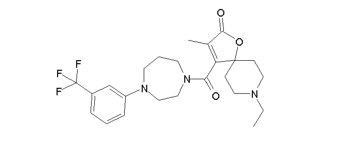 | +++ | n.d. |
| **91** | 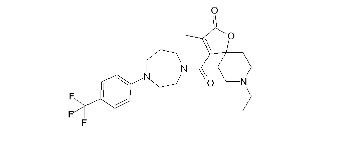 | ++ | n.d. |
| **92** | 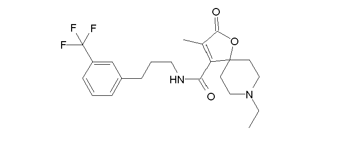 | ++ | n.d. |
| **93** | 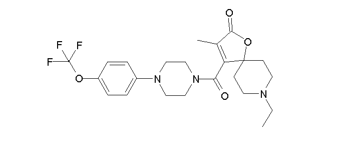 | ++ | n.d. |
| **94** | 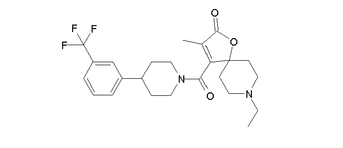 | ++ | n.d. |
| **95** | 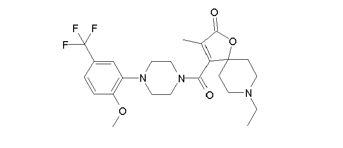 | + | n.d. |
| **96** | 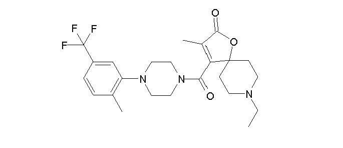 | + | n.d. |
| **97** | 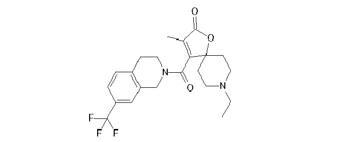 | ++++ | ++++ |
| **98** | 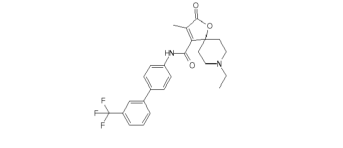 | + | n.d. |
| **99** | 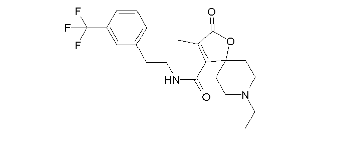 | + | n.d. |
| **100** | 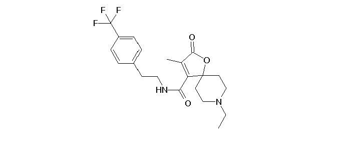 | + | n.d. |
| **101** | 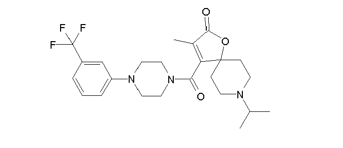 | ++++ | n.d. |
| **102** | 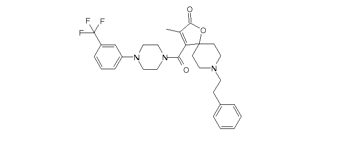 | ++++ | n.d. |
| **103** | 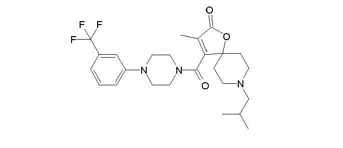 | ++++ | n.d. |
| **104** | 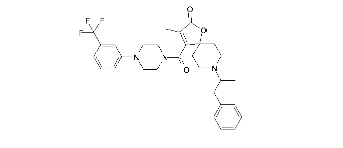 | ++++ | n.d. |
| **105** | 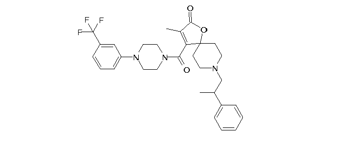 | ++++ | n.d. |
| **106** | 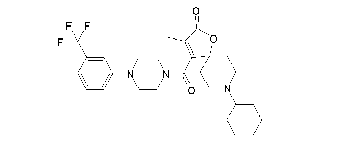 | ++++ | n.d. |
| **107** | 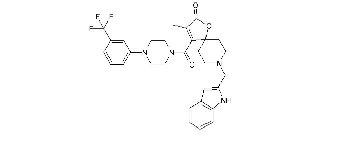 | + | n.d. |
| **108** | 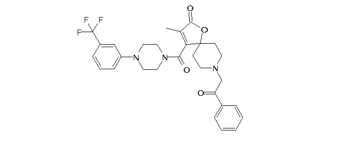 | ++ | n.d. |
| **109** | 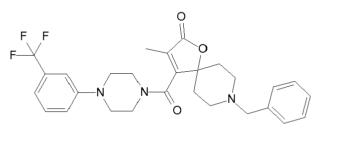 | ++ | n.d. |
| **110** | 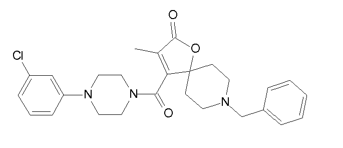 | + | n.d. |
| **111** | 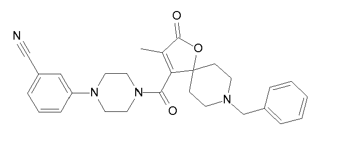 | + | n.d. |
| **112** | 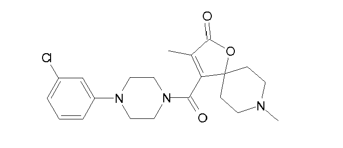 | + | n.d. |
| **113** | 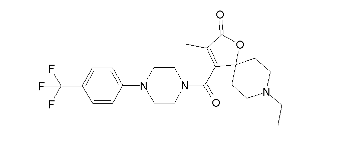 | + | n.d. |
| **114** | 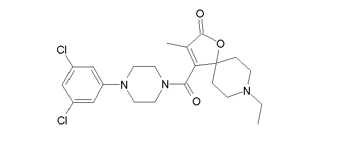 | ++ | n.d. |
| **115** | 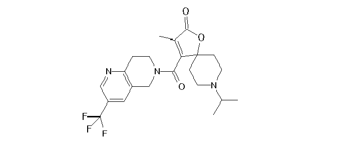 | ++++ | n.d. |
| **116** | 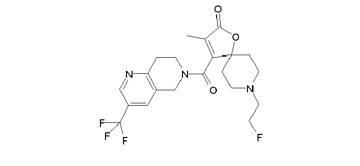 | ++++ | n.d. |
| **117** | 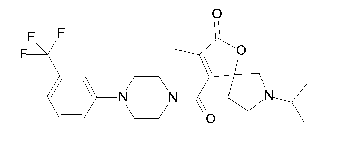 | ++++ | n.d. |
| **118** | 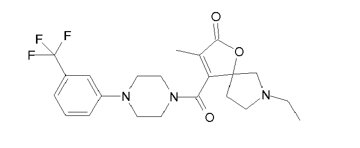 | +++ | n.d. |
| **119** | 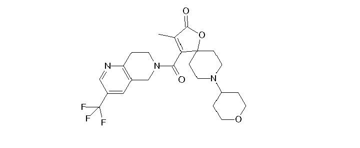 | ++++ | n.d. |
| **120** | 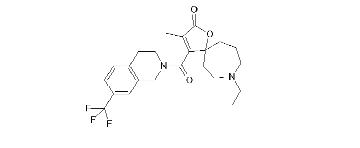 | ++++ | n.d. |
| **121** | 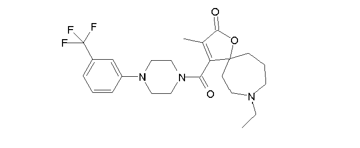 | ++++ | n.d. |
| **122** | 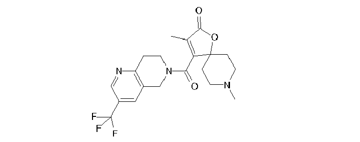 | ++++ | n.d. |

| | | | |
|---|---|---|---|
| +: 500 nM < Ki < 2000 nM ++: 100 n M < Ki < 500 nM +++: 50 nM < Ki < 100 nM ++++: Ki < 50 nM n.d.: not determined | | | |

### Example 138: Preparation of a pharmaceutical formulation

Formulation 1 - Tablets
   A compound of formula (I) is admixed as a dry powder with a dry gelatin binder in an approximate 1:2 weight ratio. A minor amount of magnesium stearate is added as a lubricant. The mixture is formed into 240-270 mg tablets (80-90 mg of active compound according to the invention per tablet) in a tablet press.
Formulation 2 - Capsules
   A compound of formula (I) is admixed as a dry powder with a starch diluent in an approximate 1:1 weight ratio. The mixture is filled into 250 mg capsules (125 mg of active compound according to the invention per capsule).
Formulation 3 - Liquid
   A compound of formula (I) (1250 mg), sucrose (1.75 g) and xanthan gum (4 mg) are blended, passed through a No. 10 mesh U.S. sieve, and then mixed with a previously prepared solution of microcrystalline cellulose and sodium carboxymethyl cellulose (11:89, 50 mg) in water. Sodium benzoate (10 mg), flavor, and color are diluted with water and added with stirring. Sufficient water is then added to produce a total volume of 5 mL.
Formulation 4 - Tablets
   A compound of formula (I) is admixed as a dry powder with a dry gelatin binder in an approximate 1:2 weight ratio. A minor amount of magnesium stearate is added as a lubricant. The mixture is formed into 450-900 mg tablets (150-300 mg of active compound according to the invention) in a tablet press.
Formulation 5 - Injection
   A compound of formula (I) is dissolved in a buffered sterile saline injectable aqueous medium to a concentration of approximately 5 mg/mL.

## Claims

1. A compound of Formula (I) 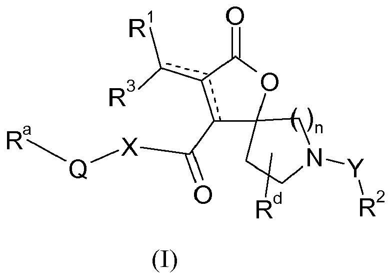 Wherein
One of the two symboles ⁻⁻⁻⁻ npendently denotes a single bond and the other one denotes a double bond.
R¹, R³ Independently from one another denotes H or a branched or linear alkyl having 1 to 6 carbon atoms,
X is selected from the groups consisting of Het^{x}, -(CH₂)_{q}Het^{x}, NR⁶-Het^{x}, wherein Het^{x} is linked to the adjacent CO group through one N atom contained in its ring, or a group selected from Het^{x}-NR⁶, Ar-NR⁶,-(CH₂)_{q}NR⁶, wherein -NR⁶ is linked to the adjacent CO group, or a group NR⁶CO(CH₂)_{q}N*R⁶, wherein N*R⁶ is linked to the adjacent CO group,
Q denotes a 5- or 6- membered saturated, unsaturated or aromatic ring optionally containing 1 or 2 nitrogen atoms and optionally substituted by 1 or 2 of R^{b} and R^{c}, or
Q-X together denote a fused bicyclic system, containing a N atom linked to the adjacent CO group, which may contain 1 to 3 additional heteroatoms independently selected from N, O, S and/or a group CO, and which may be substituted with 1 or 2 of R^{b} and R^{c},
Y denotes a single bond, a branched or linear alkylene having 1 to 6 carbon atoms or a cyclic alkylene having 3 to 7 carbon atoms, wherein 1 to 5 H atoms, in these cyclic, linear or branched alkylene, may be independently replaced by OR⁶, Hal, or CN,
R² denotes H, Cyc, Hal, Het, Ar, -(CH₂)_{q}COHet, -(CH₂)_{q}COAr, -(CH₂)_{q}COCyc, -(CH₂)_{q}CO₂R⁶, OR⁶,
R^{a} denotes CF₃, OCF₃, Hal, CN, a linear or branched alkyl having 1 to 6 carbon atoms, or when Q denotes a 5- or 6- membered saturated, unsaturated or aromatic ring optionally containing 1 or 2 nitrogen atoms and which is substituted by 1 or 2 of R^{b} and R^{c}, R^{a} also denotes H,
R^{b} denotes Hal, OR⁶, CN, CF₃, or OCF₃,
R^{c} denotes a linear or branched alkyl having 1 to 6 carbon atoms, OR⁶,
R^{d} denotes H or a linear or branched alkyl having 1 to 6 carbon atoms,
Hal denotes F, Cl, Br or I,
n is 1, 2, 3 or 4,
q is 1, 2 or 3,
Cyc denotes a unsaturated or saturated carbocyclic ring having 3 to 7 carbon atoms, which may be substituted by 1 to 5 groups independently selected from a linear or branched alkyl having 1 to 6 carbon atoms, Hal, OR⁶, CF₃, CN, NO₂, CO₂R⁶,
Het denotes a saturated, unsaturated or aromatic monocyclic or fused bicyclic ring having 1 to 3 heteroatoms independently selected from N, O and/or S, which may additionally contain a group selected from CO, SO or SO₂, and which may be substituted with 1 to 5 substituents independently selected from a linear or branched alkyl having 1 to 6 carbon atoms, Hal, OR⁶, CN, NO₂, CO₂R⁶,
Ar denotes a 5-, 6-, or 7-membered saturated, unsaturated or aromatic ring, which may be substituted with 1 to 4 groups independently selected from Hal, CF₃, OR⁶, CN or a linear or branched C₁-C₆-alkyl,
R⁶ denotes H, or a branched or linear alkyl having 1 to 6 carbon atoms, or a cycloalkyl having 3 to 6 carbon atoms,
Het^{x} denotes a 5-, 6-, or 7-membered saturated, unsaturated or aromatic heterocyclic ring containing 1 N atom, which may contain 1 to 3 additional heteroatoms independently selected from N, O, S and/or a group CO, and which may be substituted with 1 to 4 groups independently selected from Hal, CF₃, OR⁶, CN or a linear or branched C₁-C₆-alkyl, wherein 2 or more of the linear or branched C₁-C₆-alkyl may be linked to form a bridge,
As well as its pharmaceutically acceptable salts, isomers, diastereoisomers and optically active forms,
With the proviso that the following compounds are excluded.

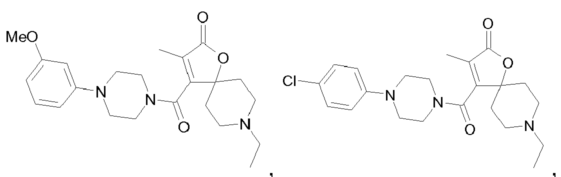

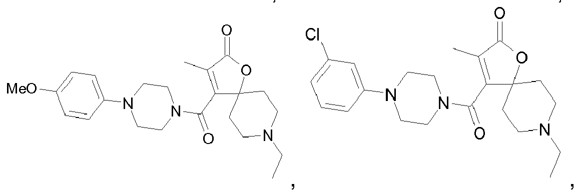

2. A compound according to Formula (I) in claim 1 wherein the compound is of Formula (Ia): 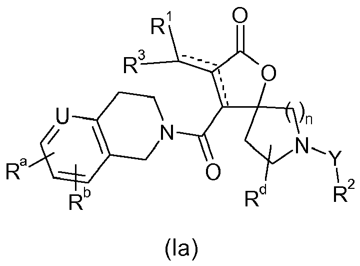 Wherein ⁻⁻⁻⁻ R^{a}, R^{b}, Y, R^{d}, R¹, R², R³ and n are as defined in claim 1, and wherein U is N or C, as well as enantiomers, diastereoisomers thereof.

3. A compound according to Formula (I) in claim 1 wherein the compound is of Formula (Ib) 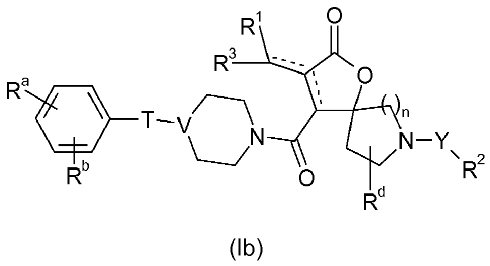 Wherein ⁻⁻⁻⁻ , R^{a}, R^{b}, R^{d}, Y, R¹, R²,R³, Y and n are as defined in claim 1, and wherein V is N or C, T is a single bond or NR⁶, (CH₂)_{q} wherein R⁶ and q are as defined in claim 1, as well as enantiomers, diastereoisomers thereof.

4. A compound according to claims 1-3 wherein R^{a} is selected from CF₃, OR⁶, OCF₃.

5. A compound according to claims 1 to 4 wherein the group Y-R² denotes H, CH₃, - CH₂-CH₃, -CH₂-CH₂-CH₃, -CH₂-CH₂-OH, -CH₂-CH₂-CH₂-OH, -CH(CH₃)₂, -CH-(C₂H₅)₂, -C(CH₃)₃, -CH₂-CH(CH₃)₂, or one of the following groups:

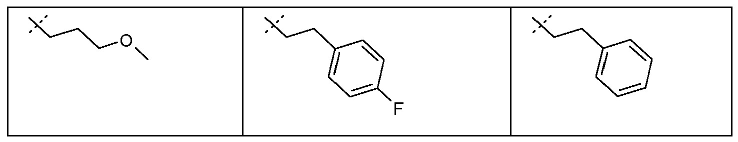

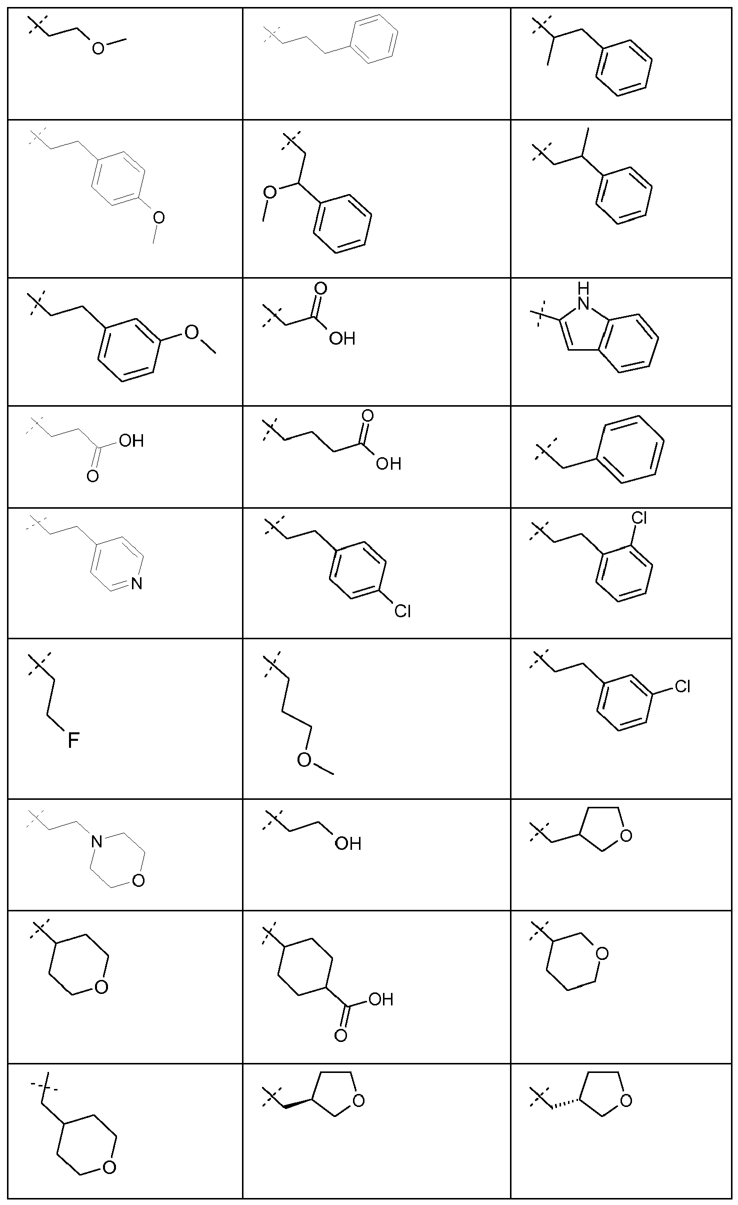

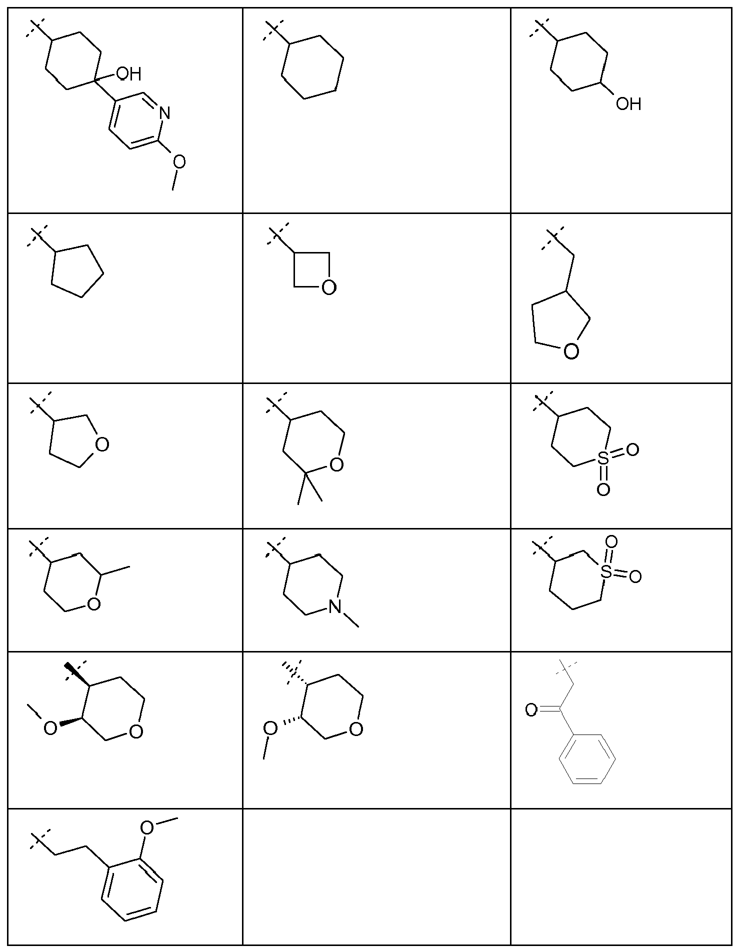

6. A compound according to claims 1 to 5 wherein the group X-Q-R^{a} in Formula (I) and related formulae denotes one of the following groups:

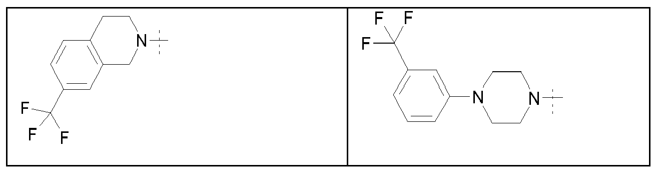

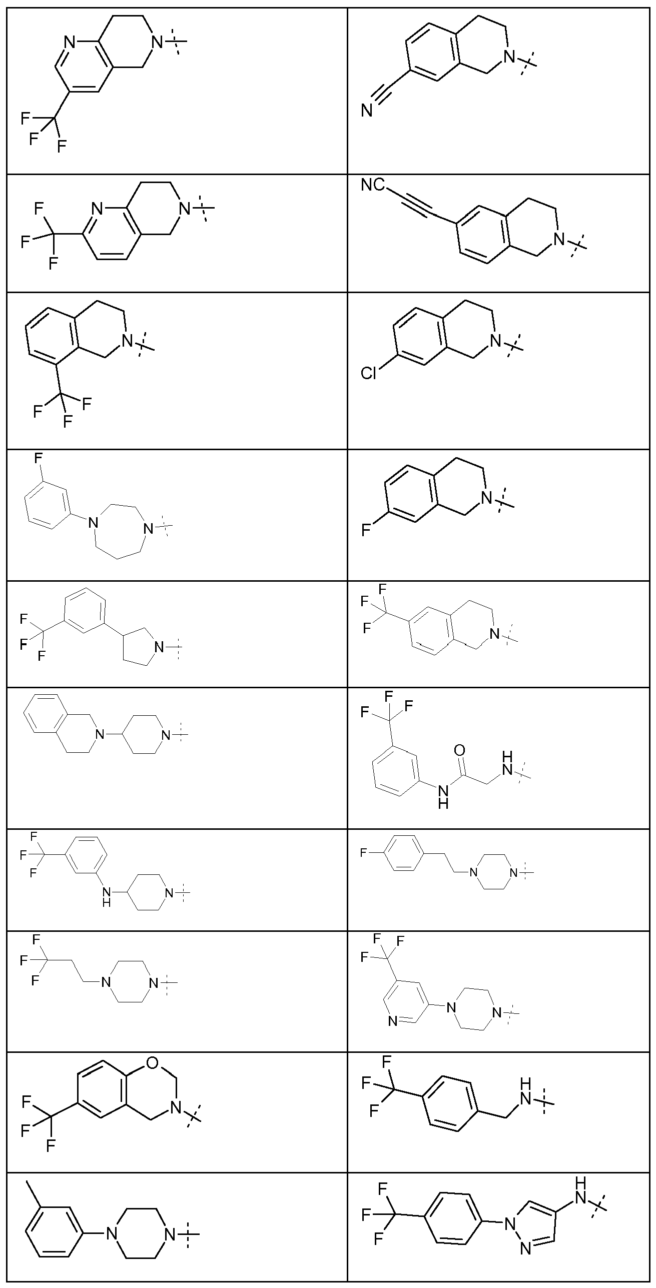

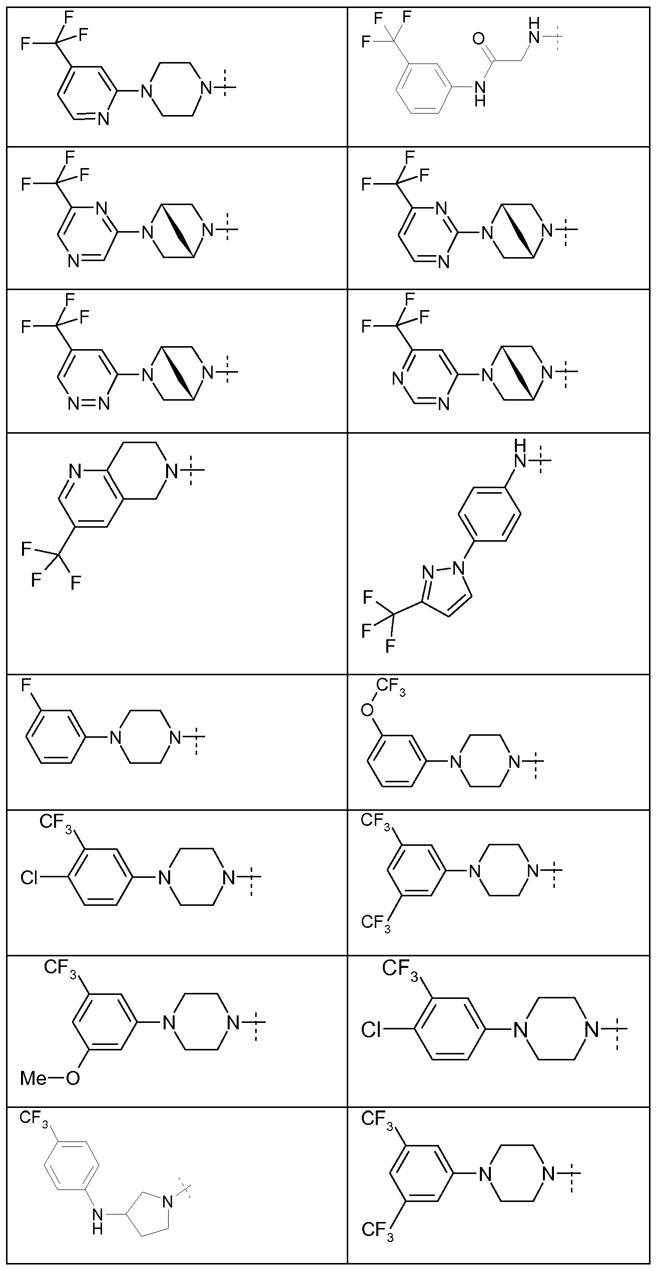

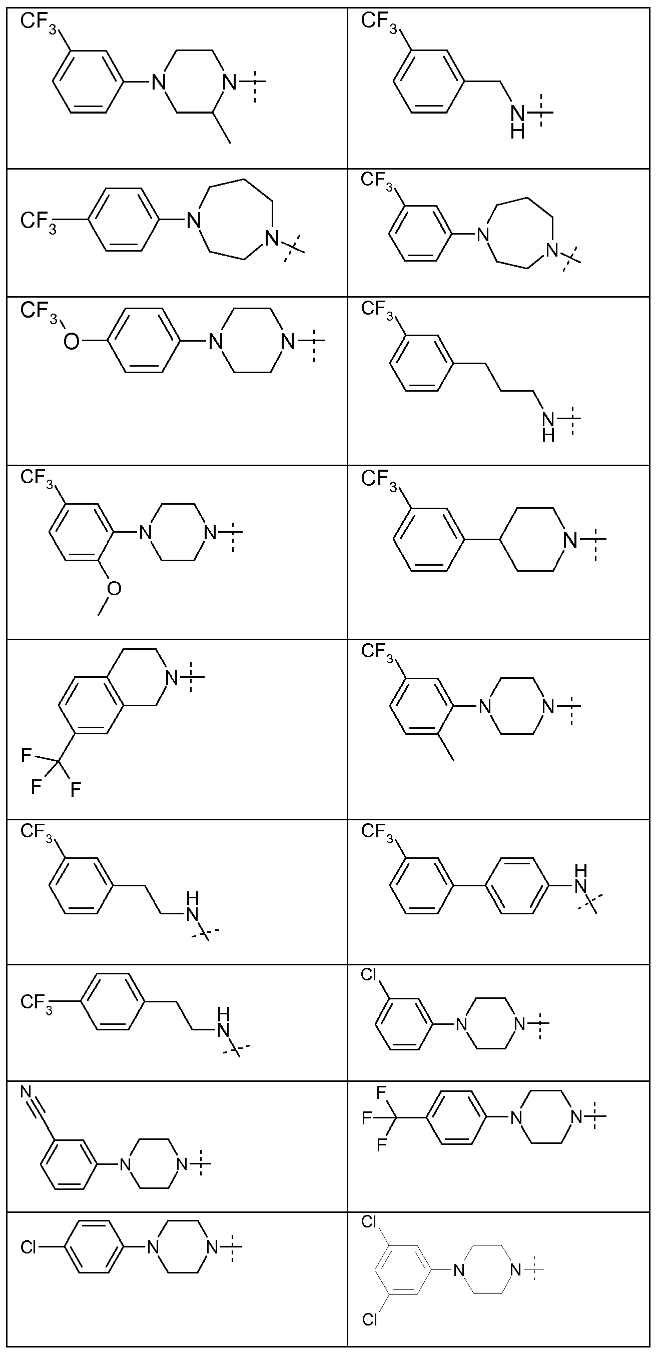

7. A compound according to claims 1 to 6 wherein the compound is selected from the following group:
| **Compound number** | **structure** |
|---|---|
| **1** | 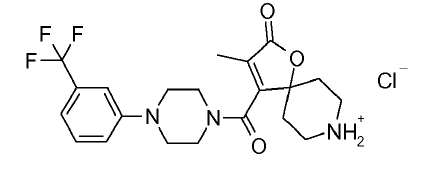 |
| **2** | 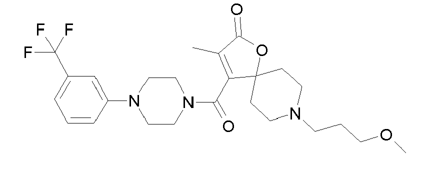 |
| **3** | 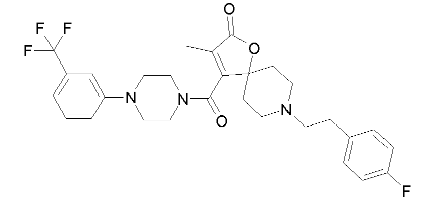 |
| **4** | 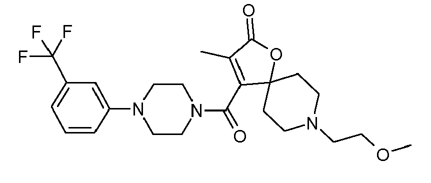 |
| **5** | 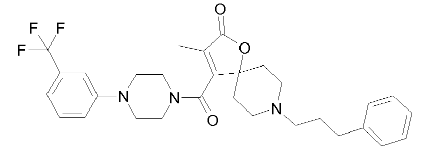 |
| **6** | 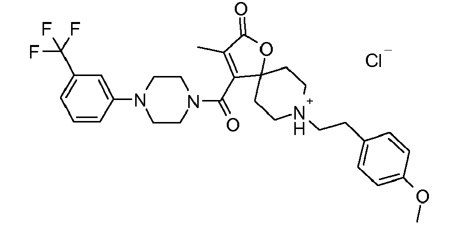 |
| **7** | 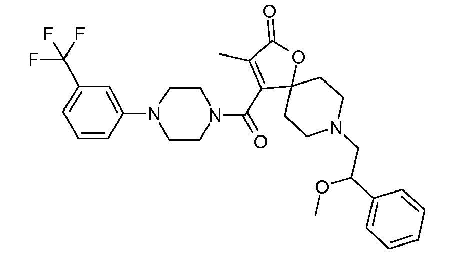 |
| **8** | 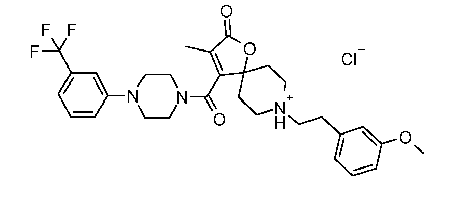 |
| **9** | 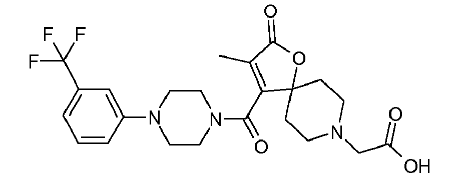 |
| **10** | 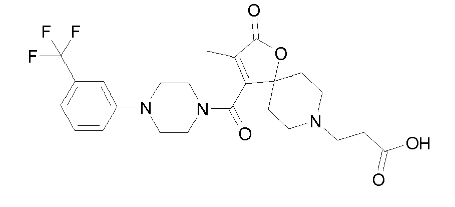 |
| **11** | 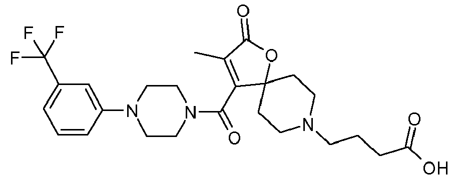 |
| **12** | 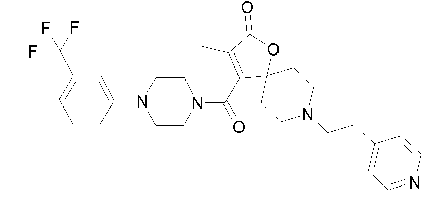 |
| **13** | 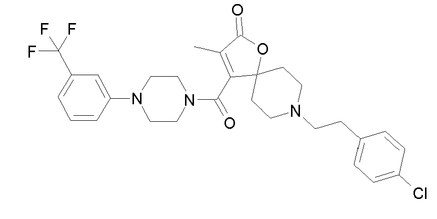 |
| **14** | 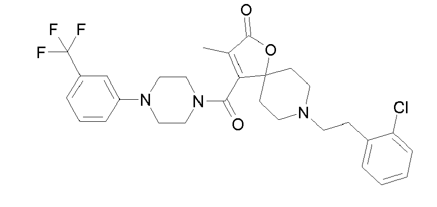 |
| **15** | 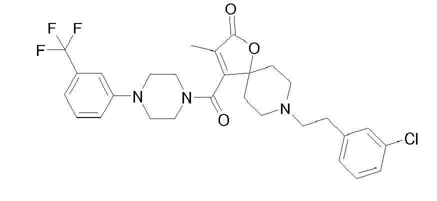 |
| **16** | 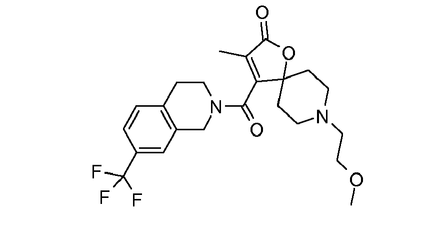 |
| **17** | 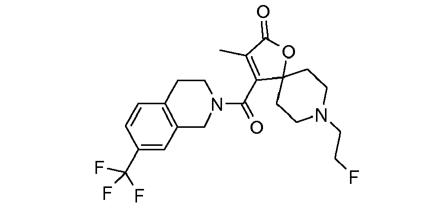 |
| **18** | 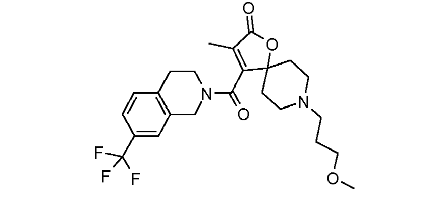 |
| **19** | 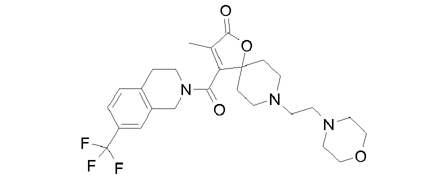 |
| **20** | 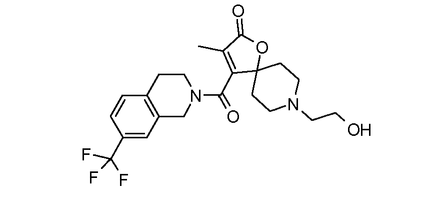 |
| **21** | 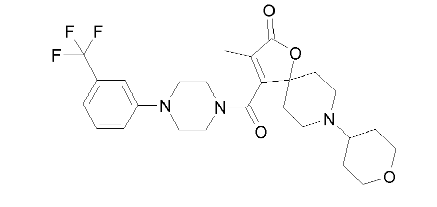 |
| **22** | 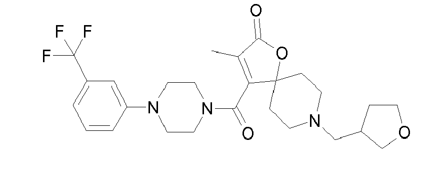 |
| **23** | 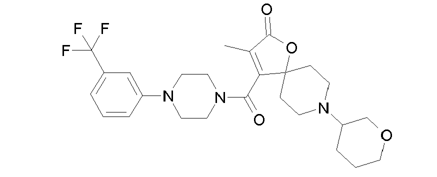 |
| **24** | 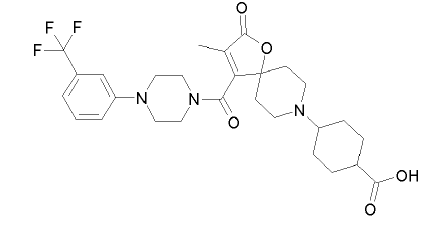 |
| **25** | 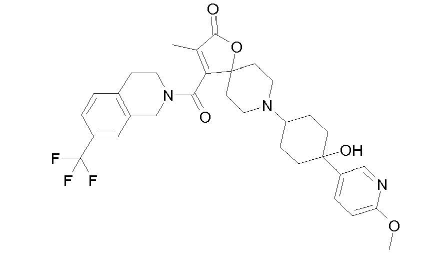 |
| **26** | 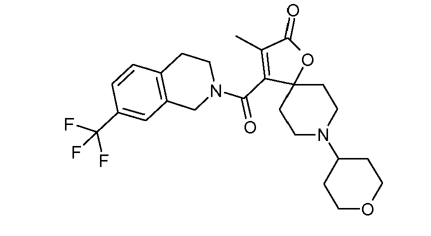 |
| **27** | 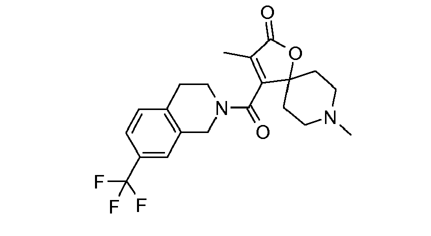 |
| **28** | 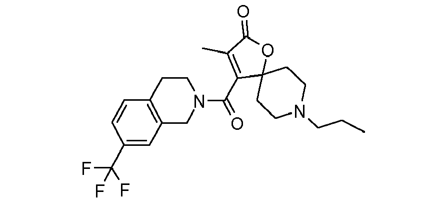 |
| **29** | 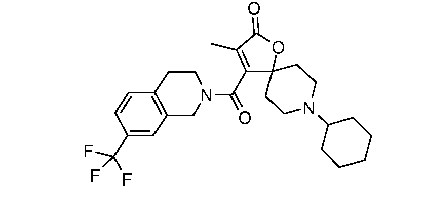 |
| **30** | 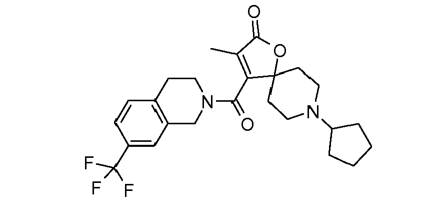 |
| **31** | 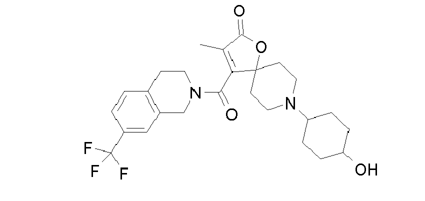 |
| **32** | 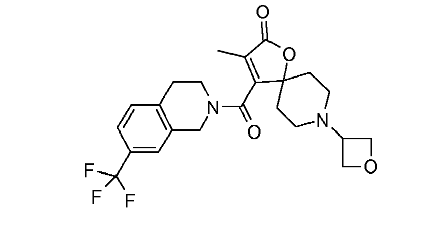 |
| **33** | 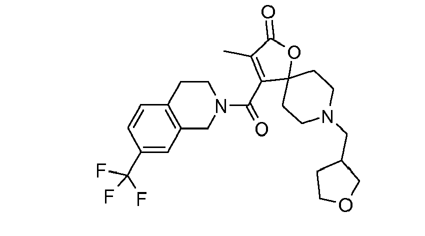 |
| **34** | 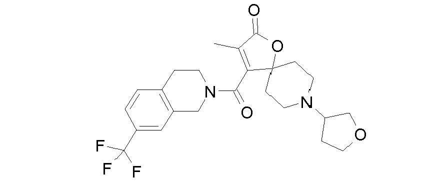 |
| **35** | 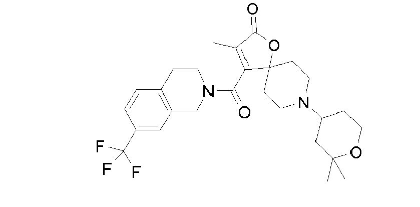 |
| **36** | 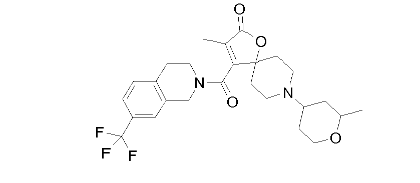 |
| **37** | 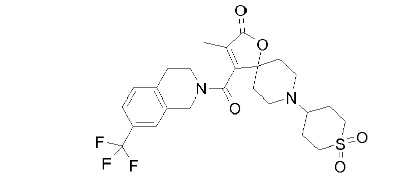 |
| **38** | 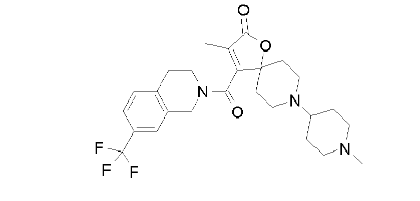 |
| **39** | 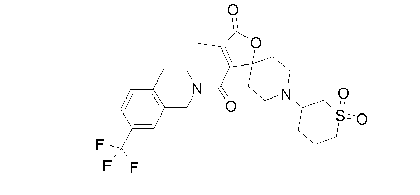 |
| **40** | 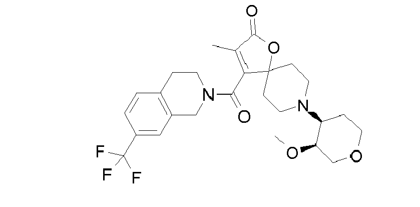 |
| **41** | 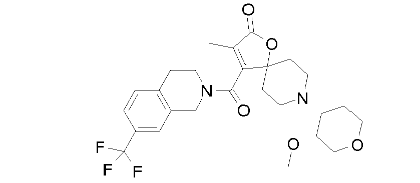 |
| **42** | 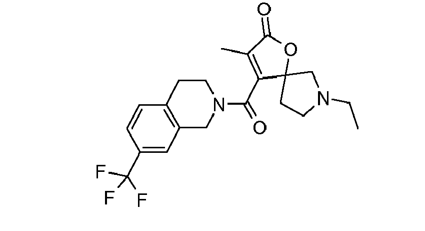 |
| **44** | 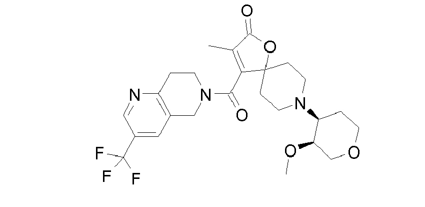 |
| **45** | 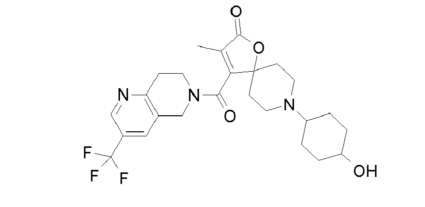 |
| **46** | 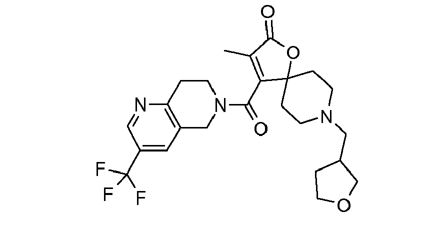 |
| **47** | 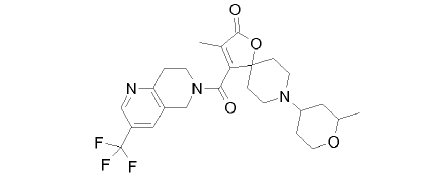 |
| **48** | 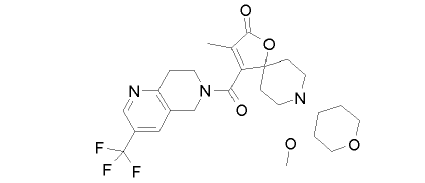 |
| **49** | 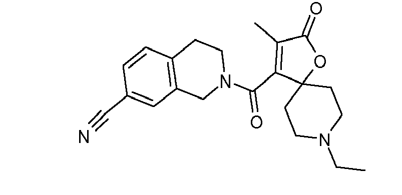 |
| **50** | 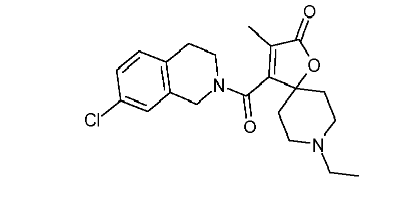 |
| **51** | 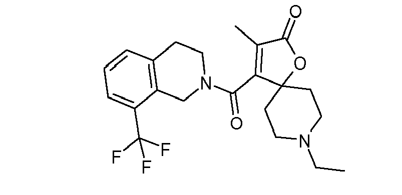 |
| **52** | 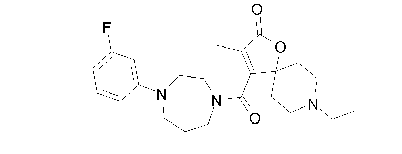 |
| **53** | 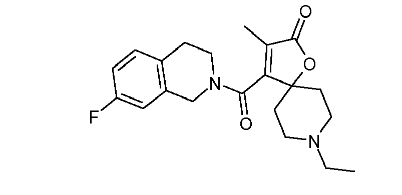 |
| **54** | 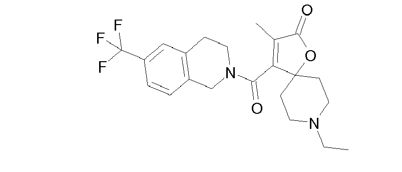 |
| **55** | 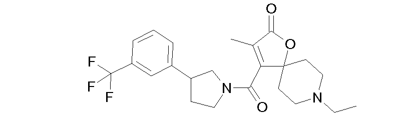 |
| **56** | 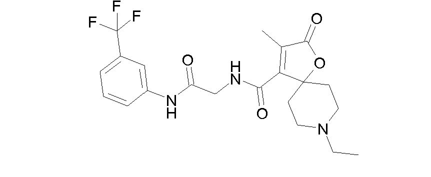 |
| **58** | 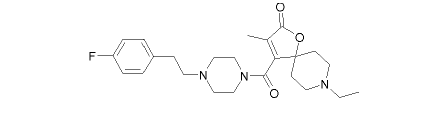 |
| **59** | 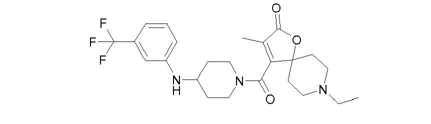 |
| **60** | 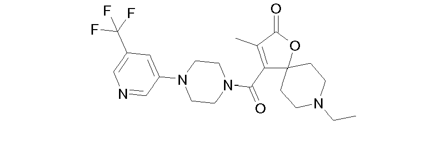 |
| **62** | 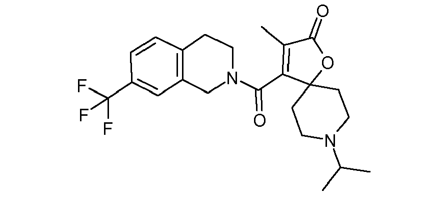 |
| **63** | 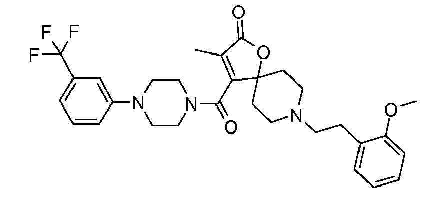 |
| **64** | 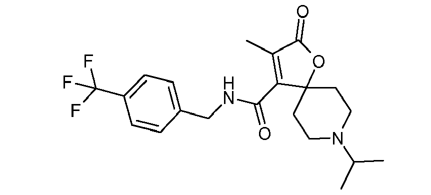 |
| **65** | 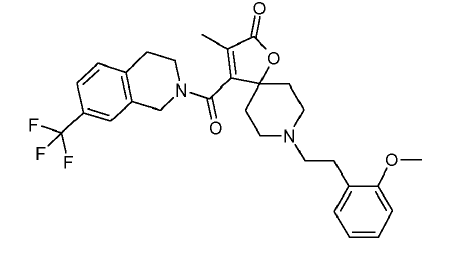 |
| **66** | 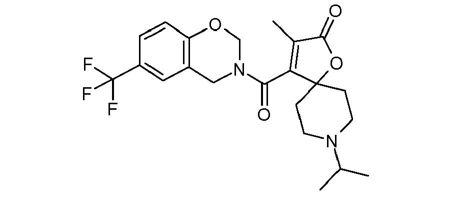 |
| **67** | 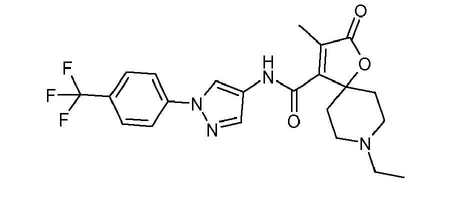 |
| **68** | 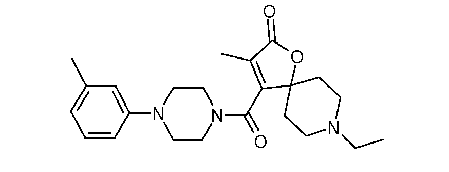 |
| **69** | 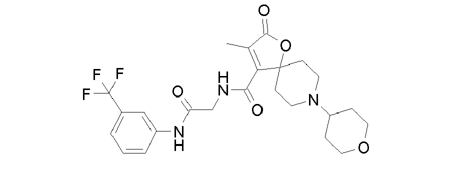 |
| **70** | 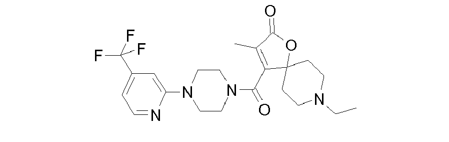 |
| **71** | 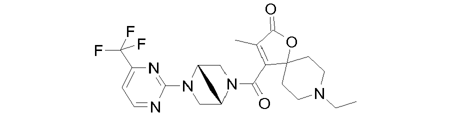 |
| **72** | 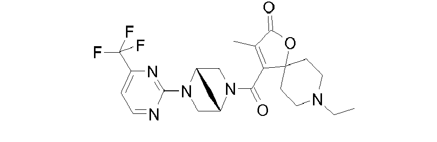 |
| **73** | 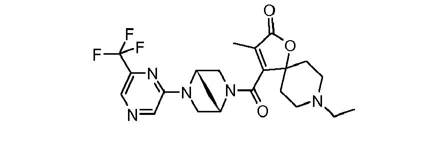 |
| **74** | 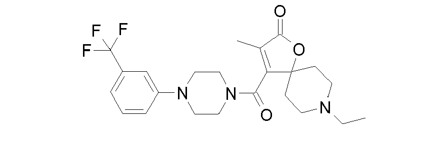 |
| **75** | 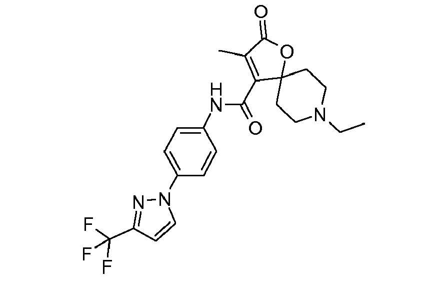 |
| **76** | 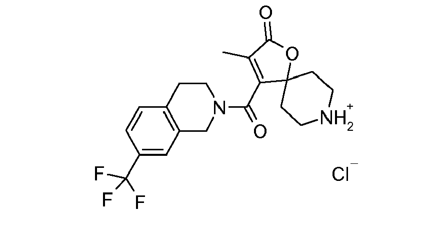 |
| **77** | 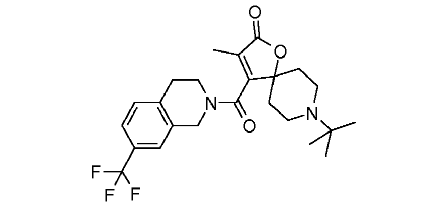 |
| **78** | 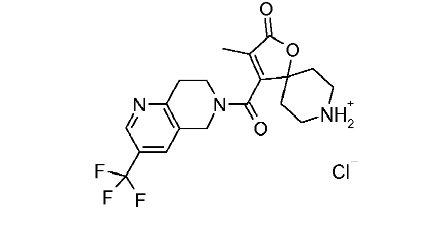 |
| **79** | 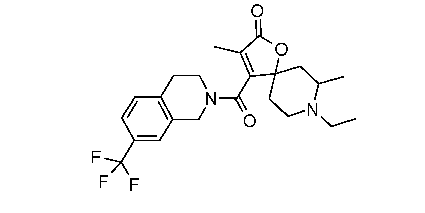 |
| **80** | 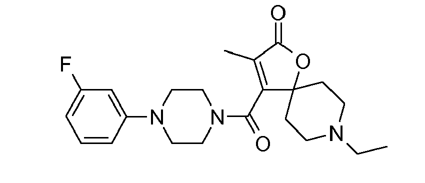 |
| **81** | 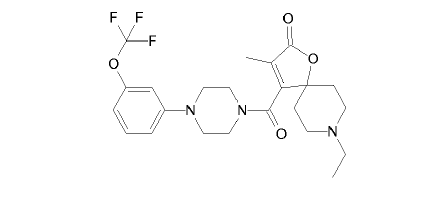 |
| **82** | 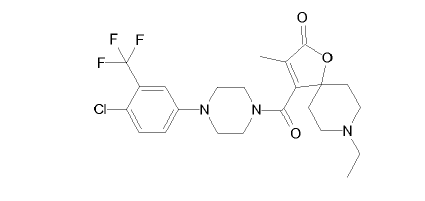 |
| **83** | 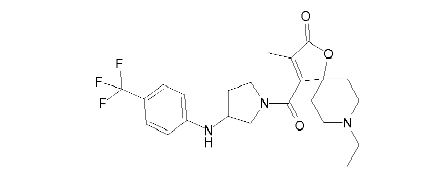 |
| **84** | 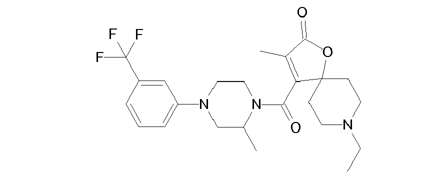 |
| **85** | 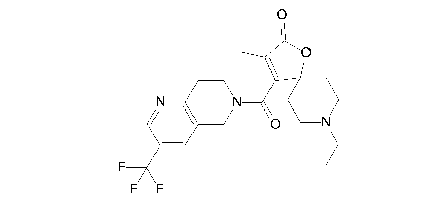 |
| **86** | 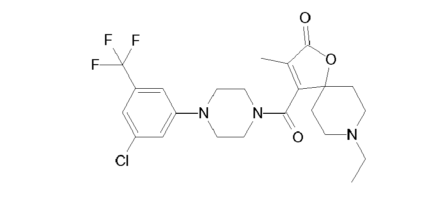 |
| **87** | 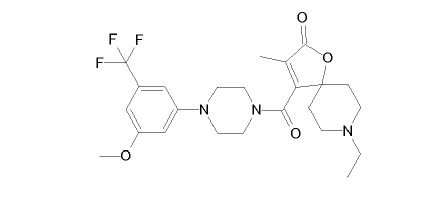 |
| **88** | 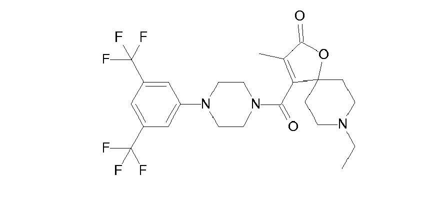 |
| **89** | 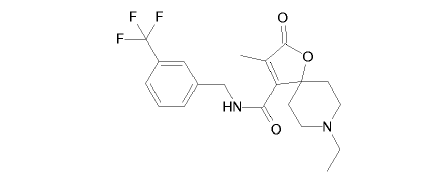 |
| **90** | 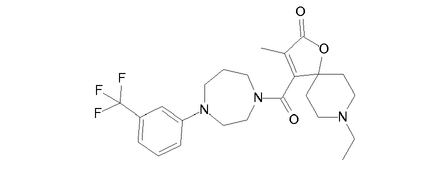 |
| **91** | 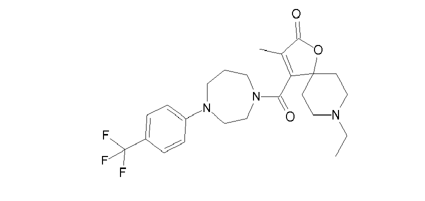 |
| **92** | 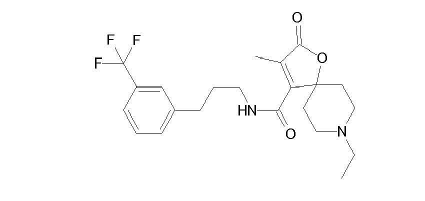 |
| **93** | 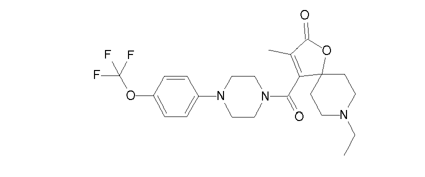 |
| **94** | 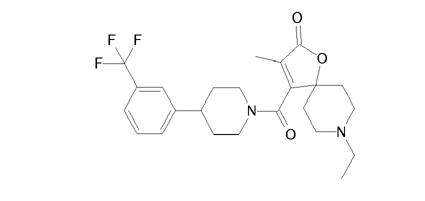 |
| **95** | 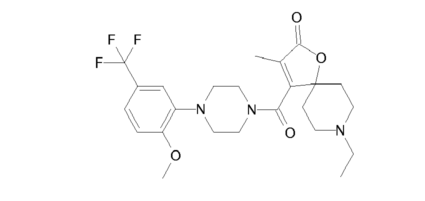 |
| **96** | 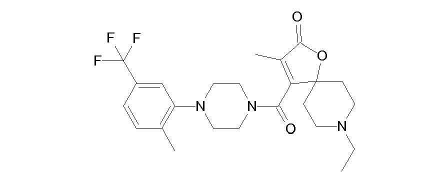 |
| **97** | 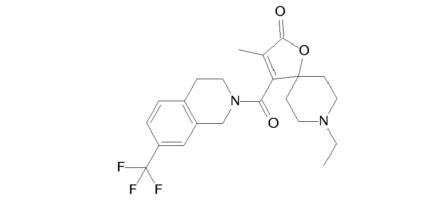 |
| **98** | 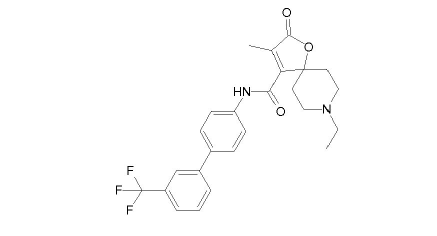 |
| **99** | 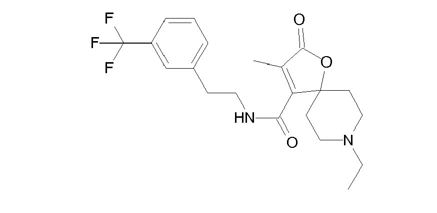 |
| **100** | 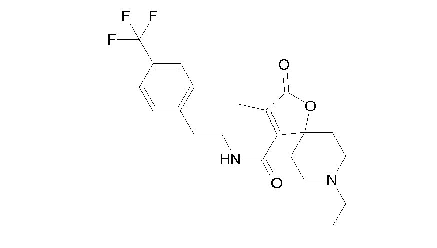 |
| **101** | 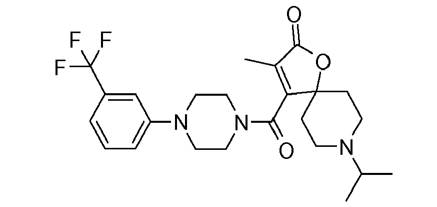 |
| **102** | 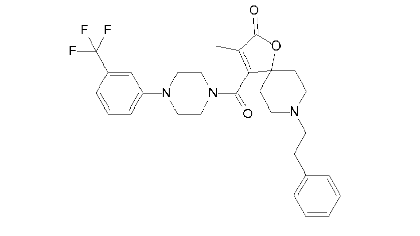 |
| **103** | 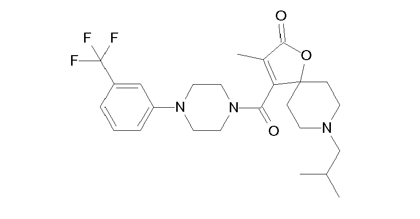 |
| **104** | 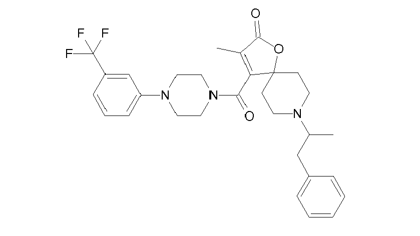 |
| **105** | 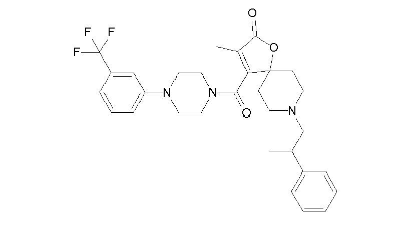 |
| **106** | 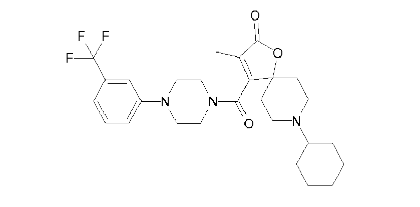 |
| **107** | 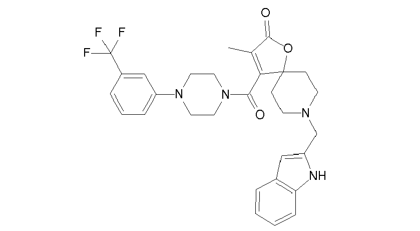 |
| **108** | 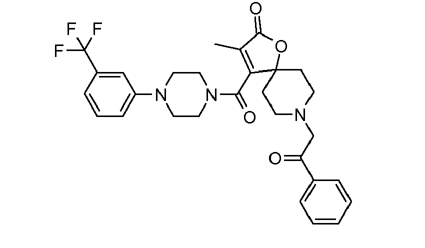 |
| **109** | 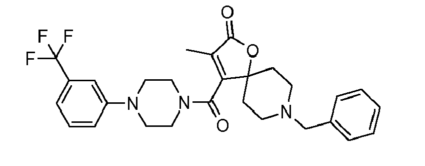 |
| **110** | 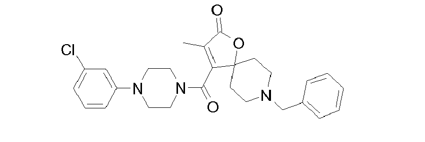 |
| **111** | 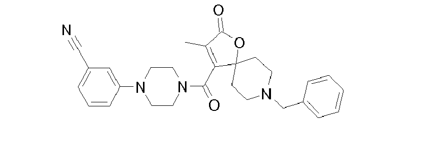 |
| **112** | 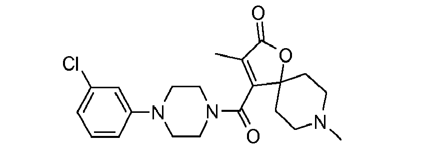 |
| **113** | 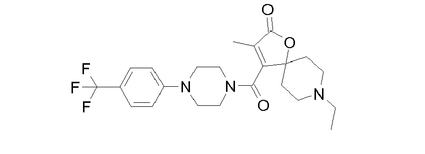 |
| **114** | 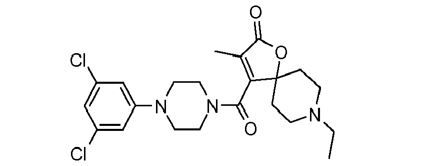 |
| **115** | 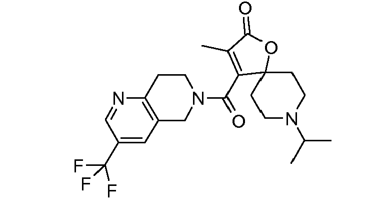 |
| **116** | 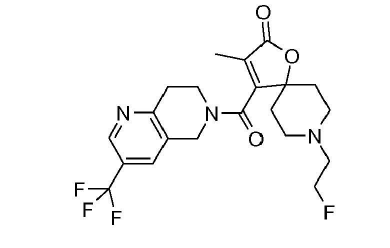 |
| **117** | 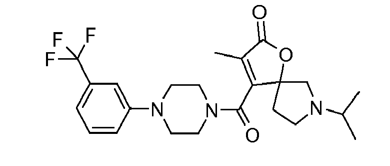 |
| **118** | 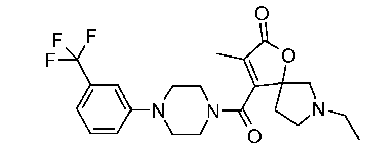 |
| **119** | 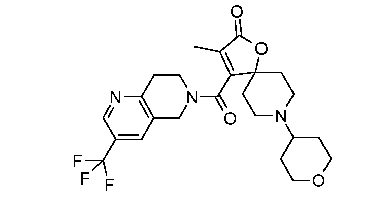 |
| **120** | 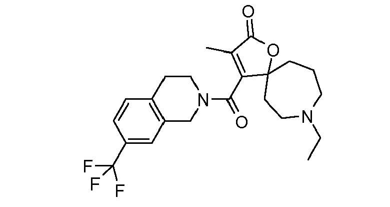 |
| **121** | 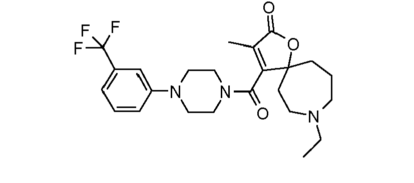 |
| **122** | 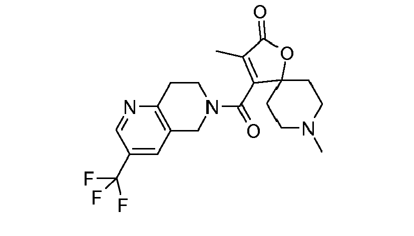 |
| **123** | 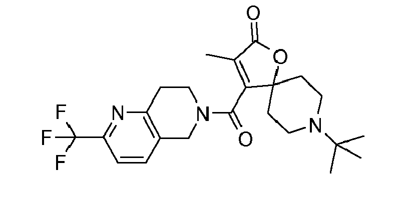 |
| **124** | 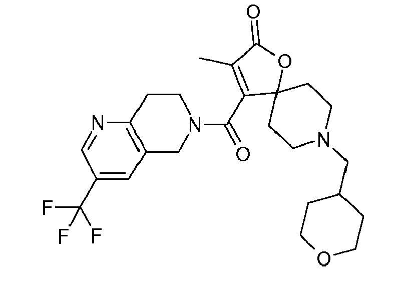 |
| **125** | 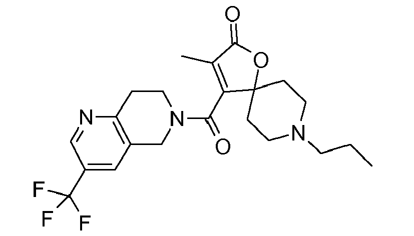 |
| **126** | 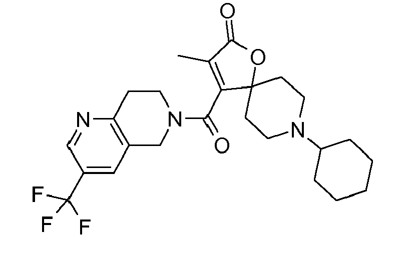 |
| **127** | 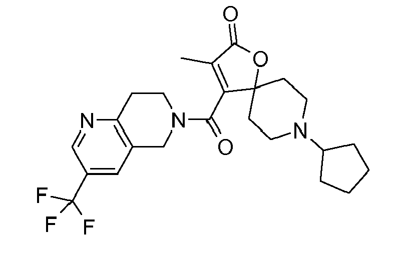 |
| **128** | 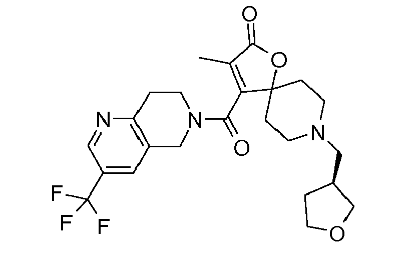 |
| **129** | 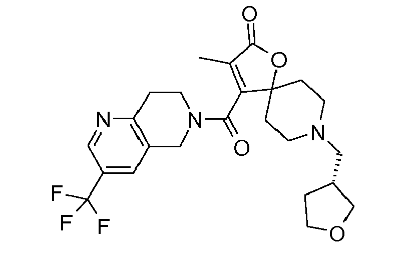 |
| **130** | 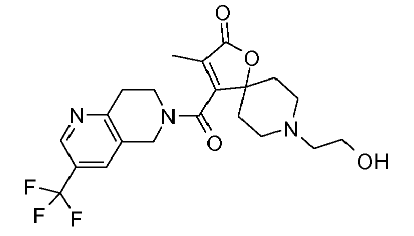 |
| **131** | 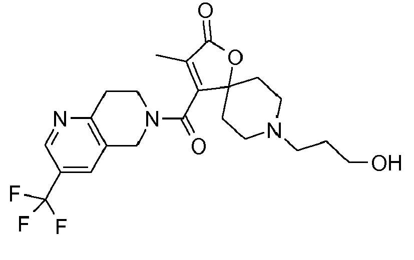 |
| **132** | 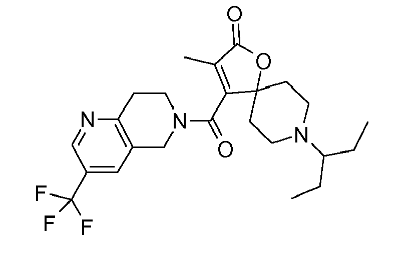 |
| **133** | 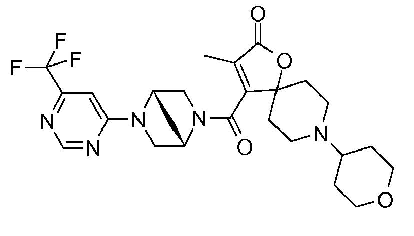 |
| **134** | 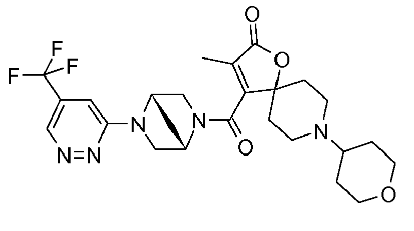 |
| **135** | 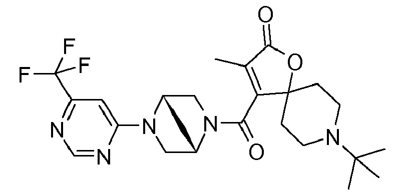 |
| **136** | 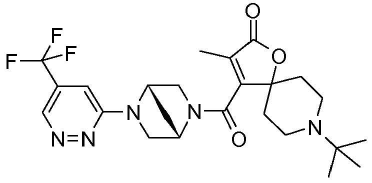 |

8. A compound of Formula (I) 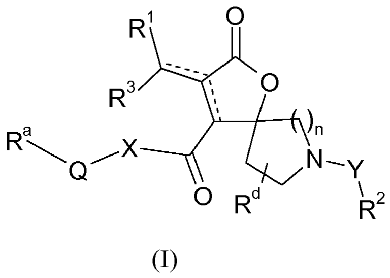 Wherein
One of the two symboles ⁻⁻⁻⁻ independently denotes a single bond and the other one denotes a double bond.
R¹, R³ Independently from one another denotes H or a branched or linear alkyl having 1 to 6 carbon atoms,
X is selected from the groups consisting of Het^{x}, -(CH₂)_{q}Het^{x}, NR⁶-Het^{x}, wherein Het^{x} is linked to the adjacent CO group through one N atom contained in its ring, or a group selected from Het^{x}-NR⁶, Ar-NR⁶,-(CH₂)_{q}NR⁶, wherein -NR⁶ is linked to the adjacent CO group, or a group NR⁶CO(CH₂)_{q}N*R⁶, wherein N*R⁶ is linked to the adjacent CO group,
Q denotes a 5- or 6- membered saturated, unsaturated or aromatic ring optionally containing 1 or 2 nitrogen atoms and optionally substituted by 1 or 2 of R^{b} and R^{c}, or
Q-X together denote a fused bicyclic system, containing a N atom linked to the adjacent CO group, which may contain 1 to 3 additional heteroatoms independently selected from N, O, S and/or a group CO, and which may be substituted with 1 or 2 of R^{b} and R^{c},
Y denotes a single bond, a branched or linear alkylene having 1 to 6 carbon atoms or a cyclic alkylene having 3 to 7 carbon atoms, wherein 1 to 5 H atoms, in these cyclic, linear or branched alkylene, may be replaced by OR⁶, Hal, or CN,
R² denotes H, Cyc, Hal, Het, Ar, -(CH₂)_{q}COHet, -(CH₂)_{q}COAr, -(CH₂)_{q}COCyc, -(CH₂)_{q}CO₂R⁶, OR⁶,
R^{a} denotes CF₃, OCF₃, Hal, CN, a linear or branched alkyl having 1 to 6 carbon atoms, or when Q denotes a 5- or 6- membered saturated, unsaturated or aromatic ring optionally containing 1 or 2 nitrogen atoms and which is substituted by 1 or 2 of R^{b} and R^{c}, R^{a} also denotes H,
R^{b} denotes Hal, OR⁶, CN, CF₃, or OCF₃,
R^{c} denotes a linear or branched alkyl having 1 to 6 carbon atoms, OR⁶,
R^{d} denotes H or a linear or branched alkyl having 1 to 6 carbon atoms,
Hal denotes F, Cl, Br or I,
n is 1, 2, 3 or 4,
q is 1, 2 or 3,
Cyc denotes a unsaturated or saturated carbocyclic ring having 3 to 7 carbon atoms, which may be substituted by a linear or branched alkyl having 1 to 6 carbon atoms, Hal, OR⁶, CF₃, CN, NO₂, CO₂R⁶,
Het denotes a saturated, unsaturated or aromatic monocyclic or fused bicyclic ring having 1 to 3 heteroatoms selected from N, O and/or S, which may additionally contain a group selected from CO, SO or SO₂, and which may be substituted with 1 to 5 substituents independently selected from a linear or branched alkyl having 1 to 6 carbon atoms, Hl, OR⁶, CN, NO₂, CO₂R⁶,
Ar denotes a 5-, 6-, or 7-membered saturated, unsaturated or aromatic ring, which may be substituted with 1 to 4 groups independently selected from Hal, CF₃, OR⁶, CN or a linear or branched C₁-C₆-alkyl,
R⁶ denotes H, or a branched or linear alkyl having 1 to 6 carbon atoms, or a cycloalkyl having 3 to 6 carbon atoms,
Het^{x} denotes a 5-, 6-, or 7-membered saturated, unsaturated or aromatic heterocyclic ring containing 1 N atom, which may contain 1 to 3 additional heteroatoms selected from N, O, S and/or a group CO, and which may be substituted with 1 to 4 groups independently selected from Hal, CF₃, OR⁶, CN or a linear or branched C₁-C₆-alkyl, wherein 2 or more of the linear or branched C₁-C₆-alkyl may be linked to form a bridge,
As well as its pharmaceutically acceptable salts, isomers, diastereoisomers and optically active forms, for use as a medicament.

9. A compound of Formula (I) according to claim 8 for use in the treatment of inflammatory diseases, autoimmune diseases or cancer.

10. A compound of Formula (I) for use according to claims 8 or 9 wherein the diseases is selected from rheumatoid arthritis, arthritis, ankylosing spondylitis, restenosis, osteoarthritis, food allergy, gout disease , systemic lupus erythematosus, lupus nephritis, glomerulonephritis, lupus, multiple sclerosis, Sjogren's Syndrome, bone metastasis, prostate cancer, breast cancer, allergic asthma, asthma, seasonal and perennial allergic rhinitis, transplant rejection, inflammatory bowel disease, Crohn's disease, ulcerative colitis, irritable bowel syndrome, colitis, nephritis, IGa nephropathy, fibrosis, glomerulonephritis fibrosis, liver fibrosis, lung fibrosis, iodiopathic pulmonary fibrosis, fibrotic kidney diseases, systemic sclerosis, scleroderma, liver cirrhosis, liver steatosis, cardiac disease, thrombotic disease, atherosclerosis, myocarditis, stroke, Ischaemia/reperfusion injury in the heart, Ischaemia/reperfusion injury in transplant rejection, pain, neuropathic pain, sarcoidosis, hypertension, psoriasis, atopic dermatitis, contact dermatitis, endometriosis, uveitis, delayed-type hypersensitivity, Behçet's disease, diabetes, obesity, insulin resistance, metabolic syndrome, Alzheimer's disease, chronic obstructive pulmonary disease, interstitial lung disease, HIV-infection, HIV with CNS manifestations - Neuro AIDS, organ transplantation, chronic allograph nephropathy, allergic eye disease, age related macular degeneration, acute kidney ischemic disease, (recurrent) Brain infarction, acute coronary syndrome, benign prostatic hyperplasia, trachoma, staphylococcal enterotoxigenic disease (food poisoning), Inner ear inflammation (otitis media), endotoxemia (sepsis), Angiotensin-II-induced cardiac hypertrophy, acute human spinal cord injury

11. A compound for use according to claim 10 wherein the disease is selected from multiple sclerosis, optic neuritis, rheumatoid arthritis, systemic lupus erythematosus, lupus nephritis, asthma, inflammatory bowel disease.

12. A kit consisting of separate packs of
(a) an effective amount of a compound according to Formula (I) or related Formulae and/or pharmaceutically usable derivatives, tautomers, salts, solvates and stereoisomers thereof, including mixtures thereof in all ratios,
and
(b) an effective amount of a further medicament active ingredient.

13. A process for the manufacture of a compound of Formula (I) according to claim 1 wherein a compound of Formula (C3), 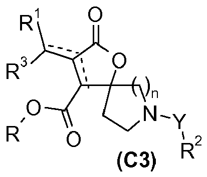 wherein R¹, R³, Y, R² and n are as defined in claim 1 and R is an alkyl or a benzyl group, is reacted with a compound of Formula R^{a}-Q-X-H (E), wherein R^{a}, Q, and X are as defined in claim 1 and wherein H is linked to the nitrogen atom of X,
or wherein a compound of Formula (D1) 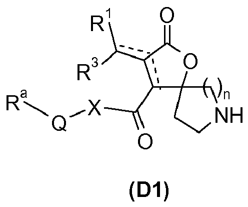 Wherein R¹, R², R^{a}, Q, X and n are as defined in claim 1, is reacted with a compound of Formula LG-Y-R² (F), wherein Y and R² are as defined in claim 1 and LG is a leaving group.

14. A process for the manufacture of a compound of Formula (I), according to claim 1, wherein Y denotes a branched or linear alkylene having 1 to 6 carbon atoms or a cyclic alkylene having 3 to 7 carbon atoms, wherein 1 to 5 H atoms, in these cyclic, linear or branched alkylene, may be replaced by OR⁶, Hal, or CN, Comprising the step of reacting a compound of Formula (C1) or a compound of Formula (D1), wherein R¹, R³, R^{d}, R^{a}, and n are as defined in claim 1 and wherein R is an alkyl or a benzyl group 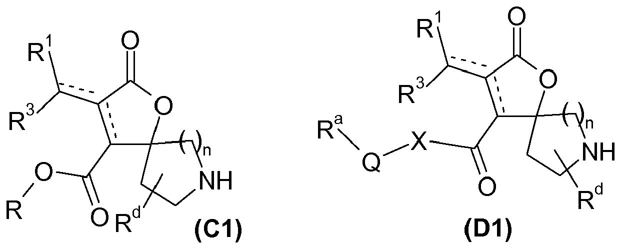 With a compound of Formula Y'-R², wherein R² is as defined in claim 1, and wherein Y' denotes a linear or branched alkyl having 1 to 6 carbon atoms or a cyclic alkyl having 3 to 7 carbon atoms, wherin one -CH₂- group, in these linear, branched, or cyclic alkyl groups, is replaced by a -CO- group, and wherein 1 to 5 H atoms, may be replaced by OR⁶, Hal, or CN.
